# EUROPEAN PATENT APPLICATION

(11) **EP 4 378 939 A1**
(43) Date of publication of application: **05.06.2024**
(21) Application number: 22849771.5
(22) Date of filing: 15.07.2022
(51) Int. Cl.: C07D 405/12, C07D 405/14, C07D 409/14, H10K 99/00, H10K 50/00

(54) **HETEROCYCLIC COMPOUND, ORGANIC LIGHT-EMITTING DEVICE COMPRISING SAME, AND COMPOSITION FOR ORGANIC LAYER**

(30) Priority: 26.07.2021 KR 20210097584
(71) Applicant: LT Materials Co., Ltd., Yongin-si, Gyeonggi-do 17118 (KR)
(72) Inventor: SEO, Young-Beom, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2022/010338
(87) International publication number: WO 2023/008795

(57) **Abstract**

The present specification relates to a heterocyclic compound represented by Chemical Formula 1, an organic light emitting device including the same and a composition for an organic material layer. The definition of each substituent in Chemical Formula 1 is as defined in the present specification. The heterocyclic compound represented by Chemical Formula 1 can lower the driving voltage of the device, improve the light efficiency of the device, and improve the service life characteristics of the device due to the thermal stability of the compound.

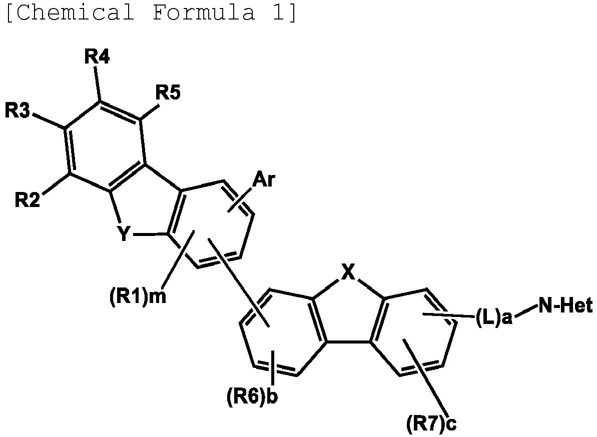

## Description

### <Cross-Reference to Related Applications>

This application claims priority to and the benefit of Korean Patent Application No. 10-2021-0097584 filed in the Korean Intellectual Property Office on July 26, 2021, the entire contents of which are incorporated herein by reference.

### [Technical Field]

The present specification relates to a heterocyclic compound, an organic light emitting device including the same and a composition for an organic material layer.

### [Background]

An electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

### <Related Art Documents>

(Patent Document 1) US Patent No. 4,356,429

### [Disclosure]

### [Technical Problem]

The present invention has been made in an effort to provide a heterocyclic compound, an organic light emitting device including the same and a composition for an organic material layer.

### [Technical Solution]

In an exemplary embodiment of the present application, provided is a heterocyclic compound represented by the following Chemical Formula 1. 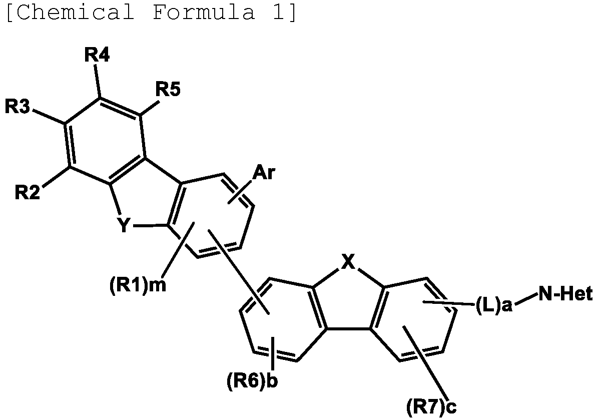 In Chemical Formula 1,
X and Y are the same as or different from each other, and are each independently O; or S,
N-Het is a monocyclic or polycyclic heterocyclic group which is substituted or unsubstituted and includes one or more N's,
R1 to R7 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
L is a direct bond; a substituted or unsubstituted C6 to C60 arylene group; or a substituted or unsubstituted C2 to C60 heteroarylene group,
Ar is a substituted or unsubstituted C1 to C60 alkyl group; a substituted or unsubstituted C6 to C60 aryl group; or a substituted or unsubstituted C2 to C60 heteroaryl group,
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
a deuterium content of the heterocyclic compound of Chemical Formula 1 is 0% to 100%,
a is an integer from 0 to 4,
b and c are an integer from 0 to 3,
m is an integer from 0 to 2, and
when a, b and c are each 2 or higher, substituents in the parenthesis are the same as or different from each other, and when m is 2, substituents in the parenthesis are the same as or different from each other.

Further, in an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In addition, an exemplary embodiment of the present application provides an organic light emitting device in which an organic material layer including the heterocyclic compound of Chemical Formula 1 further includes: a heterocyclic compound represented by the following Chemical Formula 2; or a heterocyclic compound represented by the following Chemical Formula 3. 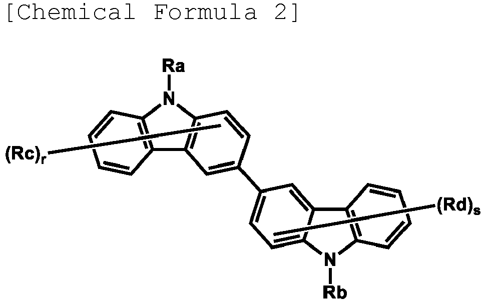 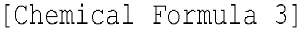 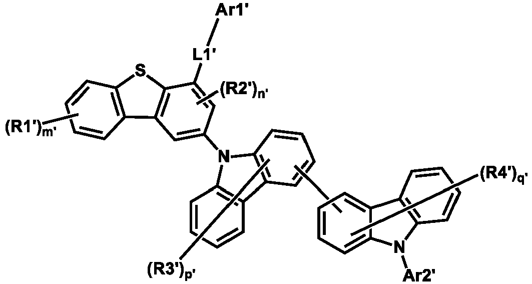 In Chemical Formulae 2 and 3,
Rc, Rd and R1' to R4' are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring,
Ra and Rb are the same as or different from each other, and are each independently a substituted or unsubstituted C6 to C60 aryl group; or a substituted or unsubstituted C2 to C60 heteroaryl group,
L1' is a direct bond; or a substituted or unsubstituted C6 to C60 arylene group,
Ar1' is a substituted or unsubstituted C6 to C60 aryl group; or a substituted or unsubstituted C2 to C60 heteroaryl group including at least one of S and O,
Ar2' is a substituted or unsubstituted C6 to C60 aryl group; or a substituted or unsubstituted C2 to C60 heteroaryl group,
m' is an integer from 0 to 4,
n' is an integer from 0 to 2,
p', q', r and s are an integer from 0 to 7, and
when m', p', q', n', r and s are each an integer of 2 or higher, substituents in the parenthesis are the same as or different from each other.

Furthermore, another exemplary embodiment of the present application provides a composition for an organic material layer of an organic light emitting device, which includes the heterocyclic compound represented by Chemical Formula 1; and the heterocyclic compound represented by Chemical Formula 2 or 3.

### [Advantageous Effects]

The compound described in the present specification can be used as a material for the organic material layer of the organic light emitting device. The compound may serve as a hole injection material, a hole transport material, a light emitting material, an electron transport material, an electron injection material, and the like in an organic light emitting device. In particular, the compound can be used as a material for a light emitting layer of an organic light emitting device.

Specifically, the compound may also be used alone as a light emitting material, and as a host material or a dopant material of the light emitting layer. When the compound represented by Chemical Formula 1 is used for an organic material layer, the driving voltage of the device can be lowered, the light efficiency of the device can be improved, and the service life characteristics of the device can be improved due to the thermal stability of the compound.

In particular, the heterocyclic compound represented by Chemical Formula 1 and the heterocyclic compound represented by Chemical Formula 2 or 3 can be used simultaneously as a material for a light emitting layer of an organic light emitting device. In this case, it is possible to lower a driving voltage of the device, improve the light efficiency, and particularly improve the service life characteristics of the device by the thermal stability of the compound.

### [Description of Drawings]

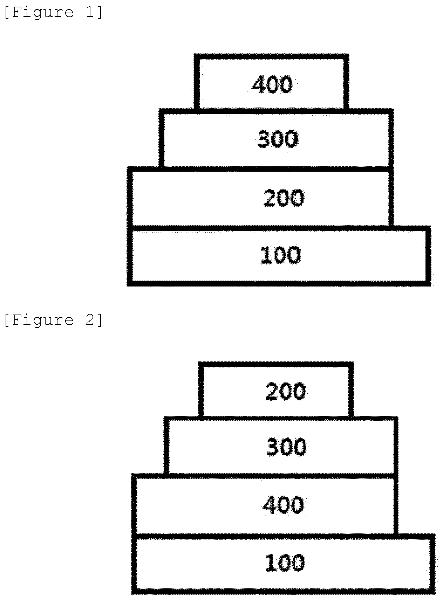

FIGS. 1 to 3 each are views schematically illustrating a stacking structure of an organic light emitting device according to an exemplary embodiment of the present application.
100: Substrate
200: Positive electrode
300: Organic material layer
301: Hole injection layer
302: Hole transport layer
303: Light emitting layer
304: Hole blocking layer
305: Electron transport layer
306: Electron injection layer
400: Negative electrode

### [Mode for Invention]

Hereinafter, the present application will be described in detail.

In the present specification, "when a substituent is not indicated in the structure of a chemical formula or compound" means that a hydrogen atom is bonded to a carbon atom. However, since deuterium (²H) and tritium (³H) are isotopes of hydrogen, some hydrogen atoms may be deuterium or tritium.

In an exemplary embodiment of the present application, "when a substituent is not indicated in the structure of a chemical formula or compound" may mean that all the positions that may be reached by the substituent are hydrogen, deuterium, or tritium. That is, deuterium or tritium is an isotope of hydrogen, some hydrogen atoms may be deuterium or tritium which are isotopes thereof, and in this case, the content of deuterium or tritium may be 0% to 100%.

In an exemplary embodiment of the present invention, in "the case where a substituent is not indicated in the structure of a chemical formula or compound", when the substituents do not explicitly exclude deuterium and tritium, such as "the content of deuterium is 0%", "the content of tritium is 0%", "the content of hydrogen is 100%", and "the substituents are all hydrogen", hydrogen, deuterium and tritium may be mixed and used in the compound.

In an exemplary embodiment of the present invention, deuterium is one of the isotopes of hydrogen, is an element that has a deuteron composed of one proton and one neutron as a nucleus, and may be represented by hydrogen-2, and the element symbol may also be expressed as D or ²H. Similarly, the element symbol for tritium may also be expressed as T or ³H.

In an exemplary embodiment of the present invention, the isotope means an atom with the same atomic number (Z), but different mass numbers (A), and the isotope may be interpreted as an element which has the same number of protons, but different number of neutrons.

In an exemplary embodiment of the present invention, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 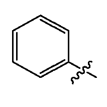 may mean the case where the total number of substituents that the phenyl group can have is 5 (T1 in the formula) and the number of deuteriums among the substituents is 1 (T2 in the formula). That is, a deuterium content of 20% in the phenyl group may be represented by the following structural formula.

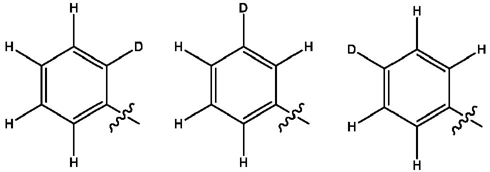

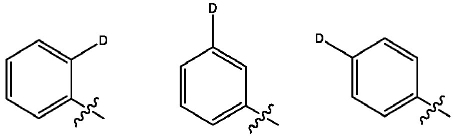

Further, in an exemplary embodiment of the present application, "a phenyl group having a deuterium content of 0%" may mean a phenyl group that does not include a deuterium atom, that is, has five hydrogen atoms.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being unsubstituted or substituted with one or more substituents selected from the group consisting of deuterium; a halogen; a cyano group; a halogen group; a C1 to C60 straight-chained or branched alkyl; a C2 to C60 straight-chained or branched alkenyl; a C2 to C60 straight-chained or branched alkynyl; a C3 to C60 monocyclic or polycyclic cycloalkyl; a C2 to C60 monocyclic or polycyclic heterocycloalkyl; a C6 to C60 monocyclic or polycyclic aryl; a C2 to C60 monocyclic or polycyclic heteroaryl; -SiRR'R"; -P(=O)RR'; a C1 to C20 alkylamine; a C6 to C60 monocyclic or polycyclic arylamine; and a C2 to C60 monocyclic or polycyclic heteroarylamine, or being unsubstituted or substituted with a substituent to which two or more substituents selected among the exemplified substituents are linked.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, a phosphine oxide group is represented by -P(=O)R101R102, and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and the above-described example may be applied to the aryl group. Examples of the phosphine oxide group include a diphenylphosphine oxide group, a dinaphthylphosphine oxide, and the like, but are not limited thereto.

In the present specification, a silyl group includes Si and is a substituent to which the Si atom is directly linked as a radical, and is represented by -SiR104R105R106, and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the fluorenyl group may be substituted, and adjacent substituents may be bonded to each other to form a ring.

When the fluorenyl group is substituted, the substituted fluorenyl group may be represented by the following structural formulae, but is not limited thereto.

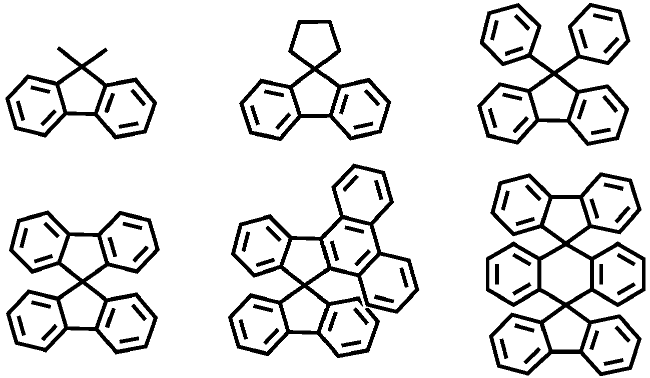

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c] [1,2,5]thiadiazolyl group, a 5,10-dihydrodibenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means that there are two bonding positions in an aryl group, that is, a divalent group. The above-described description on the aryl group may be applied to the arylene group, except that the arylene groups are each a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

In an exemplary embodiment of the present application, provided is the compound represented by Chemical Formula 1.

In an exemplary embodiment of the present application, a group not represented by a substituent; or a group represented by hydrogen may mean being substitutable with deuterium. That is, in an exemplary embodiment of the present application, it may be shown that hydrogen; or deuterium can be substituted with each other.

In an exemplary embodiment of the present application, the deuterium content of the heterocyclic compound of Chemical Formula 1 may be 0% to 100%.

In another exemplary embodiment, the deuterium content of the heterocyclic compound of Chemical Formula 1 may be 0% to 100%, 5% to 100%, 7% to 100%, 10% to 100%, 15% to 100%, or 20% to 100%.

In general, compounds bonded with hydrogen and compounds substituted with deuterium exhibit a difference in thermodynamic behavior. The reason for this is that the mass of a deuterium atom is 2-fold higher than that of hydrogen, but due to the difference in the mass of atoms, deuterium is characterized by having even lower vibration energy. In addition, the bond length of carbon and deuterium is shorter than that of hydrogen, and a dissociation energy used to break the bond is also stronger than that of hydrogen. This is because the van der Waals radius of deuterium is smaller than that of hydrogen, and thus the extension amplitude of a bond between carbon and deuterium becomes even narrower.

The deuterium-substituted compound in the heterocyclic compound of Chemical Formula 1 of the present invention is characterized in that the energy in the ground state is further lower than that of the hydrogen-substituted compound, and the shorter the bond length between carbon and deuterium is, the smaller the molecular hardcore volume is. Accordingly, the electrical polarizability may be reduced and the intermolecular interaction can be weakened, so that the volume of the device thin film may be increased. These characteristics induce an effect of lowering the crystallinity by creating the amorphous state of a thin film. Therefore, deuterium substitution in the heterocyclic compound of Chemical Formula 1 may be effective in improving the heat resistance of an OLED device, thereby improving the service life and driving characteristics.

In an exemplary embodiment of the present application, Chemical Formula 1 may be represented by any one of the following Chemical Formulae 4 to 7.

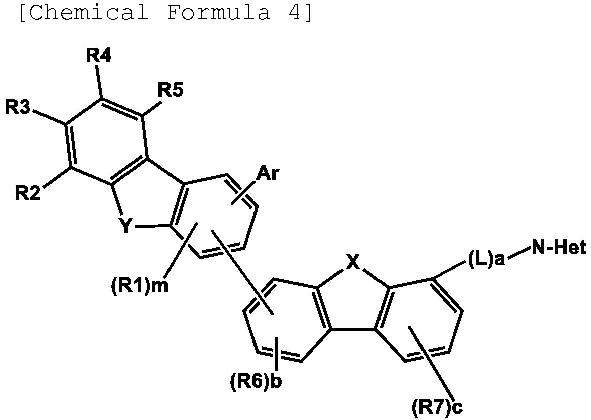

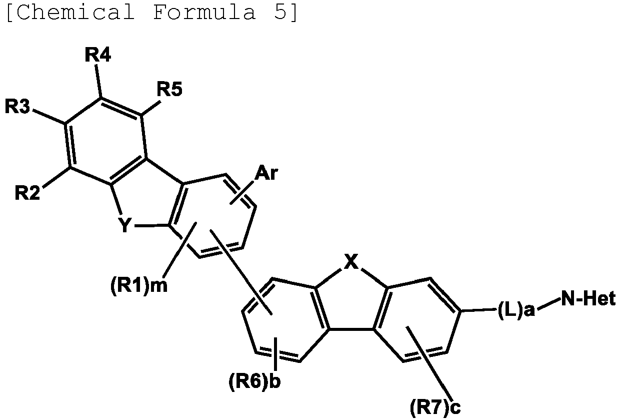

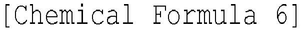

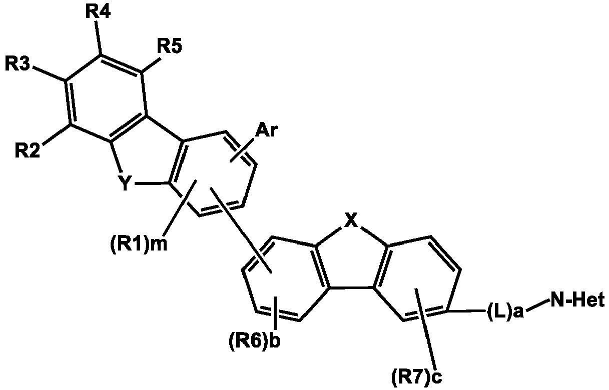

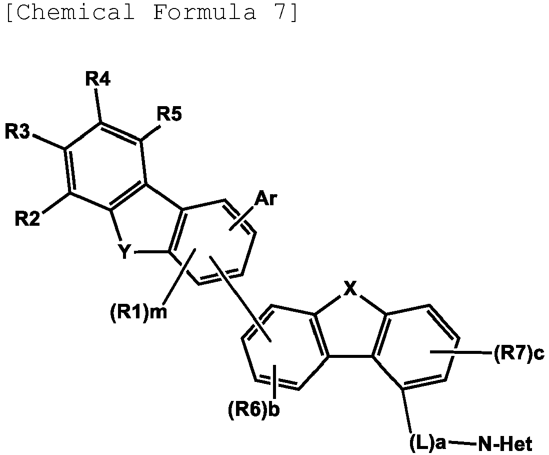

In Chemical Formulae 4 to 7,
the definition of each substituent is the same as the definition in Chemical Formula 1.

In an exemplary embodiment of the present application, R1 to R7 are the same as or different from each other, and may be each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR'.

In another exemplary embodiment, R1 to R7 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In still another exemplary embodiment, R1 to R7 are the same as or different from each other, and may be each independently hydrogen; deuterium; a substituted or unsubstituted C1 to C40 alkyl group; a substituted or unsubstituted C6 to C40 aryl group; or a substituted or unsubstituted C2 to C40 heteroaryl group.

In yet another exemplary embodiment, R1 to R7 are the same as or different from each other, and may be each independently hydrogen; deuterium; a C1 to C40 alkyl group; a C6 to C40 aryl group; or a C2 to C40 heteroaryl group. In yet another exemplary embodiment, R1 to R7 are the same as or different from each other, and may be each independently hydrogen; deuterium; a C1 to C20 alkyl group; a C6 to C20 aryl group; or a C2 to C20 heteroaryl group.

In yet another exemplary embodiment, R1 to R7 are the same as or different from each other, and may be each independently hydrogen; or deuterium.

In an exemplary embodiment of the present application, N-Het may be a monocyclic or polycyclic heterocyclic group which is substituted or unsubstituted and includes one or more N's.

In another exemplary embodiment, N-Het may be a monocyclic or polycyclic heterocyclic group which is substituted or unsubstituted and includes one or more and three or more N's.

In still another exemplary embodiment, N-Het may be a monocyclic or polycyclic C2 to C60 heterocyclic group which is substituted or unsubstituted and includes one or more and three or less N's.

In yet another exemplary embodiment, N-Het may be a monocyclic or polycyclic C2 to C40 heterocyclic group which is substituted or unsubstituted and includes one or more and three or less N's.

In yet another exemplary embodiment, N-Het may be a monocyclic or polycyclic C2 to C40 heterocyclic group which is unsubstituted or substituted with deuterium or a C6 to C40 aryl group, and includes one or more and three or less N's.

In yet another exemplary embodiment, N-Het may be a monocyclic or polycyclic C2 to C40 heterocyclic group which is unsubstituted or substituted with deuterium or a C6 to C40 aryl group, and includes one or more and three or less N's.

In yet another exemplary embodiment, N-Het may be a substituted or unsubstituted pyridine group; a substituted or unsubstituted pyrimidine group; a substituted or unsubstituted triazine group; a substituted or unsubstituted quinoline group; a substituted or unsubstituted quinazoline group; a substituted or unsubstituted phenanthroline group; or a substituted or unsubstituted quinoxaline group.

In yet another exemplary embodiment, N-Het may be a pyridine group which is unsubstituted or substituted with a phenyl group or deuterium; a pyrimidine group which is unsubstituted or substituted with a phenyl group or deuterium; a triazine group which is unsubstituted or substituted with a phenyl group or deuterium; a quinoline group which is unsubstituted or substituted with a phenyl group or deuterium; a quinazoline group which is unsubstituted or substituted with a phenyl group or deuterium; a phenanthroline group which is unsubstituted or substituted with a phenyl group or deuterium; or a quinoxaline group which is unsubstituted or substituted with a phenyl group or deuterium.

In an exemplary embodiment of the present application, L may be a direct bond; a substituted or unsubstituted C6 to C60 arylene group; or a substituted or unsubstituted C2 to C60 heteroarylene group.

In another exemplary embodiment, L may be a direct bond; a substituted or unsubstituted C6 to C40 arylene group; or a substituted or unsubstituted C2 to C40 heteroarylene group.

In still another exemplary embodiment, L may be a direct bond; or a substituted or unsubstituted C6 to C40 arylene group.

In yet another exemplary embodiment, L may be a direct bond; or a C6 to C40 arylene group.
In yet another exemplary embodiment, L may be a direct bond; or a C6 to C20 arylene group.

In yet another exemplary embodiment, L may be a direct bond; a monocyclic C6 to C10 arylene group; or a polycyclic C10 to C20 arylene group.

In an exemplary embodiment of the present application, L may be further substituted with deuterium.

In an exemplary embodiment of the present application, in 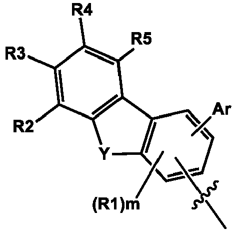 of Chemical Formula 1, a linking group linked to 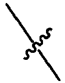 and a substituent of -Ar may be bonded to a metha; or para position each other.

That is, as described above, an example that in 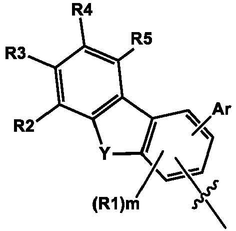 of Chemical Formula 1, a linking group linked to 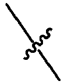 and a substituent of -Ar is bonded to a metha; or para position each other may be as follows.

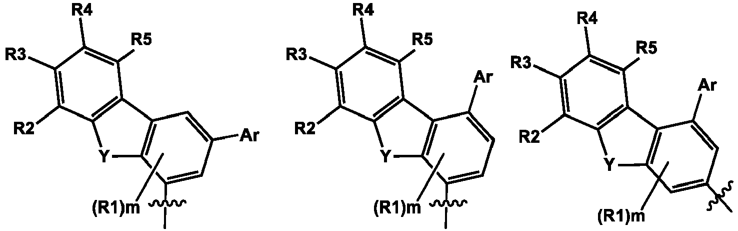

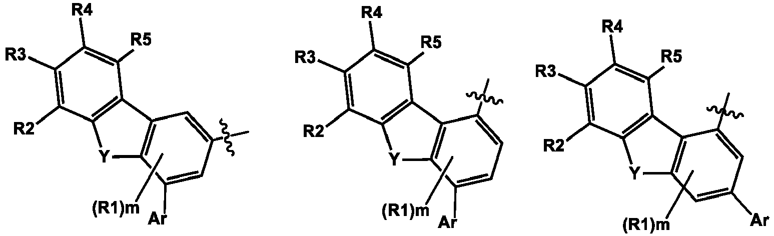

In an exemplary embodiment of the present application, X and Y may be the same as each other.

In an exemplary embodiment of the present application, X and Y may be different from each other.

In an exemplary embodiment of the present application, Ar may be a substituted or unsubstituted C1 to C60 alkyl group; a substituted or unsubstituted C6 to C60 aryl group; or a substituted or unsubstituted C2 to C60 heteroaryl group.

In another exemplary embodiment, Ar may be a substituted or unsubstituted C1 to C40 alkyl group; a substituted or unsubstituted C6 to C40 aryl group; or a substituted or unsubstituted C2 to C40 heteroaryl group.

In another exemplary embodiment, Ar may be a substituted or unsubstituted C2 to C40 heteroaryl group.

In still another exemplary embodiment, Ar may be a C2 to C20 heteroaryl group including O or S, which is unsubstituted or substituted with deuterium.

In yet another exemplary embodiment, Ar may be a dibenzofuran group which is unsubstituted or substituted with deuterium; or a dibenzothiophene group which is unsubstituted or substituted with deuterium.

In an exemplary embodiment of the present application, Chemical Formula 1 may be represented by any one of the following Chemical Formulae 8 to 11.

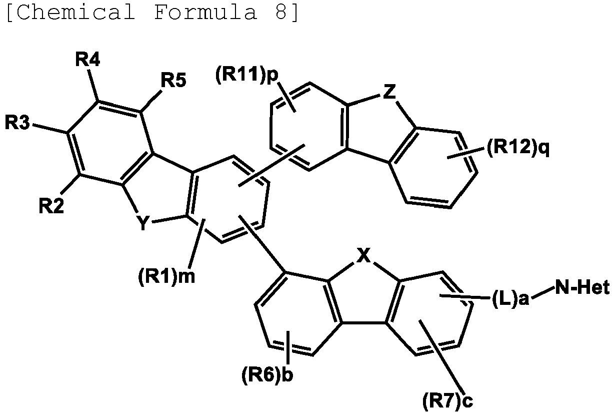

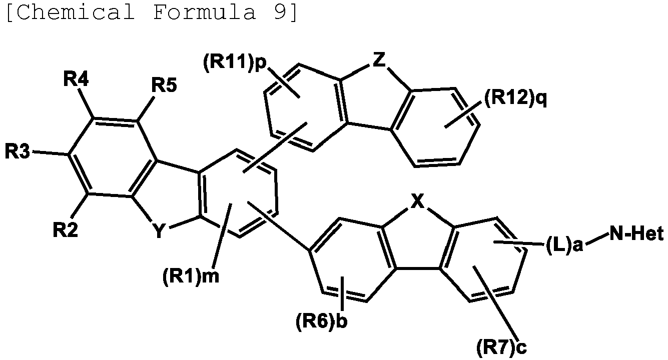

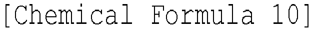

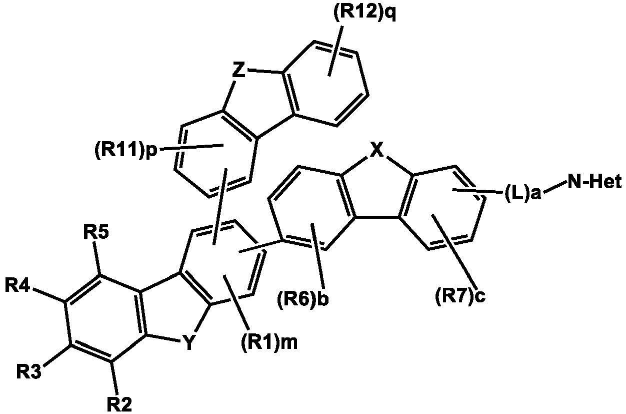

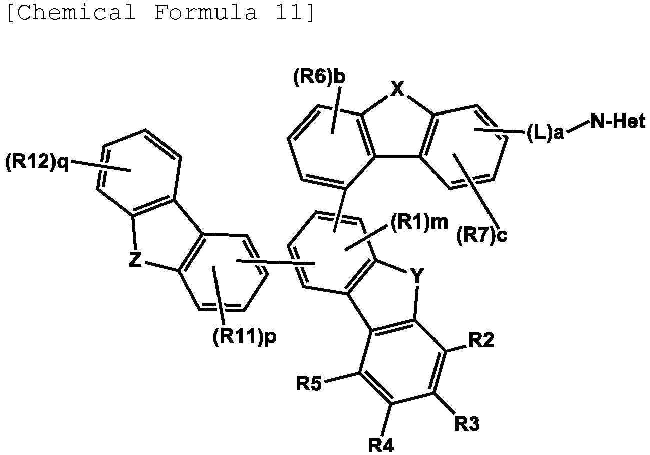

In Chemical Formulae 8 to 11,
the definitions of R1 to R7, X, Y, L, N-Het, a, b, c and m are the same as the definitions in Chemical Formula 1,
Z is O; or S,
R11 and R12 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
q is an integer of 0 to 4,
p is an integer from 0 to 3, and
when p and q are each an integer of 2 or higher, substituents in the parenthesis are the same as or different from each other.

In an exemplary embodiment of the present application, R11 and R12 are the same as or different from each other, and may be each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR'.

In another exemplary embodiment, R11 and R12 are the same as or different from each other, and may be each independently hydrogen; deuterium; a substituted or unsubstituted C1 to C60 alkyl group; a substituted or unsubstituted C6 to C60 aryl group; a substituted or unsubstituted C2 to C60 heteroaryl group; SiRR'R"; or - P(=O)RR'.

In still another exemplary embodiment, R11 and R12 are the same as or different from each other, and may be each independently hydrogen; deuterium; a C6 to C60 aryl group; a C2 to C60 heteroaryl group; SiRR'R"; or -P(=O)RR'.

In yet another exemplary embodiment, R11 and R12 are the same as or different from each other, and may be each independently hydrogen; or deuterium.

In an exemplary embodiment of the present application, in 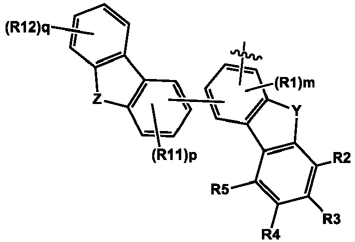 of Chemical Formulae 8 to 11, a linking group linked to 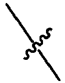 and a substituent of 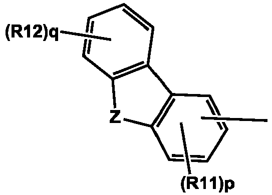 may be bonded to a metha; or para position each other.

In an exemplary embodiment of the present application, R, R' and R" are the same as or different from each other, and may be each independently a substituted or unsubstituted alkyl group; a substituted or unsubstituted aryl group; or a substituted or unsubstituted heteroaryl group.

In another exemplary embodiment, R, R' and R" are the same as different from each other, and may be each independently a substituted or unsubstituted alkyl group; or a substituted or unsubstituted aryl group.

In still another exemplary embodiment, R, R' and R" are the same as or different from each other, and may be each independently a substituted or unsubstituted C1 to C60 alkyl group; or a substituted or unsubstituted C6 to C60 aryl group.

In yet another exemplary embodiment, R, R' and R" are the same as or different from each other, and may be each independently a C1 to C60 alkyl group; or a C6 to C60 aryl group.

In yet another exemplary embodiment, R, R' and R" are the same as or different from each other, and may be each independently a methyl group; or a phenyl group.

In yet another exemplary embodiment, R, R' and R" may be a phenyl group.

In an exemplary embodiment of the present application, provided is a heterocyclic compound in which Chemical Formula 1 is represented by any one of the following compounds.

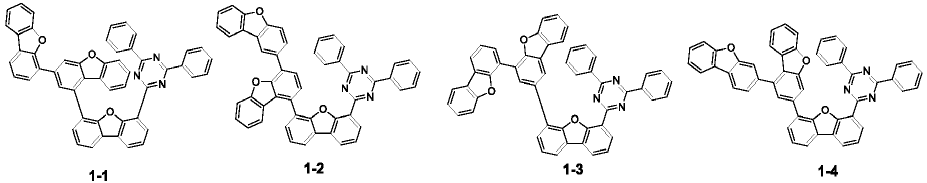

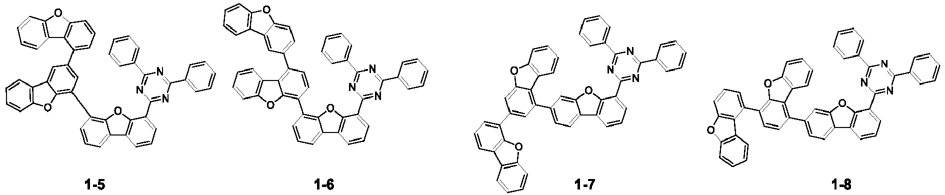

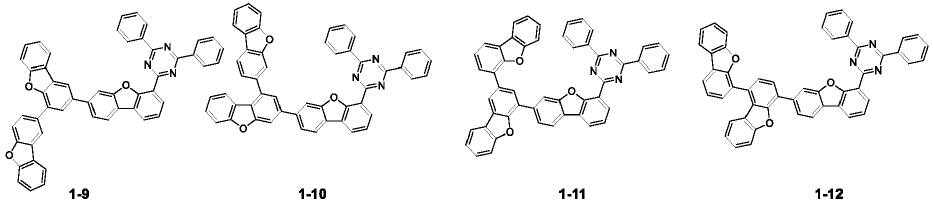

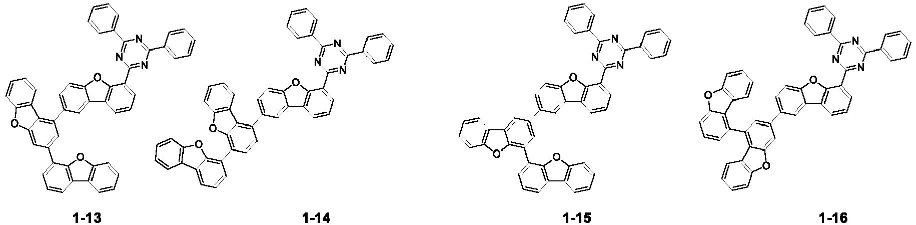

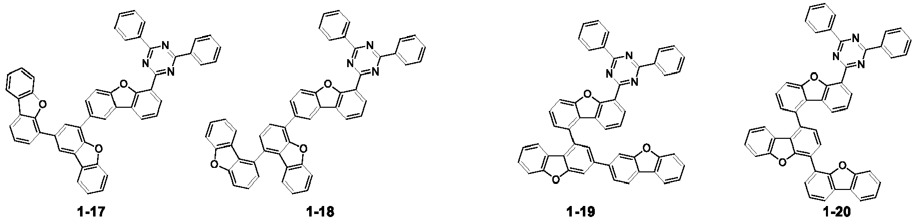

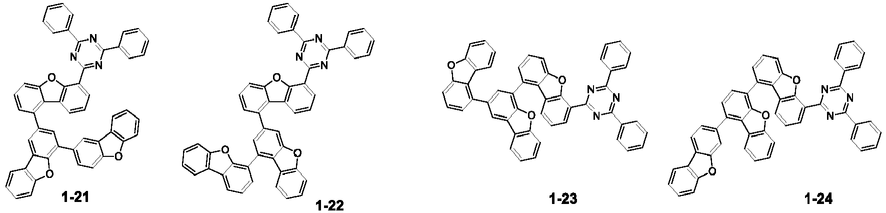

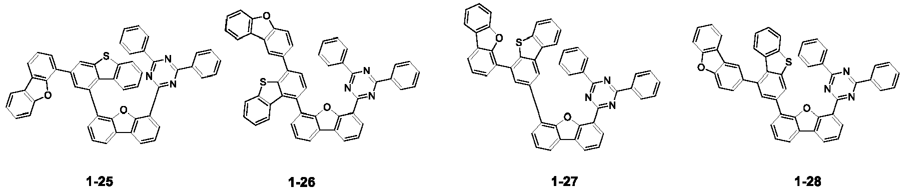

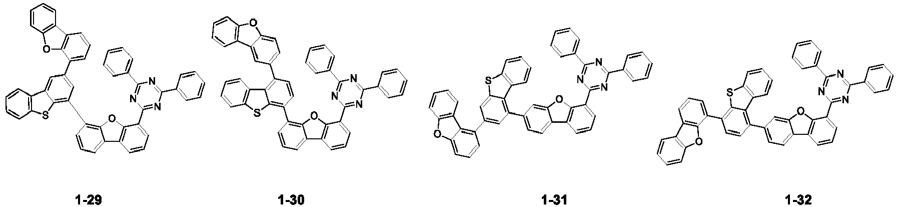

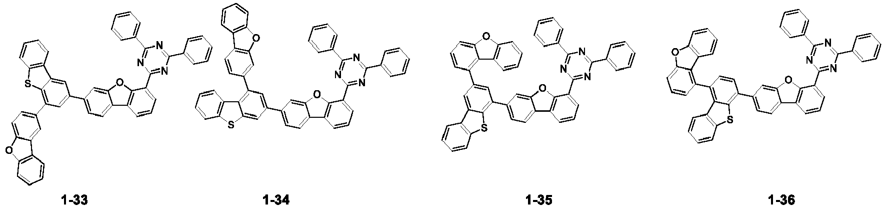

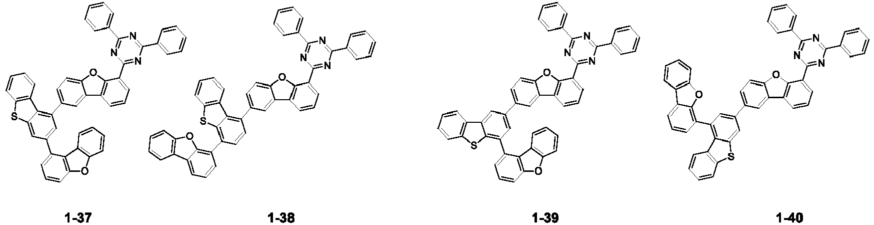

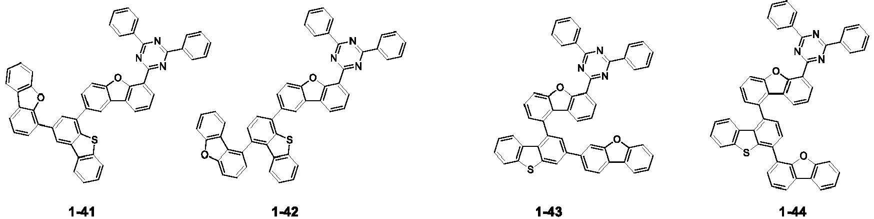

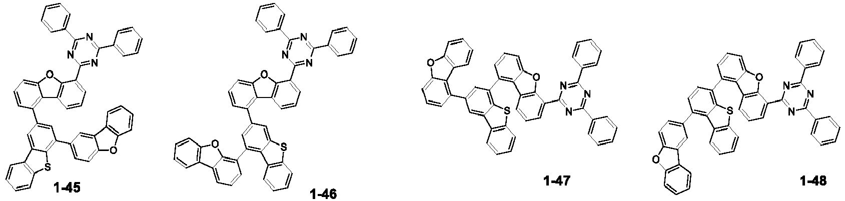

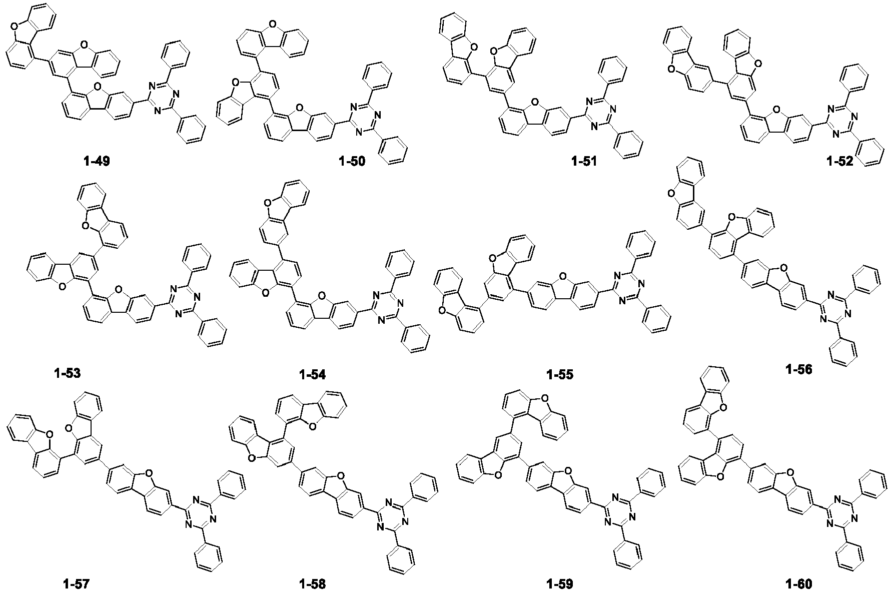

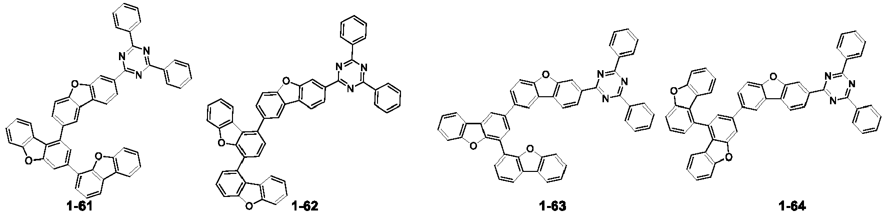

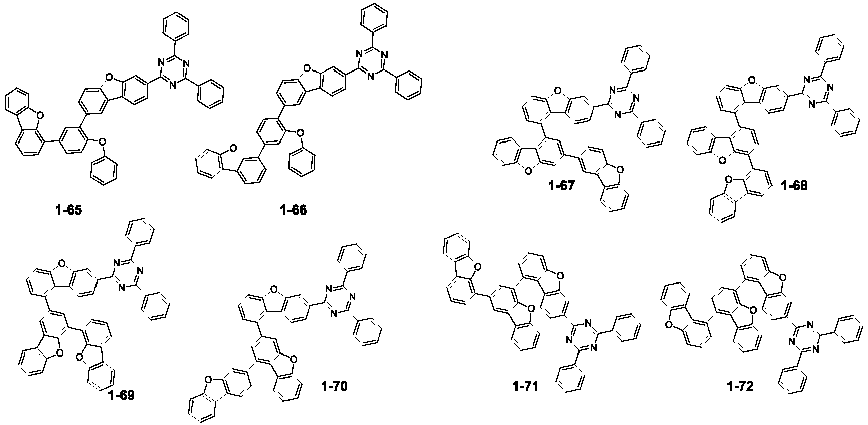

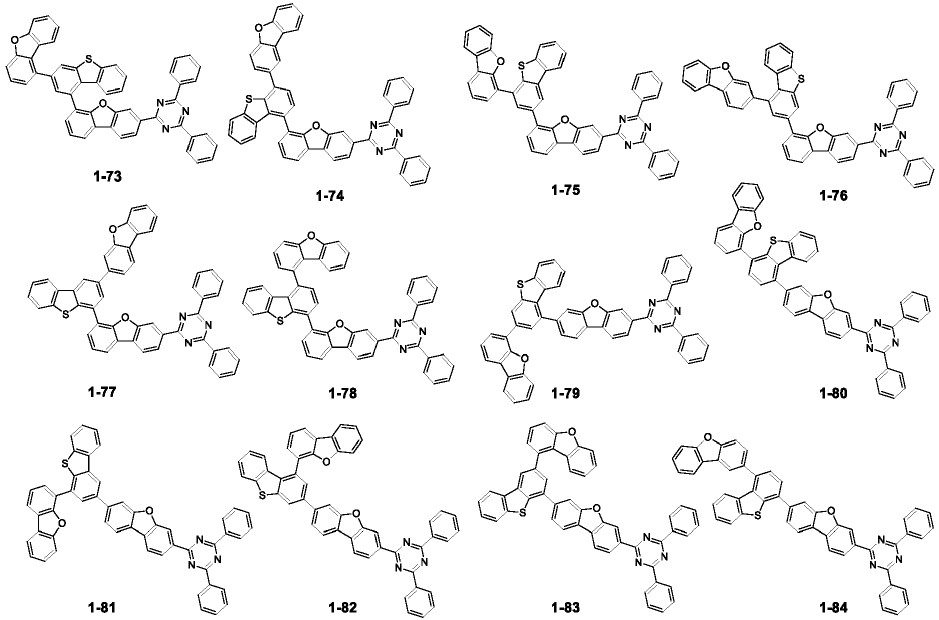

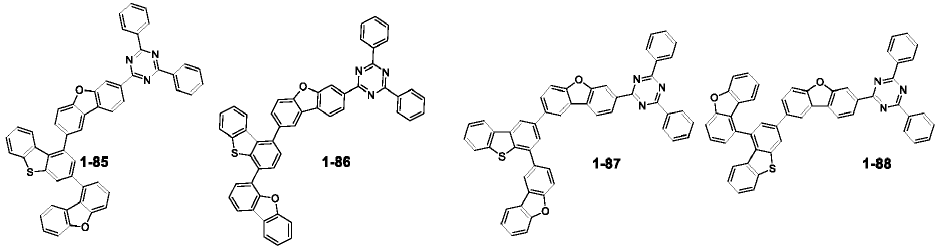

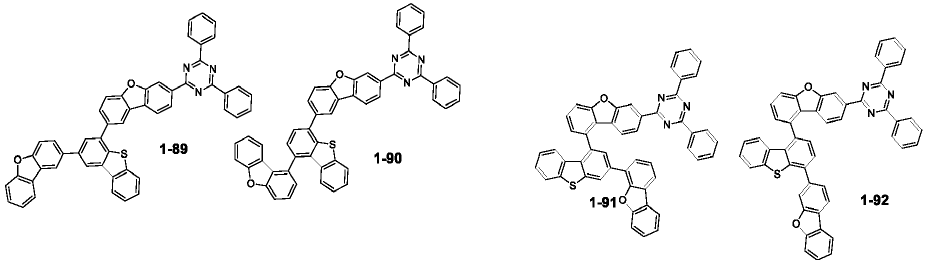

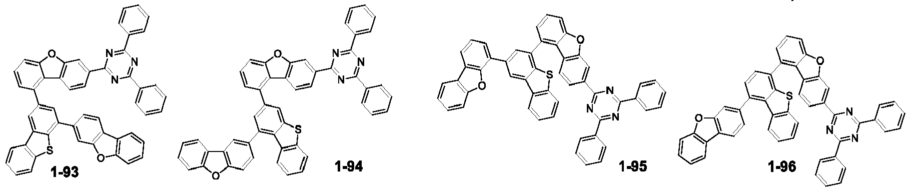

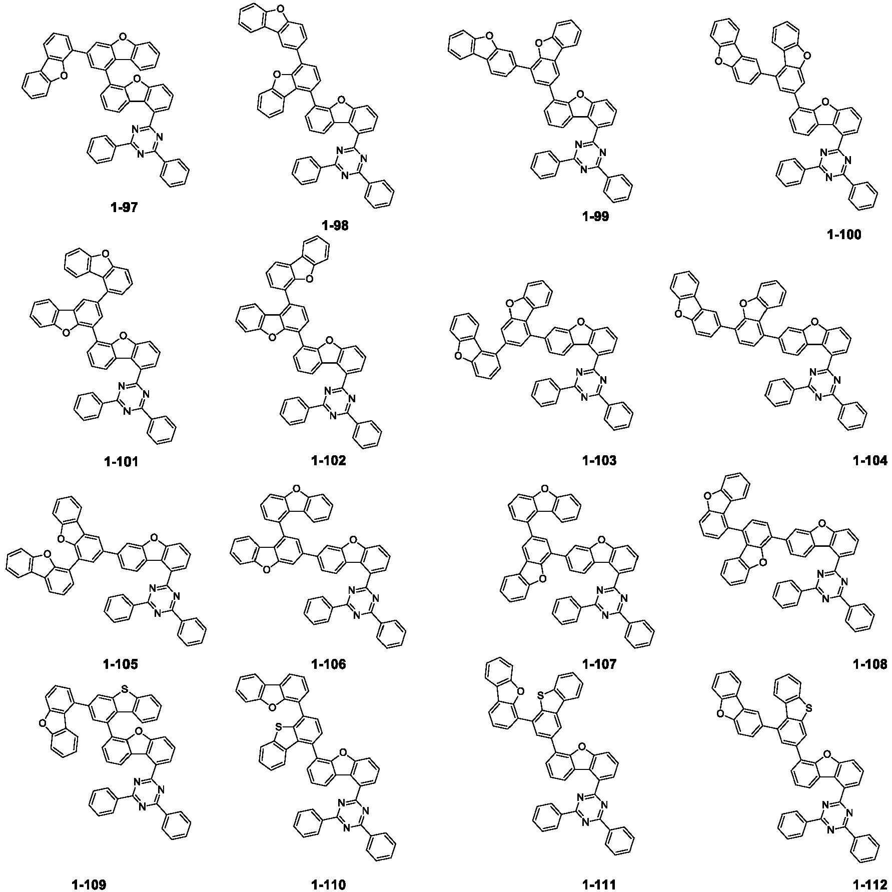

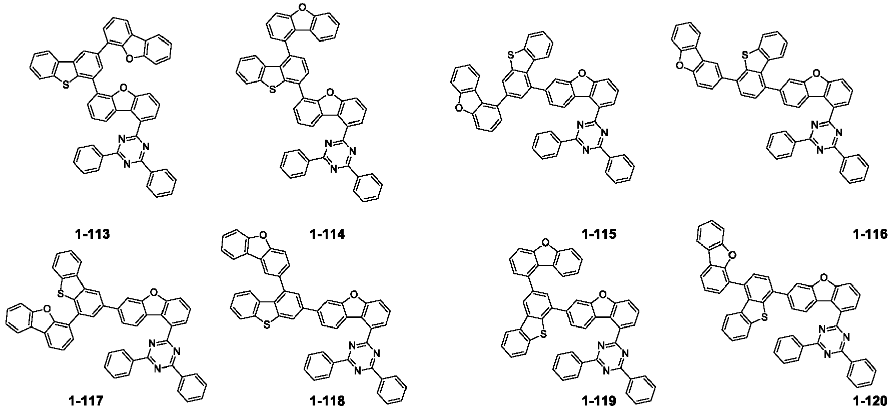

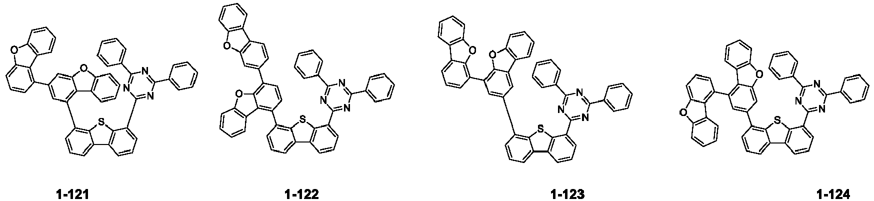

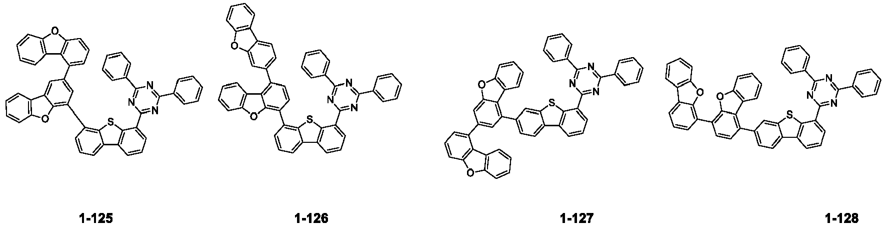

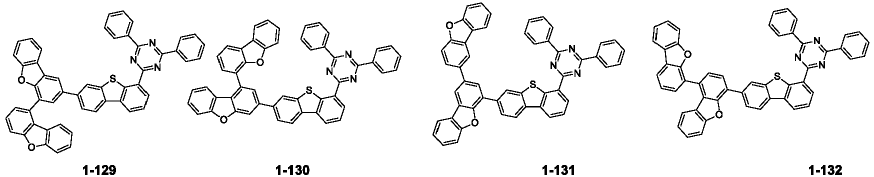

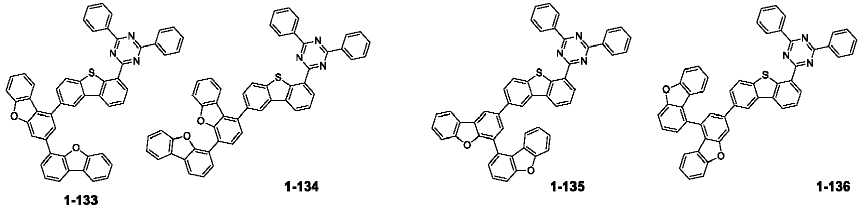

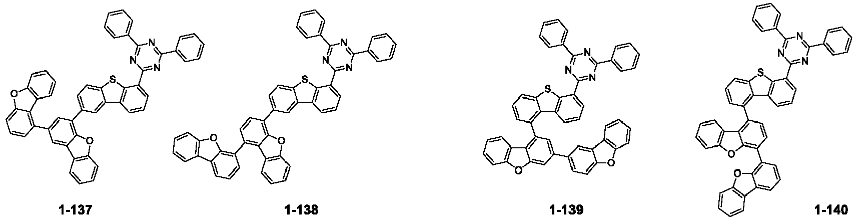

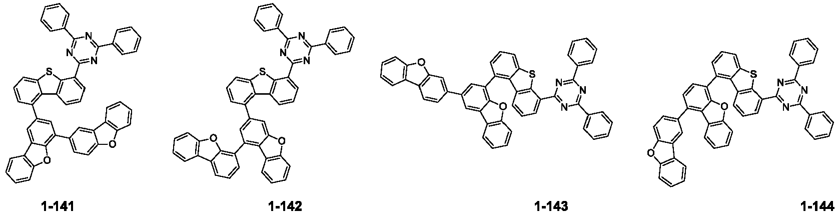

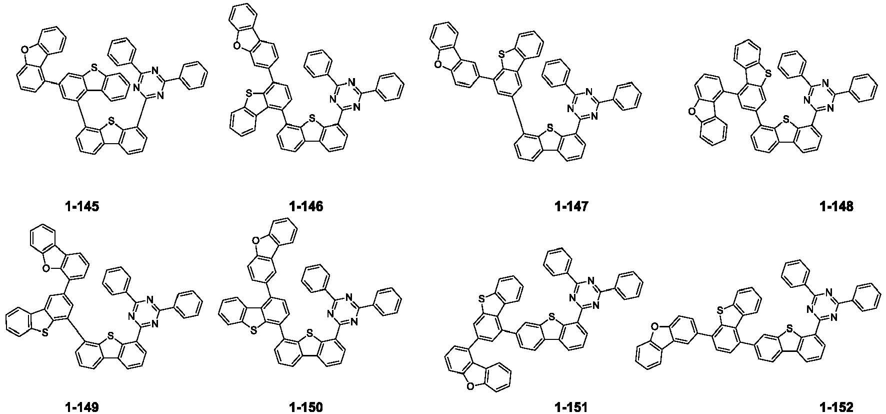

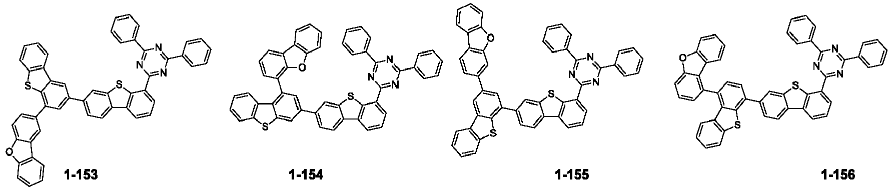

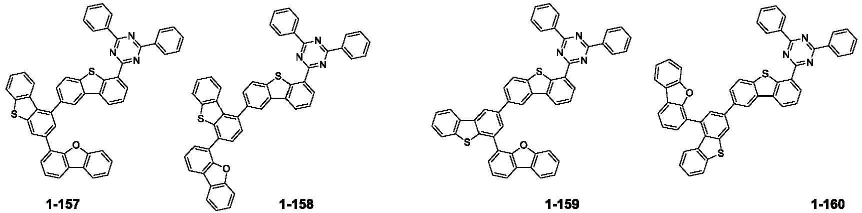

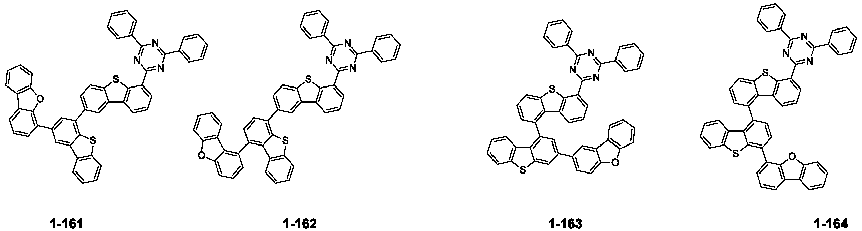

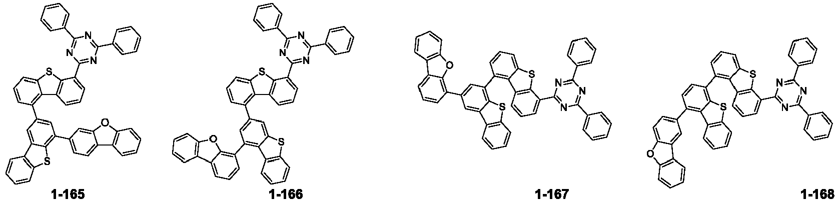

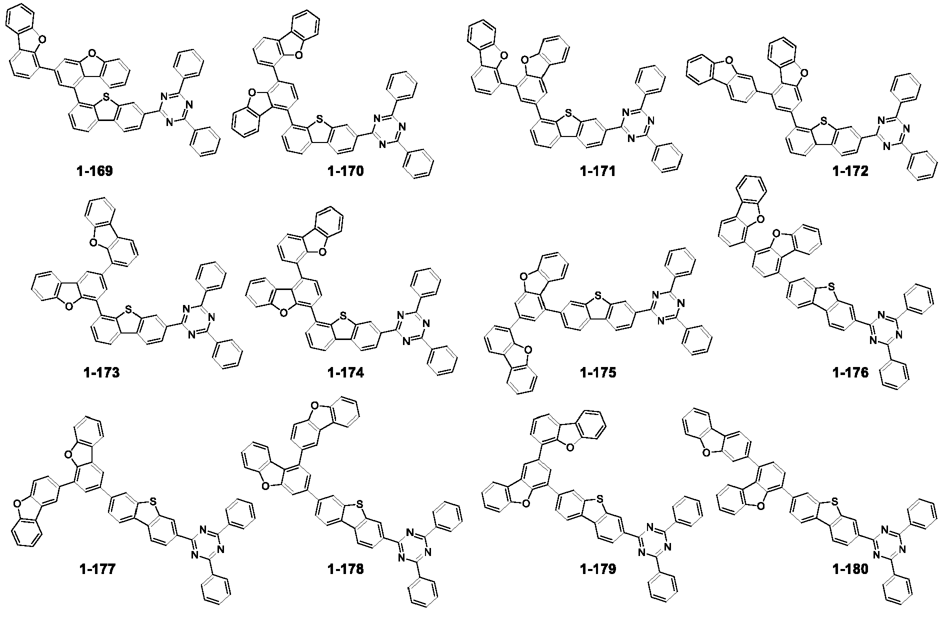

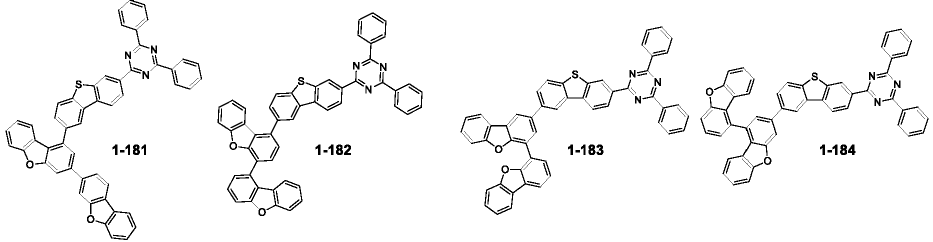

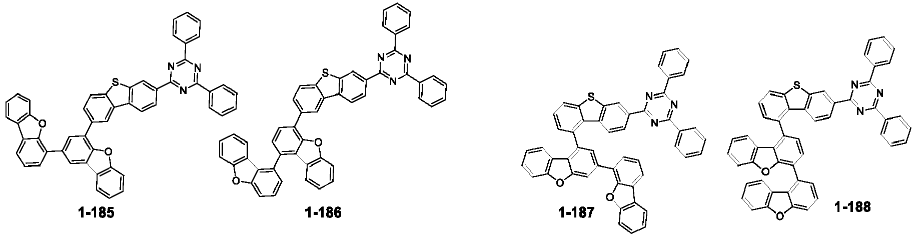

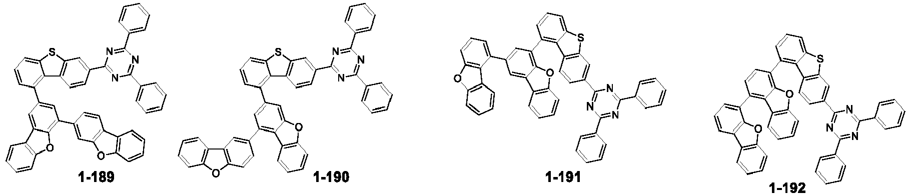

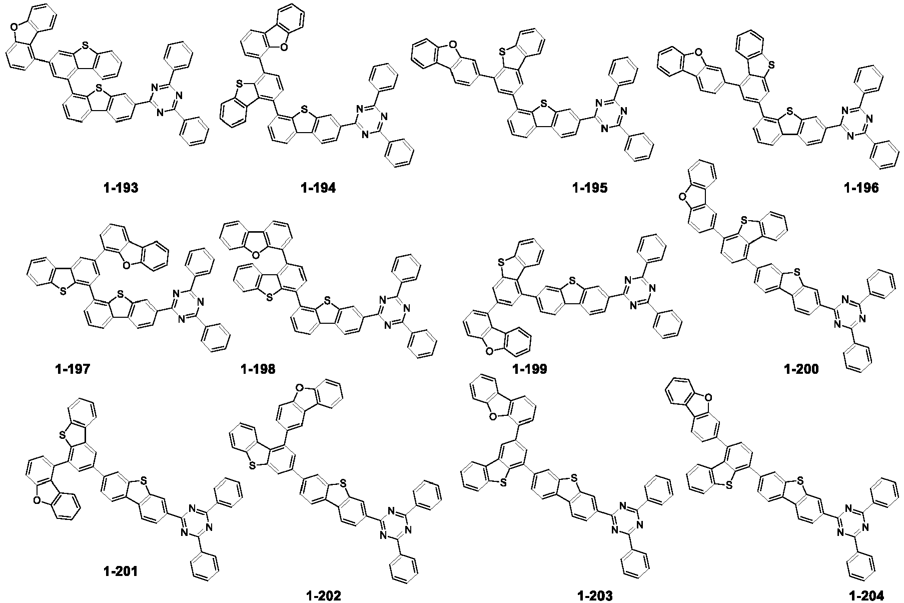

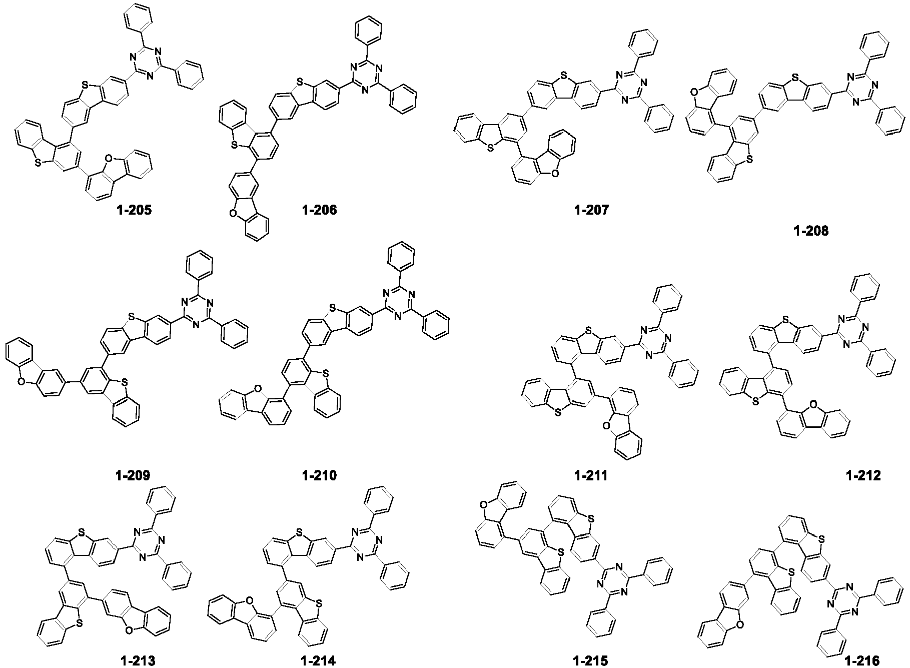

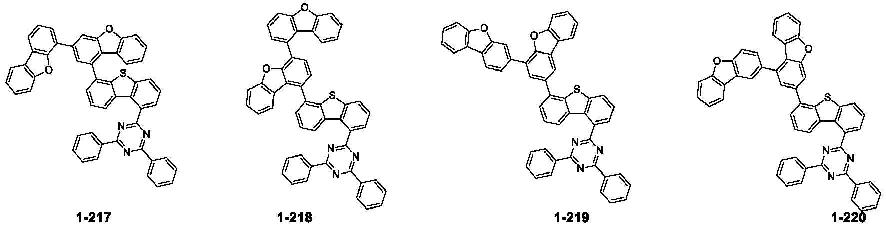

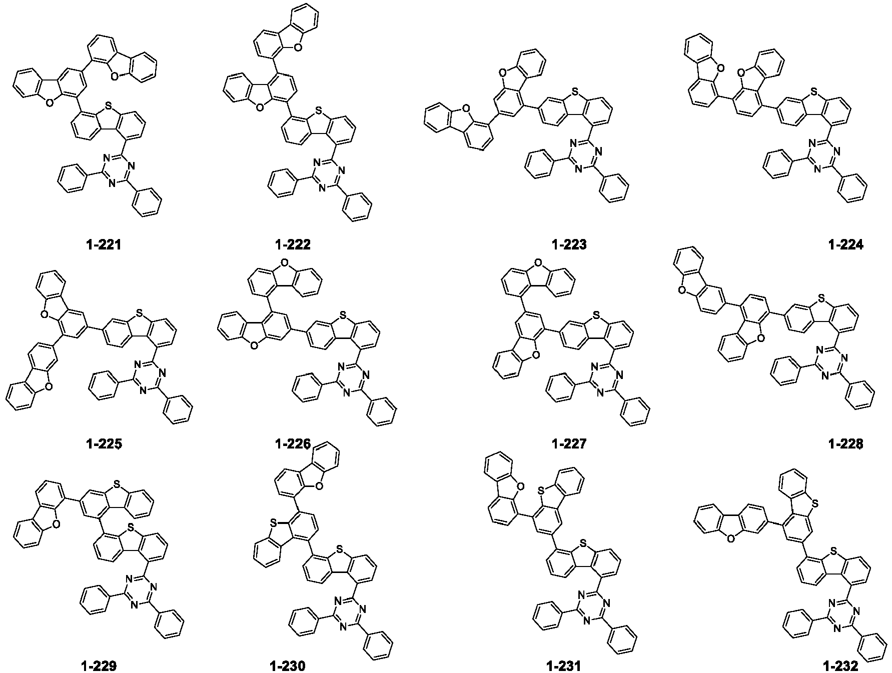

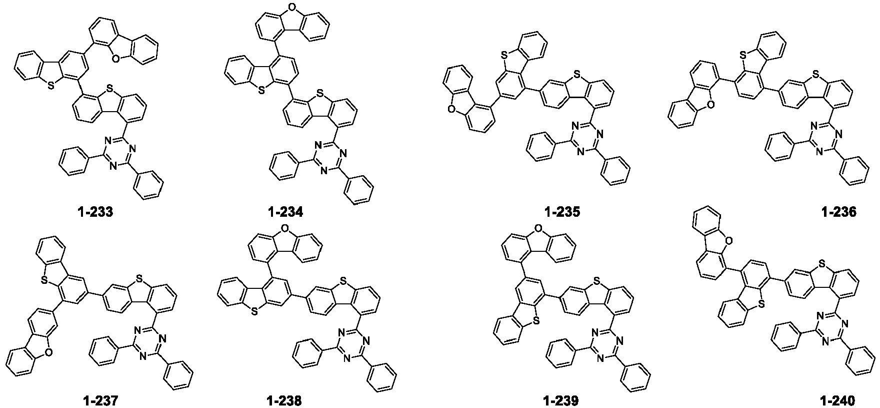

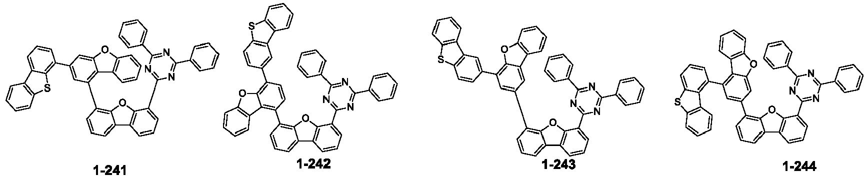

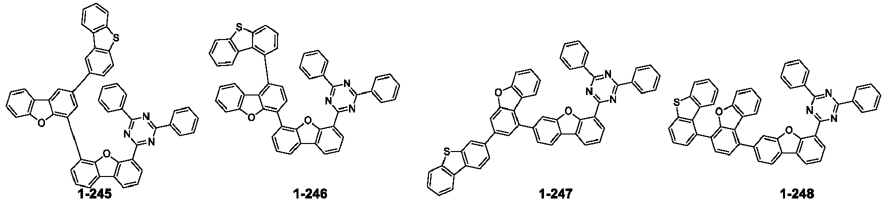

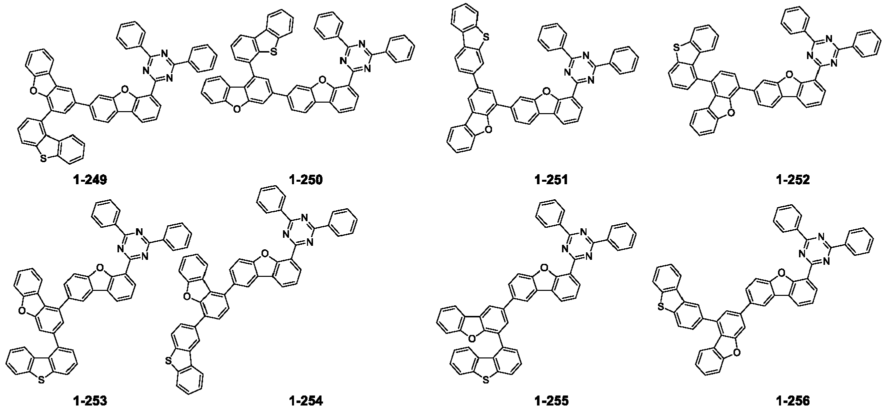

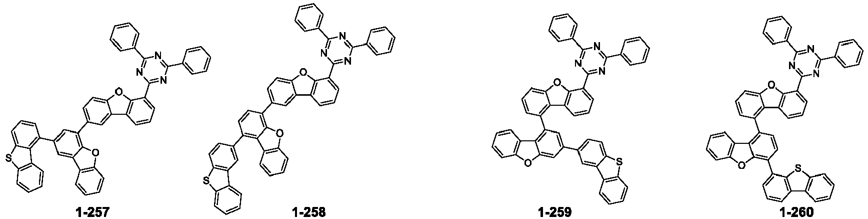

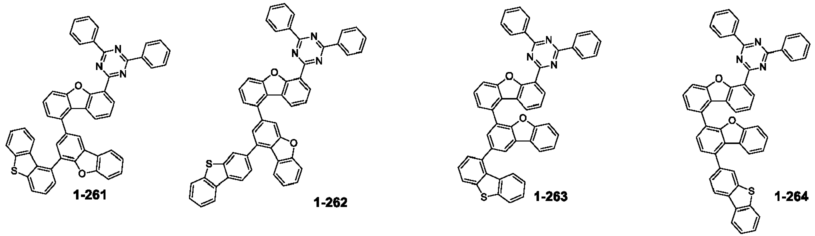

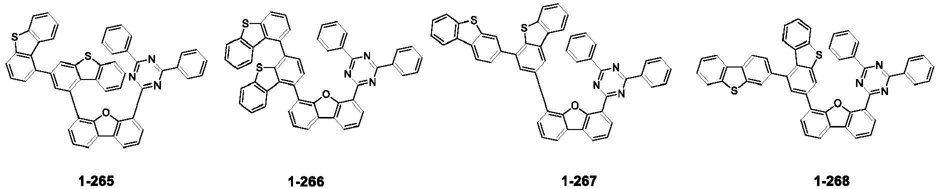

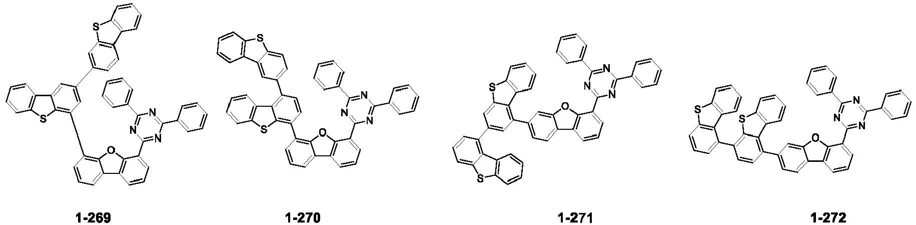

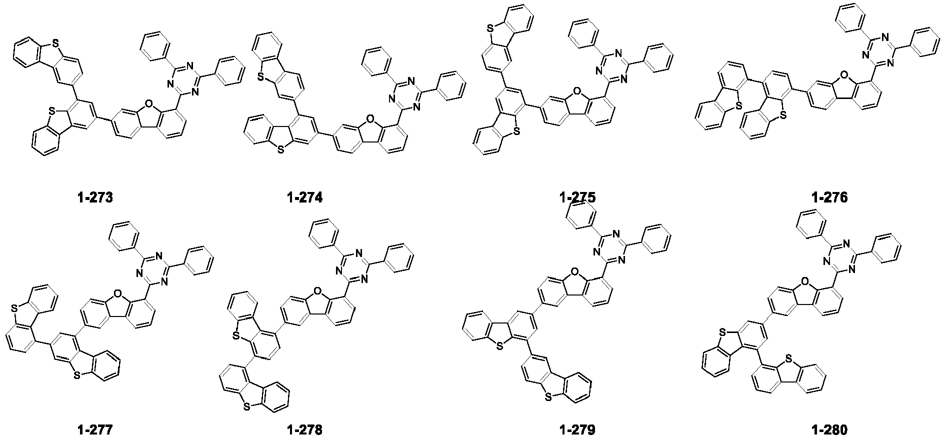

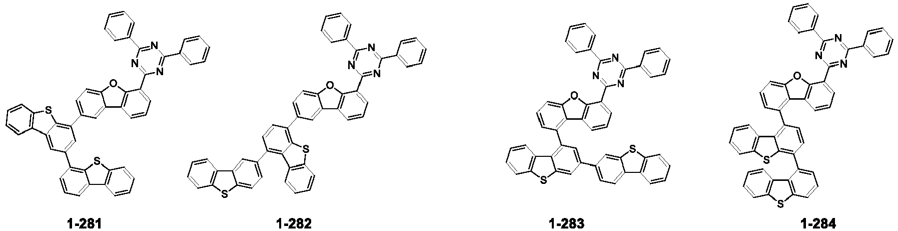

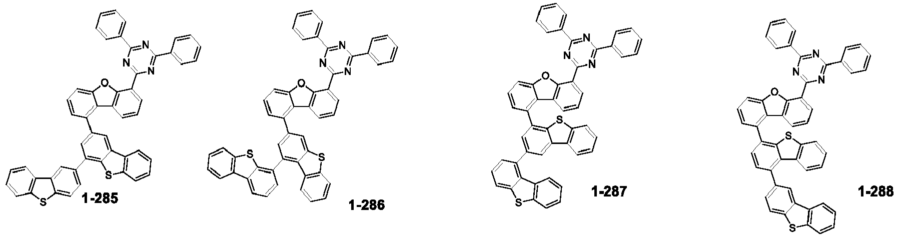

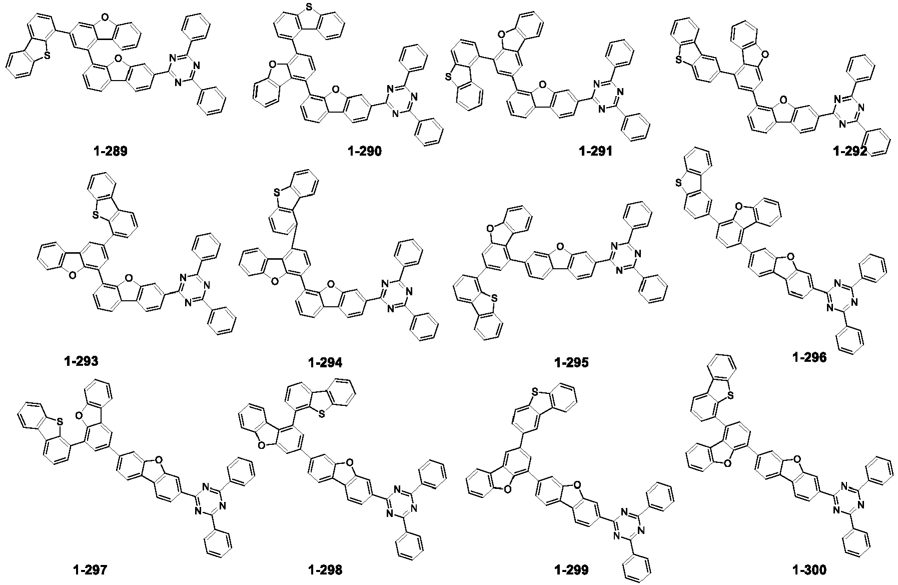

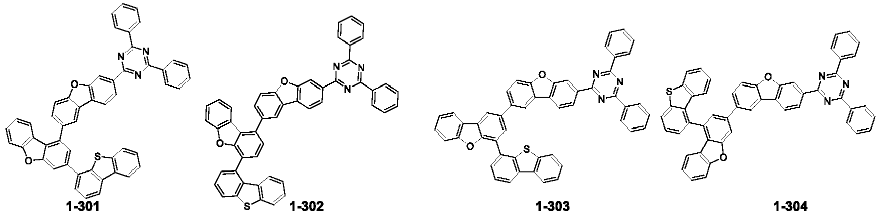

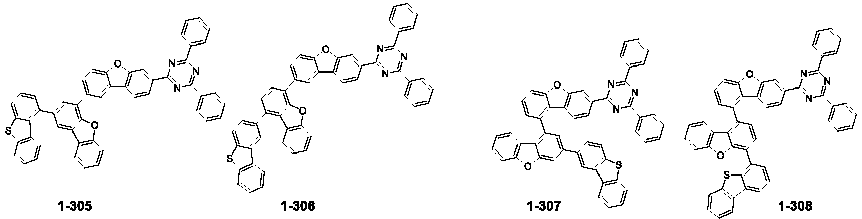

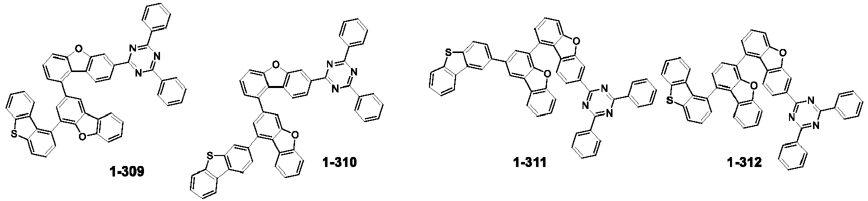

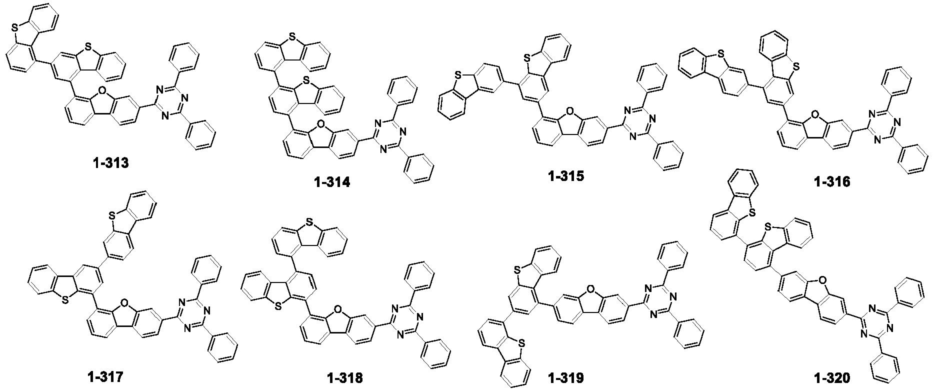

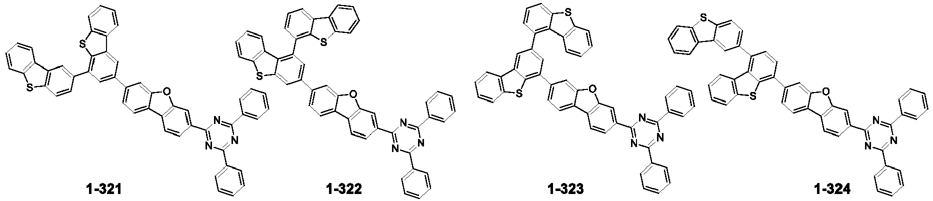

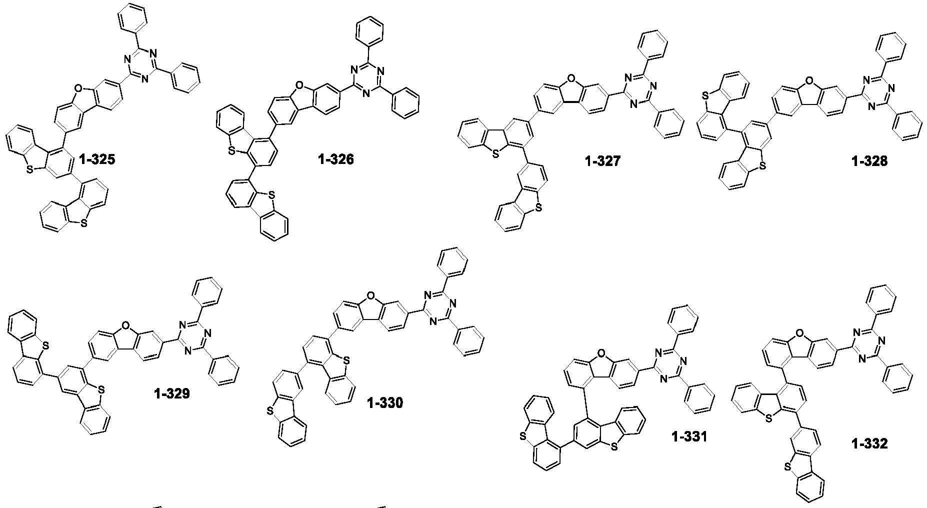

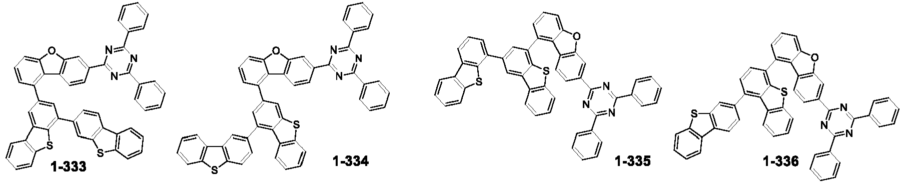

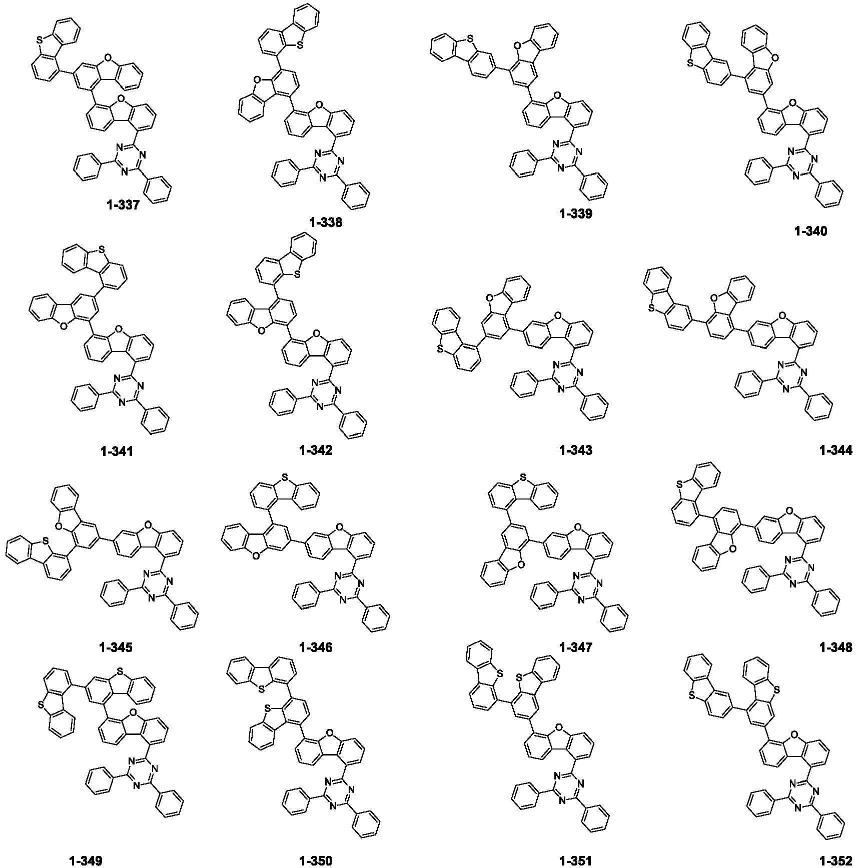

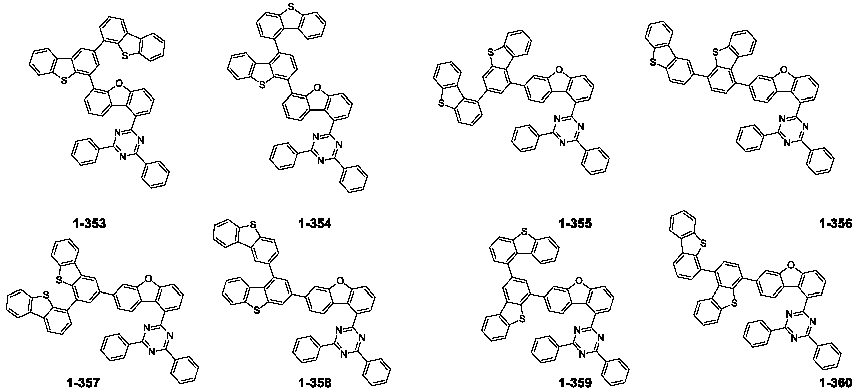

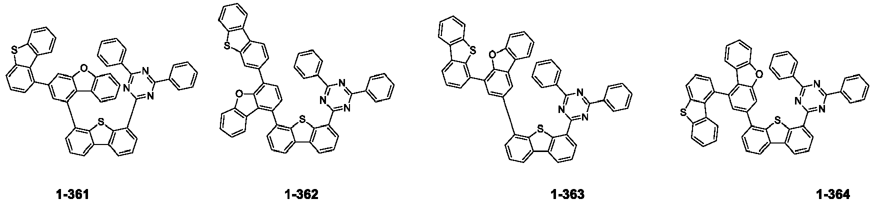

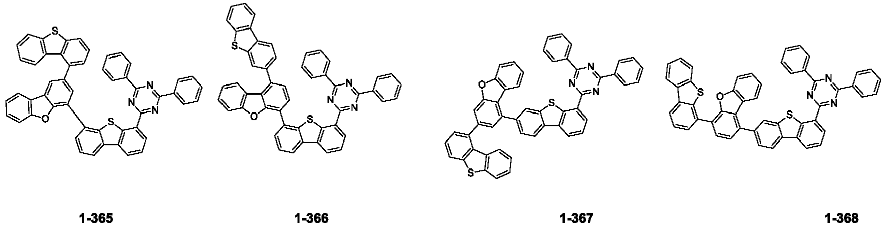

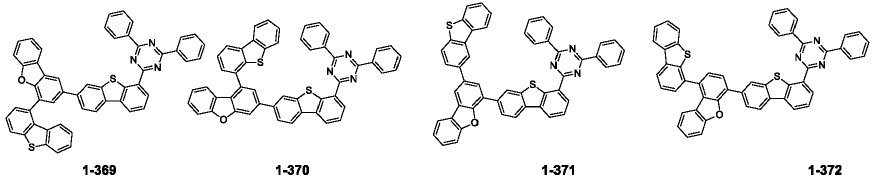

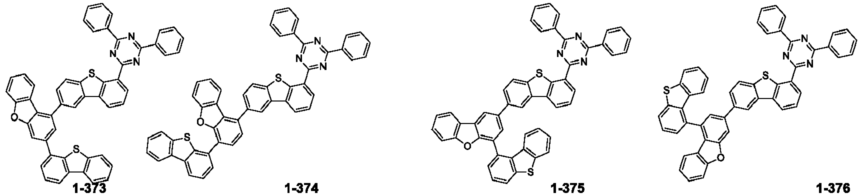

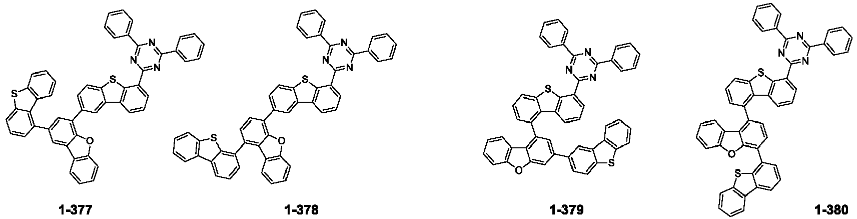

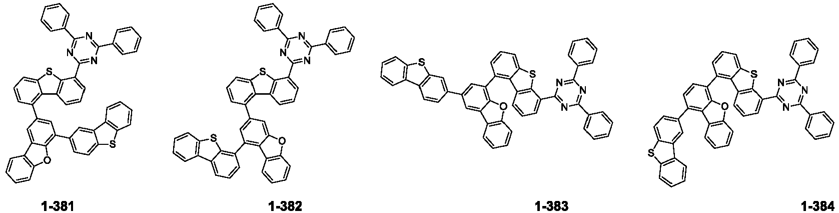

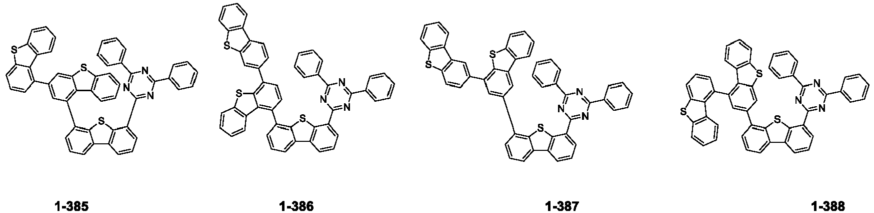

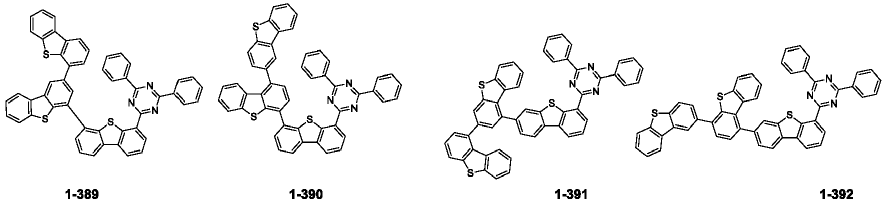

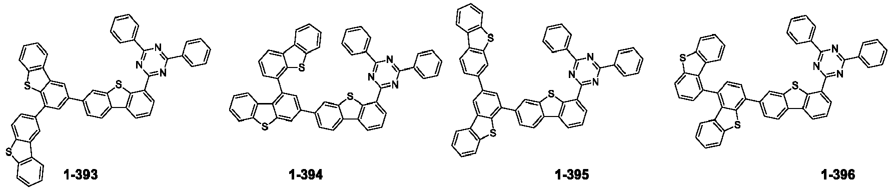

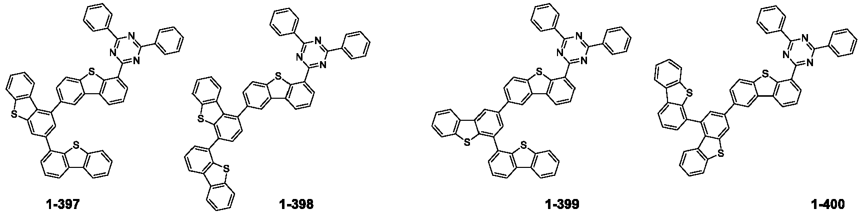

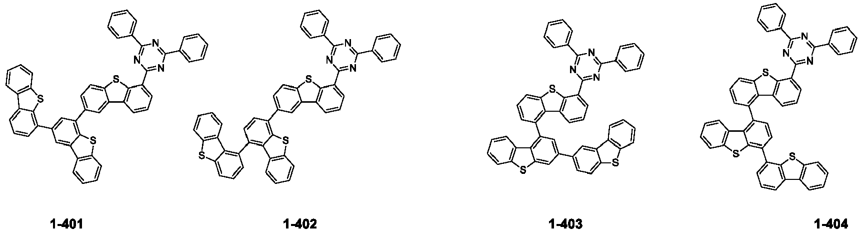

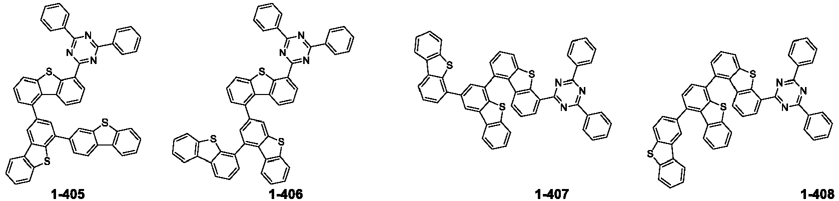

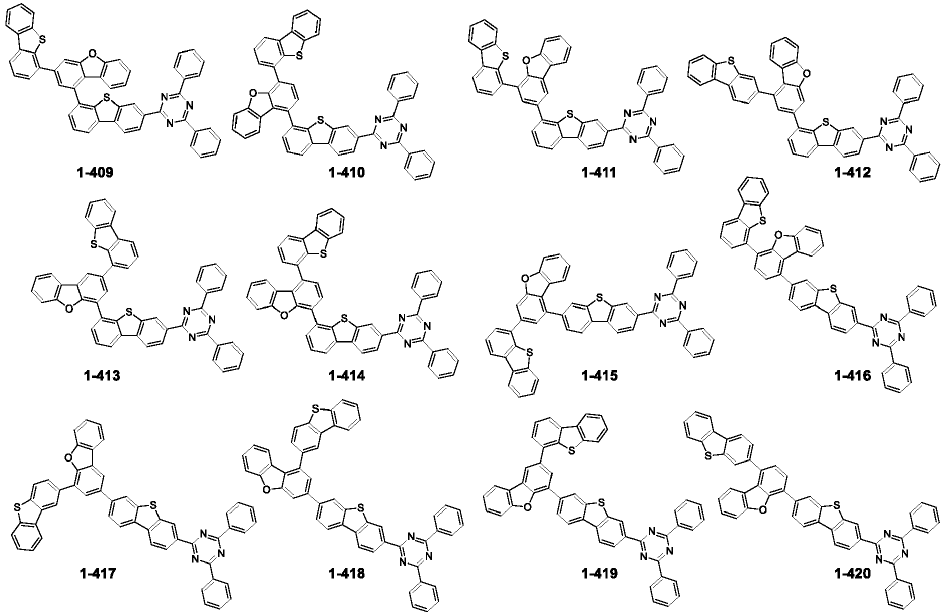

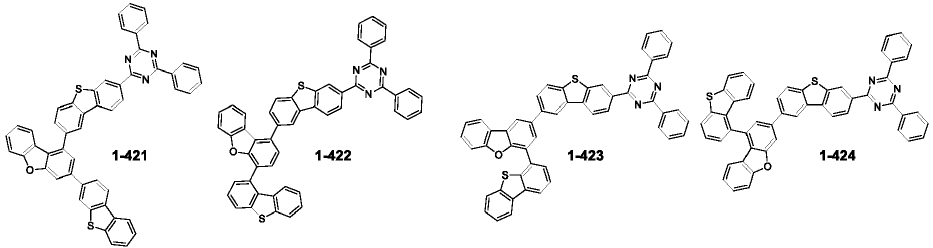

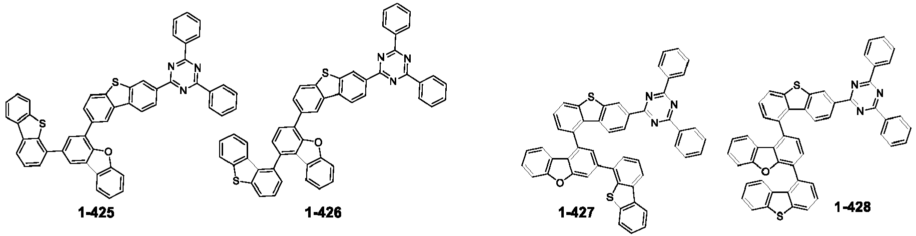

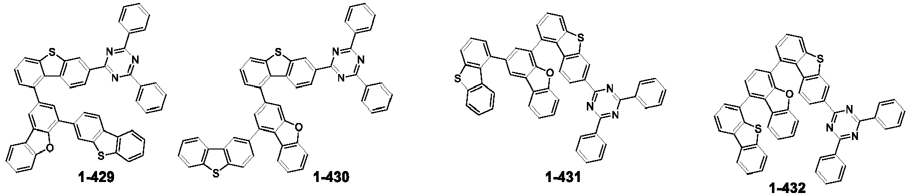

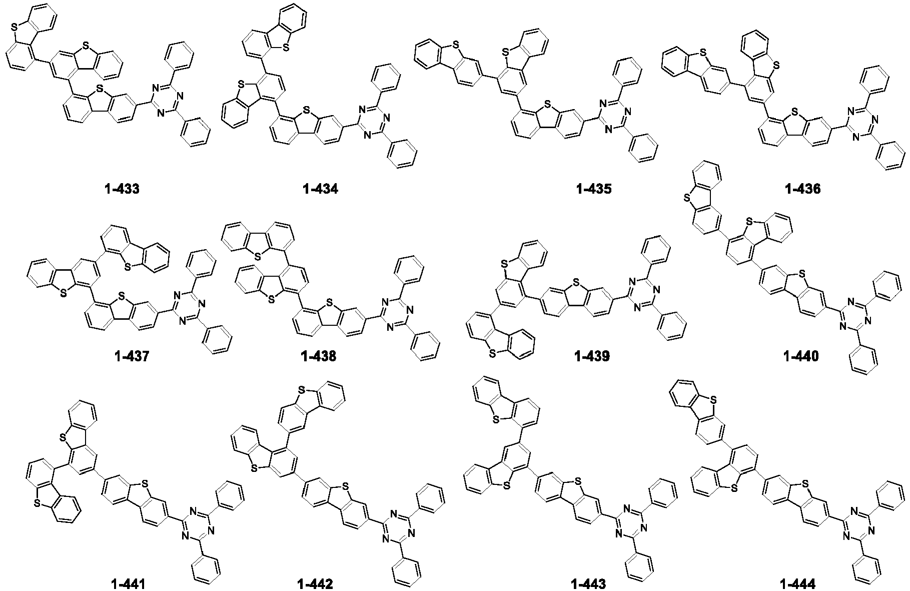

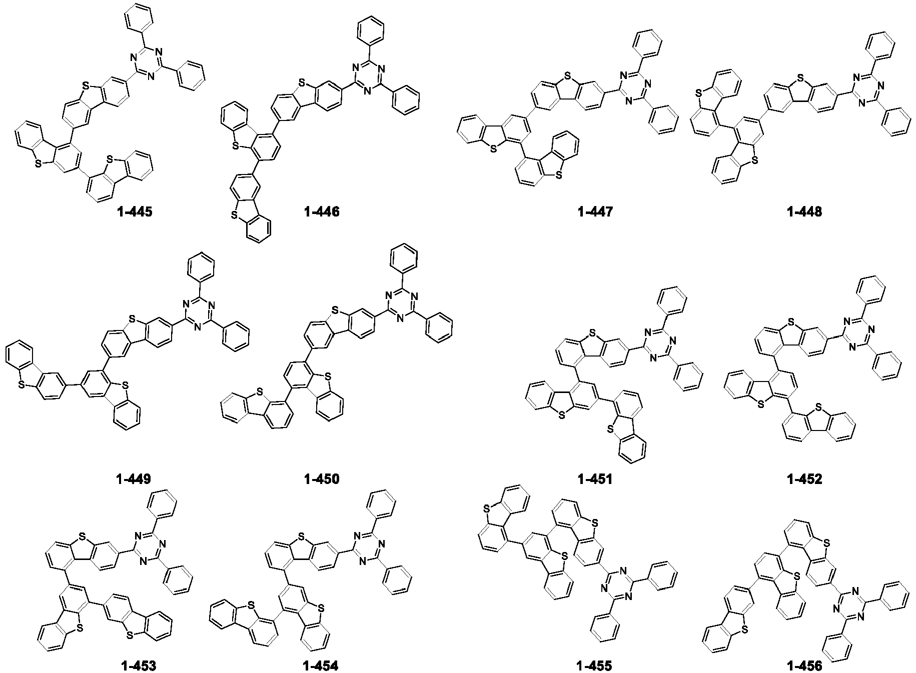

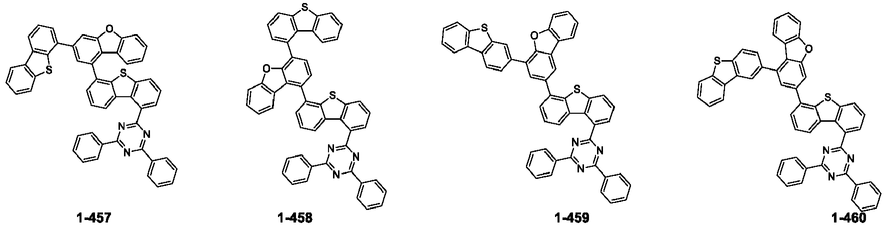

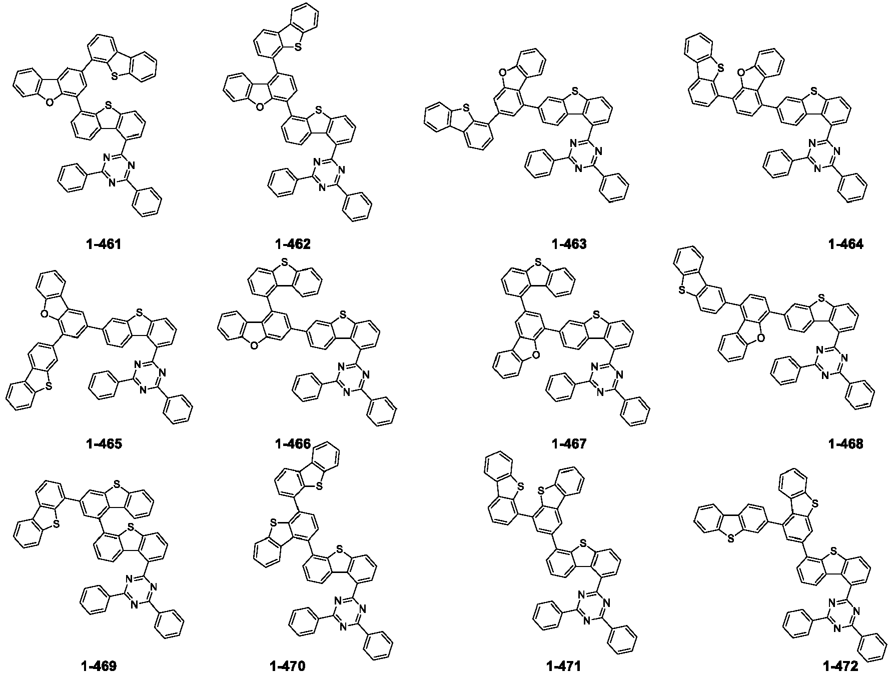

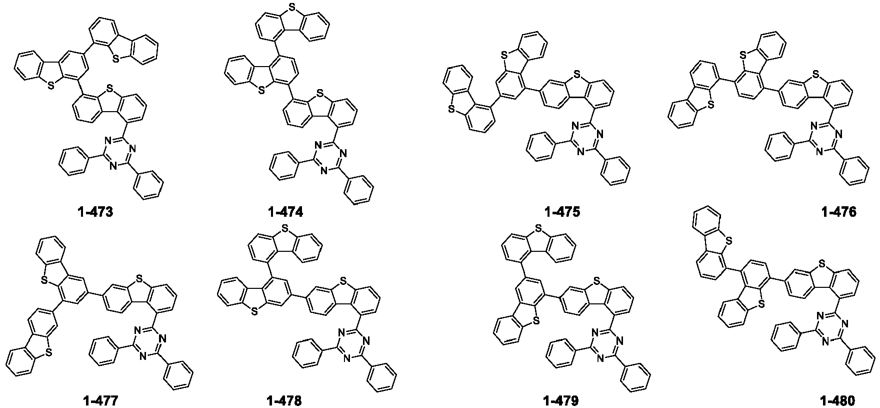

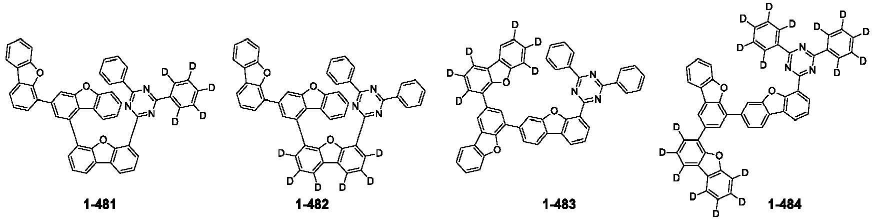

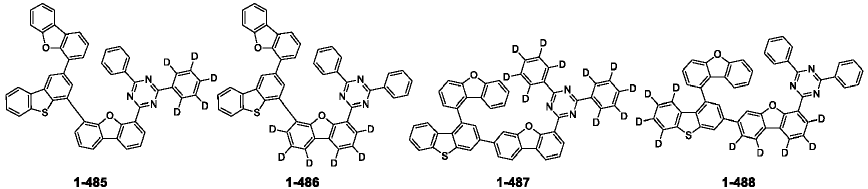

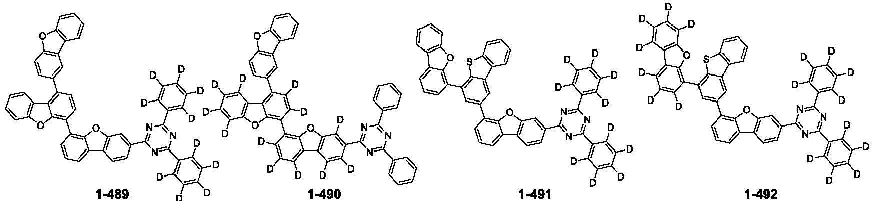

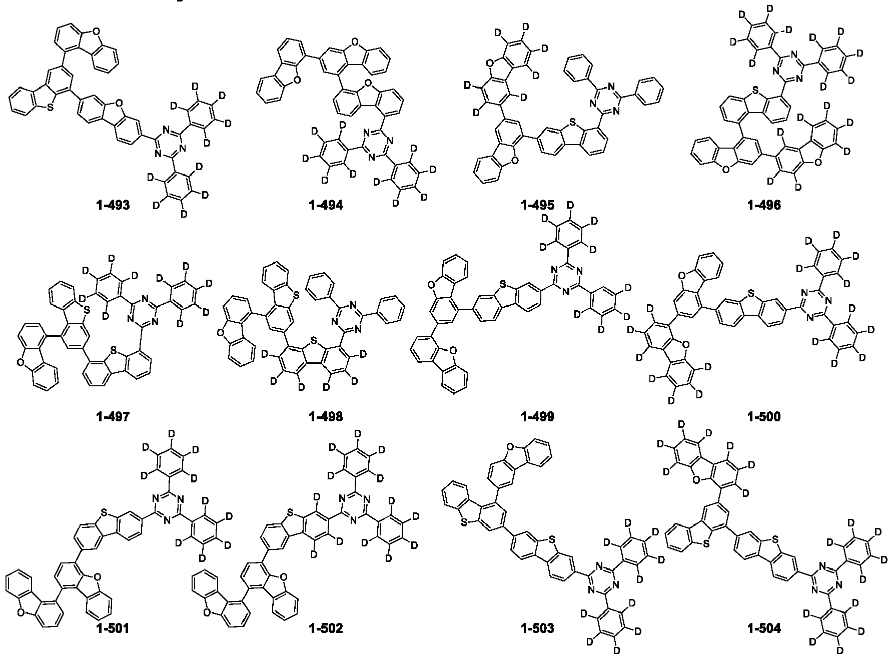

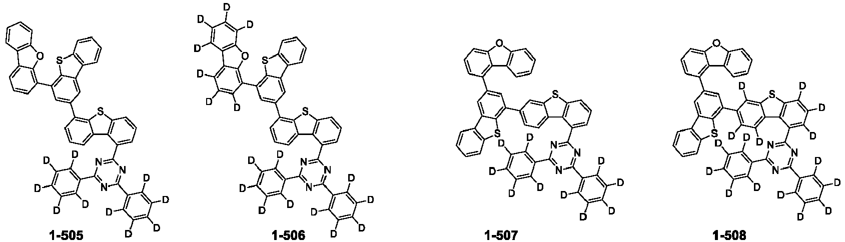

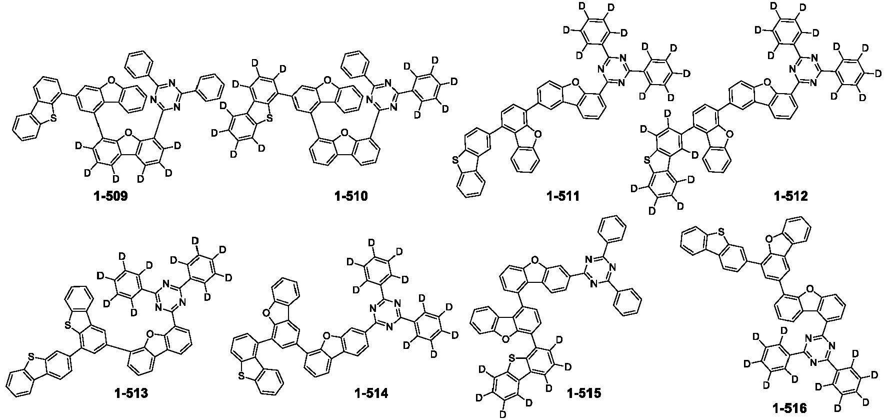

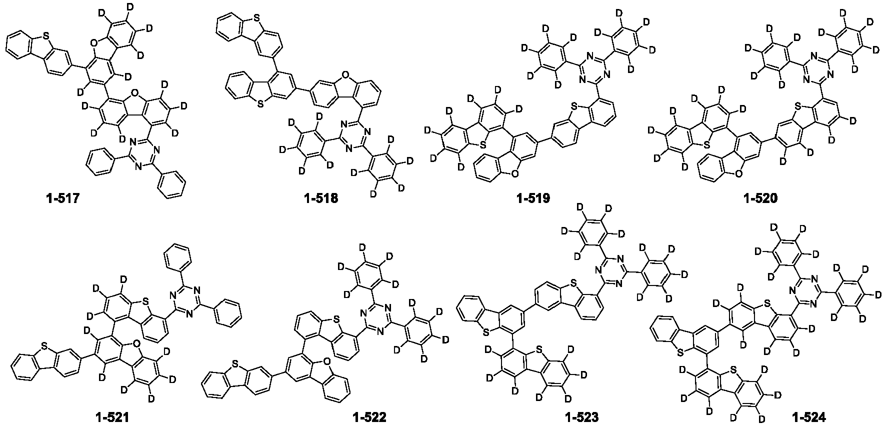

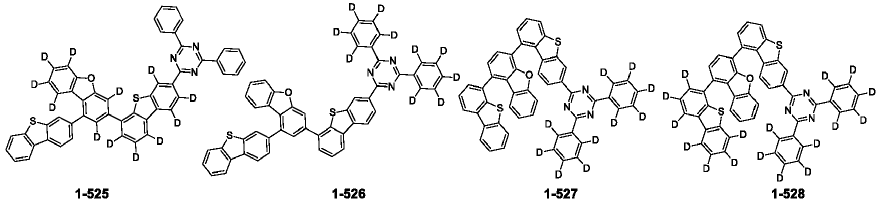

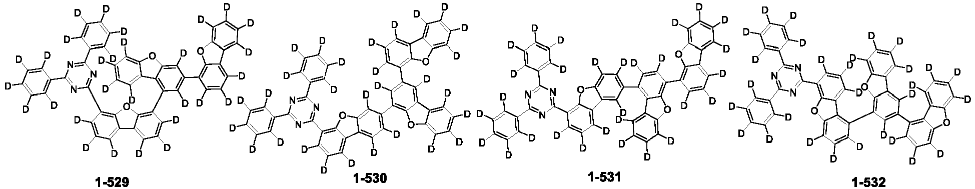

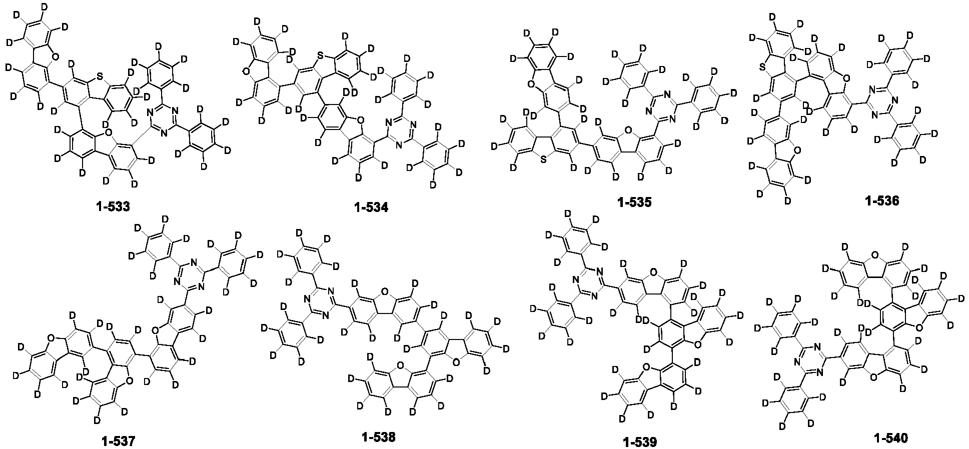

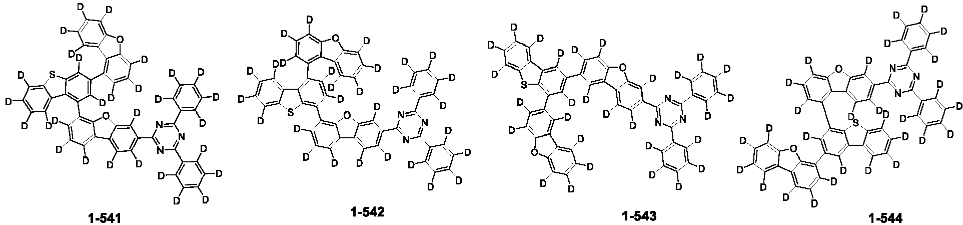

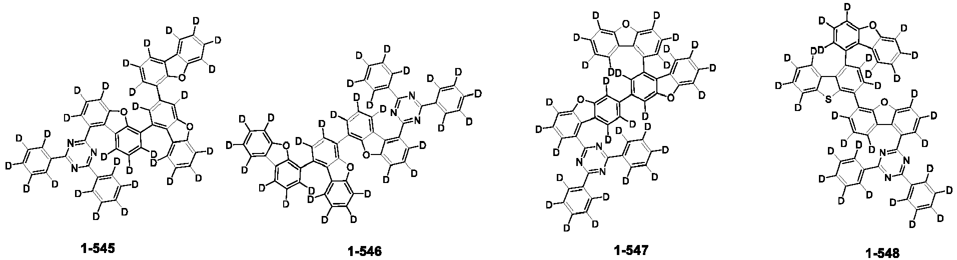

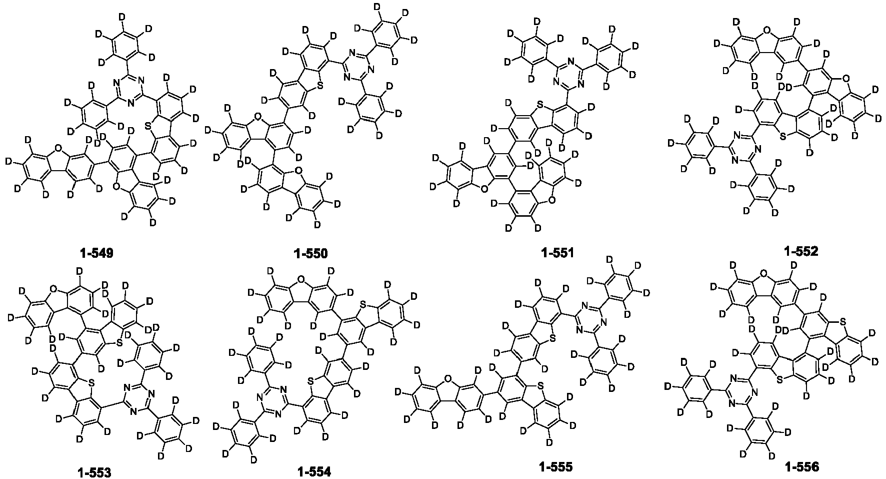

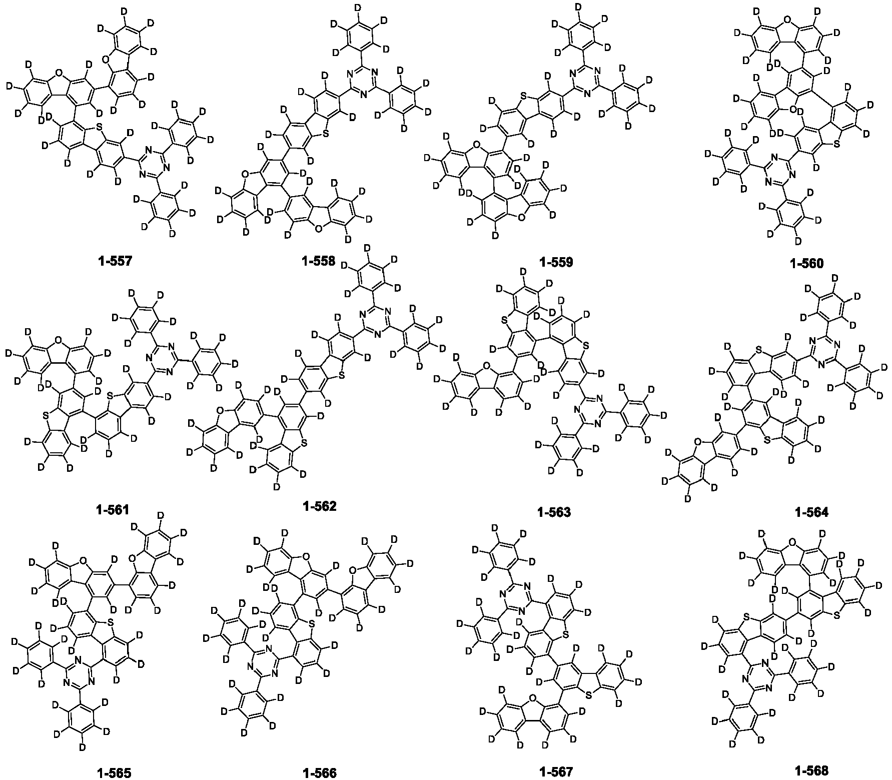

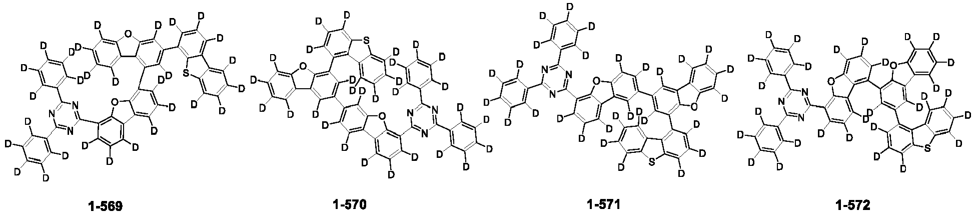

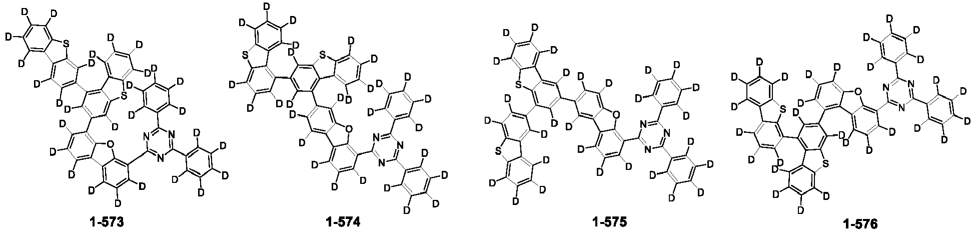

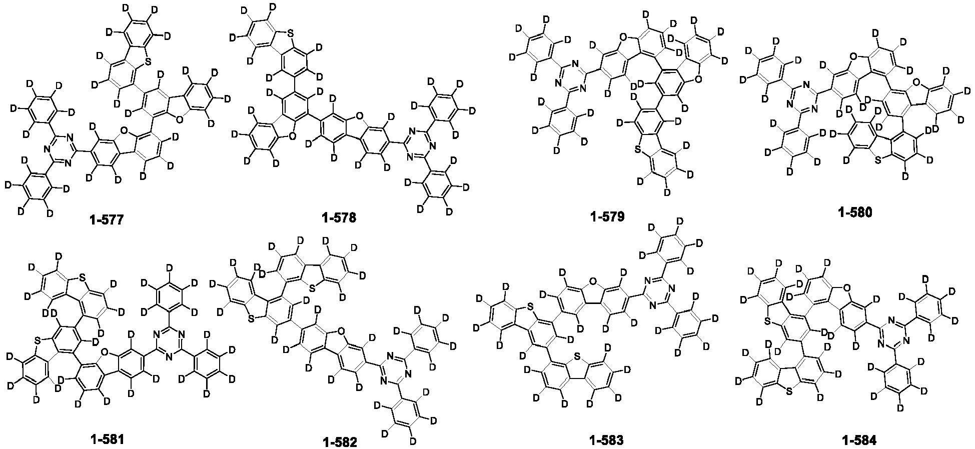

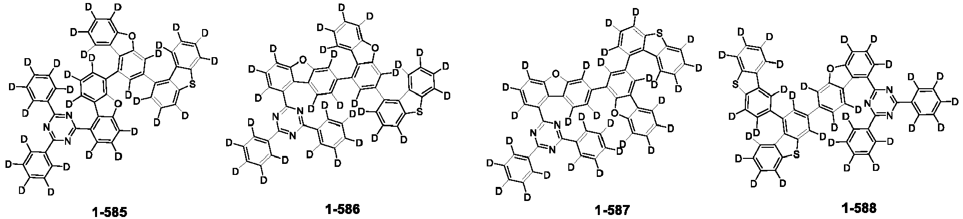

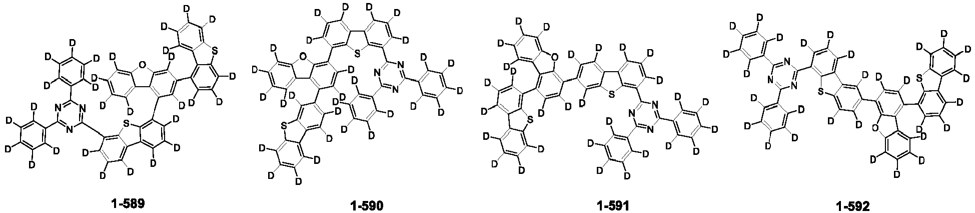

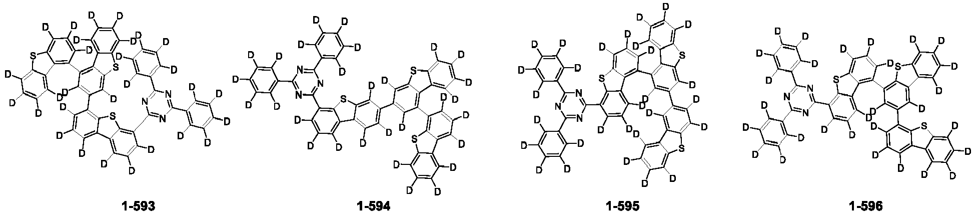

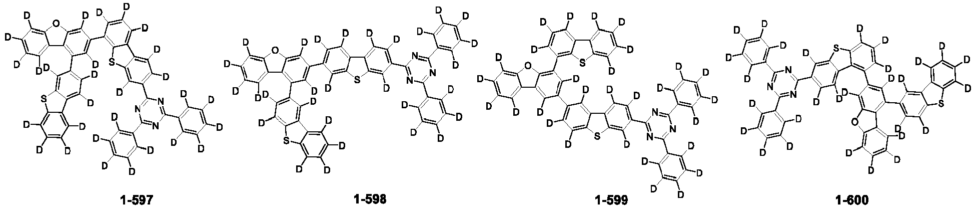

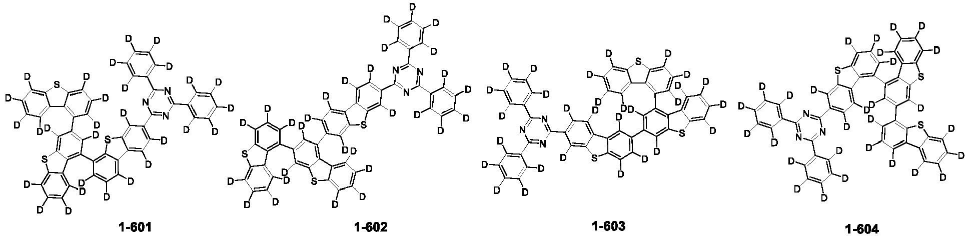

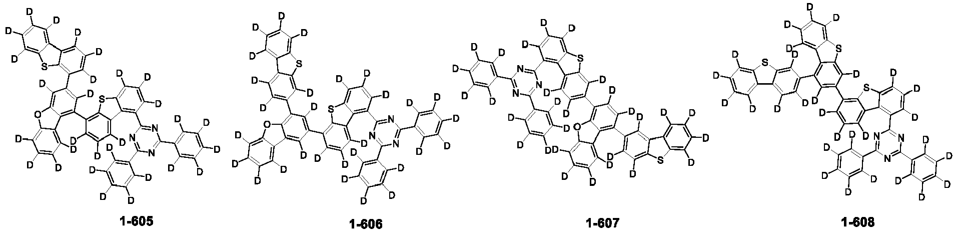

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a material for transporting holes, a light emitting layer material, an electron transport layer material, and a charge generation layer material, which are used for preparing an organic light emitting device, into the core structure.

In addition, it is possible to finely adjust an energy band-gap by introducing various substituents into the structure of Chemical Formula 1, and meanwhile, it is possible to improve characteristics at the interface between organic materials and diversify the use of the material.

Meanwhile, the compound has a high glass transition temperature (Tg) and thus has excellent thermal stability. The increase in thermal stability becomes an important factor for providing a device with driving stability.

Further, in an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocycle according to Chemical Formula 1 may be used as a material for the blue organic light emitting device.

In an exemplary embodiment of the present application, the organic light emitting device may be a green organic light emitting device, and the compound represented by Chemical Formula 1 may be used as a material for the green organic light emitting device.

In an exemplary embodiment of the present application, the organic light emitting device may be a red organic light emitting device, and the compound represented by Chemical Formula 1 may be used as a material for the red organic light emitting device.

In an exemplary embodiment of the present application, the organic light emitting device may be a blue organic light emitting device, and the heterocycle according to Chemical Formula 1 may be used as a material for a light emitting layer of the blue organic light emitting device.

In an exemplary embodiment of the present application, the organic light emitting device may be a green organic light emitting device, and the compound represented by Chemical Formula 1 may be used as a material for a light emitting layer of the green organic light emitting device.

In an exemplary embodiment of the present application, the organic light emitting device may be a red organic light emitting device, and the compound represented by Chemical Formula 1 may be used as a material for a light emitting layer of the red organic light emitting device.

The specific content on the heterocyclic compound represented by Chemical Formula 1 is the same as that described above.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In the organic light emitting device according to an exemplary embodiment of the present application, provided is an organic light emitting device in which an organic material layer including the heterocyclic compound of Chemical Formula 1 further includes: a heterocyclic compound represented by the following Chemical Formula 2; or a heterocyclic compound represented by the following Chemical Formula 3.

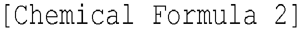

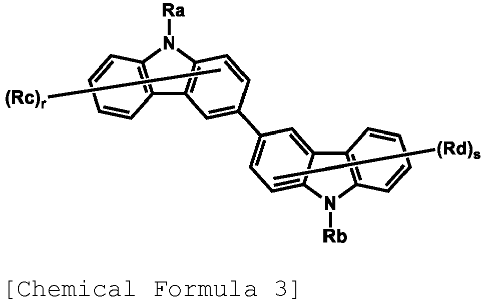

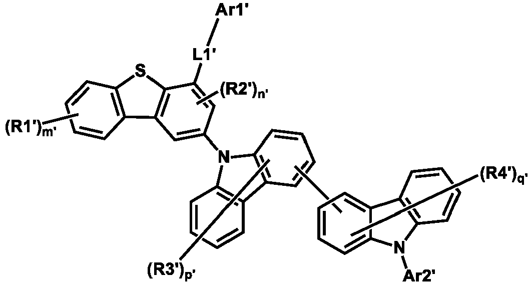

In Chemical Formulae 2 and 3,
Rc, Rd and R1' to R4' are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring,
Ra and Rb are the same as or different from each other, and are each independently a substituted or unsubstituted C6 to C60 aryl group; or a substituted or unsubstituted C2 to C60 heteroaryl group,
L1' is a direct bond; or a substituted or unsubstituted C6 to C60 arylene group,
Ar1' is a substituted or unsubstituted C6 to C60 aryl group; or a substituted or unsubstituted C2 to C60 heteroaryl group including at least one of S and O,
Ar2' is a substituted or unsubstituted C6 to C60 aryl group; or a substituted or unsubstituted C2 to C60 heteroaryl group,
m' is an integer from 0 to 4,
n' is an integer from 0 to 2,
p', q', r and s are an integer from 0 to 7, and
when m', p', q', n', r and s are each an integer of 2 or higher, substituents in the parenthesis are the same as or different from each other.

In the organic light emitting device according to an exemplary embodiment of the present application, Rc and Rd of Chemical Formula 2 may be hydrogen; or deuterium.

In the organic light emitting device according to an exemplary embodiment of the present application, Ra and Rb of Chemical Formula 2 are the same as or different from each other, and may be each independently a substituted or unsubstituted C6 to C60 aryl group; or a substituted or unsubstituted C2 to C60 heteroaryl group.

In the organic light emitting device according to another exemplary embodiment, Ra and Rb of Chemical Formula 2 are the same as or different from each other, and may be each independently a C6 to C40 aryl group which is unsubstituted or substituted with one or more substituents selected from the group consisting of a C1 to C40 alkyl group, a C6 to C40 aryl group, -CN and -SiR110R111R112; or a C2 to C40 heteroaryl group which is unsubstituted or substituted with one or more substituents selected from the group consisting of a C6 to C40 aryl group and a C2 to C40 heteroaryl group.

In the organic light emitting device according to still another exemplary embodiment, Ra and Rb of Chemical Formula 2 are the same as or different from each other, and may be each independently a C6 to C40 aryl group which is unsubstituted or substituted with one or more substituents selected from the group consisting of a C1 to C40 alkyl group, a C6 to C40 aryl group, -CN and -SiR110R111R112.

In the organic light emitting device according to yet another exemplary embodiment, Ra and Rb of Chemical Formula 2 are the same as or different from each other, and may be each independently a phenyl group which is unsubstituted or substituted with a phenyl group, -CN or -SiR110R111R112; a biphenyl group which is unsubstituted or substituted with a phenyl group; a naphthyl group; a fluorenyl group which is unsubstituted or substituted with a methyl group or a phenyl group; a spirobifluorenyl group; or a triphenylenyl group.

The definitions of R110, R111 and R112 may be the same as the definitions of R, R' and R".

In an exemplary embodiment of the present application, the deuterium content of the heterocyclic compound represented by Chemical Formula 2 may be 0% to 100%.

In another exemplary embodiment, the deuterium content of the heterocyclic compound represented by Chemical Formula 2 may be 0% to 100%, 5% to 100%, 7% to 100%, 10% to 100%, 13% to 100%, 15% to 100% or 20% to 100%.

In an exemplary embodiment of the present application, the deuterium content of the heterocyclic compound represented by Chemical Formula 2 may be 100%.

In an exemplary embodiment of the present application, 100% of Ra and Rb of Chemical Formula 2 may be substituted with deuterium.

In an exemplary embodiment of the present application, the deuterium content of the heterocyclic compound represented by Chemical Formula 3 may be 0% to 100%.

In another exemplary embodiment, the deuterium content of the heterocyclic compound represented by Chemical Formula 3 may be 0% to 100%, 5% to 100%, 7% to 100%, 10% to 100%, 13% to 100%, 15% to 100% or 20% to 100%.

In an exemplary embodiment of the present application, the deuterium content of the heterocyclic compound represented by Chemical Formula 3 may be 100%.

In the organic light emitting device according to an exemplary embodiment of the present application, R1' to R4' of Chemical Formula 3 may be hydrogen; or deuterium.

In an exemplary embodiment of the present application, Ar2' of Chemical Formula 3 may be a substituted or unsubstituted C6 to C60 aryl group; or a substituted or unsubstituted C2 to C60 heteroaryl group.

In another exemplary embodiment, Ar2' of Chemical Formula 3 may be a substituted or unsubstituted C6 to C40 aryl group; or a substituted or unsubstituted C2 to C40 heteroaryl group.

In still another exemplary embodiment, Ar2' of Chemical Formula 3 may be a substituted or unsubstituted C6 to C40 aryl group.

In yet another exemplary embodiment, Ar2' of Chemical Formula 3 may be a C6 to C40 aryl group.

In yet another exemplary embodiment, Ar2' of Chemical Formula 3 may be a monocyclic C6 to C10 aryl group; or a polycyclic C10 to C20 aryl group.

In yet another exemplary embodiment, Ar2' of Chemical Formula 3 may be a phenyl group; or a biphenyl group.

In an exemplary embodiment of the present application, L1' of Chemical Formula 3 may be a direct bond; or a substituted or unsubstituted C6 to C60 arylene group.

In another exemplary embodiment, L1' of Chemical Formula 3 may be a direct bond; or a substituted or unsubstituted C6 to C40 arylene group.

In still another exemplary embodiment, L1' of Chemical Formula 3 may be a direct bond; or a substituted or unsubstituted C6 to C20 arylene group.

In yet another exemplary embodiment, L1' of Chemical Formula 3 may be a direct bond; or a C6 to C20 arylene group.

In yet another exemplary embodiment, L1' of Chemical Formula 3 may be a direct bond; a phenylene group; or a biphenylene group.

In an exemplary embodiment of the present application, Ar1' may be a substituted or unsubstituted C6 to C60 aryl group; or a C2 to C60 heteroaryl group which is unsubstituted or substituted, and includes at least one of S and O.

In another exemplary embodiment, Ar1' may be a substituted or unsubstituted C6 to C40 aryl group; or a C2 to C40 heteroaryl group which is unsubstituted or substituted, and includes at least one of S and O.

In still another exemplary embodiment, Ar1' may be a C6 to C40 aryl group which is unsubstituted or substituted with a C1 to C20 alkyl group or a C6 to C20 aryl group; or a C2 to C40 heteroaryl group including at least one of S and O.

In yet another exemplary embodiment, Ar1' may be a phenyl group; a biphenyl group; a naphthyl group; a dimethylfluorenyl group; a diphenylfluorenyl group; a dibenzofuran group; or a dibenzothiophene group.

When the compound of Chemical Formula 1; and the compound of Chemical Formula 2 or 3 are included in the organic material layer of the organic light emitting device, better efficiency and service life effects are exhibited. From this result, it can be expected that an exciplex phenomenon will occur when two compounds are simultaneously included.

The exciplex phenomenon is a phenomenon in which energy with a magnitude of the HOMO level of a donor (p-host) and the LUMO level of an acceptor (n-host) is released due to an electron exchange between two molecules. When the exciplex phenomenon between two molecules occurs, a reverse intersystem crossing (RISC) occurs, and the internal quantum efficiency of fluorescence can be increased to 100% due to the RISC. When a donor with a good hole transport capacity (p-host) and an acceptor with a good electron transport capacity (n-host) are used as hosts for the light emitting layer, holes are injected into the p-host and electrons are injected into the n-host, so that the driving voltage can be lowered, which can help to improve the service life.

In an exemplary embodiment of the present application, the heterocyclic compound represented by Chemical Formula 2 may be any one selected among the following compounds.

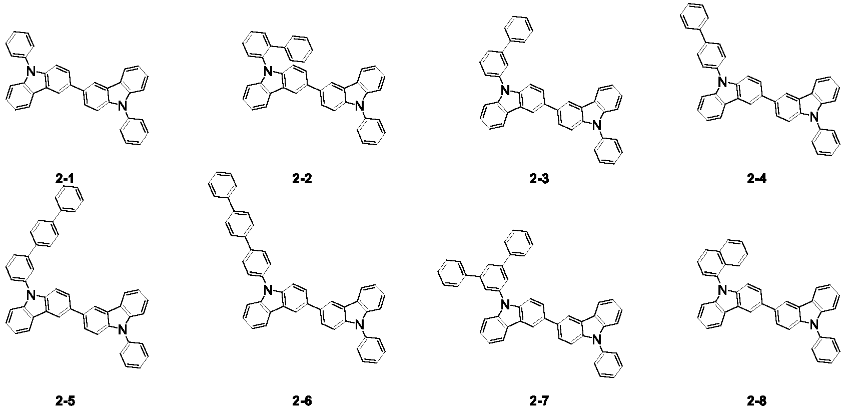

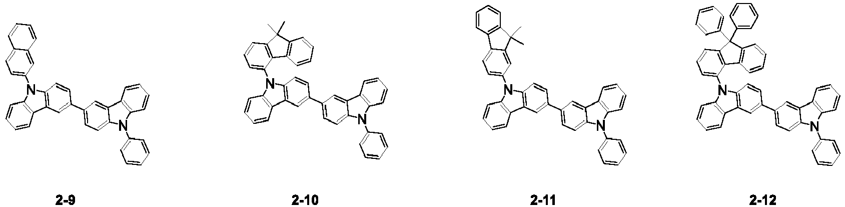

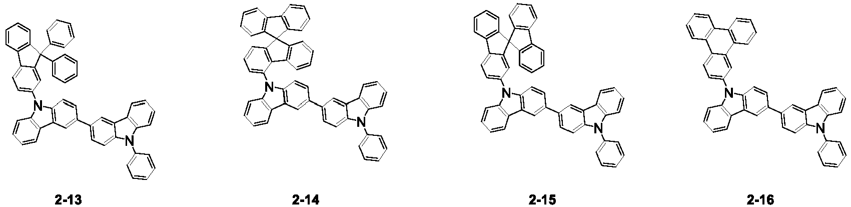

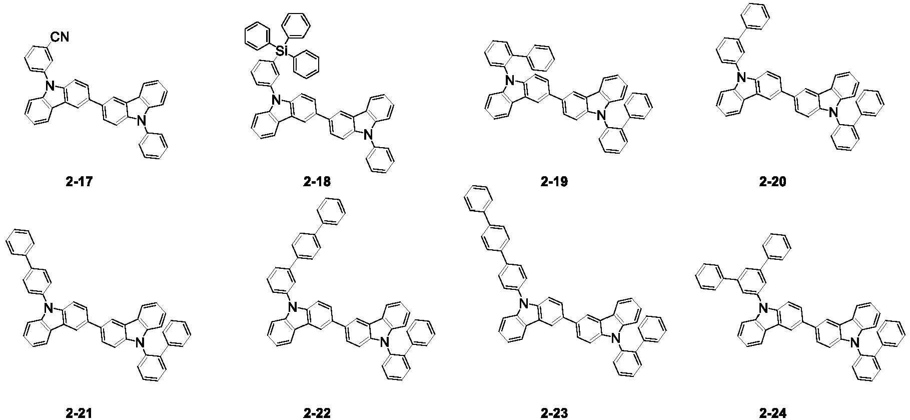

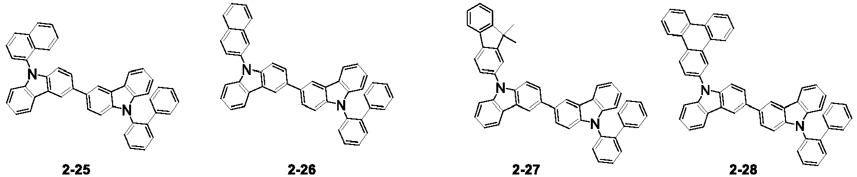

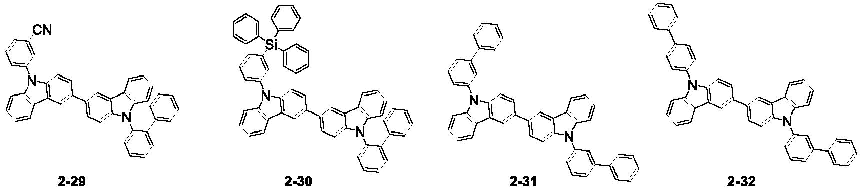

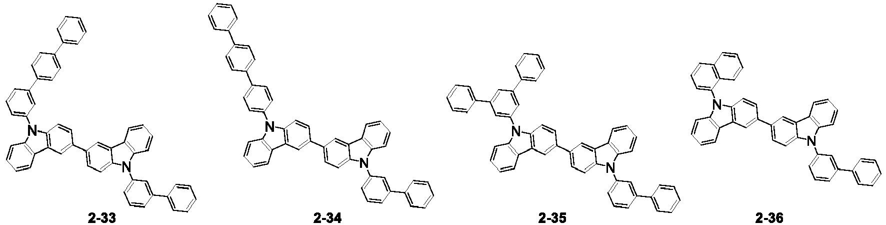

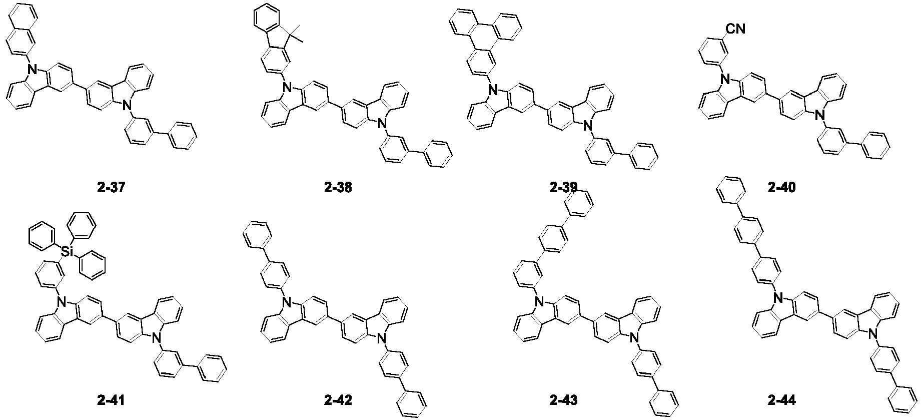

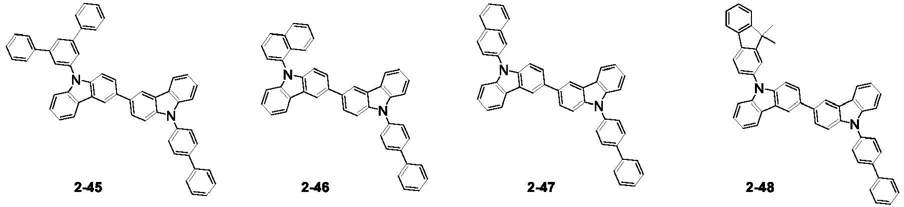

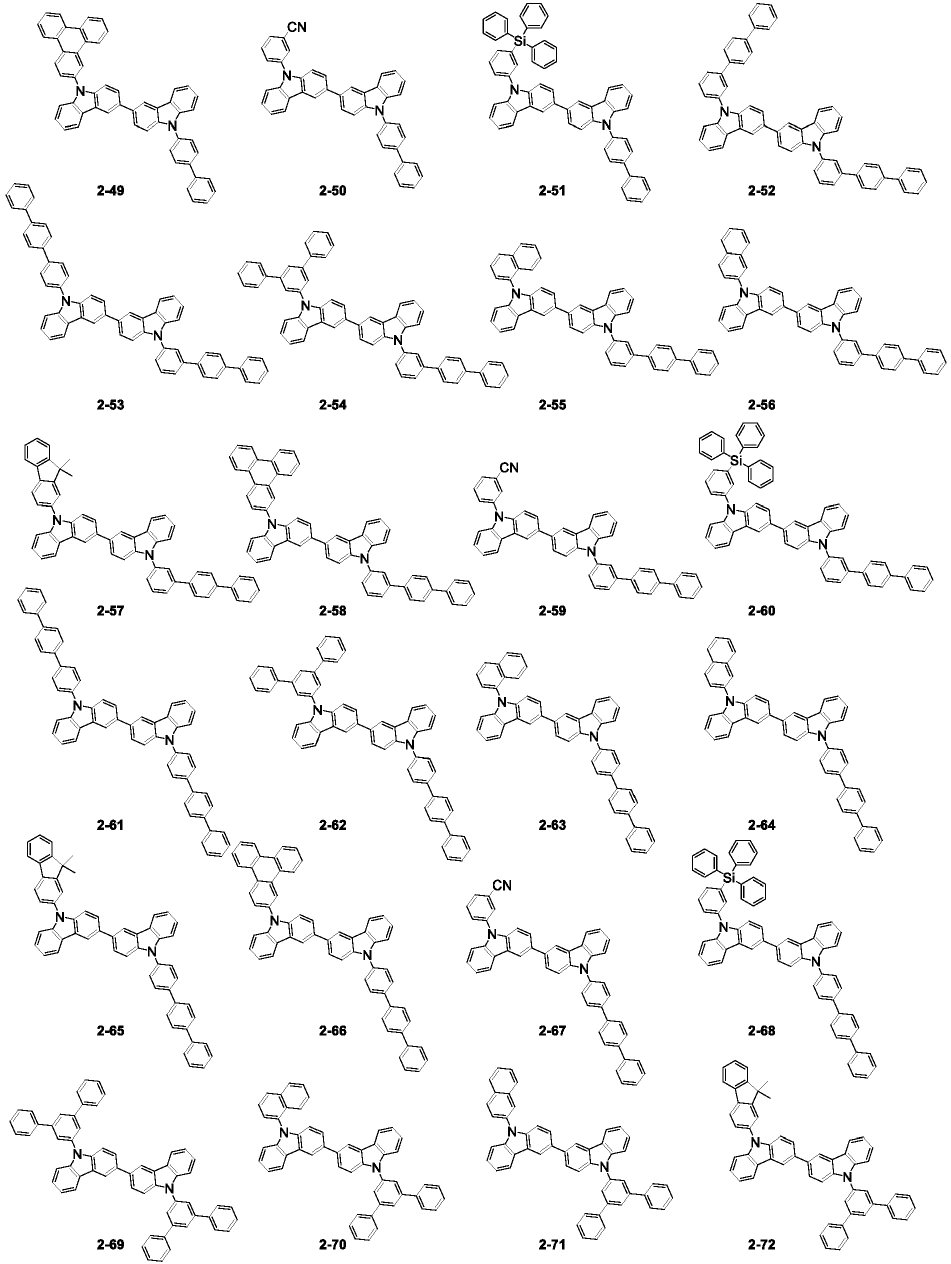

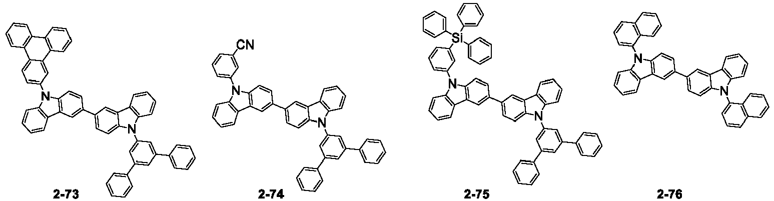

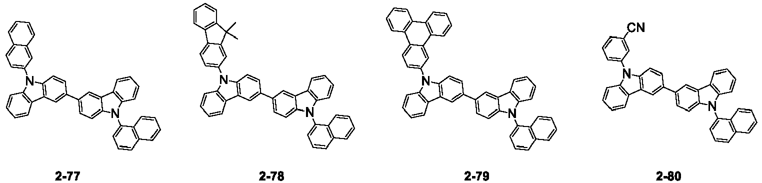

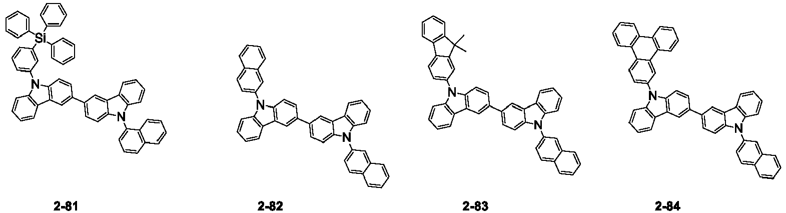

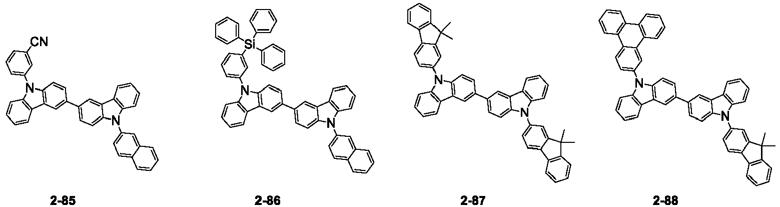

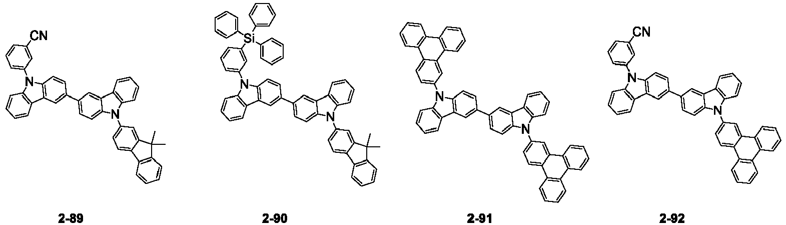

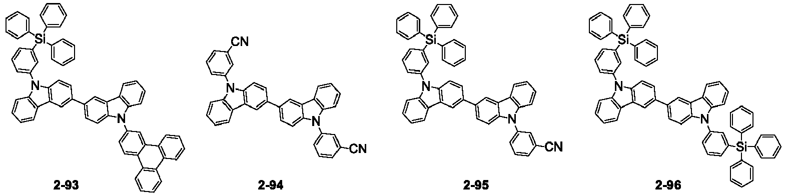

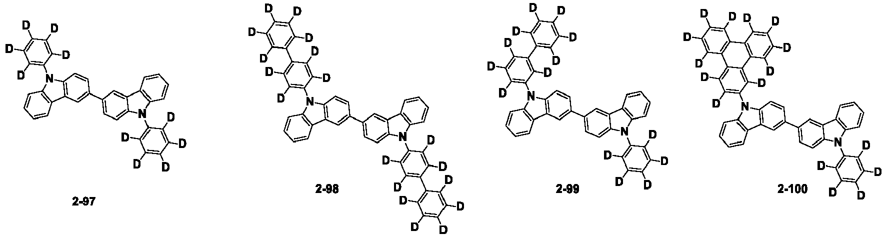

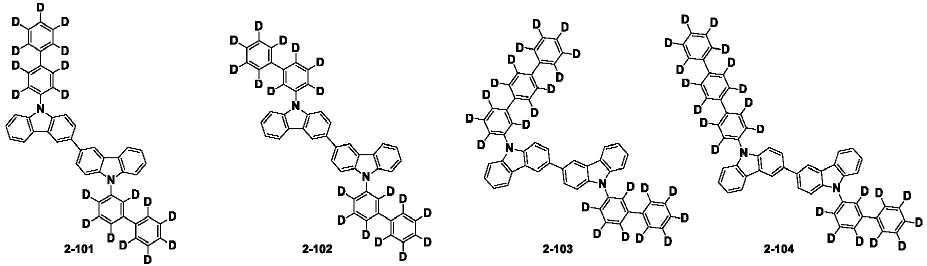

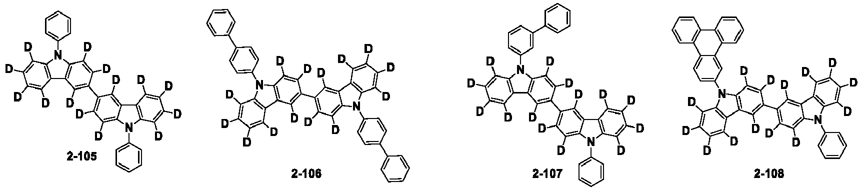

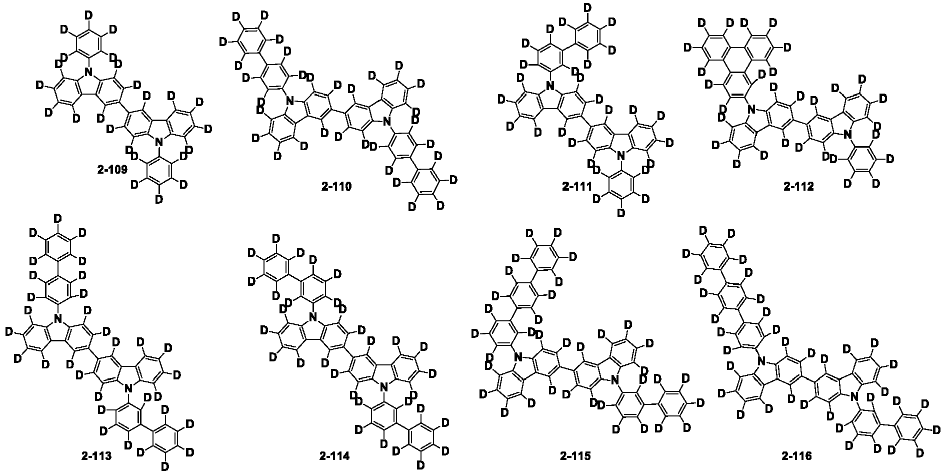

In an exemplary embodiment of the present application, the heterocyclic compound represented by Chemical Formula 3 may be any one selected among the following compounds.

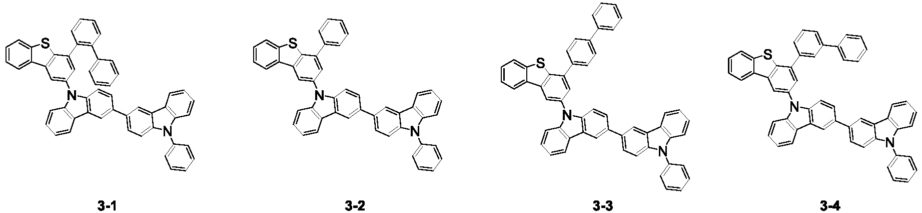

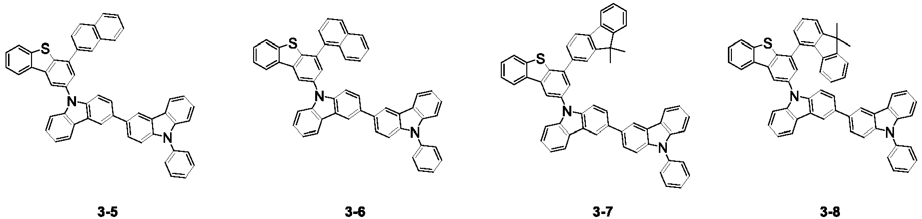

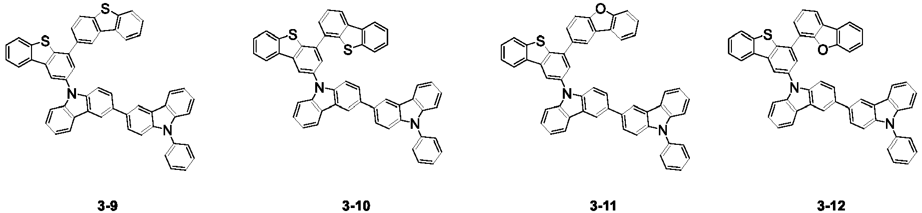

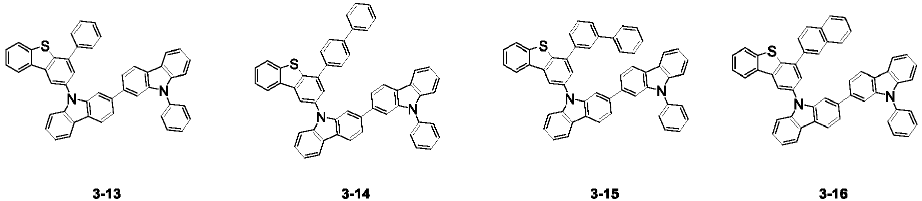

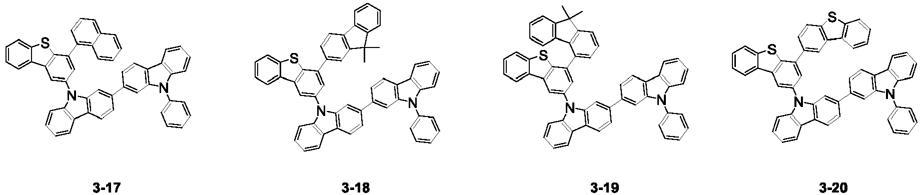

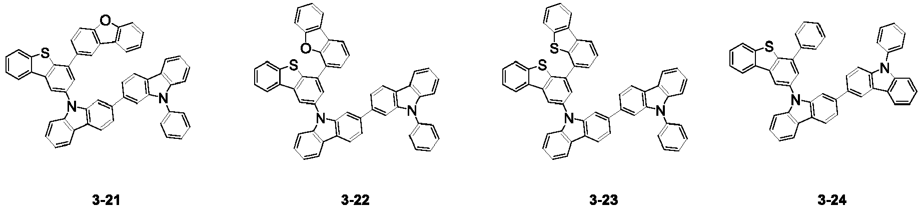

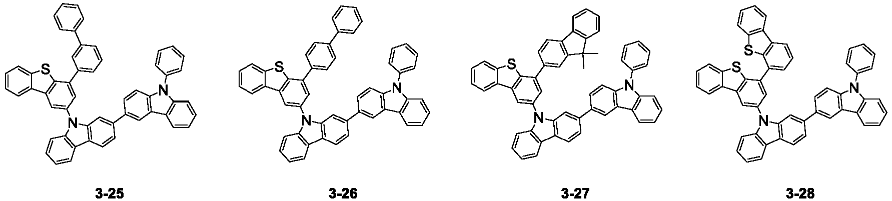

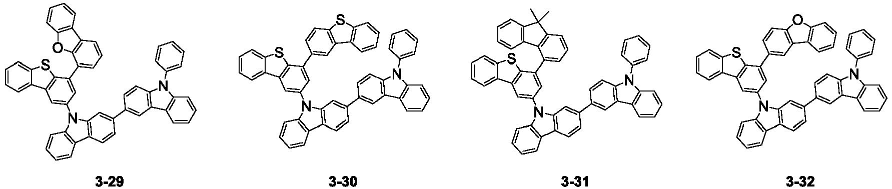

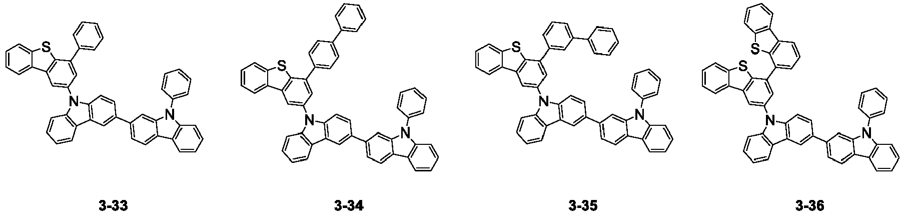

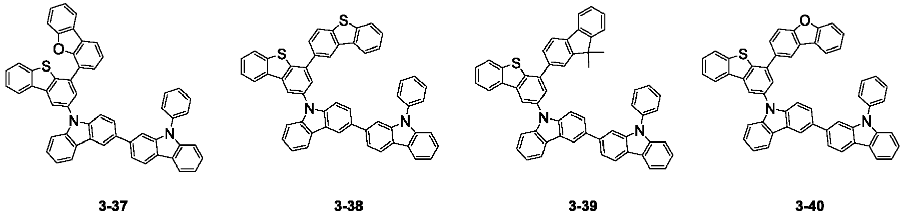

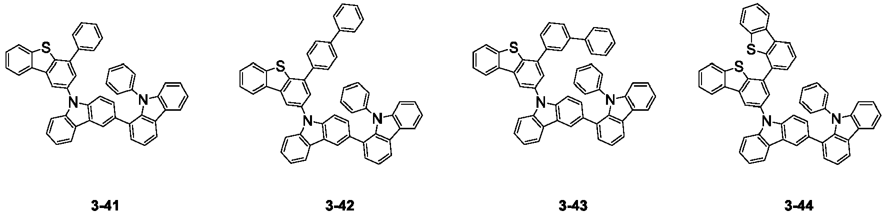

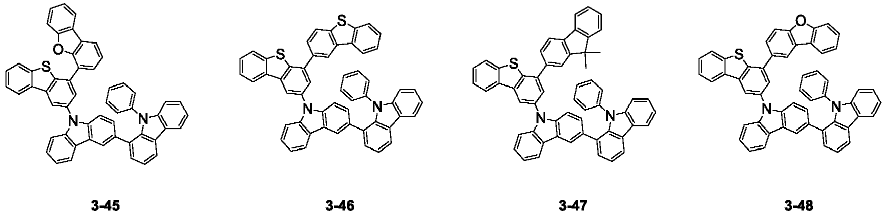

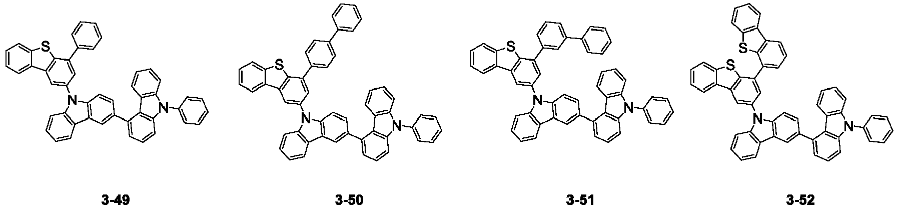

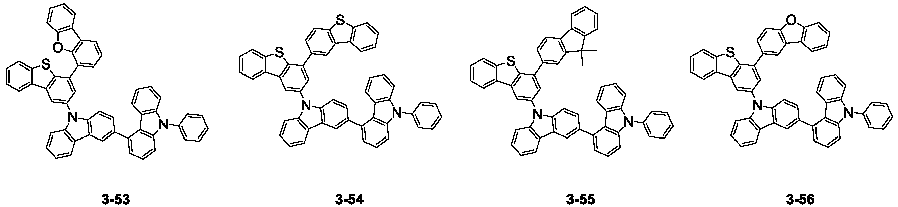

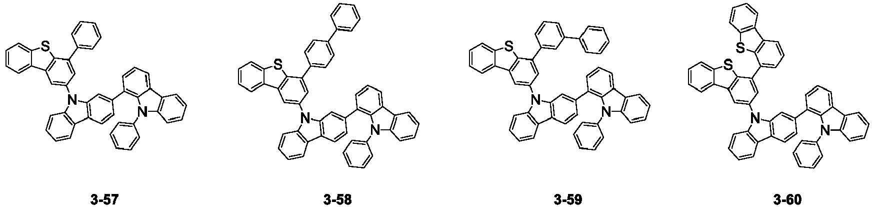

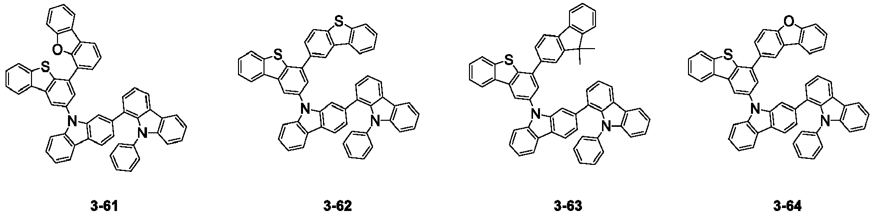

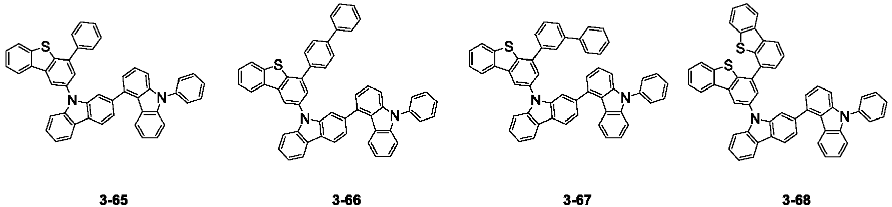

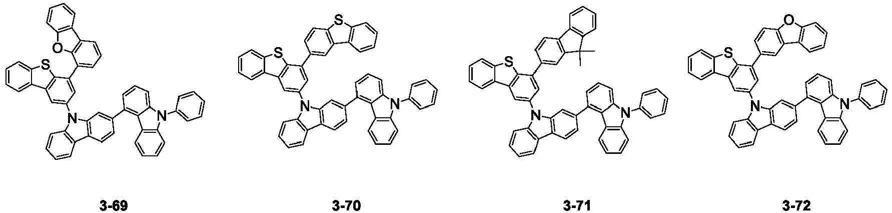

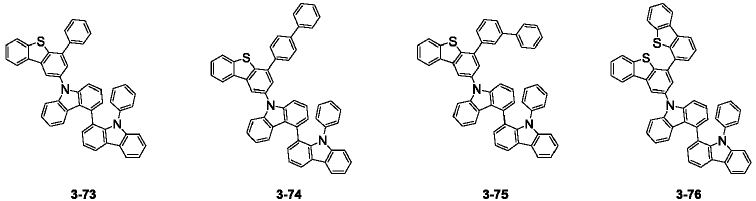

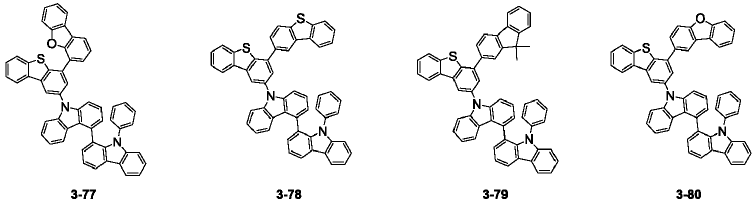

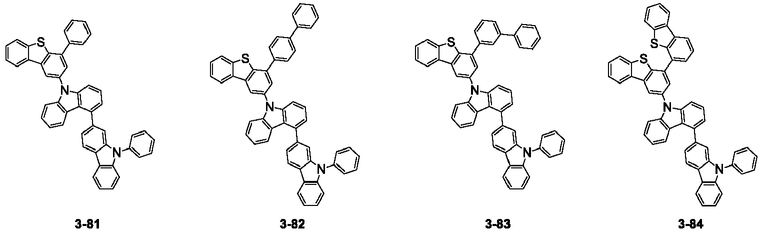

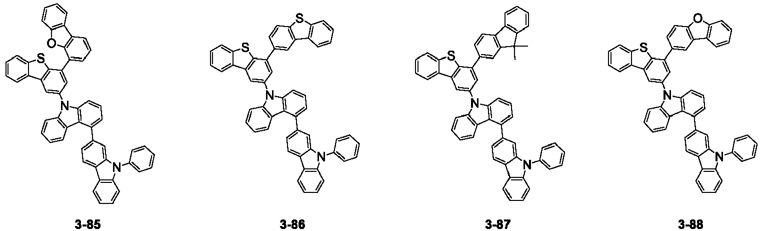

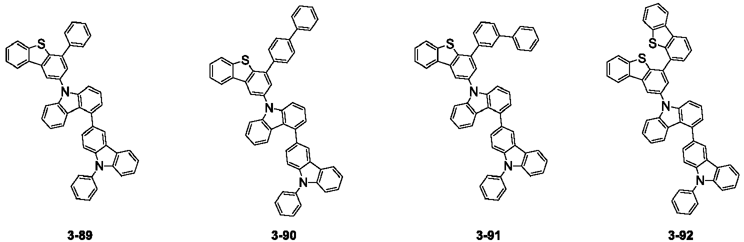

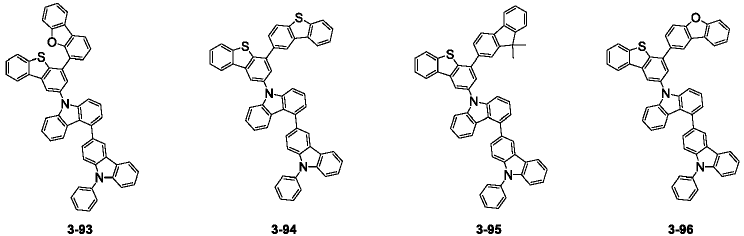

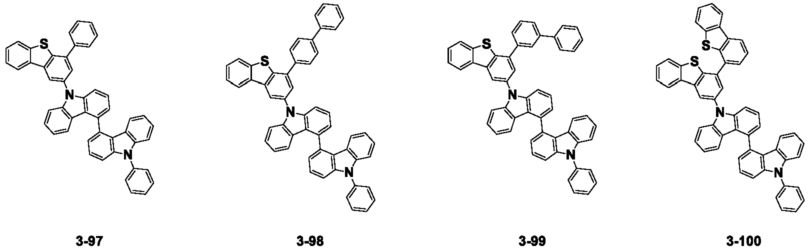

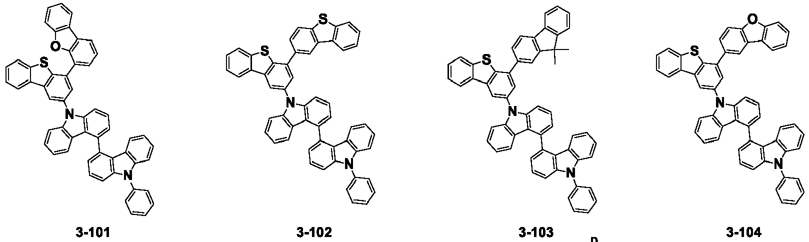

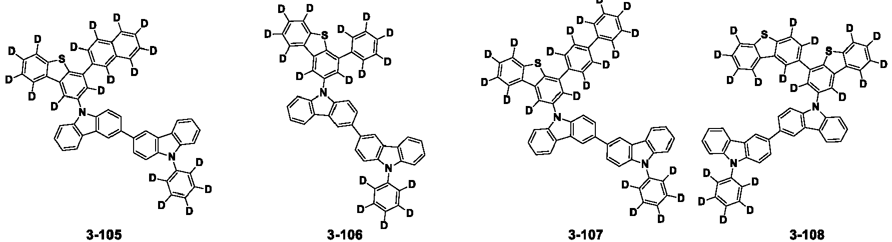

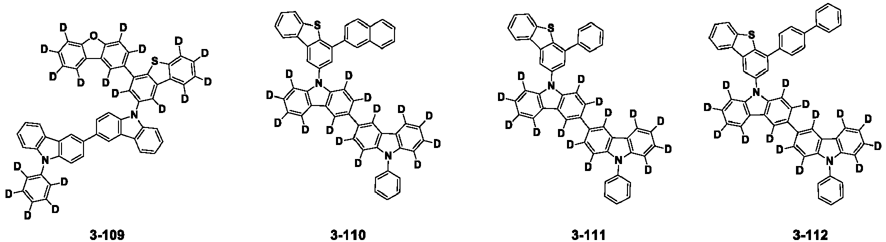

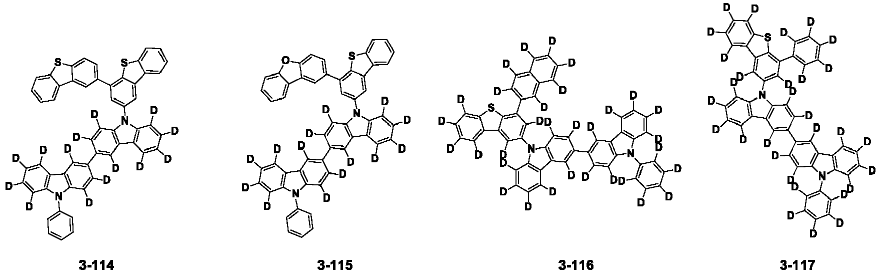

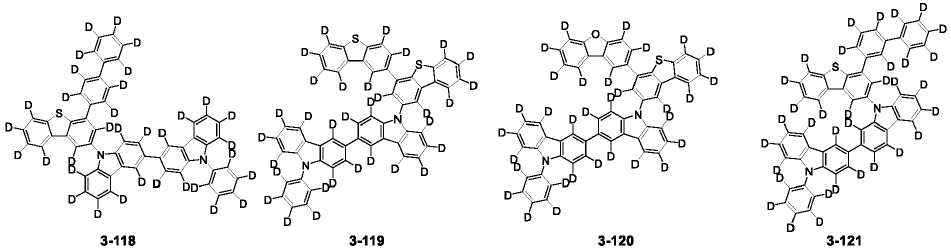

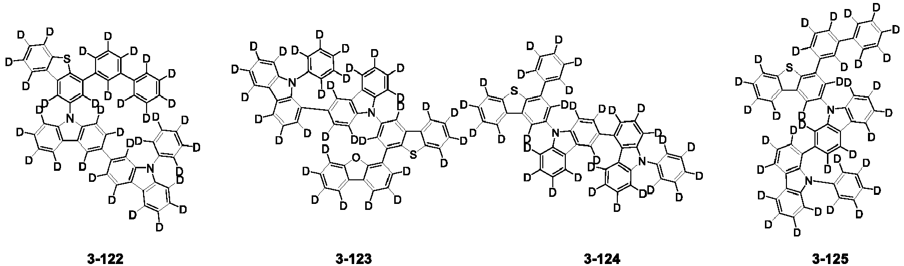

Furthermore, another exemplary embodiment of the present application provides a composition for an organic material layer of an organic light emitting device, which includes the heterocyclic compound represented by Chemical Formula 1; and the heterocyclic compound represented by Chemical Formula 2 or 3.

The specific contents on the heterocyclic compound represented by Chemical Formula 1, the heterocyclic compound represented by Chemical Formula 2 and the heterocyclic compound represented by Chemical Formula 3 are the same as those described above.

The weight ratio of the heterocyclic compound represented by Chemical Formula 1 : the heterocyclic compound represented by Chemical Formula 2 or 3 in the composition may be 1 : 10 to 10 : 1, 1 : 8 to 8 : 1, 1 : 5 to 5 : 1, and 1 : 2 to 2 : 1, but is not limited thereto. The composition may be used when an organic material for an organic light emitting device is formed, and particularly, may be more preferably used when a host of a light emitting layer is formed.

In an exemplary embodiment of the present application, the organic material layer includes: the heterocyclic compound represented by Chemical Formula 1; and the heterocyclic compound represented by Chemical Formula 2 or 3, and may be used together with a phosphorescent dopant.

In an exemplary embodiment of the present application, the organic material layer includes: the heterocyclic compound represented by Chemical Formula 1; and the heterocyclic compound represented by Chemical Formula 2 or 3, and may be used together with an iridium-based dopant.

As the phosphorescent dopant material, those known in the art may be used.

For example, phosphorescent dopant materials represented by LL'MX', LL'L"M, LMX'X", L2MX' and L3M may be used, but the scope of the present invention is not limited by these examples.

Here, L, L', L", X' and X" are bidentate ligands different from each other, and M is a metal forming an octahedral complex.

M may be iridium, platinum, osmium, and the like.

L is an anionic, bidendate ligand coordinated on M by the iridium-based dopant by sp2 carbon and a heteroatom, and X may perform a function of trapping electrons or holes. Non-limiting examples of L include 2-(1-naphthyl)benzoxazole, (2-phenylbenzoxazole), (2-phenylbenzothiazole), (2-phenylbenzothiazole), (7,8-benzoquinoline), (thiophene group pyrizine), phenylpyridine, benzothiophene group pyrizine, 3-methoxy-2-phenylpyridine, thiophene group pyrizine, tolylpyridine, and the like. Non-limiting examples of X' and X" include acetylacetonate (acac), hexafluoroacetylacetonate, salicylidene, picolinate, 8-hydroxyquinolinate, and the like.

More specific examples thereof will be shown below, but the present application is not limited only to these examples.

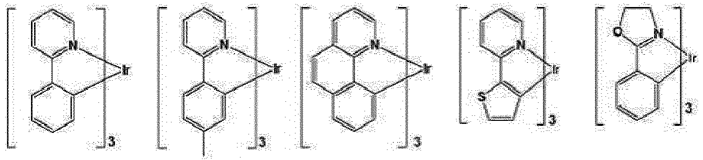

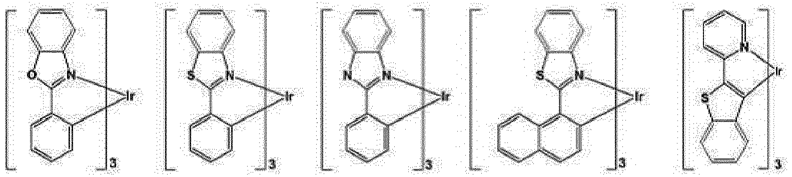

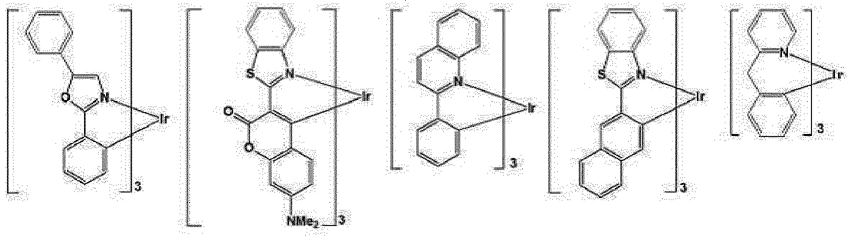

In an exemplary embodiment of the present application, as the iridium-based dopant, Ir(ppy)₃ as a green phosphorescent dopant may be used.

In an exemplary embodiment of the present application, the content of the dopant may be 1% to 15%, preferably 3% to 10%, and more preferably 5% to 10% based on the entire light emitting layer.

In an exemplary embodiment of the present application, provided is an organic light emitting device, in which the organic material layer of the organic light emitting device includes a light emitting layer, and the light emitting layer includes the heterocyclic compound.

In an exemplary embodiment of the present application, provided is an organic light emitting device, in which the organic material layer of the organic light emitting device includes a light emitting layer, and the light emitting layer includes a host material, and the host material includes the heterocyclic compound.

In the organic light emitting device of the present invention, the organic material layer includes an electron injection layer or an electron transport layer, and the electron injection layer or electron transport layer may include the heterocyclic compound.

In another organic light emitting device, the organic material layer includes an electron blocking layer or a hole blocking layer, and the electron blocking layer or the hole blocking layer may include the heterocyclic compound.

In still another organic light emitting device, the organic material layer includes an electron transport layer, a light emitting layer or a hole blocking layer, and the electron transport layer, the light emitting layer or the hole blocking layer may include the heterocyclic compound.

The organic light emitting device of the present invention may further include one or two or more layers selected from the group consisting of a light emitting layer, a hole injection layer, a hole transport layer, an electron injection layer, an electron transport layer, an electron blocking layer, and a hole blocking layer.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

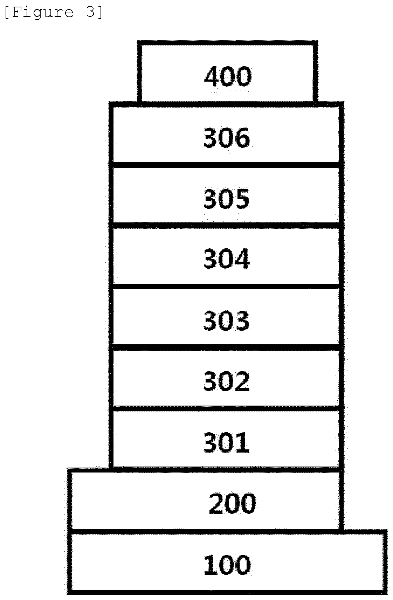

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

In an exemplary embodiment of the present application, provided is a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer according to an exemplary embodiment of the present application.

In an exemplary embodiment of the present application, provided is a method for manufacturing an organic light emitting device, in which the forming of the organic material layer forms the organic material layer by pre-mixing the heterocyclic compound of Chemical Formula 1 and the heterocyclic compound of Chemical Formula 2 or 3, and using a thermal vacuum deposition method.

The pre-mixing means that before the heterocyclic compound of Chemical Formula 1 and the heterocyclic compound of Chemical Formula 2 or 3 are deposited onto an organic material layer, the materials are first mixed and the mixture is contained in one common container and mixed.

The pre-mixed material may be referred to as a composition for an organic material layer according to an exemplary embodiment of the present application.

An organic material layer including Chemical Formula 1 may additionally include another material, if necessary.

An organic material layer simultaneously including: Chemical Formula 1; and Chemical Formula 2 or 3 may additionally include another material, if necessary.

In the organic light emitting device according to an exemplary embodiment of the present application, materials other than the compound of Chemical Formula 1, Chemical Formula 2 or Chemical Formula 3 will be exemplified below, but these materials are provided only for exemplification and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials may be deposited or used as an individual supply source, or pre-mixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

Hereinafter, the present specification will be described in more detail through Examples, but these Examples are provided only for exemplifying the present application, and are not intended to limit the scope of the present application.

### <Preparation Examples>

### [Preparation Example 1] Preparation of Intermediate C

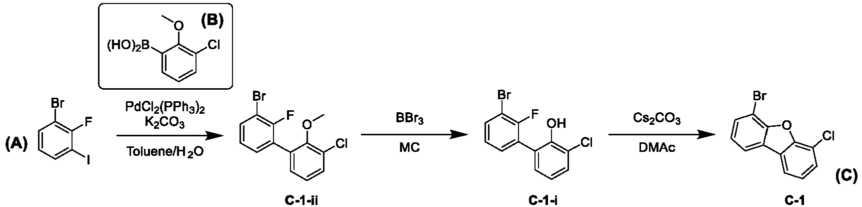

### Preparation of Compound C-1-ii

1-bromo-2-fluoro-3-iodobenzene) (A) (20 g/66.47 mmol), (3-chloro-2-methoxyphenyl)boronic acid (B) (14.87 g/79.76 mmol), PdCl₂(PPh₃)₂ (2.33 g/3.32 mmol), and K₂CO₃ (18.37 g/132.93 mmol) were put into a 1 L one neck round bottom flask and dissolved in 200 ml of toluene and 60 ml of water, and the resulting solution was reacted under reflux at 111°C for 3 hours.

After the reaction was completed, the resulting product was cooled to room temperature (25°C) and extracted using water and MC, and then an organic layer was concentrated. The concentrated organic layer was purified by performing column chromatography with MC and HX. Next, an excess amount of methanol was added thereto, a precipitated solid was removed by filtration, the filtrate was concentrated and dried to obtain a target compound 3-bromo-3'-chloro-2-fluoro-2'-methoxy-1,1'-biphenyl [C-1-ii] (14.7 g/70%).

### Preparation of Compound C-1-i

3-bromo-3'-chloro-2-fluoro-2'-methoxy-1,1'-biphenyl [C-1-ii], (14.7 g/46.53 mmol) was put into a 1 L one neck round bottom flask and dissolved in 150 ml of MC, and the resulting solution was stirred. After the solution was sufficiently stirred, BBr₃ (23.31 g/93.06 mmol) was added dropwise for 10 minutes while maintaining the temperature at room temperature, and the resulting mixture was stirred at room temperature of 2 hours.

After the reaction was completed, an excess amount of water (300 ml) was added thereto, the resulting mixture was stirred for 1 hour, and then a solid was filtered by filtration. An organic layer was separated from the filtrate and concentrated, and then purified by performing column chromatography with MC and HX. The filtrate subjected to column chromatography was concentrated and dried under vacuum to obtain a target compound 3'-bromo-3-chloro-2'-fluoro-[1,1'-biphenyl]-2-ol [C-1-i] (11.5 g/82%).

### Preparation of Compound C-1

3'-bromo-3-chloro-2'-fluoro-[1,1'-biphenyl]-2-ol [C-1-i], (11.5 g/ 38.15 mmol) was put into a 1 L one neck round bottom flask, dissolved in 110 ml of DMAc, and then stirred while being heated to 120°C. In this case, Cs₂CO₃ (24.86 g/76.31 mmol) was slowly added thereto, and the resulting mixture was heated to 165°C, and reacted under reflux for 3 hours.

After the reaction was completed, the reaction solution was cooled to room temperature and filtered to remove a solid. The filtrate was concentrated, and then purified by performing column chromatography with MC and HX. After the filtrate was concentrated, the concentrated filtrate was stirred in 6 T MeOH for 3 hours, and then filtered to obtain a target compound 4-bromo-6-chlorodibenzo[b,d]furan [C-1] (9.1 g/85%) as a white solid.

The following target compound C was prepared in the same manner as in the compound C-1 by using (A) and (B) in the following Table 1 as intermediates instead of 1-bromo-2-fluoro-3-iodobenzene and (3-chloro-2-methoxyphenyl)boronic acid, respectively, in Preparation Example 1.

**[Table 1]**

| **Compound** | **A** | **B** | **C** | **Total yield** |
|---|---|---|---|---|
| C-1 | 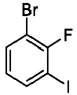 | 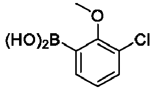 | 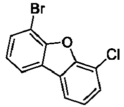 | **49%** |
| C-2 | 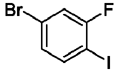 | 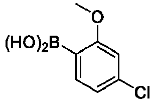 | 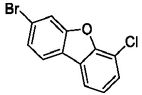 | **52%** |
| C-3 | 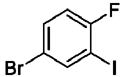 | 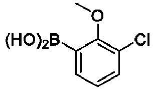 | 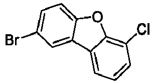 | **55%** |
| C-4 | 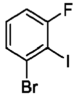 | 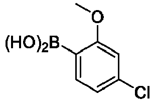 | 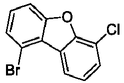 | **45%** |
| C-5 | 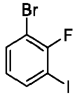 | 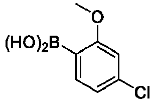 | 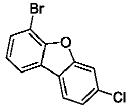 | **52%** |
| C-6 | 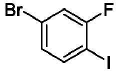 | 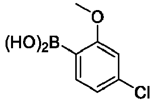 | 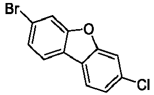 | **55%** |
| C-7 | 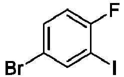 | 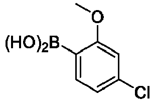 | 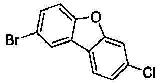 | **52%** |
| C-8 | 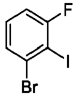 | 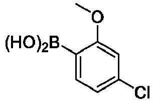 | 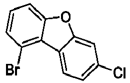 | **49%** |
| C-9 | 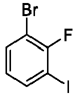 | 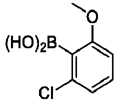 | 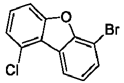 | **46%** |
| C-10 | 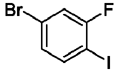 | 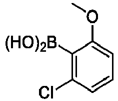 | 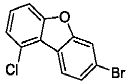 | **48%** |
| C-11 | 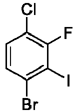 | 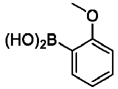 | 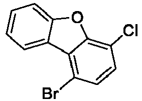 | **42%** |
| C-12 | 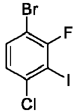 | 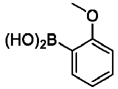 | 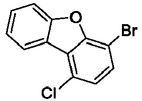 | **39%** |
| C-13 | 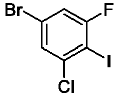 | 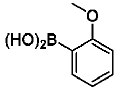 | 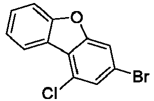 | **45%** |
| C-14 | 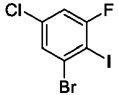 | 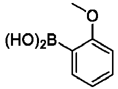 | 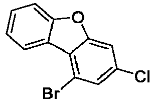 | **44%** |
| C-15 | 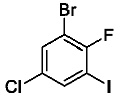 | 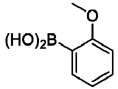 | 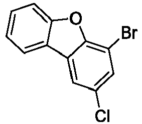 | **48%** |
| C-16 | 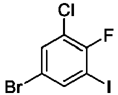 | 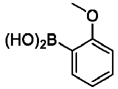 | 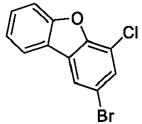 | **45%** |

### [Preparation Example 2] Synthesis of Intermediate C-17

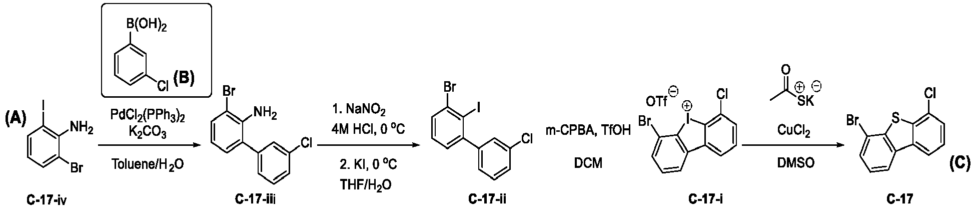

### Preparation of Compound C-17-iii

After 2-bromo-6-iodoaniline [A] (100 g, 335.56 mmol), (4-chlorophenyl)boronic acid [B] (63.0 g/402.79 mmol), Pd(PPh₃)₄ (19.39 g/16.78 mmol), and K₂CO₃ (92.8 g/671.32 mmol) were put into a 2 L one neck round bottom flask, 500 ml of 1,4-dioxane and 120 ml of H₂O were added thereto, and the resulting mixture was stirred under reflux at 101°C for 6 hours. After the reaction was completed, the reaction solution was cooled to room temperature, and then an organic layer was extracted with water and dichloromethane and concentrated.

After adsorption, column purification was performed with MC/HX to obtain a target compound 3-bromo-3'-chloro-[1,1'-biphenyl]-2-amine [C-17-iii] (73 g/77%).

### Preparation of Compound C-17-ii

After 700 ml of THF and 1050 ml of 4 M HCl were added to 3-bromo-3'-chloro-[1,1'-biphenyl]-2-amine [C-17-iii] (73 g/258.38 mmol) in a 3 L one neck round bottom flask, the reaction temperature was lowered to 0°C. After a NaNO₂ aqueous solution (52.2 g/90 ml) was added dropwise thereto at 0°C, the resulting mixture was stirred while maintaining the temperature for 1 hour, and then a KI aqueous solution (280 g/1680 ml) was slowly added thereto.

After the reaction was completed, sodium thiosulfate was added to neutralize the acid.

After an organic layer was extracted and separated using water and dichloromethane, the organic material layer was adsorbed, and then subjected to column purification with MC/HX to obtain a target compound 3-bromo-3'-chloro-2-iodo-1,1'-biphenyl [C-17-ii] (88 g/87%).

### Preparation of Compound C-17-i

After 3-bromo-3'-chloro-2-iodo-1,1'-biphenyl[C-17-iiJ (58.24 g/224.8mmol) was put into a 2 L one neck round bottom flask and sufficiently dissolved in 900 ml of dichloromethane, m-CPBA (58.24 g/337.19 mmol) and TfOH (59.6 ml/674.4 mmol) were added thereto in a state in which the temperature was maintained at 0°C, and the resulting mixture was stirred for 5 hours. After the reaction was completed, the resulting solid was filtered, dried, and then dissolved in acetone, the undissolved solid was again removed by filtration, and only the filtrate was concentrated. After the concentrated filtrate was stirred in dichloromethane, a precipitated solid was filtered to obtain a target compound ((trifluoromethyl)sulfonyl)-l1-oxidane, 4-bromo-6-chlorodibenzo[b,d]iodol-5-ium salt [C-17-i] (86.4 g/71%).

### Preparation of Compound C-17

After ((trifluoromethyl)sulfonyl)-l1-oxidane, 4-bromo-6-chlorodibenzo[b,d]iodol-5-ium salt [C-17-i] (86.4 g/159.6 mmol), potassium ethanethioate (91 g/798 mmol), and Cu(OAc)₂ (1.4 g, 8.0 mmol) were put into a 2 L one neck round bottom flask, the resulting mixture was dissolved in 1200 ml of DMSO, and then the resulting solution was stirred while being heated at 110°C for 24 hours.

After the reaction was completed, the resulting product was cooled to room temperature, a precipitated solid was removed by filtration and washed sufficiently with dichloromethane, and then the filtrate was concentrated. The concentrated organic material was adsorbed, and then subjected to column purification with MC/HX to obtain a target compound 4-bromo-6-chlorodibenzo[b,d]thiophene [C-17] (22 g/46%).

The following target compound C was prepared in the same manner as in the compound C-17 by using (A) and (B) in the following Table 2 as intermediates instead of 2-bromo-6-iodoaniline and (2-chlorophenyl)boronic acid, respectively, in Preparation Example 2.

**[Table 2]**

| **Compound** | **A** | **B** | **C** | **Total yield** |
|---|---|---|---|---|
| C-17 | 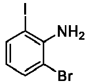 | 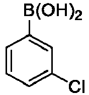 | 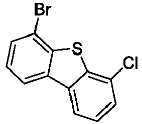 | **21%** |
| C-18 | 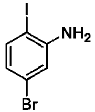 | 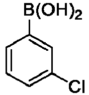 | 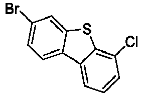 | **21%** |
| C-19 | 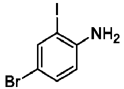 | 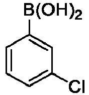 | 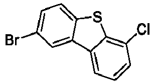 | **22%** |
| C-20 | 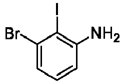 | 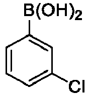 | 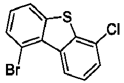 | **24%** |
| C-21 | 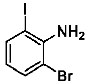 | 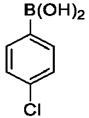 | 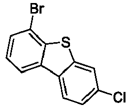 | **20%** |
| C-22 | 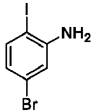 | 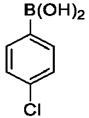 | 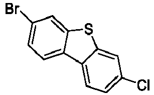 | **22%** |
| C-23 | 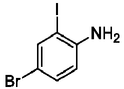 | 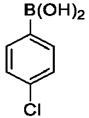 | 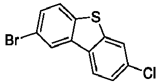 | **21%** |
| C-24 | 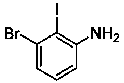 | 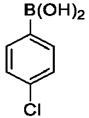 | 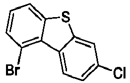 | **17%** |
| C-25 | 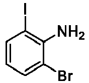 | 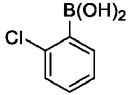 | 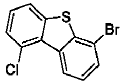 | **23%** |
| C-26 | 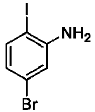 | 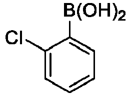 | 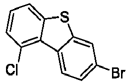 | **19%** |
| C-27 | 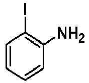 | 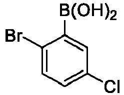 | 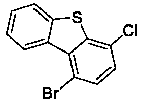 | **19%** |
| C-28 | 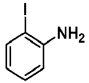 | 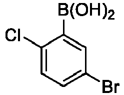 | 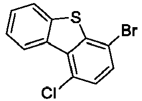 | **20%** |
| C-29 | 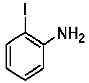 | 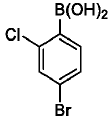 | 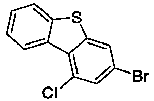 | **15%** |
| C-30 | 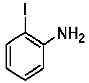 | 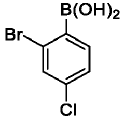 | 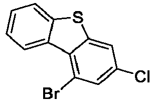 | **14%** |
| C-31 | 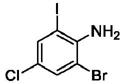 | 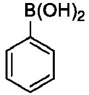 | 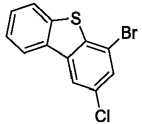 | **16%** |
| C-32 | 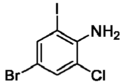 | 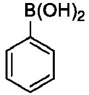 | 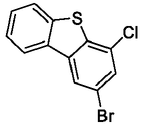 | 15% |

### [Preparation Example 3] Synthesis of Compound 1-1 (F)

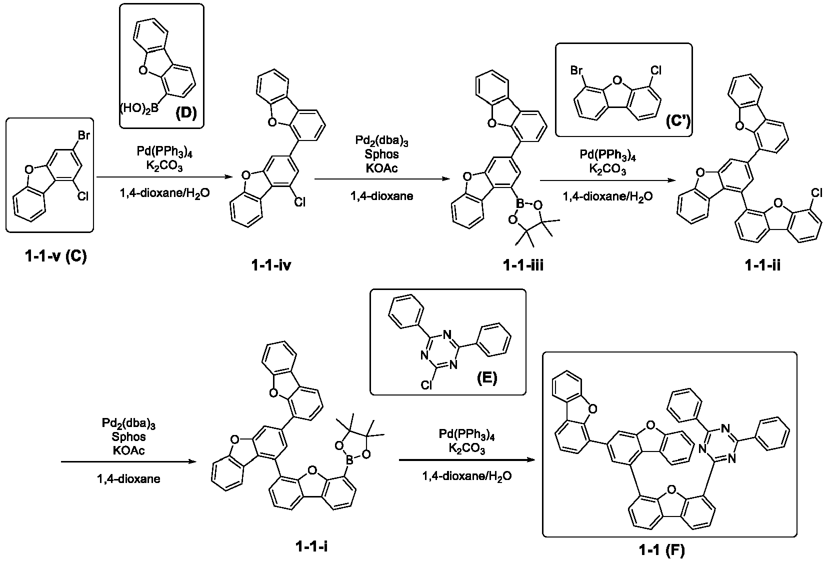

### Preparation of Compound 1-1-iv

3-bromo-1-chlorodibenzo [b, dJ furan) (C) (20 g/71.04 mmol), dibenzo[b,d]furan-4-ylboronic acid) (D) (16.56 g/78.11 mmol), Pd(PPh₃)₄ (2.36 g/3.36 mmol), and K₂CO₃ (18.56 g, 134.26 mmol) were put into a 1 L one neck round bottom flask and dissolved in 200 ml of 1,4-dioxane and 60 ml of water, and then the resulting solution was reacted under reflux at 101°C for 3 hours.

After the reaction was completed, the resulting product was cooled to room temperature, and then an organic layer was extracted with water and MC.

After the extract was concentrated and then dissolved in MC, the resulting solution was loaded onto a column, and subjected to column purification with MC/HX. After the filtrate was concentrated, a precipitated solid was filtered by stirring the concentrated filtrate in 100 mL of methanol, and a white solid, 1-chloro-3,4'-bidibenzo[b,d]furan) [1-1-iv] (21 g/80%) was obtained.

### Preparation of Compound 1-1-iii

1-chloro-3,4'-bidibenzo[b,d]furan) [1-1-iv] (21 g/56.83 mmol), 4,4,5,5-tetramethyl-2-(4,4,5,5-tetramethyl-1,3,2-dioxaborolan-2-yl)-1,3,2-dioxaborolane (21.6 g/85.24 mmol), Pd₂(dba)₃ (5.2 g/5.68 mmol), Sphos (4.66 g/11.36 mmol), and KOAc (10.92 g/113.66 mmol) were put into a 1 L one neck round bottom flask and dissolved in 200 mL of 1,4-dioxane, and then the resulting solution was stirred under reflux at 101°C for 1 hour.

After the reaction was completed, the resulting product was cooled to room temperature, and then an organic layer was extracted with water and MC.

After the organic layer was concentrated and then dissolved in a small amount of toluene, the resulting solution was filtered with silica gel and subjected to column purification with toluene. The filtered filtrate was concentrated, and then dried under vacuum to obtain 2-([3,4'-bidibenzo[b,d]furan]-1-yl)-4,4,5,5-tetramethyl-1,3,2-dioxaborolane [1-1-iii](22.2 g/85%).

### Preparation of Compound 1-1-ii

2-([3,4'-bidibenzo[b,d]furan]-1-yl)-4,4,5,5-tetramethyl-1,3,2-dioxaborolane) [1-1-iii] (22.2 g/48.31 mmol), 4-bromo-6-chlorodibenzo[b,d]furan (C') (13.6 g/48.31 mmol), Pd(PPh₃)₄ (2.79 g/2.41 mmol), and K₂CO₃ (13.35 g/96.62 mmol) were put into a 1000 ml one neck round bottom flask and dissolved in 250 mL of 1,4-dioxane and 80 mL of water, and then the resulting solution was stirred under reflux at 101°C for 3 hours.

After the reaction was completed, the resulting product was cooled to room temperature, and then water and methanol were added thereto, and the resulting mixture was stirred. A precipitated solid was filtrated and washed with an excess amount of water and methanol.

The solid was dried under vacuum to obtain 6-chloro-4,1':3',4"-terdibenzo[b,d]furan) [1-1-ii] (20.1 g/78%) as a white solid.

### Preparation of Compound 1-1-i

6-chloro-4,1':3',4"-terdibenzo[b,d]furan) [1-1-ii] (20.1 g/37.68 mmol), 4,4,5,5-tetramethyl-2-(4,4,5,5-tetramethyl-1,3,2-dioxaborolan-2-yl)-1,3,2-dioxaborolane) (14.35 g/56.52 mmol), Pd₂(dba)₃ (3.45 g/3.76 mmol), Sphos (3.86 g/9.4 mmol), and KOAc (7.4 g/75.36 mmol) were put into a 1 L one neck round bottom flask and dissolved in 180 mL of 1,4-dioxane, and the resulting solution was stirred under reflux at 101°C for 1.5 hours.

After the reaction was completed, an organic layer was extracted with water and MC from the reaction product cooled to room temperature.

After the extracted organic layer was concentrated and then dissolved in a small amount of toluene, the resulting solution was filtered with silica gel and subjected to column purification with toluene. After the filtered filtrate was concentrated, the concentrated filtrate was stirred in 100 ml of ethyl acetate, and then a precipitated solid was filtered to obtain 2-([4,1':3',4"-terdibenzo[b,d]furan]-6-yl)-4,4,5,5-tetramethyl-1,3,2-dioxaborolane [1-1-i] (19.3 g/82%).

### Preparation of Compound 1-1

2-([4,1':3',4"-terdibenzo[b,d]furan]-6-yl)-4,4,5,5-tetramethyl-1,3,2-dioxaborolane) [1-1-i] (6.3 g/10 mmol), 2-chloro-4,6-diphenyl-1,3,5-triazine (E) (2.68 g/10 mmol), Pd(PPh₃)₄ (0.57 g/0.5 mmol), and K₂CO₃ (2.76 g/20 mmol) were put into a 500 ml one neck round bottom flask and dissolved in 90 mL of 1,4-dioxane and 30 mL of water, and then the resulting solution was stirred under reflux at 101°C for 12 hours.

After the reaction was completed, the resulting product was cooled to room temperature, a precipitated solid was filtered and washed with an excess amount of water and acetone. The sufficiently dried solid was dissolved in DCB, and the resulting solution was filtered with silica gel and subjected to column purification with toluene. The filtered filtrate was concentrated and white crystals obtained by stirring the concentrated filtrate in 100 mL of EA were filtered to obtain a target compound 2-([4,1':3',4"-terdibenzo[b,d]furan]-6-yl)-4,6-diphenyl-1,3,5-triazine [1-1] (5.9 g/80% yield).

The following target compound F was prepared in the same manner as in the compound C-17 by using (C), (D), (C') and (E) in the following Table 3 as intermediates instead of 3-bromo-1-chlorodibenzo[b,d]furan, dibenzo[b,d]furan-4-ylboronic acid, 4-bromo-6-chlorodibenzo[b,d]furan, and 2-chloro-4,6-diphenyl-1,3,5-triazine, respectively, in Preparation Example 3.

**[Table 3]**

| **Compound** | **C** | **D** | **C'** | **E** | **F** | **Total yield** |
|---|---|---|---|---|---|---|
| 1-1 | 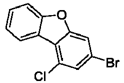 | 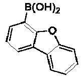 | 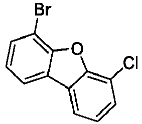 | 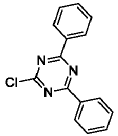 | 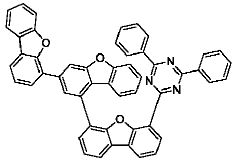 | 35% |
| 1-2 | 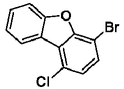 | 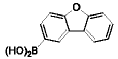 | 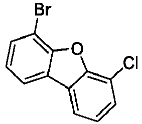 | 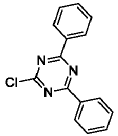 | 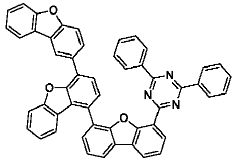 | 38% |
| 1-5 | 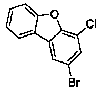 | 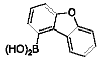 | 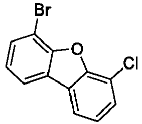 | 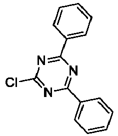 | 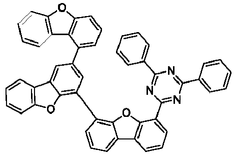 | 38% |
| 1-11 | 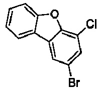 | 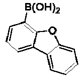 | 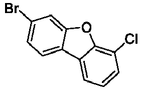 | 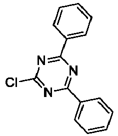 | 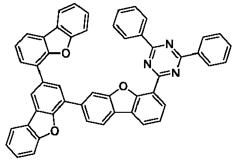 | 36% |
| 1-14 | 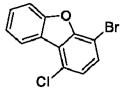 | 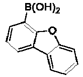 | 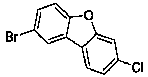 | 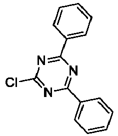 | 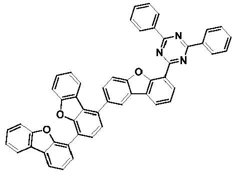 | 37% |
| 1-19 | 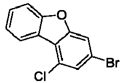 | 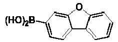 | 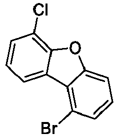 | 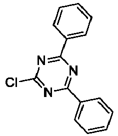 | 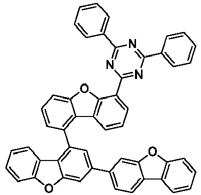 | 34% |
| 1-23 | 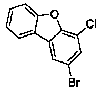 | 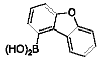 | 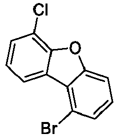 | 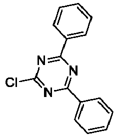 | 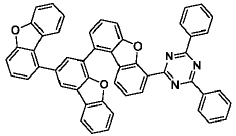 | 37% |
| 1-25 | 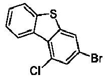 | 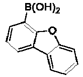 | 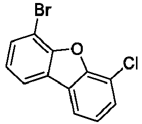 | 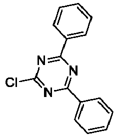 | 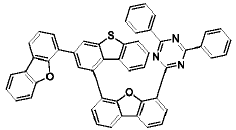 | 39% |
| 1-31 | 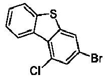 | 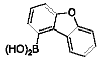 | 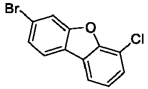 | 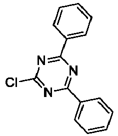 | 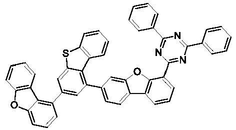 | 33% |
| 1-34 | 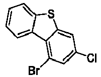 | 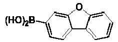 | 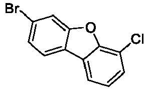 | 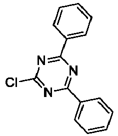 | 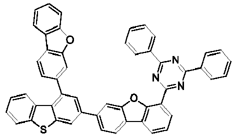 | 35% |
| 1-43 | 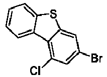 | 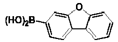 | 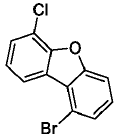 | 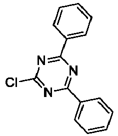 | 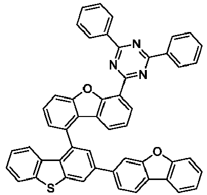 | 33% |
| 1-54 | 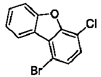 | 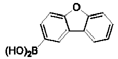 | 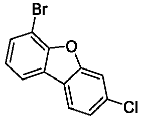 | 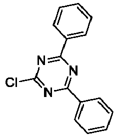 | 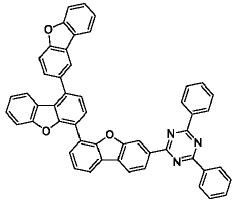 | 38% |
| 1-63 | 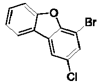 | 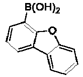 | 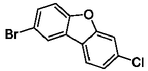 | 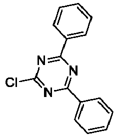 | 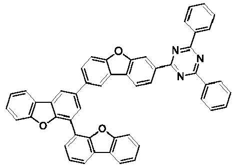 | 40% |
| 1-68 | 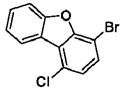 | 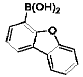 | 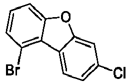 | 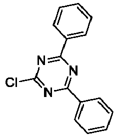 | 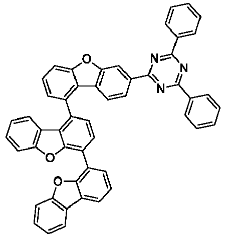 | 34% |
| 1-72 | 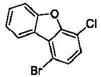 | 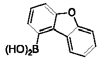 | 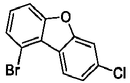 | 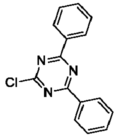 | 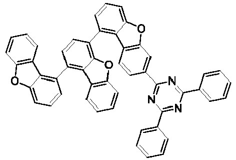 | 36% |
| 1-73 | 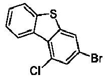 | 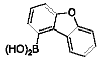 | 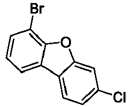 | 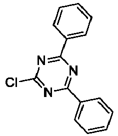 | 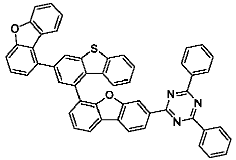 | 32% |
| 1-78 | 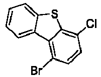 | 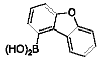 | 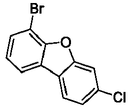 | 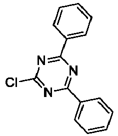 | 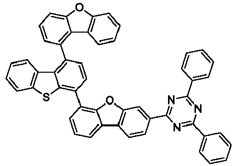 | 34% |
| 1-87 | 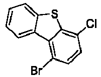 | 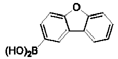 | 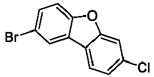 | 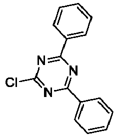 | 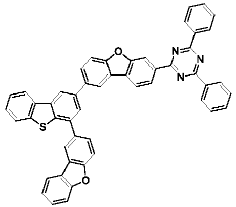 | 32% |
| 1-88 | 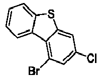 | 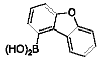 | 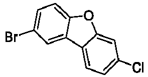 | 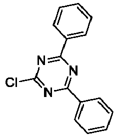 | 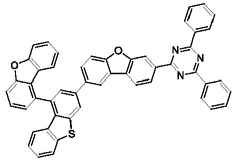 | 34% |
| 1-95 | 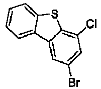 | 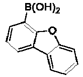 | 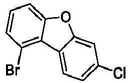 | 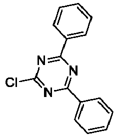 | 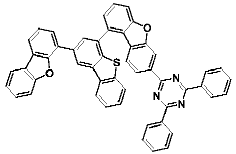 | 39% |
| 1-96 | 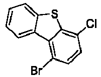 | 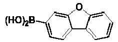 | 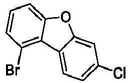 | 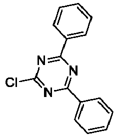 | 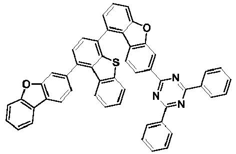 | 38% |
| 1-97 | 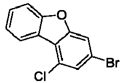 | 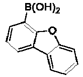 | 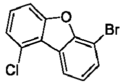 | 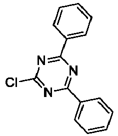 | 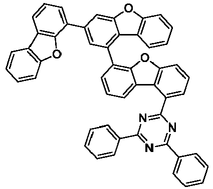 | 37% |
| 1-102 | 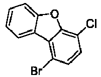 | 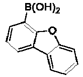 | 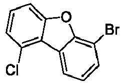 | 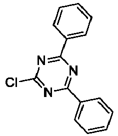 | 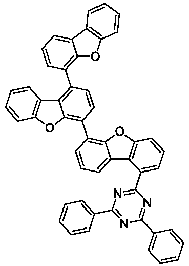 | 38% |
| 1-106 | 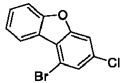 | 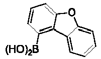 | 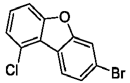 | 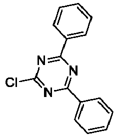 | 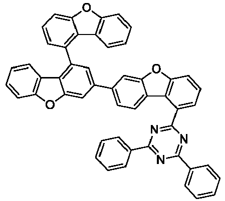 | 34% |
| 1-114 | 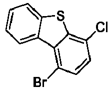 | 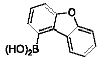 | 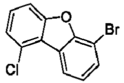 | 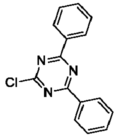 | 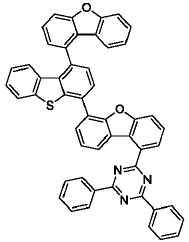 | 32% |
| 1-118 | 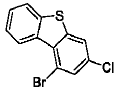 | 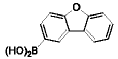 | 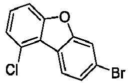 | 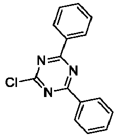 | 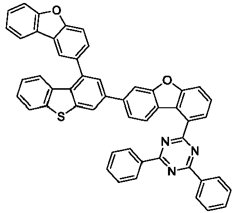 | 33% |
| 1-119 | 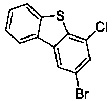 | 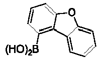 | 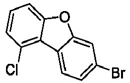 | 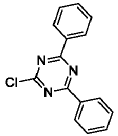 | 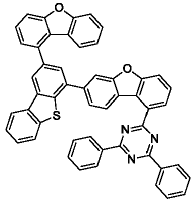 | 32% |
| 1-122 | 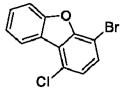 | 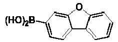 | 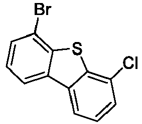 | 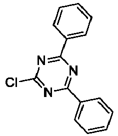 | 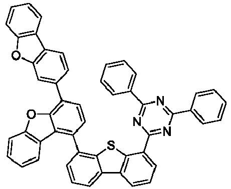 | 31% |
| 1-132 | 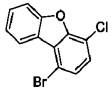 | 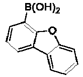 | 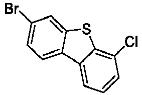 | 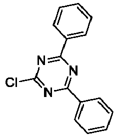 | 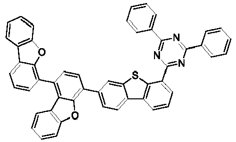 | 36% |
| 1-135 | 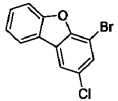 | 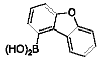 | 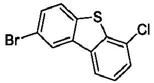 | 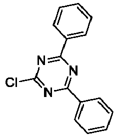 | 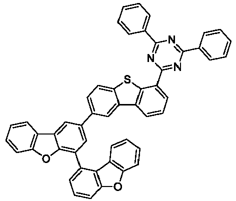 | 33% |
| 1-137 | 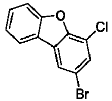 | 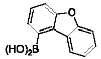 | 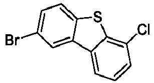 | 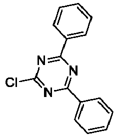 | 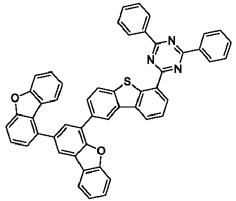 | 35% |
| 1-139 | 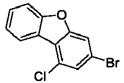 | 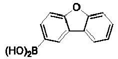 | 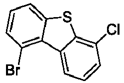 | 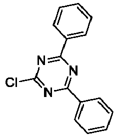 | 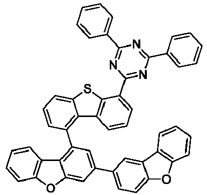 | 37% |
| 1-148 | 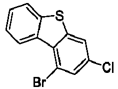 | 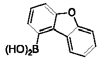 | 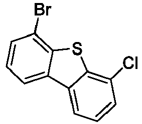 | 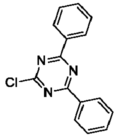 | 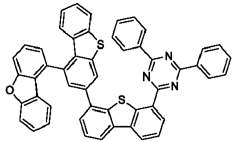 | 33% |
| 1-153 | 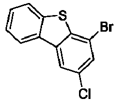 | 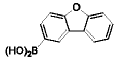 | 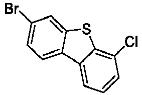 | 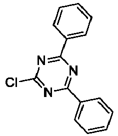 | 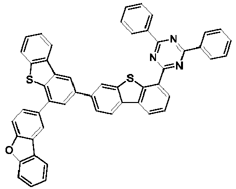 | 32% |
| 1-155 | 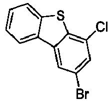 | 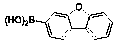 | 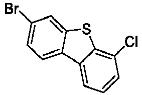 | 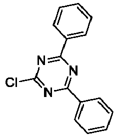 | 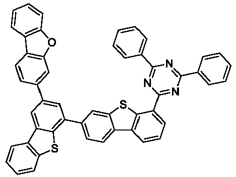 | 37% |
| 1-163 | 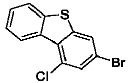 | 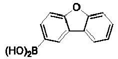 | 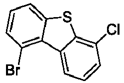 | 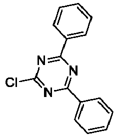 | 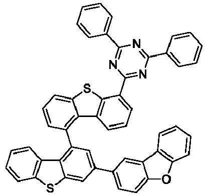 | 36% |
| 1-164 | 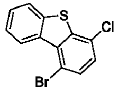 | 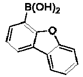 | 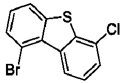 | 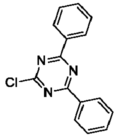 | 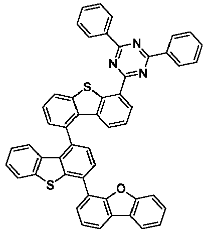 | 35% |
| 1-169 | 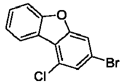 | 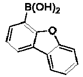 | 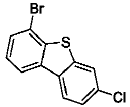 | 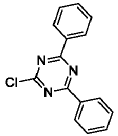 | 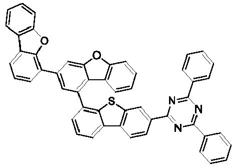 | 34% |
| 1-171 | 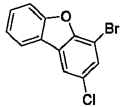 | 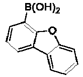 | 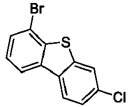 | 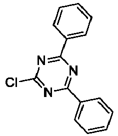 | 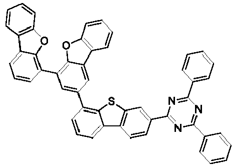 | 30% |
| 1-178 | 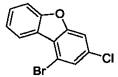 | 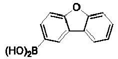 | 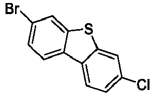 | 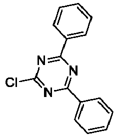 | 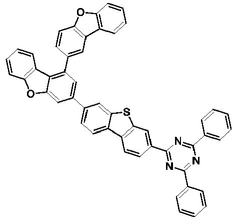 | 32% |
| 1-180 | 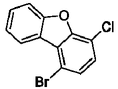 | 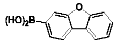 | 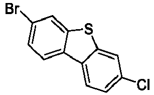 | 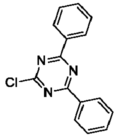 | 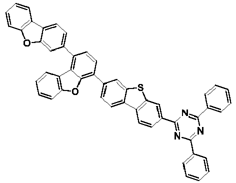 | 36% |
| 1-186 | 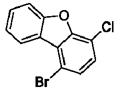 | 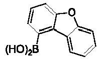 | 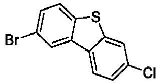 | 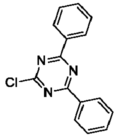 | 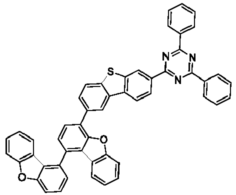 | 34% |
| 1-191 | 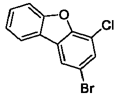 | 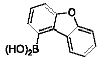 | 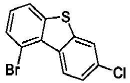 | 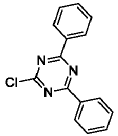 | 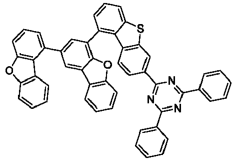 | 34% |
| 1-193 | 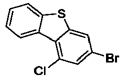 | 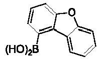 | 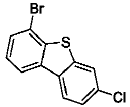 | 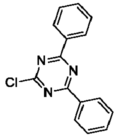 | 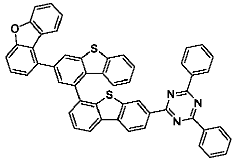 | 36% |
| 1-196 | 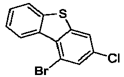 | 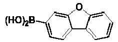 | 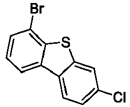 | 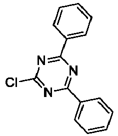 | 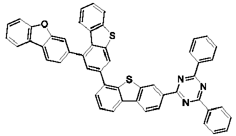 | 35% |
| 1-202 | 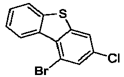 | 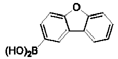 | 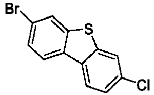 | 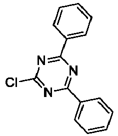 | 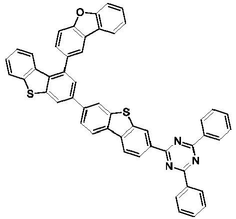 | 37% |
| 1-207 | 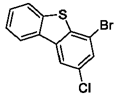 | 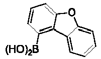 | 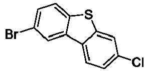 | 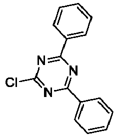 | 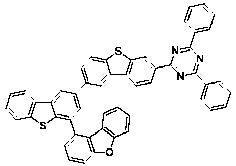 | 38% |
| 1-211 | 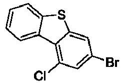 | 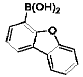 | 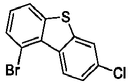 | 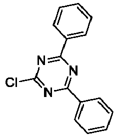 | 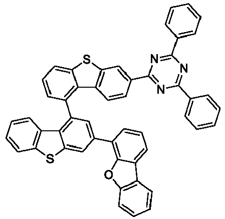 | 33% |
| 1-213 | 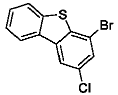 | 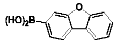 | 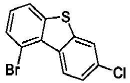 | 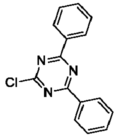 | 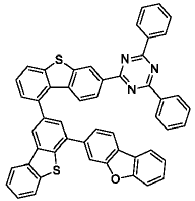 | 32% |
| 1-217 | 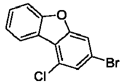 | 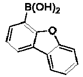 | 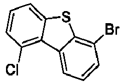 | 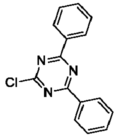 | 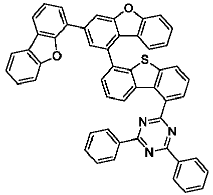 | 33% |
| 1-221 | 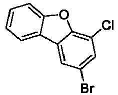 | 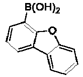 | 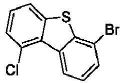 | 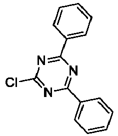 | 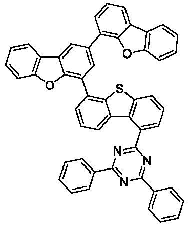 | 35% |
| 1-223 | 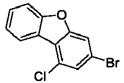 | 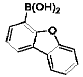 | 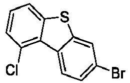 | 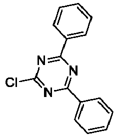 | 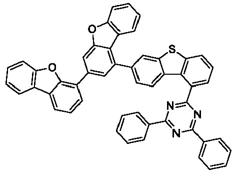 | 31% |
| 1-231 | 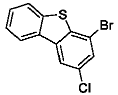 | 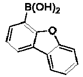 | 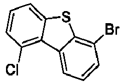 | 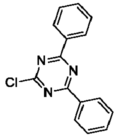 | 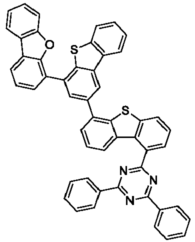 | 34% |
| 1-233 | 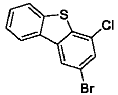 | 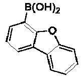 | 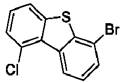 | 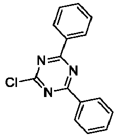 | 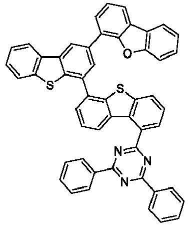 | 35% |
| 1-235 | 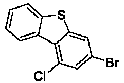 | 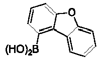 | 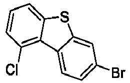 | 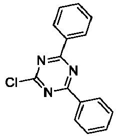 | 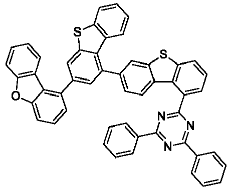 | 32% |
| 1-238 | 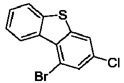 | 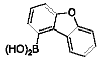 | 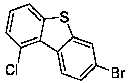 | 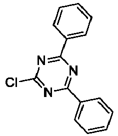 | 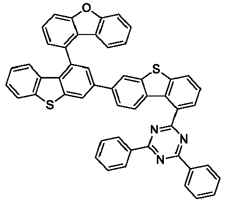 | 34% |
| 1-239 | 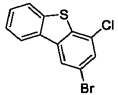 | 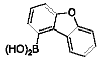 | 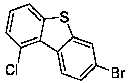 | 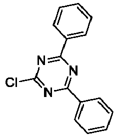 | 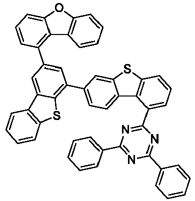 | 31% |
| 1-241 | 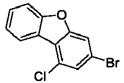 | 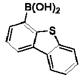 | 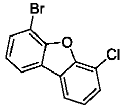 | 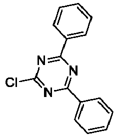 | 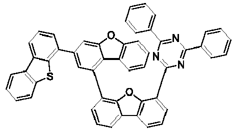 | 33% |
| 1-242 | 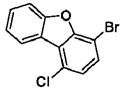 | 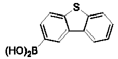 | 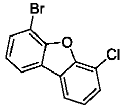 | 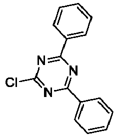 | 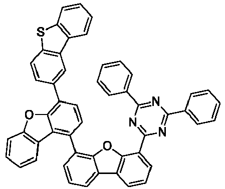 | 32% |
| 1-249 | 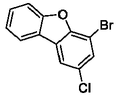 | 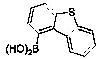 | 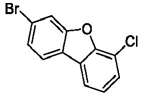 | 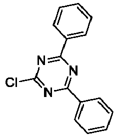 | 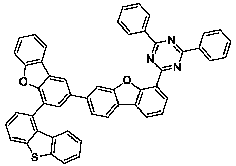 | 35% |
| 1-251 | 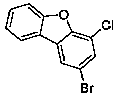 | 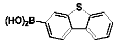 | 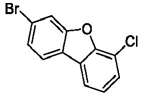 | 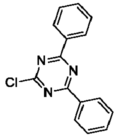 | 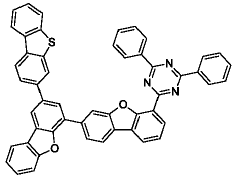 | 36% |
| 1-255 | 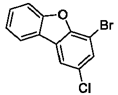 | 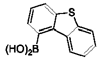 | 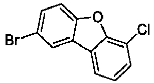 | 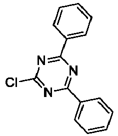 | 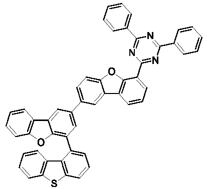 | 31% |
| 1-263 | 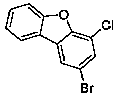 | 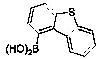 | 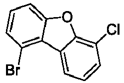 | 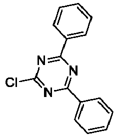 | 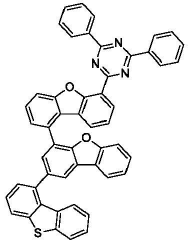 | 36% |
| 1-268 | 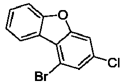 | 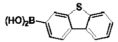 | 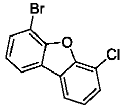 | 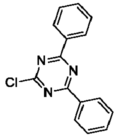 | 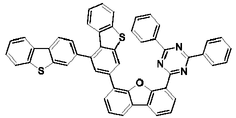 | 33% |
| 1-271 | 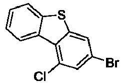 | 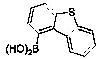 | 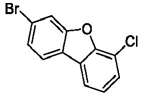 | 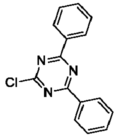 | 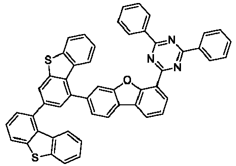 | 34% |
| 1-279 | 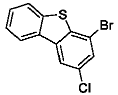 | 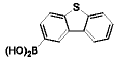 | 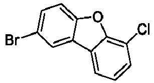 | 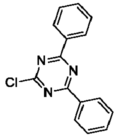 | 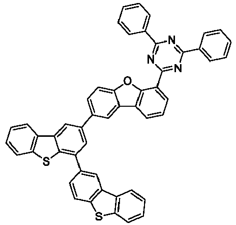 | 37% |
| 1-286 | 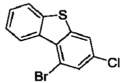 | 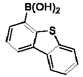 | 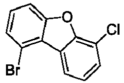 | 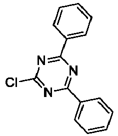 | 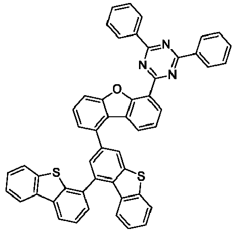 | 35% |
| 1-293 | 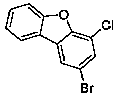 | 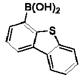 | 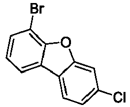 | 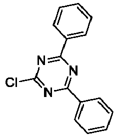 | 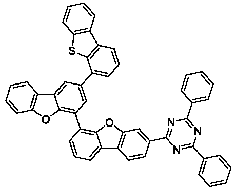 | 31% |
| 1-299 | 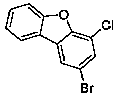 | 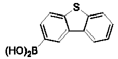 | 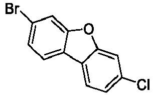 | 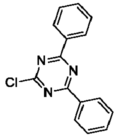 | 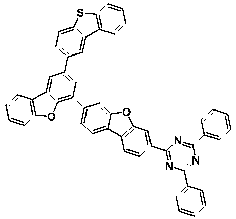 | 32% |
| 1-307 | 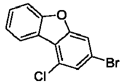 | 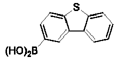 | 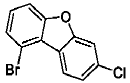 | 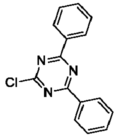 | 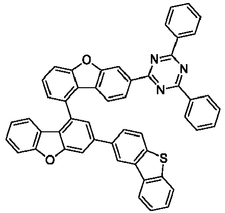 | 30% |
| 1-312 | 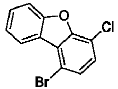 | 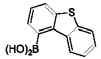 | 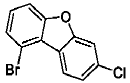 | 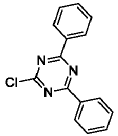 | 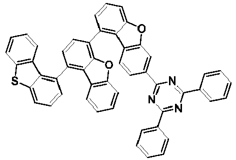 | 31% |
| 1-313 | 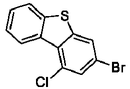 | 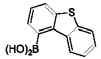 | 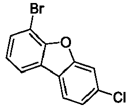 | 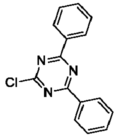 | 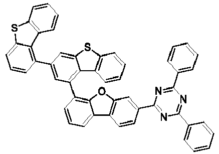 | 30% |
| 1-322 | 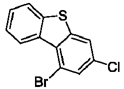 | 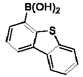 | 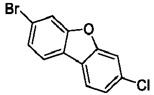 | 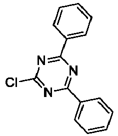 | 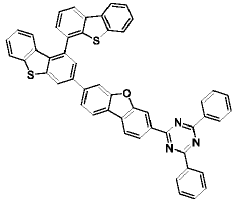 | 32% |
| 1-329 | 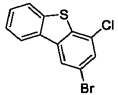 | 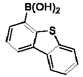 | 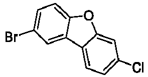 | 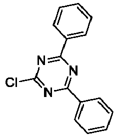 | 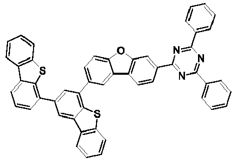 | 35% |
| 1-331 | 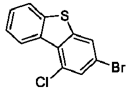 | 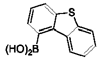 | 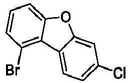 | 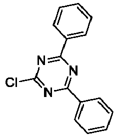 | 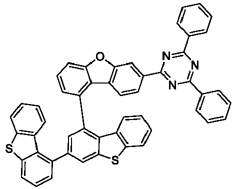 | 34% |
| 1-337 | 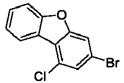 | 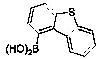 | 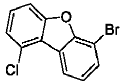 | 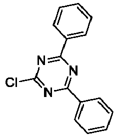 | 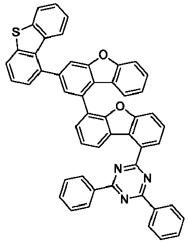 | 31% |
| 1-343 | 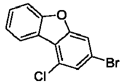 | 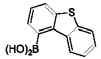 | 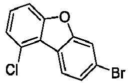 | 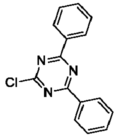 | 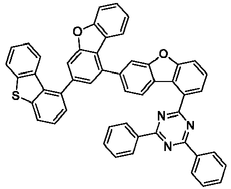 | 38% |
| 1-347 | 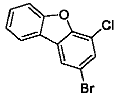 | 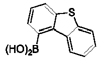 | 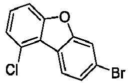 | 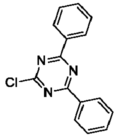 | 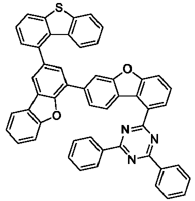 | 39% |
| 1-358 | 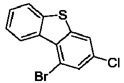 | 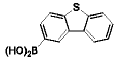 | 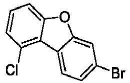 | 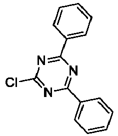 | 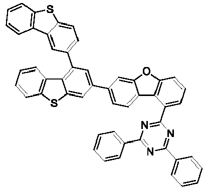 | 35% |
| 1-361 | 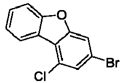 | 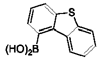 | 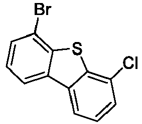 | 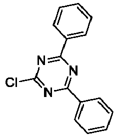 | 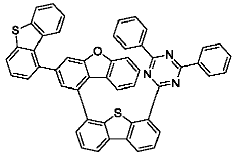 | 32% |
| 1-366 | 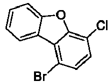 | 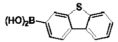 | 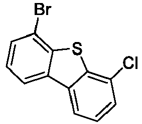 | 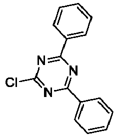 | 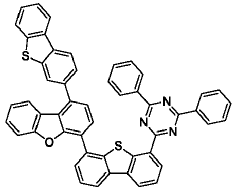 | 31% |
| 1-372 | 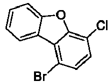 | 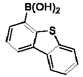 | 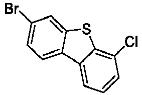 | 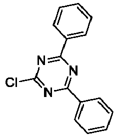 | 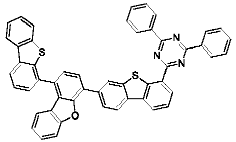 | 31% |
| 1-378 | 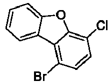 | 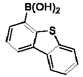 | 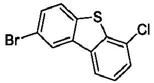 | 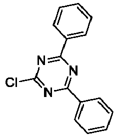 | 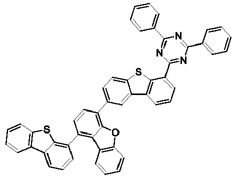 | 32% |
| 1-388 | 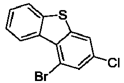 | 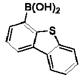 | 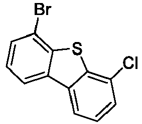 | 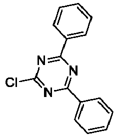 | 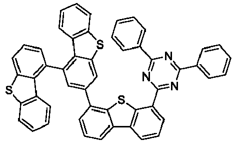 | 31% |
| 1-394 | 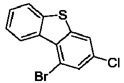 | 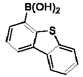 | 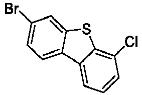 | 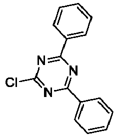 | 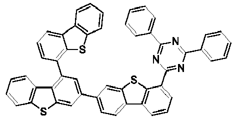 | 30% |
| 1-403 | 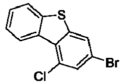 | 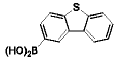 | 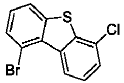 | 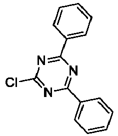 | 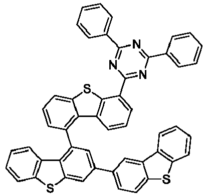 | 33% |
| 1-407 | 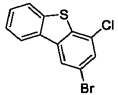 | 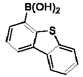 | 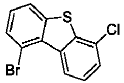 | 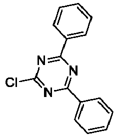 | 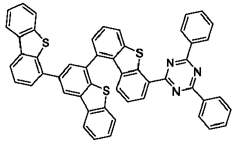 | 39% |
| 1-412 | 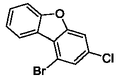 | 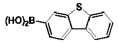 | 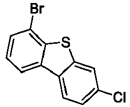 | 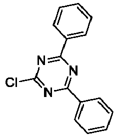 | 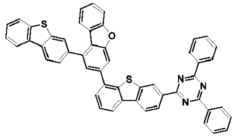 | 32% |
| 1-423 | 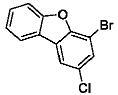 | 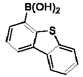 | 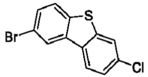 | 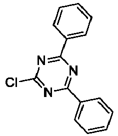 | 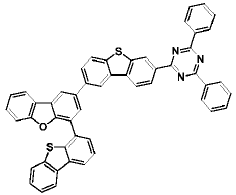 | 34% |
| 1-439 | 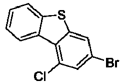 | 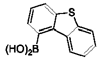 | 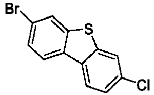 | 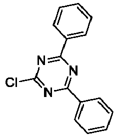 | 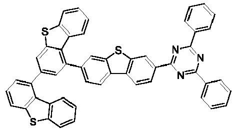 | 34% |
| 1-448 | 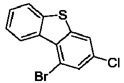 | 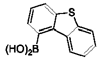 | 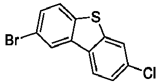 | 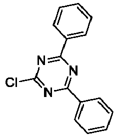 | 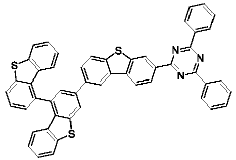 | 35% |
| 1-451 | 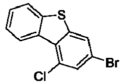 | 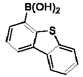 | 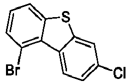 | 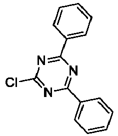 | 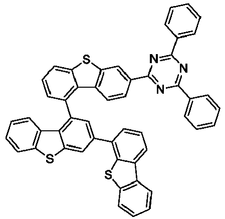 | 31% |
| 1-457 | 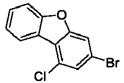 | 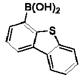 | 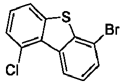 | 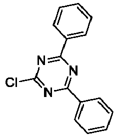 | 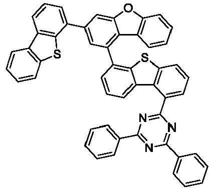 | 36% |
| 1-459 | 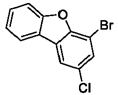 | 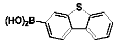 | 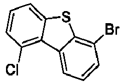 | 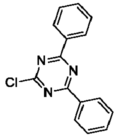 | 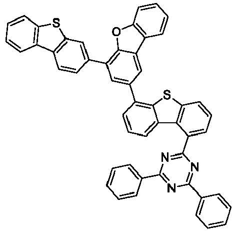 | 35% |
| 1-468 | 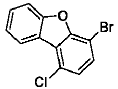 | 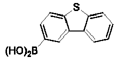 | 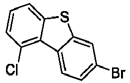 | 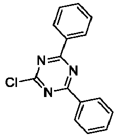 | 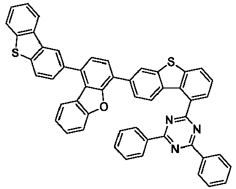 | 33% |
| 1-472 | 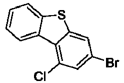 | 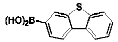 | 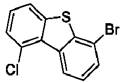 | 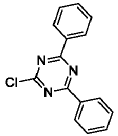 | 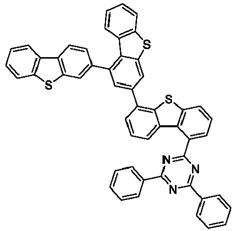 | 34% |
| 1-481 | 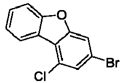 | 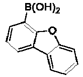 | 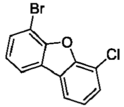 | 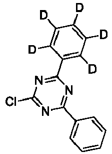 | 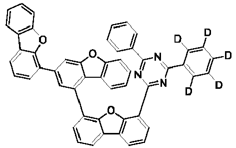 | 31% |
| 1-483 | 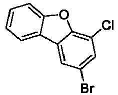 | 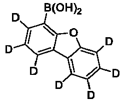 | 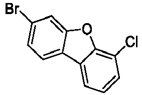 | 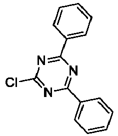 | 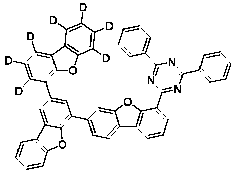 | 32% |
| 1-484 | 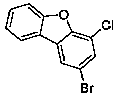 | 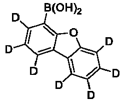 | 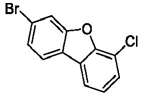 | 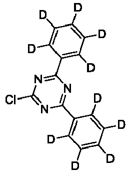 | 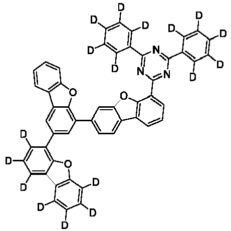 | 33% |
| 1-487 | 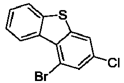 | 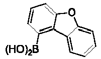 | 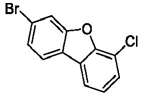 | 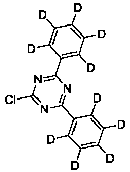 | 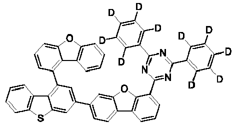 | 36% |
| 1-489 | 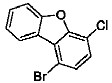 | 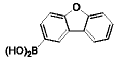 | 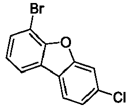 | 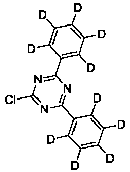 | 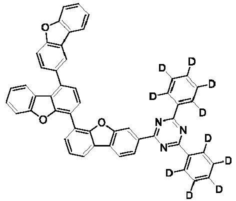 | 32% |
| 1-494 | 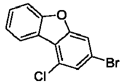 | 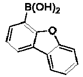 | 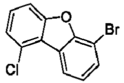 | 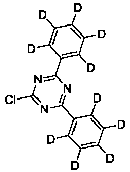 | 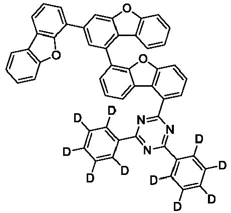 | 37% |
| 1-496 | 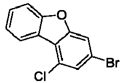 | 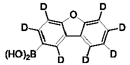 | 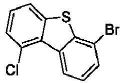 | 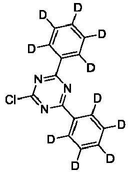 | 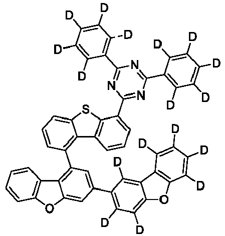 | 32% |
| 1-497 | 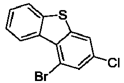 | 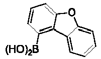 | 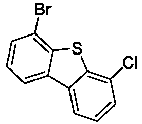 | 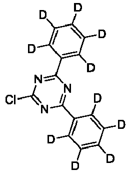 | 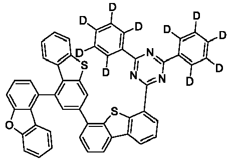 | 33% |
| 1-500 | 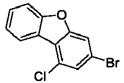 | 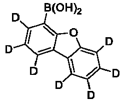 | 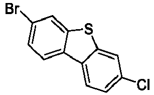 | 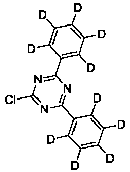 | 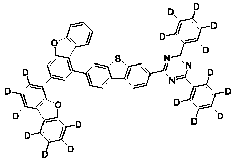 | 32% |
| 1-503 | 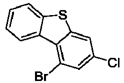 | 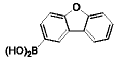 | 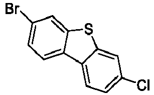 | 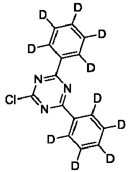 | 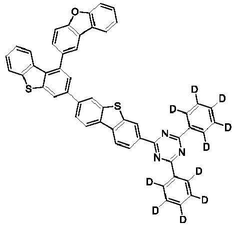 | 31% |
| 1-505 | 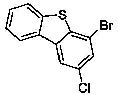 | 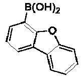 | 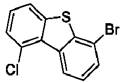 | 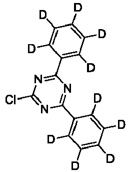 | 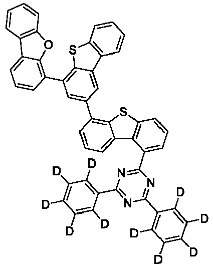 | 32% |
| 1-510 | 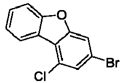 | 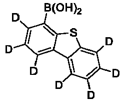 | 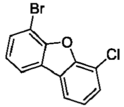 | 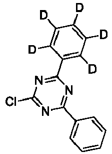 | 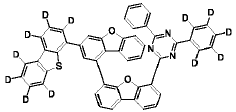 | 30% |
| 1-518 | 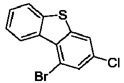 | 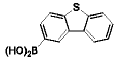 | 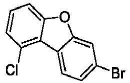 | 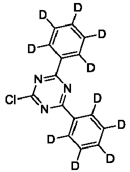 | 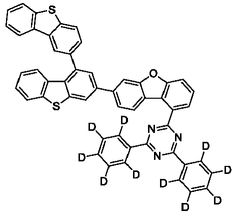 | 32% |

### [Preparation Example 4] Synthesis of Compound 1-529

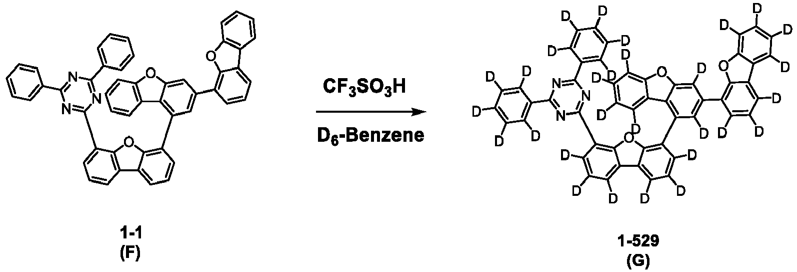

### Preparation of Compound 1-529

A mixture of Compound 1-1 (10 g, 0.014 mol), triflic acid (33.6 g, 0.27 mol), and D₆-benzene (100 ml) was refluxed at 50°C in a one neck round bottom flask. After extraction with dichloromethane and water was performed to dry an organic layer over MgSO₄, the dried organic layer was concentrated and then purified with column chromatography (DCM : Hex = 1 : 2). After concentration, the concentrated product was recrystallized with methanol to obtain 9.8 g (95%) of a target compound 1-529.

The following Target Compound G was synthesized in the same manner as in Preparation Example 4, except that a compound in the following Table 4 was used instead of Compound 1-1 (F) in Preparation Example 4.

**[Table 4]**

| **Compound** | **F** | **G** | **Total yield** |
|---|---|---|---|
| 1-531 | 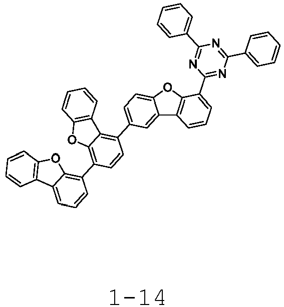 | 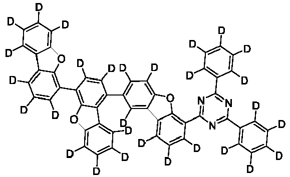 | **92%** |
| 1-541 | 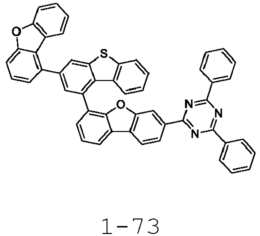 | 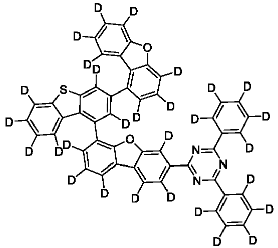 | **92%** |
| 1-547 | 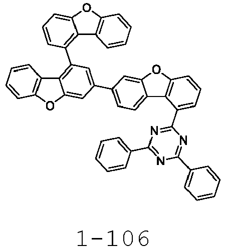 | 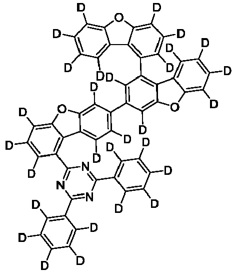 | **95%** |
| 1-549 | 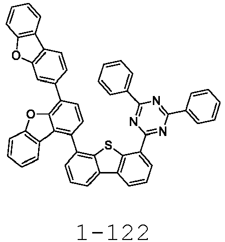 | 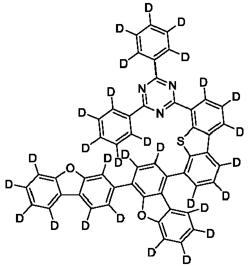 | **95%** |
| 1-554 | 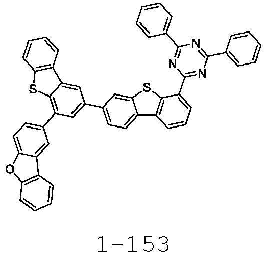 | 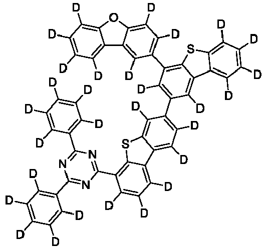 | **94%** |
| 1-559 | 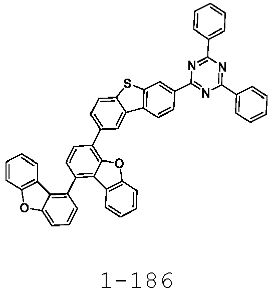 | 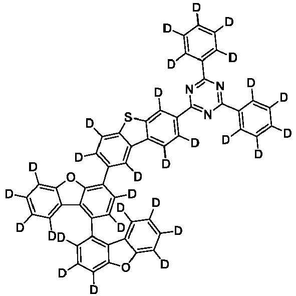 | **93%** |
| 1-567 | 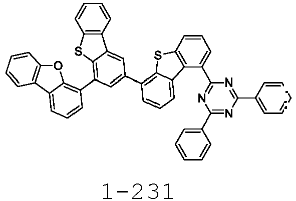 | 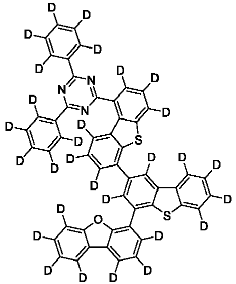 | **92%** |
| 1-569 | 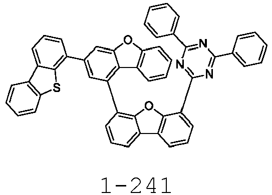 | 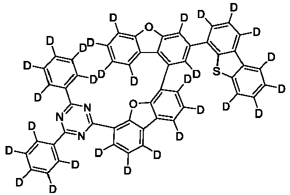 | **91%** |
| 1-575 | 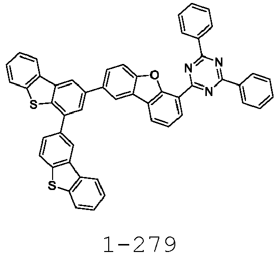 | 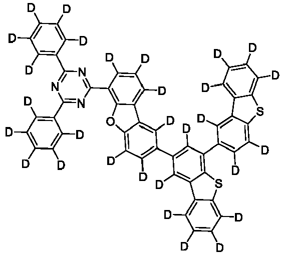 | **96%** |
| 1-582 | 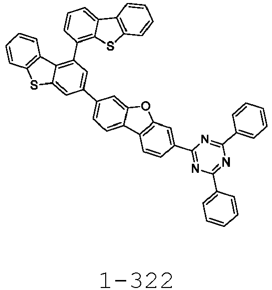 | 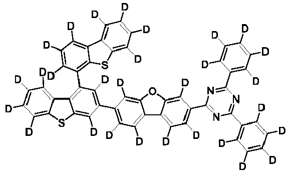 | **94%** |
| 1-587 | 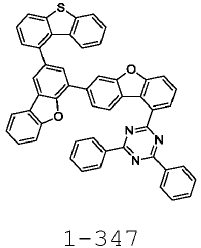 | 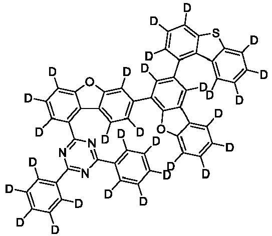 | **93%** |
| 1-590 | 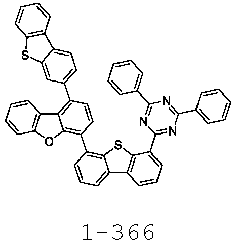 | 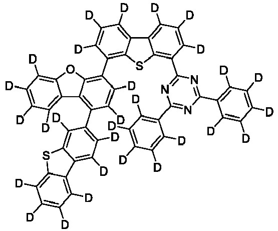 | **93%** |
| 1-595 | 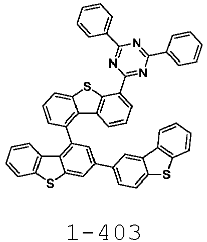 | 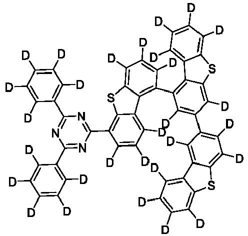 | **95%** |
| 1-598 | 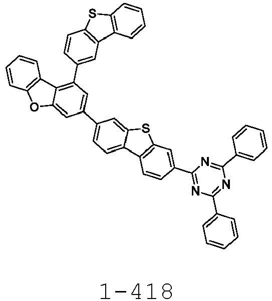 | 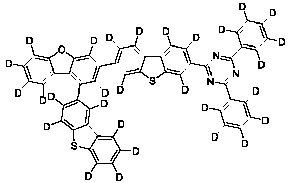 | **92%** |
| 1-607 | 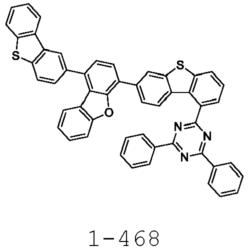 | 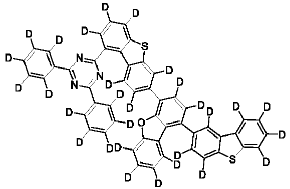 | **94%** |

### [Preparation Example 5] Synthesis of Compound 2-3

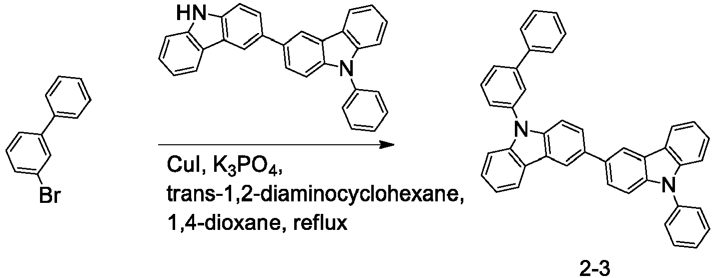

### 1) Preparation of Compound 2-3

After 3.7 g (15.8 mM) of 3-bromo-1,1'-biphenyl, 6.5 g (15.8 mM) of 9-phenyl-9H,9'H-3,3'-bicarbazole, 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-oxane, the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 7.5g (85%) of Target Compound 2-3.

Target Compound A was prepared and synthesized in the same manner as in Preparation Example 5, except that Intermediate A in the following Table 5 was used instead of 3-bromo-1,1"-biphenyl, and Intermediate B in the following Table 5 was used instead of 9-phenyl-9H,9'H-3,3'-bicarbazole in Preparation Example 5.

**[Table 5]**

| Compound No. | Intermediate A | Intermediate B | Target Compound A | Total yield |
|---|---|---|---|---|
| 2-3 | 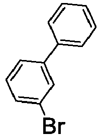 | 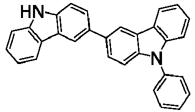 | 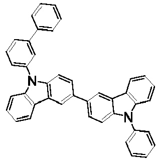 | 85% |
| 2-4 | 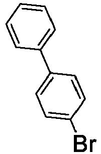 | | 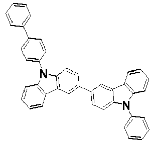 | 83% |
| 2-5 | 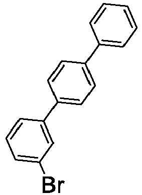 | | 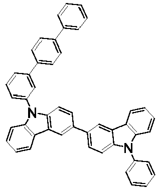 | 85% |
| 2-7 | 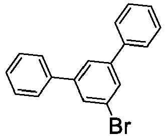 | | 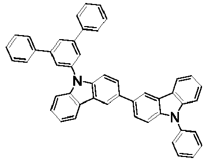 | 84% |
| 2-31 | 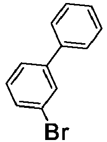 | 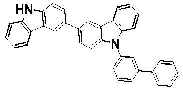 | 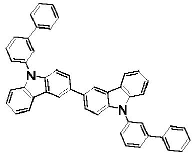 | 81% |
| 2-32 | 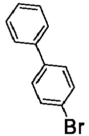 | | 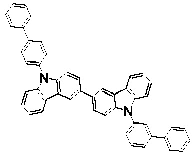 | 80% |
| 2-42 | 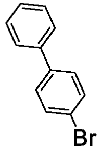 | 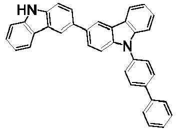 | 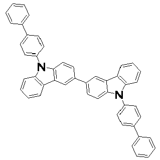 | 82% |

### [Preparation Example 6] Synthesis of Compound 2-98

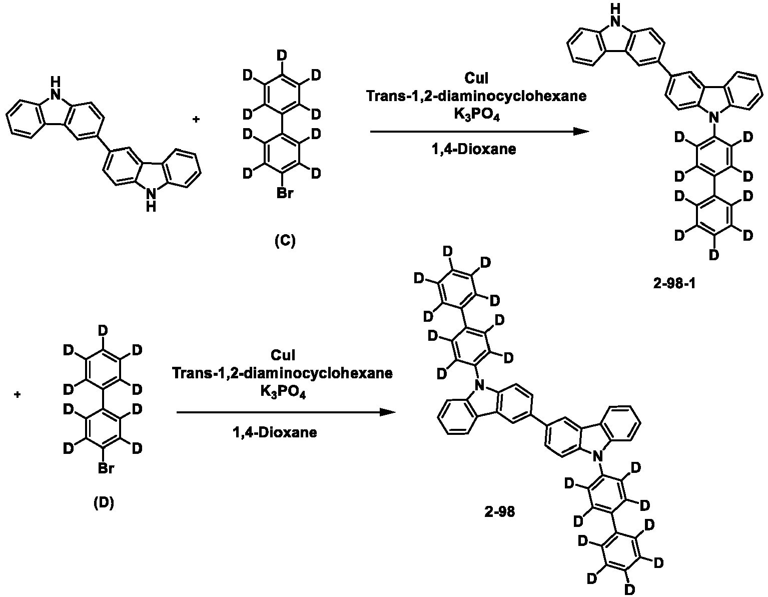

### Preparation of Compound 2-98-1

After 9H,9'H-3,3'-bicarbazole (10 g, 0.030 mol), 4-bromo-1,1'-biphenyl-2,2',3,3', 4',5,5',6,6'-D₉ (C) (7.26 g, 0.030 mol), CuI (0.57 g, 0.003 mol), trans-1,2-diaminocyclohexane (0.34 g, 0.003 mol), and K₃PO₄ (12.7 g, 0.06 mol) were dissolved in 1,4-dioxane (100 ml) in a one neck round bottom flask, the resulting solution was refluxed at 125°C for 8 hours. After the reaction was completed, the resulting product was cooled, extracted with dichloromethane and then dried over MgSO₄. The filtered organic layer was concentrated under reduced pressure, and then separated by column chromatography to obtain Compound 2-98-1 (13.92 g, 94%) .

### Preparation of Compound 2-98

After Intermediate 2-98-1 (13.92 g, 0.028 mol), 4-bromo-1,1'-biphenyl-2,2',3,3',4',5,5',6,6'-D₉ (C) (6.83 g, 0.028 mol), CuI (0.53 g, 0.0028 mol), trans-1,2-diaminocyclohexane (0.32 g, 0.0028 mol), and K₃PO₄ (11.89 g, 0.056 mol) were dissolved in 1,4-dioxane (140 ml) in a one neck round bottom flask, the resulting solution was refluxed at 125°C for 8 hours. After the reaction was completed, the resulting product was cooled, extracted with dichloromethane and then dried over MgSO₄. The filtered organic layer was concentrated under reduced pressure, and then separated by column chromatography to obtain Compound 2-98 (16.14 g, 88%).

When Compound M and Compound N are the same, the target compound may be immediately synthesized by adding 2 equivalents of Compound M in the method for synthesizing Intermediate 2-98-1 in Preparation Example 6.

The following target compound B was synthesized in the same manner as in Preparation Example 6, except that Compounds C and D in the following Table 6 were used instead of 4-bromo-1,1'-biphenyl-2,2',3,3',4',5,5',6,6'-D₉ (Intermediate C) and 4-bromo-1,1'-biphenyl-2,2',3,3',4',5,5',6,6'-D₉ (Intermediate D), respectively, in Preparation Example 6.

**[Table 6]**

| Compound No. | Intermediate C | Intermediate D | Target Compound B | Yield |
|---|---|---|---|---|
| 2-98 | 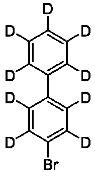 | 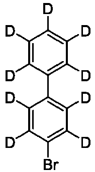 | 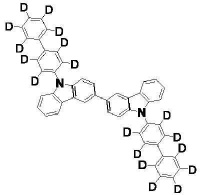 | 88% |
| 2-99 | 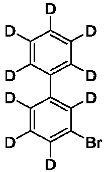 | 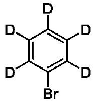 | 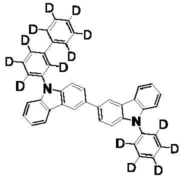 | 95% |
| 2-101 | 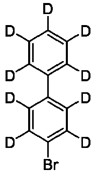 | 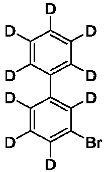 | 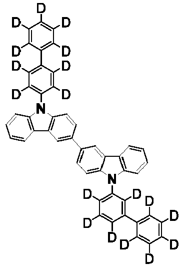 | 74% |
| 2-104 | 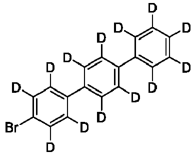 | 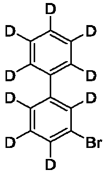 | 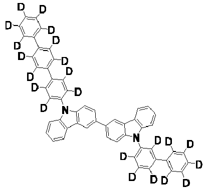 | 69% |

### [Preparation Example 7] Synthesis of Compound 2-106

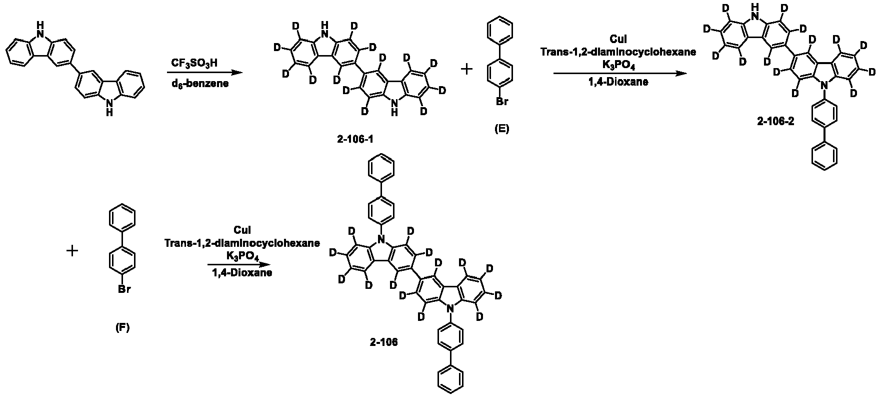

### Preparation of Compound 2-106-1

A mixture of 9H,9'H-3,3'-biscarbazole (10 g, 0.030 mol), triflic acid (34 g, 0.023 mol), and D₆-benzene (100 ml) was refluxed at 50°C in a one neck round bottom flask. After the mixture was cooled, the resulting product was extracted with dichloromethane, and then dried over MgSO₄. The product was separated by column chromatography to obtain Intermediate 2-106-1 (7.07 g, 68%).

### Preparation of Compound 2-106-2

After Intermediate 2-106-1 (7.07 g, 0.02 mol), CuI (0.38 g, 0.002 mol), 4-bromo-1,1'-biphenyl (E) (4.66 g, 0.02 mol), trans-1,4-diaminocyclohexane (0.23 g, 0.002 mol), and K₃PO₄ (8.49 g, 0.04 mol) were dissolved in 1,4-dioxane (70 ml) in a one-neck round bottom flask, the resulting solution was refluxed at 125°C for 8 hours. After the reaction was completed, distilled water and dichloromethane were added thereto at room temperature, extraction was performed, the organic layer was dried over MgSO₄, and then the solvent was removed by a rotary evaporator. The reaction product was purified by column chromatograph (DCM:Hexane = 1:3) and recrystallized with methanol to obtain Intermediate 2-106-2 (8.28 g, 83%).

### Preparation of Compound 2-106

After Intermediate 2-106-2 (8.28 g, 0.017 mol), CuI (0.32 g, 0.0017 mol), 4-bromo-1,1'-biphenyl (F) (3.96 g, 0.017 mol), trans-1,4-diaminocyclohexane (0.19 g, 0.0017 mol), and K₃PO₄ (7.22 g, 0.034 mol) were dissolved in 1,4-dioxane (80 ml) in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hexane = 1:3) and recrystallized with methanol to obtain Target Compound 2-106 (8.63 g, 78%).

When Intermediate Compound E and Compound F are the same, the target compound may be immediately synthesized by adding 2 equivalents of compound E in the method for synthesizing 2-106-1 in Preparation Example 7.

The following Target Compound C was synthesized in the same manner as in Preparation Example 7, except that Compounds E and F in the following Table 7 were used instead of 4-bromo-1,1'-biphenyl (E) and 4-bromo-1,1'-biphenyl (F), respectively, in Preparation Example 7.

**[Table 7]**

| Compound No. | Intermediate E | Intermediate F | Target Compound C | Yield |
|---|---|---|---|---|
| 2-106 | 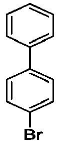 | 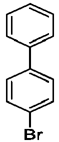 | 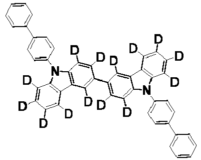 | 78% |
| 2-107 | 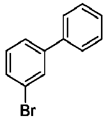 | 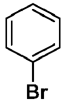 | 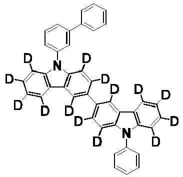 | 90% |
| 2-108 | 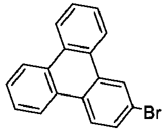 | 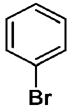 | 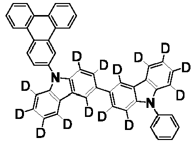 | 72% |

### [Preparation Example 8] Synthesis of Compound 2-110

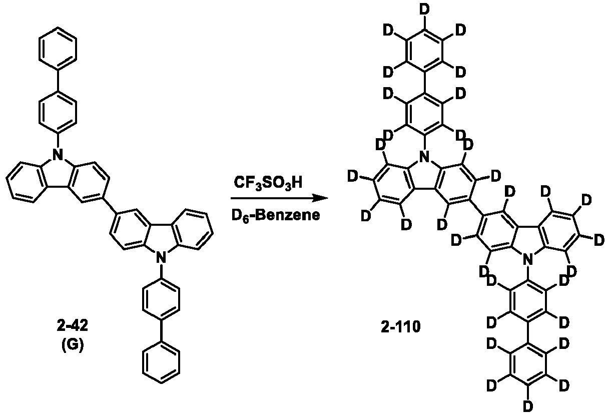

### Preparation of Compound 2-110

A mixture of Intermediate Compound 2-42 (12.17 g, 0.017 mol), triflic acid (40.8 g, 0.27 mol), and D₆-benzene (120 ml) was refluxed at 50°C in a one neck round bottom flask. Extraction was performed with dichloromethane and water, and the organic layer was dried over MgSO₄, then concentrated, and then filtered with silica gel. After concentration, the concentrated product was recrystallized with methanol to obtain Target Compound 2-110 (8.87 g, 78%).

The following Target Compound D was synthesized in the same manner as in Preparation Example 8, except that an intermediate Compound G in the following Table 8 was used instead of an intermediate Compound 2-42 (G) in Preparation Example 8.

**[Table 8]**

| Compound No. | Intermediate G | Target Compound D | Yield |
|---|---|---|---|
| 2-110 | 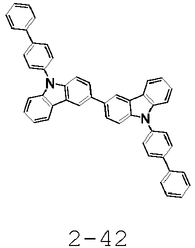 | 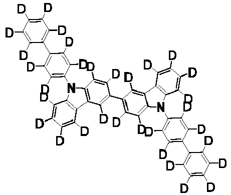 | 78% |
| 2-111 | 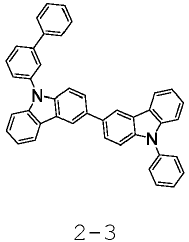 | 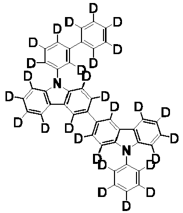 | 91% |
| 2-113 | 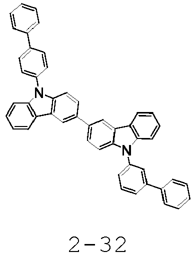 | 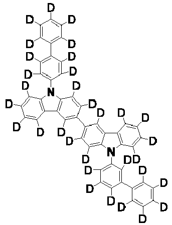 | 74% |
| 2-114 | 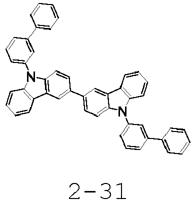 | 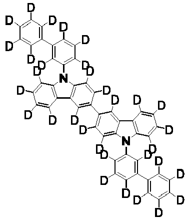 | 84% |
| 2-116 | 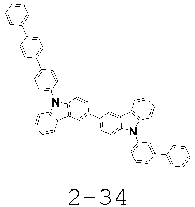 | 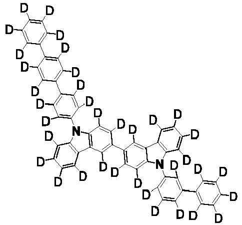 | 72% |

### [Preparation Example 9] Synthesis of Compound 3-2

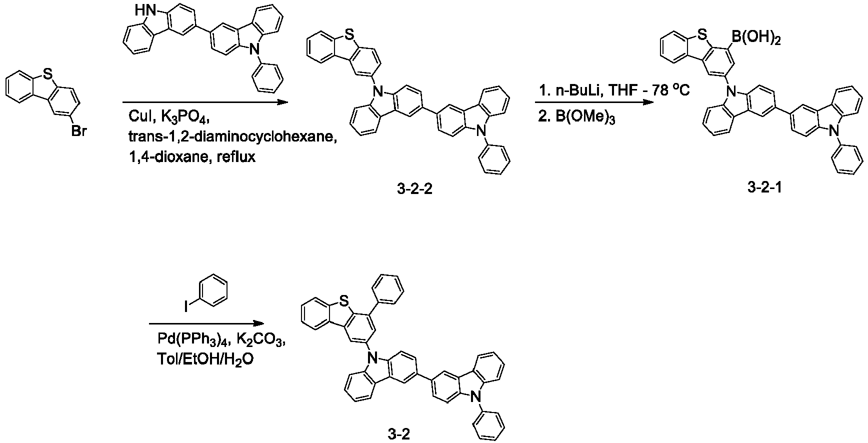

### 1) Preparation of Compound 3-2-2

After 4.2 g (15.8 mM) of 2-bromodibenzo[b,d]thiophene, 6.5 g (15.8 mM) of 9-phenyl-9H,9'H-3,3'-bicarbazole, 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-oxane, the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM : Hex = 1 : 3) and recrystallized with methanol to obtain 7.9 g (85%) of Target Compound 3-2-2.

### 2) Preparation of Compound 3-2-1

7.4 mL (18.6 mmol) of 2.5 M n-BuLi was added dropwise to a mixed solution containing 8.4g (14.3 mmol) of Compound 3-2-2 and 100 mL of THF at -78°C, and the resulting mixture was stirred at room temperature for 1 hour. 4.8 mL (42.9 mmol) of trimethyl borate was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM : MeOH = 100 : 3) and recrystallized with DCM to obtain 3.9 g (70%) of Target Compound 3-2-1.

### 3) Preparation of Compound 3-2

After 6.7 g (10.5 mM) of Compound 3-2-1, 2.1 g (10.5 mM) of iodobenzene, 606 mg (0.52 mM) of Pd(PPh₃)₄, and 2.9 g (21.0 mM) of K₂CO₃ were dissolved in 100/20/20 mL of toluene/EtOH/H₂O, the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM : Hex = 1 : 3) and recrystallized with methanol to obtain 4.9 g (70%) of Target Compound 3-2.

The following Target Compound E was obtained in the same manner as in the preparation of 3-2 by using the following Compound A instead of iodobenzene in Preparation Example 9.

**[Table 9]**

| Compound No. | Intermediate 3-2-1 | Intermediate A | Target Compound E | Total yield |
|---|---|---|---|---|
| 3-2 | 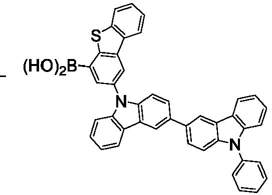 | 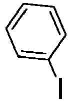 | 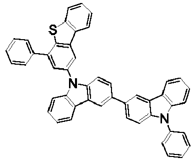 | 83% |
| 3-3 | | 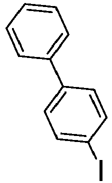 | 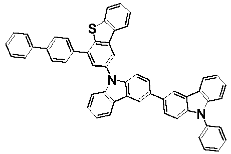 | 84% |

### [Preparation Example 10] Synthesis of Compound 3-106

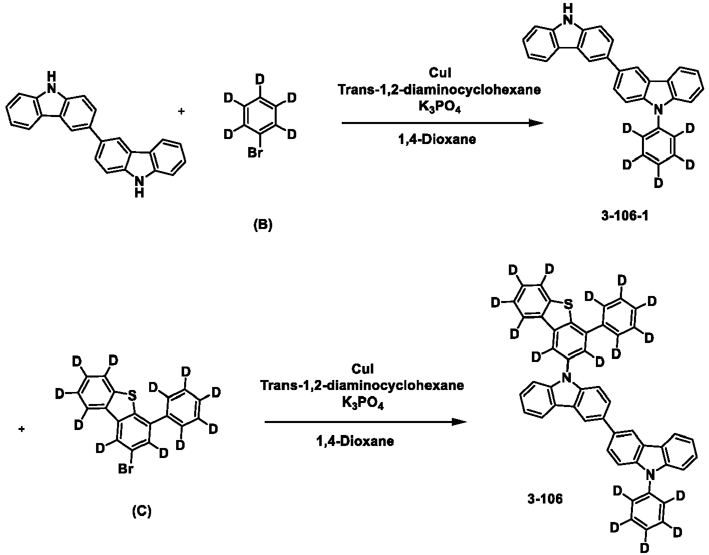

### Preparation of Compound 3-106-1

After 9H,9'H-3,3'-bicarbazole (10 g, 0.030 mol), 1-bromobenzene-2,3,4,5,6-D₅ (B) (4.86 g, 0.030 mol), CuI (0.57 g, 0.003 mol), trans-1,2-diaminocyclohexane (0.34 g, 0.003 mol), and K₃PO₄ (12.7 g, 0.06 mol) were dissolved in 1,4-dioxane (100 ml) in a one neck round bottom flask, the resulting solution was refluxed at 125°C for 8 hours. After the reaction was completed, the resulting product was cooled, extracted with dichloromethane and then dried over MgSO₄. The filtered organic layer was concentrated under reduced pressure, and then separated by column chromatography to obtain Compound 3-106-1 (10 g, 81%).

### Preparation of Compound 3-106

After Intermediate 3-106-1 (10 g, 0.024 mol), 2-bromo-4-(phenyl-D₅)dibenzo[b,d]thiophene 1,3,6,7,8,9-D₆ (C) (8.4 g, 0.024 mol), CuI (0.45 g, 0.0024 mol), trans-1,2-diaminocyclohexane (0.27 g, 0.0024 mol), and K₃PO₄ (10.2 g, 0.048 mol) were dissolved in 1,4-dioxane (100 ml) in a one neck round bottom flask, the resulting solution was refluxed at 125°C for 8 hours. After the reaction was completed, the resulting product was cooled, extracted with dichloromethane and then dried over MgSO₄. The filtered organic layer was concentrated under reduced pressure, and then separated by column chromatography to obtain Compound 3-106 (14.4 g, 88%).

The following Target Compound F was synthesized in the same manner as in Preparation Example 10, except that Compounds B and C in the following Table 10 were used instead of 1-bromobenzene-2,3,4,5,6-D₅ (Intermediate B) and 2-bromo-4-(phenyl-D₅)dibenzo[b,d]thiophene 1,3,6,7,8,9-D₆ (Intermediate C), respectively, in Preparation Example 10.

**[Table 10]**

| Compound No. | Intermediate B | Intermediate C | Target Compound F | Yield |
|---|---|---|---|---|
| 3-107 | 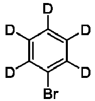 | 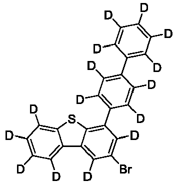 | 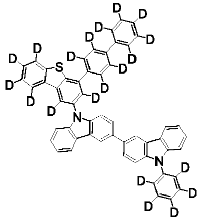 | 86% |
| 3-108 | 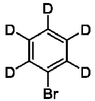 | 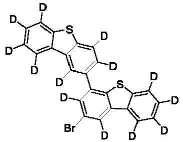 | 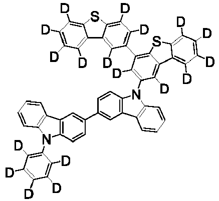 | 82% |
| 3-109 | 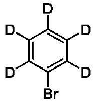 | 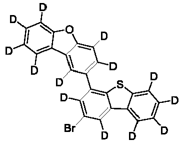 | 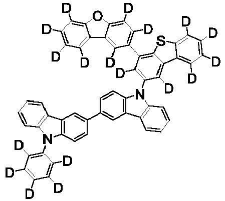 | 77% |

### [Preparation Example 11] Synthesis of Compound 3-111

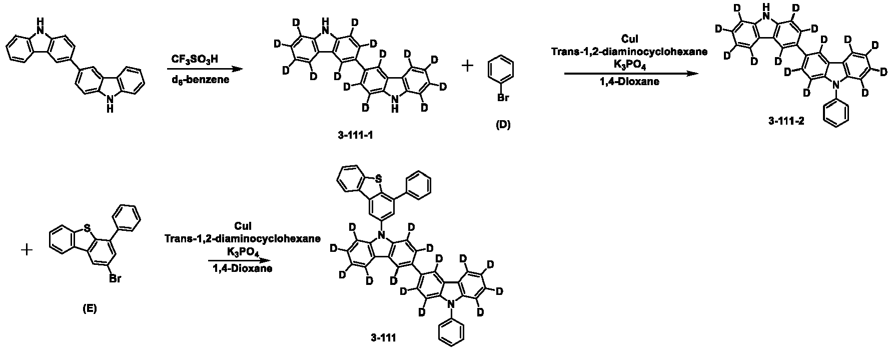

### Preparation of Compound 3-111-1

A mixture of 9H,9'H-3,3'-biscarbazole (10 g, 0.030 mol), triflic acid (34 g, 0.023 mol), and D₆-benzene (100 ml) was refluxed at 50°C in a one neck round bottom flask. After the mixture was cooled, the resulting product was extracted with dichloromethane, and then dried over MgSO₄. The product was separated by column chromatography to obtain Intermediate 3-111-1 (7.07 g, 68%).

### Preparation of Compound 3-111-2

After Intermediate 3-111-1 (7.07 g, 0.02 mol), CuI (0.38 g, 0.002 mol), bromobenzene (D) (3.14 g, 0.02 mol), trans-1,4-diaminocyclohexane (0.23 g, 0.002 mol), and K₃PO₄ (8.49 g, 0.04 mol) were dissolved in 1,4-dioxane (70 ml) in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reaction product was purified by column chromatograph (DCM:Hexane = 1:3) and recrystallized with methanol to obtain Intermediate 3-111-2 (6.85 g, 81%).

### Preparation of Compound 3-111

After Intermediate 3-111-2 (6.85 g, 0.016 mol), CuI (0.30 g, 0.0016 mol), 2-bromo-4-phenyldibenzo[b,d]thiophene (E) (3.73 g, 0.016 mol), trans-1,4-diaminocyclohexane (0.18 g, 0.0016 mol), and K₃PO₄ (6.79 g, 0.032 mol) were dissolved in 1,4-dioxane (70 ml) in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed by a rotary evaporator. The reaction product was purified by column chromatography (DCM:Hexane = 1:3) and recrystallized with methanol to obtain Target Compound 3-111 (8.72 g, 72%).

The following Target Compound G was synthesized in the same manner as in Preparation Example 11, except that Compounds D and E in the following Table 11 were used instead of bromobenzene (D) and 2-bromo-4-phenyldibenzo[b,d]thiophene (E), respectively, in Preparation Example 11.

**[Table 11]**

| Compound No. | Intermediate D | Intermediate E | Target Compound G | Yield |
|---|---|---|---|---|
| 3-112 | 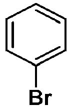 | 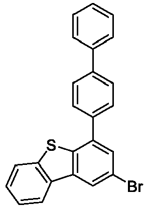 | 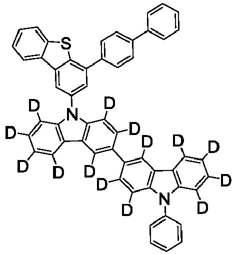 | 76% |
| 3-114 | 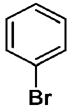 | 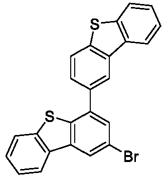 | 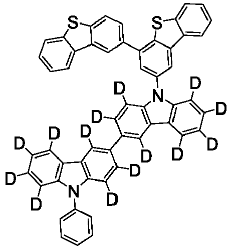 | 81% |
| 3-115 | 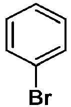 | 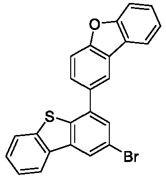 | 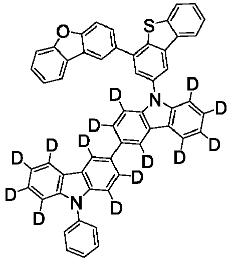 | 74% |

### [Preparation Example 12] Synthesis of Compound 3-117

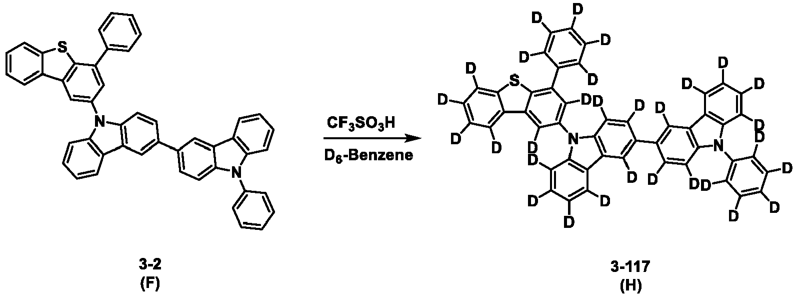

### Preparation of Compound 3-117

A mixture of Intermediate Compound 3-2 (10 g, 0.015 mol), triflic acid (34.8 g, 0.23 mol), and D₆-benzene (100 ml) was refluxed at 50°C in a one neck round bottom flask. Extraction was performed with dichloromethane and water, and the organic layer was dried over MgSO₄, and then concentrated. The reaction product was purified by column chromatography (DCM:Hexane = 1:2) and recrystallized with methanol to obtain Target Compound 3-117 (9.62 g, 92%).

The following Target Compound H was synthesized in the same manner as in Preparation Example 12, except that an intermediate F in the following Table 12 was used instead of an intermediate Compound 3-2 (F) in Preparation Example 12.

**[Table 12]**

| Compound No. | Intermediate F | Target Compound H | Yield |
|---|---|---|---|
| 3-118 | 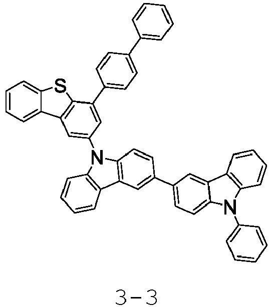 | 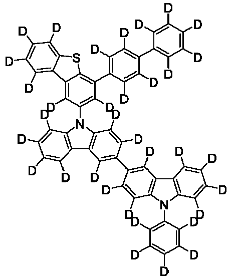 | 85% |
| 3-119 | 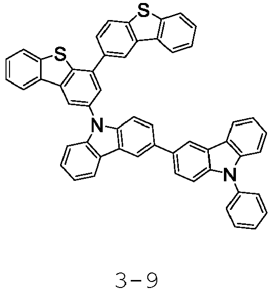 | 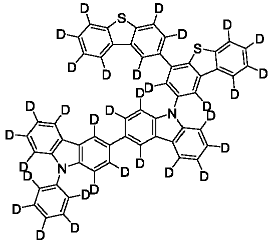 | 91% |
| 3-120 | 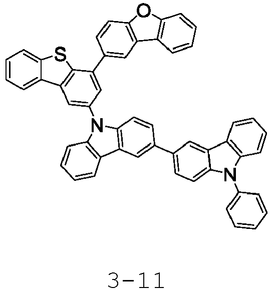 | 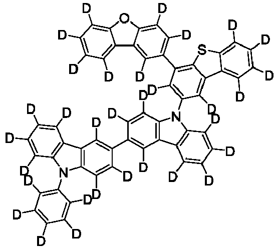 | 88% |
| 3-123 | 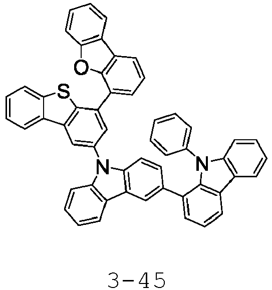 | 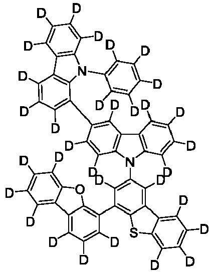 | 89% |
| 3-125 | 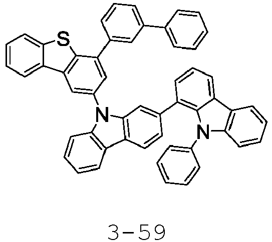 | 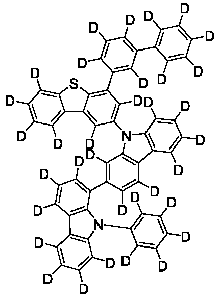 | 92% |

The remaining compounds related to Chemical Formula 1 other than the compounds shown in Preparation Examples 1 to 4 and Tables 1 to 4 above, the remaining compounds related to Chemical Formula 2 other than the compounds shown in Preparation Examples 5 to 8 and Tables 5 to 8 and the remaining compounds related to Chemical Formula 3 other than the compounds shown in Preparation Examples 9 to 12 and Tables 9 to 12 were also prepared in the same manner as in the methods described in the above-described Preparation Examples.

The following Tables 13 to 18 are the FD-MS data and 1H NMR data of the synthesized compounds, and it can be confirmed through the following data that the desired compound was synthesized.

**[Table 13]**

| Compound | FD-Mass | Compound | FD-Mass |
|---|---|---|---|
| 1-1 | m/z= 731.22 (C₅₁H₂₉N₃O₃=731.81) | 1-268 | m/z= 763.18 (C₅₁H₂₉N₃OS₂=763.93) |
| 1-2 | m/z= 731.22 (C₅₁H₂₉N₃O₃=731.81) | 1-271 | m/z= 763.18 (C₅₁H₂₉N₃OS₂=763.93) |
| 1-5 | m/z= 731.22 (C₅₁H₂₉N₃O₃=731.81) | 1-279 | m/z= 763.18 (C₅₁H₂₉N₃OS₂=763.93) |
| 1-11 | m/z= 731.22 (C₅₁H₂₉N₃O₃=731.81) | 1-286 | m/z= 763.18 (C₅₁H₂₉N₃OS₂=763.93) |
| 1-14 | m/z= 731.22 (C₅₁H₂₉N₃O₃=731.81) | 1-293 | m/z= 747.20 (C₅₁H₂₉N₃O₂S=747.87) |
| 1-19 | m/z= 731.22 (C₅₁H₂₉N₃O₃=731.81) | 1-299 | m/z= 747.20 (C₅₁H₂₉N₃O₂S=747.87) |
| 1-23 | m/z= 731.22 (C₅₁H₂₉N₃O₃=731.81) | 1-307 | m/z= 747.20 (C₅₁H₂₉N₃O₂S=747.87) |
| 1-25 | m/z= 747.20 (C₅₁H₂₉N₃O₂S=747.87) | 1-312 | m/z= 747.20 (C₅₁H₂₉N₃O₂S=747.87) |
| 1-31 | m/z= 747.20 (C₅₁H₂₉N₃O₂S=747.87) | 1-313 | m/z= 763.18 (C₅₁H₂₉N₃OS₂=763.93) |
| 1-34 | m/z= 747.20 (C₅₁H₂₉N₃O₂S=747.87) | 1-322 | m/z= 763.18 (C₅₁H₂₉N₃OS₂=763.93) |
| 1-43 | m/z= 747.20 (C₅₁H₂₉N₃O₂S=747.87) | 1-329 | m/z= 763.18 (C₅₁H₂₉N₃OS₂=763.93) |
| 1-54 | m/z= 731.22 ( C₅₁H₂₉N₃O₃=731.81) | 1-331 | m/z= 763.18 (C₅₁H₂₉N₃OS₂=763.93) |
| 1-63 | m/z= 731.22 (C₅₁H₂₉N₃O₃=731.81) | 1-337 | m/z= 747.20 (C₅₁H₂₉N₃O₂S=747.87) |
| 1-68 | m/z= 731.22 (C₅₁H₂₉N₃O₃=731.81) | 1-343 | m/z= 747.20 (C₅₁H₂₉N₃O₂S=747.87) |
| 1-72 | m/z= 731.22 (C₅₁H₂₉N₃O₃=731.81) | 1-347 | m/z= 747.20 (C₅₁H₂₉N₃O₂S=747.87) |
| 1-73 | m/z= 747.20 (C₅₁H₂₉N₃O₂S=747.87) | 1-358 | m/z= 763.18 (C₅₁H₂₉N₃OS₂=763.93) |
| 1-78 | m/z= 747.20 (C₅₁H₂₉N₃O₂S=747.87) | 1-361 | m/z= 763.18 (C₅₁H₂₉N₃OS₂=763.93) |
| 1-87 | m/z= 747.20 (C₅₁H₂₉N₃O₂S=747.87) | 1-366 | m/z= 763.18 (C₅₁H₂₉N₃OS₂=763.93) |
| 1-88 | m/z= 747.20 (C₅₁H₂₉N₃O₂S=747.87) | 1-372 | m/z= 763.18 (C₅₁H₂₉N₃OS₂=763.93) |
| 1-95 | m/z= 747.20 (C₅₁H₂₉N₃O₂S=747.87) | 1-378 | m/z= 763.18 (C₅₁H₂₉N₃OS₂=763.93) |
| 1-96 | m/z= 747.20 (C₅₁H₂₉N₃O₂S=747.87) | 1-388 | m/z= 779.15 (C₅₁H₂₉N₃S₃=779.99) |
| 1-97 | m/z= 731.22 (C₅₁H₂₉N₃O₃=731.81) | 1-394 | m/z= 779.15 (C₅₁H₂₉N₃S₃=779.99) |
| 1-102 | m/z= 731.22 (C₅₁H₂₉N₃O₃=731.81) | 1-403 | m/z= 779.15 (C₅₁H₂₉N₃S₃=779.99) |
| 1-106 | m/z= 731.22 (C₅₁H₂₉N₃O₃=731.81) | 1-407 | m/z= 779.15 (C₅₁H₂₉N₃S₃=779.99) |
| 1-114 | m/z= 747.20 (C₅₁H₂₉N₃O₂S=747.87) | 1-412 | m/z= 747.20 (C₅₁H₂₉N₃O₂S=747.87) |
| 1-118 | m/z= 747.20 (C₅₁H₂₉N₃O₂S=747.87) | 1-423 | m/z= 747.20 (C₅₁H₂₉N₃O₂S=747.87) |
| 1-119 | m/z= 747.20 (C₅₁H₂₉N₃O₂S=747.87) | 1-439 | m/z= 779.15 (C₅₁H₂₉N₃S₃=779.99) |
| 1-122 | m/z= 747.20 (C₅₁H₂₉N₃O₂S=747.87) | 1-448 | m/z= 779.15 (C₅₁H₂₉N₃S₃=779.99) |
| 1-132 | m/z= 747.20 (C₅₁H₂₉N₃O₂S=747.87) | 1-451 | m/z= 779.15 (C₅₁H₂₉N₃S₃=779.99) |
| 1-135 | m/z= 747.20 (C₅₁H₂₉N₃O₂S=747.87) | 1-457 | m/z= 763.18 (C₅₁H₂₉N₃OS₂=763.93) |
| 1-137 | m/z= 747.20 (C₅₁H₂₉N₃O₂S=747.87) | 1-459 | m/z= 763.18 (C₅₁H₂₉N₃OS₂=763.93) |
| 1-139 | m/z= 747.20 (C₅₁H₂₉N₃O₂S=747.87) | 1-468 | m/z= 763.18 (C₅₁H₂₉N₃OS₂=763.93) |
| 1-148 | m/z= 763.18 (C₅₁H₂₉N₃OS₂=763.93) | 1-472 | m/z= 763.18 (C₅₁H₂₉N₃OS₂=763.93) |
| 1-153 | m/z= 763.18 (C₅₁H₂₉N₃OS₂=763.93) | 1-481 | m/z= 736.25 (C₅₁H₂₄D₅N₃O₃=736.84) |
| 1-155 | m/z= 763.18 (C₅₁H₂₉N₃OS₂=763.93) | 1-483 | m/z= 738.26 (C₅₁H₂₂D₇N₃O₃=738.85) |
| 1-163 | m/z= 763.18 (C₅₁H₂₉N₃OS₂=763.93) | 1-484 | m/z= 748.33 (C₅₁H₁₂D₁₇N₃O₃=748.91) |
| 1-164 | m/z= 763.18 (C₅₁H₂₉N₃OS₂=763.93) | 1-487 | m/z= 757.26 (C₅₁H₁₉D₁₀N₃O₂S=757.93) |
| 1-169 | m/z= 747.20 (C₅₁H₂₉N₃O₂S=747.87) | 1-489 | m/z= 741.28 (C₅₁H₁₉D₁₀N₃O₃=741.87) |
| 1-171 | m/z= 747.20 (C₅₁H₂₉N₃O₂S=747.87) | 1-494 | m/z= 741.28 (C₅₁H₁₉D₁₀N₃O₃=741.87) |
| 1-178 | m/z= 747.20 (C₅₁H₂₉N₃O₂S=747.87) | 1-496 | m/z= 764.30 (C₅₁H₁₂D₁₇N₃O₂S=764.98) |
| 1-180 | m/z= 747.20 (C₅₁H₂₉N₃O₂S=747.87) | 1-497 | m/z= 773.24 (C5₁H₁₉D₁₀N₃OS₂=773.99) |
| 1-186 | m/z= 747.20 (C₅₁H₂₉N₃O₂S=747.87) | 1-500 | m/z= 764.30 (C₅₁H₁₂D₁₇N₃O₂S=764.98) |
| 1-191 | m/z= 747.20 (C₅₁H₂₉N₃O₂S=747.87) | 1-503 | m/z= 773.24 (C₅₁H₁₉D₁₀N₃OS₂=773.99) |
| 1-193 | m/z= 763.18 (C₅₁H₂₉N₃OS₂=763.93) | 1-505 | m/z= 773.24 (C₅₁H₁₉D₁₀N₃OS₂=773.99) |
| 1-196 | m/z= 763.18 (C₅₁H₂₉N₃OS₂=763.93) | 1-510 | m/z= 759.27 (C₅₁H₁₇D₁₂N₃O₂S=759.95) |
| 1-202 | m/z= 763.18 (C₅₁H₂₉N₃OS₂=763.93) | 1-518 | m/z= 773.24 (C₅₁H₁₉D₁₀N₃OS₂=773.99) |
| 1-207 | m/z= 763.18 (C₅₁H₂₉N₃OS₂=763.93) | 1-529 | m/z= 760.40 (C₅₁D₂₉N₃O₃=760.99) |
| 1-211 | m/z= 763.18 (C₅₁H₂₉N₃OS₂=763.93) | 1-531 | m/z= 760.40 (C₅₁D₂₉N₃O₃=760.99) |
| 1-213 | m/z= 763.18 (C₅₁H₂₉N₃OS₂=763.93) | 1-541 | m/z= 776.38 (C₅₁D₂₉N₃O₂S=777.05) |
| 1-217 | m/z= 747.20 (C₅₁H₂₉N₃O₂S=747.87) | 1-547 | m/z= 760.40 (C₅₁D₂₉N₃O₃=760.99) |
| 1-221 | m/z= 747.20 (C₅₁H₂₉N₃O₂S=747.87) | 1-549 | m/z= 776.38 (C₅₁D₂₉N₃O₂S=777.05) |
| 1-223 | m/z= 747.20 (C₅₁H₂₉N₃O₂S=747.87) | 1-554 | m/z= 792.36 (C₅₁D₂₉N₃OS₂=793.11) |
| 1-231 | m/z= 763.18 (C₅₁H₂₉N₃OS₂=763.93) | 1-559 | m/z= 776.38 (C₅₁D₂₉N₃O₂S=777.05) |
| 1-233 | m/z= 763.18 (C₅₁H₂₉N₃OS₂=763.93) | 1-567 | m/z= 792.36 (C₅₁D₂₉N₃OS₂=793.11) |
| 1-235 | m/z= 763.18 (C₅₁H₂₉N₃OS₂=763.93) | 1-569 | m/z= 776.38 (C₅₁D₂₉N₃O₂S=777.05) |
| 1-238 | m/z= 763.18 (C₅₁H₂₉N₃OS₂=763.93) | 1-575 | m/z= 792.36 (C₅₁D₂₉N₃OS₂=793.11) |
| 1-239 | m/z= 763.18 (C₅₁H₂₉N₃OS₂=763.93) | 1-582 | m/z= 792.36 (C₅₁D₂₉N₃OS₂=793.11) |
| 1-241 | m/z= 747.20 (C₅₁H₂₉N₃O₂S=747.87) | 1-587 | m/z= 776.38 (C₅₁D₂₉N₃O₂S=777.05) |
| 1-242 | m/z= 747.20 (C₅₁H₂₉N₃O₂S=747.87) | 1-590 | m/z= 792.36 (C₅₁D₂₉N₃OS₂=793.11) |
| 1-249 | m/z= 747.20 (C₅₁H₂₉N₃O₂S=747.87) | 1-595 | m/z= 808.33 (C₅₁D₂₉N₃S₃=809.17) |
| 1-251 | m/z= 747.20 (C₅₁H₂₉N₃O₂S=747.87) | 1-598 | m/z= 792.36 (C₅₁D₂₉N₃OS₂=793.11) |
| 1-255 | m/z= 747.20 (C₅₁H₂₉N₃O₂S=747.87) | 1-607 | m/z= 792.36 (C₅₁D₂₉N₃OS₂=793.11) |
| 1-263 | m/z= 747.20 (C₅₁H₂₉N₃O₂S=747.87) | | |

**[Table 14]**

| Compound | FD-Mass | Compound | FD-Mass |
|---|---|---|---|
| 2-3 | m/z= 560.23 (C₄₂H₂₈N₂=560.70) | 2-4 | m/z= 560.23 (C₄₂H₂₈N₂=560.70) |
| 2-5 | m/z= 636.26 (C₄₈H₃₂N₂=636.80) | 2-7 | m/z= 636.26 (C₄₈H₃₂N₂=636.80) |
| 2-31 | m/z= 636.26 (C₄₈H₃₂N₂=636.80) | 2-32 | m/z= 636.26 (C₄₈H₃₂N₂=636.80) |
| 2-42 | m/z= 636.26 (C₄₈H₃₂N₂=636.80) | 2-98 | m/z= 654.91 (C₄₈H₁₄D₁₈N₂=654.37) |
| 2-99 | m/z= 574.79 (C₄₂H₁₄D₁₄N₂=574.31) | 2-101 | m/z= 654.91 (C₄₈H₁₄D₁₈N₂=654.37) |
| 2-102 | m/z= 735.03 (C₅₄H₁₄D₂₂N₂=734.43) | 2-104 | m/z= 734.43 (C₅₄H₁₄D₂₂N₂=735.03) |
| 2-106 | m/z= 650.88 (C₄₈H₁₈D₁₄N₂=650.34) | 2-107 | m/z= 574.79 (C₄₂H₁₄D₁₄N₂=574.31) |
| 2-108 | m/z= 648.33 (C₄₈H₁₆D₁₄N₂=648.87) | 2-110 | m/z= 668.99 (C₄₈D₃₂N₂=668.46) |
| 2-111 | m/z= 588.87 (C₄₂D₂₈N₂=588.40) | 2-113 | m/z= 668.99 (C₄₈D₃₂N₂=668.46) |
| 2-114 | m/z= 668.99 (C₄₈D₃₂N₂=668.46) | 2-115 | m/z= 749.12 (C₅₄D₃₂N₂=748.51) |

**[Table 15]**

| Compound | FD-Mass | Compound | FD-Mass |
|---|---|---|---|
| 3-2 | m/z= 666.84 (C₄₈H₃₀N₂=666.21) | 3-3 | m/z= 742.24 (C₅₄H₃₄N₂S=742.94) |
| 3-106 | m/z= 682.31 (C₄₈H₁₄D₁₆N₂S=682.94) | 3-107 | m/z= 762.37 (C₅₄H₁₄D₂0N₂S=763.06) |
| 3-108 | m/z= 790.31 (C₅₄H₁₄D₁₈N₂S₂=791.09) | 3-109 | m/z= 774.34 (C₅₄H₁₄D₁₈N₂OS=775.03) |
| 3-111 | m/z= 680.30 (C₄₈H₁₆D₁₄N₂S=680.93) | 3-112 | m/z= 756.33 (C₅₄H₂₀D₁₄N₂S=757.03) |
| 3-114 | m/z= 786.29 (C₅₄H₁₈D₁₄N₂S₂=787.07) | 3-115 | m/z= 770.31 (C₅₄H₁₈D₁₄N₂OS=771.01) |
| 3-117 | m/z= 696.40 (C₄₈D₃₀N₂S=697.03) | 3-118 | m/z= 776.46 (C₅₄D₃₄N₂S=777.15) |
| 3-119 | m/z= 804.40 (C₅₄D₃₂N₂S₂=805.18) | 3-120 | m/z= 788.42 (C₅₄D₃₂N₂OS=789.12) |
| 3-123 | m/z= 788.42 (C₅₄D₃₂N₂OS=789.12) | 3-125 | m/z= 776.46 (C₅₄D₃₄N₂S=777.15) |

**[Table 16]**

| Compound | ¹H NMR(CDCl₃, 200Mz) |
|---|---|
| 1-1 | δ = 8.36(4H, d), 8.08(3H, d), 8.02 (2H, d), 7.98(2H, d), 7.88(2H, d), 7.81(1H, s), 7.54-7.50(11H, m), 7.39(1H, t), 7.31(1H, t) |
| 1-2 | δ = 8.36(4H, d),8.08-7.98(7H, m), 7.88(2H, d), 7.83(1H, s), 7.79(1H, d), 7.54-7.50 (10H, m), 7.39(1H, t), 7.31(1H, t) |
| 1-5 | δ = 8.36(4H, d), 8.08(2H, d), 8.02 (2H, d), 7.98-7.87 (5H, m), .69(1H, d), 757-7.50(11H, m), 7.39(2H, t), 7.31(2H, t) |
| 1-11 | δ = 8.36(4H, d), 8.08(2H, m), 8.03-7.76(9H, m), 7.54-7.50(10H, m), 7.39(2H, t), 7.31(2H, t) |
| 1-14 | δ = 8.36(4H, d), 8.08(3H, d), 8.01(1H, d), 7.98(2H, d), 7.88(2H, d), 7.83(1H, s), 7.79(1H, s), 7.57-7.50(10H, m), 7.39(2H, t), 7.31(2H, t) |
| 1-19 | δ = 8.36(4H, d), 8.03(2H, d), 7.98(2h, d), 7.87(1H, s), 7.82(2H, d), 7.76(2H, s), 7.69(1H, d), 7.57-7.50(10H, m), 7.39(2H, t), 7.31(2H, t) |
| 1-23 | δ = 8.36(4H, d), 8.08(1H, d), 7.98(2H, d), 7.93(1H, s), 7.87(1H, d), 7.82(2H, d), 7.69(2H, d), 7.57-7.50(11H, m), 7.39(2H, t), 7.31(2H, t) |
| 1-25 | δ = 8.45(1H, d) 8.36(4H, d), 8.30(1H, s), 8.08(2h, d), 8.03(2H, d), 8.01(1H, s), 7.98(1H, d), 7.93(2H, d) 7.88(1H, d), 7.56-7.50(12H, m), 7.39(1H, t), 7.31(1H, t) |
| 1-31 | δ = 8.45(1H, d), 8.36(4H, d), 8.30(1H, s), 8.08(1H, d), 8.03(2H, d), 8.01(1H, s), 7.98(2H, d), 7.93(1H ,d), 7.88(1H, s), 7.82(1H, d), 7.69(1H, d), 7.57-7.49(11H, m), 7.39(1H, t), 7.31(1H, t) |
| 1-34 | δ = 8.45(1H, d), 8.36(4H, d), 8.30(1H, s), 8.08(1H, d), 8.03(2H, d), 8.01(1H, s), 7.98(2H, d), 7.93(1H, d), 7.88(1H, d), 7.82(2h, d), 7.76(1H, d), 7.56-7.50(10H, m), 7.39(1H, t), 7.31(1H, t) |
| 1-43 | δ = 8.45(1H, d), 8.36(4H, d), 8.30(1H, s), 8.03(1H, d), 8.01(1H, s), 7.98-.7.88(6h, m), 7.76(1H, s), 7.69(1H, s), 7.57-7.50(11H, m), 7.39(1H, t), 7.31(1H, t) |
| 1-54 | δ = 8.36(4H, d), 8.08-7.98 (7H, m), 7.88(2H, d), 7.82(2h, d), 7.76(1H, s), 7.54-7.50(9H, m), 7.39(2H, t), 7.31(2H, t) |
| 1-63 | δ = 8.36(4H, d), 8.08-7.87(8H, m), 7.83(1H, s), 7.79(2H, d), 7.76(1H, s), 7.54-7.50(9H, m), 7.39(2H, t), 7.31(2H, t) |
| 1-68 | δ = 8.36(4H, d), 8.08-7.98(7H, m), 7.82 (2H, d), 7.76 (1H, s), 7.69(1H, d), 7.57-7.50(10H, m), 7.39(2H, t), 7.31(2H, t) |
| 1-72 | δ = 8.36(4H, d), 8.07-7.98(5H, m), 7.82(2H, d), 7.76(1H, s), 7.69(2H, d), 7.57-7.50(10H, m), 7.39(2H, t), 7.31(2H, t) |
| 1-73 | δ = 8.45(1H, s), 8.36(4H, d), 8.30(1H, s), 8.08-7.93(6Hh, m), 7.82(2H, d), 7.76(1H, s), 7.69(1H, d), 7.57-7.49(11H, m), 7.39(1H, t), 7.31(1H, t) |
| 1-78 | δ = 8.45(1H, d), 8.36(4H, d), 8.13-7.93 (7H, m), 7.82 (2H, d), 7.76(1H, s), 7.69(1H, d), 7.57-7.4(11H, m), 7.39(1H, t), 7.31(1H, t) |
| 1-87 | δ = 8.45(1H, d), 8.36(4H, d), 8.22(1H, s), 8.03-7.76 (12H, m), 7.56-7.50 (9H, m), 7.39(1H, t), 7.31(1H, t) |
| 1-88 | δ = 8.45(1H, d), 8.36(4H, d), 8.30(1H, s), 8.03-7.76(10H, m), 7.69(1H, d), 7.57-7.49(10H, m), 7.39(1H, t), 7.31(1H, t) |
| 1-95 | δ = 8.45(1H, d),8.36(4H, d), 8.22(1H, s), 8.08(1h, d), 8.03-7.98(4H, m), 7.93(1H, d), 7.82(2H, d), 7.76(1H, s), 7.69(1H, d), 7.57-7.50(12H, m), 7.39(1H, t), 7.31(1H, t) |
| 1-96 | δ = 8.45(1H, d), 8.36(4H, d), 8.12(2H, d), 8.03(2H, d), 7.98(1H, d), 7.93(1h, d), 7.82(3H, d), 7.76(2H, s), 7.69(1H, d), 7.57-7.49(10H, m), 7.39(1H, t), 7.31(1H, t) |
| 1-97 | δ = 8.36(4H, d), 8.08(2H, d), 8.02-7.98(4h, m), 7.86(1H, s), 7.82(2H, d), 7.69(1H, d), 7.57-7.49(11H, m), 7.39(2H, t), 7.31(2H, t) |
| 1-102 | δ = 8.36(4H, d), 8.08-6.98(8H, m), 7.82(1H, d), 7.69(1H, d), 7.57-7.50(11H, m), 7.39(2H, t), 7.31(2H, t) |
| 1-106 | δ = 8.36(4H, d), 8.03(1h, d), 7.98(2H, d), 7.86-7.82(5H, m), 7.76(1H, s), 7.69(2H, d), 7.57-7.50(10H, m), 7.39(2H, t), 7.31(2H, t) |
| 1-114 | δ = 8.45(1H, d), 8.36(4H, d), 8.11-7.93(6h, m), 7.82(2H, d), 7.69(2H, d), 7.57-7.49(12H, m), 7.39(1H, t), 7.31(1H, t) |
| 1-118 | δ = 8.45(1H, d), 8.36(4H, d), 8.30(1H, s), 8.03-7.76(10H, ), 7.69(1H, d), 7.57-7.49(10H, m), 7.39(1H, t), 7.31(1H, t) |
| 1-119 | δ = 8.45(1H, d), 8.36(4H, d), 8.22(1H, s), 8.03(1H, d), 7.98(2H, d), 7.93(1H, d), 7.82(3H, d), 7.76(1H, ), 7.69(2H, d), 7.57-7.49(11H, m), 7.39(1H, t), 7.31(1H, t) |
| 1-122 | δ = 8.5(2H, d), 8.36(4H, d), 8.31(1H, d), 8.07-7.98(5H, m), 7.92(1H, d), 7.82(1h, d), 7.76(1H, s), 7.70(2H, d), 7.54-7.49(8H, m), 7.39(2H, t), 7.31(2H, t) |
| 1-132 | δ = 8.55(1H, d), 8.36(4H, d), 8.24-8.17(3H, m), 8.07(2H, d), 8.02 (2H, d), 7.98(2H, d), 7.92(1H, d), 7.70(1H, t), 7.54-7.50(9H, m), 7.39(2H, t), 7.31(2H, t) |
| 1-135 | δ = 8.55(1H, d), 8.36(4H, d), 8.12(2H, d), 7.98(2H, d), 7.93-7.82(5H, m) 7.70(2H, d). 7.57-7.49(9H, m), 7.39(2H, t), 7.31(2H, t) |
| 1-137 | δ = 8.55(1H, d), 8.36(4H, d), 8.12(2H, d), 7.98)2H, d), 7.93-7.82(5H, m), 7.70(2H, d), 7.57-7.50(9H, m), 7.39(2H, t), 7.31(2H, t) |
| 1-139 | δ = 8.55(1H, d), 8.36(4H, d), 8.03-7.81(9H, m), 7.79(1h, s), 7.70(2H, d), 7.55-7.49 (8H, m), 7.39(2H, t), 7.31(2H, t) |
| 1-148 | δ = 8.55 (2H, d), 8.45(1h, d), 8.36(4H, d), 8.30(2H, s) 8.01(2H, d), 7.91 (2H, d), 7.82 (1H, d), 7.70(3H, d), 7.57-7.49(9H, m), 7.39(1H, t), 7.31(1H, t) |
| 1-153 | δ = 8.55(1H, d), 8.45(1H, d), 8.36(4H, d), 8.24-8.17(4H, m), 7.99-7.79(7H, m), 7.70(1H, d), 7.56-7.49(9H, m), 7.39(1H, t), 7.31(1H, t) |
| 1-155 | δ = 8.55(1H, d), 8.45(1H, d), 8.36(4H, d), 8.24-8.17(4H, m), 8.03-7.92(5H, m), 7.82(1H, d), 7.76(1H, s), 7.70(1H, d), 7.56-7.49(9H, m), 7.39(1H, t), 7.31(1H, t) |
| 1-163 | δ = 8.55(1H, d), 8.45(1H, d), 8.36(4H, d), 8.30(1H, s), 8.03-7.79(9H, m), 7.70(2H, d), 7.56-7.49(9H, m), 7.39(1H, t), 7.31(1H, t) |
| 1-164 | δ = 8.55(1H, d), 8.45(1H, d), 8.36(4H, d), 8.08-7.92(9H, m0, 7.70(1H, s), 7.69(1H, d), 7.56-7.49(10H, m), 7.39(1H, t), 7.31(1H, t) |
| 1-169 | δ = 8.55(1H, d), 8.36(4H, d) , 8.23(1H, d), 8.24(2H, d), 8.08-7.94(5h, m), 7.86(1H, s), 7.81(1H, s), 7.70(1h, d), 7.54-7.50(9H, m), 7.39(2H, t), 7.31(2H, t) |
| 1-171 | δ = 8.55(1H, d), 8.36(4H, d), 8.32(1H, d), 8.24(1H, d), 8.20(1H, s), 8.08-.7-93(6h, m), 7.87(1H, s), 7.70(1H, d), 7.54-7.49(9H, m), 7.39(2H, t), 7.31(2H, t) |
| 1-178 | δ = 8.36(4H, d), 8.24-8.17(4H, m), 7.98-7.79(8H, m), 7.54-7.50(8H, m), 7.39(2H, t), 7.31(2H, t) |
| 1-180 | δ = 8.36(4H, d), 8.24-8.17(4H, m), 8.07-7.94(6H, m), 7.82(1H, d), 7.76(1H, s), 7.54-7.50(8H, m), 7.39(2H, t), 7.31(2H, t) |
| 1-186 | δ = 8.36(4H, d), 8.24(1H, d), 8.20(1h, s), 8.12-7.94 (8H, m0, 7.82(1H, d), 7.69(1H, d), 7.57-7.50(9H, m), 7.39(2H, t), 7.31(2H, t) |
| 1-191 | δ = 8.36(4H, d), 8.24(1H, d), 8.20(1H, s), 8.03-7.93(8H, m), 7.569(2H, d), 7.57-7.49(9H, m), 7.39(2H, t), 7.31(2H, t) |
| 1-193 | δ = 8.55(1H, d), 8.45(1H, d), 8.36(4H, d), 8.30(2H, s), 8.24(1H, d), 8.20(1H, s), 8.01-7.94(4H, m), 7.82(1H, d), 7.70(2H, d), 7.57-7.49(10H, m), 7.39(1H, t), 7.31(1H, t) |
| 1-196 | δ = 8.55(1H, d), 8.45(1H, d), 8.36(4H, d), 8.30(2H, s), 8.24(1H, s), 8.20(1H, s), 8.01-7.93 (5H, m), 7.82(1H, d), 7.76(1H, s), 7.70(1H, d), 7.56-7.49 (9H, m), 7.39(1H, t), 7.31(1H, t) |
| 1-202 | δ = 8.45(1H, d), 8.36(4H, d), 8.30-8.20(6H, m), 8.01-7.94(5, m), 7.83(1H, s), 7.79(1h, d), 7.55-7.49(9H, m), 7.39(1H, t), 7.31(1H, t) |
| 1-207 | δ = 8.45(1H, d), 8.36(4H, d), 8.24(1H, s), 8.20(2H, s), 8.12(2H, s), 7.99-7.94(5H, m), 7.82(1H, d), 7.69(1H, d), 7.57-7.50(10H, m), 7.39(1H, t), 7.31(1H, t) |
| 1-211 | δ = 8.45(1H, d), 8.36(4H, d), 8.30(1H, d), 8.24(1H, d), 8.20(1H, s), 8.08-7.94(8H, m), 7.68(1H, d), 7.56-7.50(10H, m), 7.39(1H, t), 7.31(1H, t) |
| 1-213 | δ = 8.45(1H, d), 8.36(4H, d), 8.24(1H, d), 8.20(1H, s), 8.03-7.93(7H, m), 7.82(1H, d), 7.76(1H, s), 7.68(1H, d), 7.56-7.50(9H, m), 7.39(1H, t), 7.31(1H, t) |
| 1-217 | δ = 8.55(1H, d), 8.36(4H, d), 8.31(1H, d), 8.08-7.94(6h, m), 7.86(1H, s), 7.82(1H, s), 7.70(2H, d), 7.54-7.50(8H, m), 7.39(2H, t), 7.31(2H, t) |
| 1-221 | δ = 8.55(1H, d), 8.36(4H, d), 8.32(1H, d), 8.08-7.93(7h, m0, 7.87(1H, s), 7.68(2h, d), 7.54-7.50(9H, m), 7.39(2H, t), 7.31(2H, t) |
| 1-223 | δ = 8.36(4H, d), 8.24-8.17(3H, m), 8.08-7.94(6H, m), 7.86(1H, s), 7.81(1H, s), 7.68(1H, d), 7.54-7.50(9h, m), 7.39(2H, t), 7.31(2H, t) |
| 1-231 | δ = 8.55(1H, d), 8.45(1H, d), 8.36(4H, d), 8.30(1h, s0, 8.22(1H, s), 8.03-7.93(7h, m), 7.69(2H, d), 7.56-7.49(9H, m), 7.39(1H, t), 7.31(1H, t) |
| 1-233 | δ = 8.55(1H, d), 8.45(1H, d), 8.36(4H, d), 8.32(1H, d), 8.22(1H, s), 8.08-7.94(7H, m), 7.70(2H,d), 7.56-7.49(10H, m), 7.39(1H, t), 7.31(1H, t) |
| 1-235 | δ = 8.45(1H, d), 8.36(4H, d), 8.30-8.20(4H, m), 8.03-7.93(5H, m), 7.82(1H, d), 7.69(2H, d), 7.56-7.49(10H, m), 7.39(1H, t), 7.31(1H, t) |
| 1-238 | δ = 8.45(1H, d), 8.36(4H, d), 8.30(1H, d), 8.24(1H, d), 8.20(1H, s), 8.17(1H, d), 8.03-7.94(5H, m), 7.82(1H, d), 7.68(2H, d), 7.57-7.49(10H, m), 7.39(1H, t), 7.31(1H, t) |
| 1-239 | δ = 8.45(1H, d), 8.36(4H, d), 8.24-8.17(4h, m), 8.03-7.94(5H, m), 7.82(1H, d(, 7.68(2H, d), 7.57-7.49(10H, m), 7.39(1H, t), 7.31(1H, t) |
| 1-241 | δ = 8.55(1H, d), 8.45(1H, d), 8.36(4H, d), 8.32(1H, d(, 8.08-7.88(7H, m), 7.81(1H, s), 7.70(2H, d), 7.57-7.49(11H, m), 7.39(1H, t), 7.31(1H, t) |
| 1-242 | δ = 8.45(1H, d), 8.36(4H, d), 8.12-7.88(11H, m), 7.57-7.49(11H, m), 7.39(1H, t), 7.31(1H, t) |
| 1-249 | δ = 8.45(1H, d), 8.36(4H, d), 8.03-7.83(10H, m), 7.76(1H, s), 7.70(1H, d), 7.54-7.49(9H ,m), 7.39(1H, t), 7.31(1H, t) |
| 1-251 | δ = 8.45(1H, d), 8.36(4H, d), 8.24(1H, d), 8.20(1H, s), 8.17(1H, d), 8.08-7.87(8H, m), 7.76(2H, s), 7.56-7.49(9H, m), 7.39(1H, t), 7.31(1H, t) |
| 1-255 | δ = 8.45(1H, d), 8.36(4H, d), 8.08-7.79(11H, m), 7.68(1H, d), 7.56-7.49(10H, m), 7.39(1H, t), 7.31(1H, t) |
| 1-263 | δ = 8.45(1H, d), 8.36(4H, d), 8.08-7.88(8H, m), 7.82(1H,d), 7.69(2H, d), 7.57-7.49(11H, m), 7.39(1H, t), 7.31(1H, t) |
| 1-268 | δ = 8.45(2H, d), 8.36(4H, d), 8.30(1H, s), 8.24(1H, d), 8.20(1H, s), 8.17(1H, d), 8.08-8.01(4H, m), 7.93(2H, d), 7.88(1H, d), 7.56-7.49(11H, m) |
| 1-271 | δ = 8.45 (2H, d), 8.36(4H, d), 8.30 (1H, s), 8.08-8.01 (4H, m), 7.94-7.88 (4H, m), 7.82(1H, d), 7.76(1H, s) 7.68(1H ,d), 7.56-7.49(11H, m) |
| 1-279 | δ = 8.45(2H, d), 8.36(4H, d), 8.22(1H, s), 8.11-8.08(3H, m), 7.99-7.79(8H, m), 7.56-7.49(11H, m) |
| 1-286 | δ = 8.55(1H, d), 8.45(2H, d), 8.36(4H, d), 8.32(2H, d), 8.08(1h, d), 8.01(1H, s), 7.93-7.88(4H, m), 7.70(2H, d), 7.57-7.49(12H, m) |
| 1-293 | δ = 8.55(1H, d), 8.45(1H, d), 8.36-8.32(5H, m), 8.08-7.87 (7H, m), 7.82 (1H, d), 7.76 (1H, s), 7.70 (1H, d), 7.56-7.49(9H, m), 7.39(1H, t), 7.31(1H, t) |
| 1-299 | δ = 8.45(1H, d), 8.36(4H, d), 8.12(2H, d), 8.03-7.87 (7H, m), 7.82(2H, d), 7.76(2H, s), 7.57-7.49(9H, m), 7.39(1H, t), 7.31(1H, t) |
| 1-307 | δ = 8.45(1H, d), 8.36(4H, d), 8.12(2H, d), 8.03-7.93 (4H, m), 7.86-7.82(4H, m), 7.76(1H, s), 7.69(1H, d), 7.57-7.49(10H, m), 7.39(1H, t), 7.31(1H, t) |
| 1-312 | δ = 8.45(1H, d), 8.36(4H, d), 8.07-7.94 (7H, m), 7.82 (2H, d), 7.76(1H, s), 7.68(2H, d), 7.57-7.49(10H, m), 7.39(1H, t), 7.31(1H, t) |
| 1-313 | δ = 8.45(2H, d), 8.36(4H, d), 8.30(1H, s), 8.08-8.01(5H, m), 7.94-7.93(3H, m), 7.82(1H, d), 7.76(1H, s), 7.68(1H, d), 7.57-7.49(11H, m) |
| 1-322 | δ = 8.55(1H, d), 8.45(2H, d), 8.36(4H, d), 8.30(1H, s), 8.03(3H, d), 7.93(3H, d), 7.82(2H, d), 7.76(1H, s), 7.70(1H, d), 7.56-7.49(10H, m) |
| 1-329 | δ = 8.55(1H, d), 8.45(2H, d), 8.36(4H, d), 8.32 (1H, s), 8.22(1H, s), 7.99-7.82(8H, m), 7.76(1H, s), 7.70(1H, d), 7.56-7.49(10H, m) |
| 1-331 | δ = 8.45(2H, d), 8.36(4H, d), 8.30(1H, s), 8.02(2H, d), 7.93(3H, d), 7.82(2H, d), 7.76(1H, s), 7.69(2H, d), 7.57-7.49(11H, m) |
| 1-337 | δ = 8.45(1H, d), 8.36(4H, d), 8.08-7.93(6H, m), 7.86(1H, s), 7.82 (2H, d), 7.69(2H, d), 7.56-7.50(11H, m), 7.39(1H, t), 7.31(1H, t) |
| 1-343 | δ = 8.45(1H, d), 8.36(4H, d), 8.03-7.94(5h, m), 7.86(1H, s), 7.82(3H, d), 7.76 (1H, s), 7.69(2H, d), 7.57-7.49(10H, m), 7.39(1H, t), 7.31(1H, t) |
| 1-347 | δ = 8.45(1H, d), 8.36(4H, d), 8.03-7.87 (7h, m), 7.82 (2H, d), 7.76(1H, s), 7.69(2H, d), 7.57-7.49(10H, m), 7.39(1H, t), 7.31(1H, t) |
| 1-358 | δ = 8.45(1H, d), 8.36(4H, d) , 8.30(1H, s), 8.12(2H, d), 8.03-7.93(5h, m), 7.82(2h, d), 7.76(1H, s), 7.69(1H, d), 7.75-7.49(11H, m) |
| 1-361 | δ = 8.55 (2H, d), 8.45(1H, d), 8.36(4H, d), 8.32 (1H, s), 8.03-7.92(7H, m), 7.70(2H, d), 7.68(1H, s), 7.56-7.49(9H, m), 7.39(1H, t), 7.31(1H, t) |
| 1-366 | δ = 8.55(2H, d), 8.45(1H, d), 8.36(4H, d), 8.30(1H, s), 8.24-8.20(3H, m), 8.03-7.93(5H, m), 7.70(2H, d), 7.56-7.49(9H, m), 7.39(1H, t), 7.31(1H, t) |
| 1-372 | δ = 8.55(2H, d), 8.45(1H, d), 8.36(4H, d), 8.32(1H, s), 8.24-8.20(3H, m), 8.01-7.93(5H, m), 7.70(2h, d), 7.56-7.49(9H, m), 7.39(1H, t), 7.31(1H, t) |
| 1-378 | δ = 8.55(2H, d), 8.45(1H, d), 8.36(4H, d), 8.30(1H, s), 8.12-7.93(8H, m), 7.70(2H, d), 7.56-7.49(9H, m), 7.39(1H, t), 7.31(1H, t) |
| 1-388 | δ = 8.55(2H, d), 8.45(1H, d), 8.36(4H, d), 8.30(2H, d), 8.01(2H, d), 7.96-7.92(4H, m), 7.71-7.68(3h, m), 7.56-7.49(10H, m) |
| 1-394 | δ = 8.55(2H, d), 8.45(2H, d), 8.36(4H, d), 8.36-8.24(9H, m), 8.01 (1H, s), 7.92 (2H, d), 7.70(2h, d), 7.56-7.49(10H, m) |
| 1-403 | δ = 8.55 (2H, d), 8.45(2H, d), 8.36(4H, d), 8.30(1H, s), 8.12(2H, d), 8.03-7.93(7H, m), 7.70(2h, d), 7.56-7.49(10H, m) |
| 1-407 | δ = 8.55 (2H, d), 8.45(2H, d), 8.36(4H, d), 8.30(1H, s), 8.22(1H, s), 8.03-7.93(6H, m), 7.70-7.68(3H, m), 7.56-7.49(10H, m) |
| 1-412 | δ = 8.55(1H, d), 8.45(1H, d) 8.36(4H, d), 8.32(1H, d), 8.24-8.17(6H, m), 7.98-7.95(3h, m), 7.86(1h, s), 7.81(1H, s), 7.70(1h, d), 7.56-7.49(9h, m), 7.39(1H, t), 7.31(1H, t) |
| 1-423 | δ = 8.55(1h, d), 8.45(1H, d), 8.36-8.24(7H, m), 8.12(2H, d), 7.99-7.93(5H, m), 7.87(1H, s), 7.70(1H, d), 7.56-7.49(9H, m), 7.39(1H, t), 7.31(1H, t) |
| 1-439 | δ = 8.45 (2H, d), 8.36(4H, d), 8.30(1H, s), 8.24-8.17(5h, m), 8.03(1H, d), 8.01(1h, s), 7.93(3h, d), 7.68(1H, d), 7.56-7.49(10H, m) |
| 1-448 | δ = 8.45(2H, d), 8.36(4H, d), 8.30(1H, s), 8.24(1H, d), 8.20(1H, s), 8.12(2H, d), 8.03-7.93(7H, m), 7.68(1h, d), 7.56-7.49(10H, m) |
| 1-451 | δ = 8.55(1H, d), 8.45(2H, d), 8.36-8.20(10H, m), 8.03(1H, d), 8.01(1H, s), 7.94(3H, d), 7.69(1H, d), 7.56-7.49(10H, m) |
| 1-457 | δ = 8.55(2H, d), 8.45(1H, d), 8.36(4H, d), 8.30(2H, d), 8.01-7.93(4h, m), 7.86(1H, s), 7.81(1H, s), 7.71-7.68(3H, m), 7.56-7.49(9H, m), 7.39(1H, t), 7.31(1H, t) |
| 1-459 | δ = 8.55(1H, d), 8.45(1H, d), 8.36(4H, d), 8.30(1H, s), 8.24(1H, d), 8.20(1H, s), 8.17(1H, s), 8.03-7.93(5H, m), 7.87(1H, s), 7.68(2H, d), 7.56-7.49(9H, m), 7.39(1H, t), 7.31(1H, t) |
| 1-468 | δ = 8.45(1H, d), 8.36(4H, d), 8.24-7.93(12H, m), 7.68(1H, d), 7.56-7.49(9H, m), 7.39(1H, t), 7.31(1H, t) |
| 1-472 | δ = 8.55(1H, d), 8.45(2H, d), 8.36-8.17(9H, m), 8.03(1H, d), 8.01 (1H, s), 7.93(2H, d), 7.69(2h, d), 7.57-7.49(10H, m) |
| 1-481 | δ = 8.36(2H, d), 8.08-7.98(7H, m) 7.88(2H, d), 7.81(1H, s), 7.54(-7.49(8h, m), 7.39(2H, t), 7.31(2H, t) |
| 1-483 | δ = 8.36(4H, m), 8.08-7.88(8H, m), 7.54-7.50(8H, m), 7.39(1H, t), 7.31(1H, t), |
| 1-484 | δ = 8,08-7.88(7H, m), 7.76(1H, s), 7.57(2H, d), 7.39(1H, t), 7.31(1H, t) |
| 1-487 | δ = 8.45(1H, d), 8.30(1H, s), 8.08-7.88(8H, m), 7.76(1H, s), 7.69(1H, d), 7.57-7.49(5H, m), 7.39(1H, t), 7.31(1H, t) |
| 1-489 | δ = 8.08-.7.98(7H, m), 7.88-7.76(5H, m), 7.54(2H, d), 7.50(1H, d), 7.39(2H, t), 7.31(2H, t) |
| 1-494 | δ = 8.08(2H, d), 8.02-7.98(4H, m), 7.86(1H, s), 7.82 (2H, d), 7.69(1H, d), 7.57-7.50(5H, m), 7.39(2H, t), 7.31(2H, t) |
| 1-496 | δ = 8.55(1H, d), 8.03-7.86(6H, m), 7.69(2H, d), 7.54(1H, d), 7.39(1H, t), 7.31(1H, t) |
| 1-497 | δ =8.55(2H, d), 8.45(1H, d), 8.30(2H, d), 7.98-7.93(4H, m), 7.82(1H, d), 7.70(3H, d), 7.54-7.70(4H, m), 7.39(1H, t), 7.31(1H, t) |
| 1-500 | δ = 8.24-8.17(5H, m), 7.98(1H, d), 7.94(1H, d), 7.86(1H, s), 7.81(1H, s) 7.54(1H, d), 7.39(1H, t), 7.31(1H, t) |
| 1-503 | δ = 8.45(1H, t), 8.30(1H, s), 8.24-8.17(5H, m), 8.01-7.88(5H, m), 7.83(1H, s), 7.79(1h, d), 7.54-7.49(3H, m), 7.39(1H, t), 7.31(1H, t) |
| 1-505 | δ = 8.55(1H, d), 8.45(1H, d), 8.32(1H, d), 8.22(1H, s), 8.03-7.93(7H, m), 7.69(2H, d), 7.56-7.49(4H, m), 7.39(1H, t), 7.31(1H, t) |
| 1-510 | δ = 8.36(2H, d), 8.08-7.98(4H, m), 7.88(2H, d), 7.81(1H, s), 7.54-7.50(6H, m), 7.39(1H, t), 7.31(1H, t) |
| 1-518 | δ = 8.45(2H, d), 8.30(1H, s), 8.12(2H, d), 8.03-7.93(5H, m), 7.82(2H, d), 7.76(1H, s), 7.69(1H, d), 7.57-7.49(5H, m) |
| 1-529 | δ = deuterium content of 100% with no ¹H NMR peak |
| 1-531 | δ = deuterium content of 100% with no ¹H NMR peak |
| 1-541 | δ = deuterium content of 100% with no ¹H NMR peak |
| 1-547 | δ = deuterium content of 100% with no ¹H NMR peak |
| 1-549 | δ = deuterium content of 100% with no ¹H NMR peak |
| 1-554 | δ = deuterium content of 100% with no ¹H NMR peak |
| 1-559 | δ = deuterium content of 100% with no ¹H NMR peak |
| 1-567 | δ = deuterium content of 100% with no ¹H NMR peak |
| 1-569 | δ = deuterium content of 100% with no ¹H NMR peak |
| 1-575 | δ = deuterium content of 100% with no ¹H NMR peak |
| 1-582 | δ = deuterium content of 100% with no ¹H NMR peak |
| 1-587 | δ = deuterium content of 100% with no ¹H NMR peak |
| 1-590 | δ = deuterium content of 100% with no ¹H NMR peak |
| 1-595 | δ = deuterium content of 100% with no ¹H NMR peak |
| 1-598 | δ = deuterium content of 100% with no ¹H NMR peak |
| 1-607 | δ = deuterium content of 100% with no ¹H NMR peak |

**[Table 17]**

| Compound | ¹H NMR(CDCl₃, 200Mz) |
|---|---|
| 2-3 | δ= 8.55 (1H, d), 8.30 (1H, d), 8.21-8.13 (3H, m), 7.99-7.89 (4H, m), 7.77-7.35 (17H, m), 7.20-7.16 (2H, m) |
| 2-4 | δ= 8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 2-5 | δ= 8.55 (1H, d), 8.30(1H, d), 8.21-8.13(3H, m), 7.99-7.94(2H, m), 7.89(2H, s), 7.77-7.35 (17H, m), 7.25-7.16 (6H, m) |
| 2-7 | δ= 8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 2-31 | δ= 8.55 (1H, d), 8.30 (1H, d), 8.21-8.13 (4H, m), 7.99-7.89 (4H, m), 7.77-7.35 (20H, m), 7.20-7.16 (2H, m) |
| 2-32 | δ= 8.55 (1H, d), 8.30 (1H, d), 8.21-8.13 (3H, m), 7.99-7.89 (8H, m), 7.77-7.35 (17H, m), 7.20-7.16 (2H, m) |
| 2-37 | δ= 8.55 (1H, d), 8.30 (1H, d), 8.21-8.13 (3H, m), 8.03-7.35 (23H, m), 7.20-7.16 (2H, m) |
| 2-42 | δ= 8.55 (1H, d), 8.30 (1H, d), 8.19(1H, d) 8.13 (1H, d), 7.99-7.89 (12H, m), 7.77-7.75 (5H, m), 7.50-7.35 (8H, m), 7.20-7.16 (2H, m) |
| 2-82 | δ= 8.55 (1H, d), 8.30 (1H, d), 8.19-8.13 (2H, m), 8.03-7.77 (13H, m), 7.59-7.50 (6H, m) 7.36-7.35 (3H, m), 7.20-7.16 (2H, m) |
| 2-98 | δ = 8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, t) |
| 2-99 | δ = 8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, t) |
| 2-101 | δ = 8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, t) |
| 2-104 | δ = 8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, t) |
| 2-106 | δ = 7.91-7.92 (8H, m), 7.75 (4H, d), 7.41-7.49 (6H, m) |
| 2-107 | δ = 8.21 (1H, s), 7.41-7.68 (13H, m) |
| 2-108 | δ = 9.05 (1H, s), 8.33-8.25 (4H, m), 7.94 (1H, d), 7.70-7.50 (10H, m) |
| 2-110 | δ = deuterium content of 100% with no ¹H NMR peak |
| 2-111 | δ = deuterium content of 100% with no ¹H NMR peak |
| 2-113 | δ = deuterium content of 100% with no ¹H NMR peak |
| 2-114 | δ = deuterium content of 100% with no ¹H NMR peak |
| 2-115 | δ = deuterium content of 100% with no ¹H NMR peak |
| 2-116 | δ = deuterium content of 100% with no ¹H NMR peak |

**[Table 18]**

| Compound | ¹H NMR(CDCl₃, 200Mz) |
|---|---|
| 3-2 | δ = 8.55(1H, d), 8.45(1H, d), 8.30(1H, d), 8.19(1H, d), 8.13(1H, d), 8.00∼7.89(6H, m), 7.77(2H, m), 7.62∼7.35(15H, m), 7.20∼7.16(2H, m) |
| 3-3 | δ = 8.55(1H, d), 8.45(1H, d), 8.30(1H, d), 8.19(1H, d), 8.13(1H, d), 8.00∼7.89(6H, m), 7.77-7.75(4H, m), 7.62∼7.35(13H, m), 7.25∼7.16(6H, m) |
| 3-106 | δ = 8.55 (2H, d), 7.99∼7.89 (6H, m), 7.77 (2H, d), 7.35 (2H, t), 7.16 (2H, t) |
| 3-107 | δ = 8.55 (2H, d), 7.99∼7.89 (6H, m), 7.77 (2H, d), 7.35 (2H, t), 7.16 (2H, t) |
| 3-108 | δ = 8.55 (2H, d), 7.99∼7.89 (6H, m), 7.77 (2H, d), 7.35 (2H, t), 7.16 (2H, t) |
| 3-109 | δ = 8.55 (2H, d), 7.99∼7.89 (6H, m), 7.77 (2H, d), 7.35 (2H, t), 7.16 (2H, t) |
| 3-111 | δ = 8.45 (1H, d), 8.00 (1H, s), 7.93 (1H, d), 7.77 (1H, s), 7.62∼7.41 (12H, m) |
| 3-112 | δ = 8.45 (1H, d), 8.00 (1H, s), 7.93∼7.91 (5H, m), 7.77∼7.75 (3H, m), 7.56∼7.41 (10H, m) |
| 3-114 | δ = 8.45 (2H, d), 8.12 (2H, m), 8.00∼7.93 (4H, m), 7.77 (1H, s), 7.62∼7.49 (9H, m) |
| 3-115 | δ = 8.45 (1H, d), 8.00∼7.77 (7H, m), 7.62∼7.31 (10H, m) |
| 3-117 | δ = deuterium content of 100% with no ¹H NMR peak |
| 3-118 | δ = deuterium content of 100% with no ¹H NMR peak |
| 3-119 | δ = deuterium content of 100% with no ¹H NMR peak |
| 3-120 | δ = deuterium content of 100% with no ¹H NMR peak |
| 3-123 | δ = deuterium content of 100% with no ¹H NMR peak |
| 3-125 | δ = deuterium content of 100% with no ¹H NMR peak |

### <Experimental Example 1> - Manufacture of organic light emitting device

A glass substrate, in which indium tin oxide (ITO) was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then subjected to ultraviolet ozone (UVO) treatment for 5 minutes by using UV in a UV washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

The hole injection layer 4,4',4"-tris[2-naphthyl(phenyl)amino] triphenylamine (2-TNATA) and the hole transport layer N,N'-diphenyl-(1,1'-biphenyl)-4,4'-diamine (NPB), which are common layers, were formed on the ITO transparent electrode (positive electrode).

A light emitting layer was thermally vacuum deposited thereon as follows. The light emitting layer was deposited to have a thickness of 400 Å by using one type of the compounds described in Chemical Formula 1 as in the following Table 19 as a host, and was deposited by doping the host with Ir(ppy)₃ as a green phosphorescent dopant in an amount of 7%. Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then aluminum (Al) negative electrode was deposited to have a thickness of 1,200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁶ to 10⁻⁸ torr for each material, and used for the manufacture of OLED.

For the organic electroluminescence device manufactured as described above, electroluminescence (EL) characteristics were measured by M7000 manufactured by McScience Inc., and based on the measurement result thereof, T90 was measured by a service life measurement equipment (M6000) manufactured by McScience Inc., when the reference luminance was 6,000 cd/m².

**[Table 19]**

| | Light emitting layer Compound | Driving voltage (V) | Efficiency (cd/A) | Light emission color | Service life (T90) |
|---|---|---|---|---|---|
| Example 1 | 1-1 | 4.22 | 74.1 | Green | 198 |
| Example 2 | 1-2 | 4.24 | 75.0 | Green | 202 |
| Example 3 | 1-5 | 4.20 | 74.4 | Green | 209 |
| Example 4 | 1-11 | 4.25 | 75.1 | Green | 204 |
| Example 5 | 1-14 | 4.20 | 75.2 | Green | 203 |
| Example 6 | 1-19 | 4.19 | 76.3 | Green | 209 |
| Example 7 | 1-23 | 4.18 | 76.0 | Green | 201 |
| Example 8 | 1-25 | 4.26 | 75.8 | Green | 206 |
| Example 9 | 1-31 | 4.30 | 74.9 | Green | 212 |
| Example 10 | 1-34 | 4.25 | 76.2 | Green | 213 |
| Example 11 | 1-43 | 4.26 | 76.1 | Green | 210 |
| Example 12 | 1-54 | 4.15 | 77.2 | Green | 215 |
| Example 13 | 1-63 | 4.17 | 77.0 | Green | 209 |
| Example 14 | 1-68 | 4.20 | 76.4 | Green | 201 |
| Example 15 | 1-72 | 4.22 | 75.6 | Green | 210 |
| Example 16 | 1-73 | 4.27 | 77.2 | Green | 218 |
| Example 17 | 1-78 | 4.25 | 76.4 | Green | 213 |
| Example 18 | 1-87 | 4.19 | 77.5 | Green | 217 |
| Example 19 | 1-88 | 4.19 | 76.3 | Green | 221 |
| Example 20 | 1-95 | 4.20 | 77.1 | Green | 209 |
| Example 21 | 1-96 | 4.18 | 75.9 | Green | 205 |
| Example 22 | 1-97 | 4.11 | 76.5 | Green | 195 |
| Example 23 | 1-102 | 4.09 | 78.9 | Green | 202 |
| Example 24 | 1-106 | 4.08 | 78.4 | Green | 190 |
| Example 25 | 1-114 | 4.23 | 78.0 | Green | 195 |
| Example 26 | 1-118 | 4.19 | 77.1 | Green | 219 |
| Example 27 | 1-119 | 4.15 | 79.0 | Green | 207 |
| Example 28 | 1-122 | 4.25 | 76.9 | Green | 220 |
| Example 29 | 1-132 | 4.30 | 78.5 | Green | 220 |
| Example 30 | 1-135 | 4.28 | 77.4 | Green | 211 |
| Example 31 | 1-137 | 4.25 | 79.1 | Green | 229 |
| Example 32 | 1-139 | 4.19 | 78.6 | Green | 201 |
| Example 33 | 1-148 | 4.29 | 75.4 | Green | 191 |
| Example 34 | 1-153 | 4.15 | 78.9 | Green | 225 |
| Example 35 | 1-155 | 4.28 | 80.1 | Green | 224 |
| Example 36 | 1-163 | 4.32 | 82.0 | Green | 229 |
| Example 37 | 1-164 | 4.33 | 81.5 | Green | 233 |
| Example 38 | 1-169 | 4.27 | 78.1 | Green | 211 |
| Example 39 | 1-171 | 4.25 | 77.4 | Green | 208 |
| Example 40 | 1-178 | 4.19 | 78.5 | Green | 221 |
| Example 41 | 1-180 | 4.16 | 79.5 | Green | 215 |
| Example 42 | 1-186 | 4.17 | 80.0 | Green | 219 |
| Example 43 | 1-191 | 4.20 | 78.7 | Green | 231 |
| Example 44 | 1-193 | 4.24 | 76.9 | Green | 222 |
| Example 45 | 1-196 | 4.35 | 75.5 | Green | 194 |
| Example 46 | 1-202 | 4.19 | 80.4 | Green | 237 |
| Example 47 | 1-207 | 4.20 | 83.0 | Green | 230 |
| Example 48 | 1-211 | 4.30 | 77.5 | Green | 209 |
| Example 49 | 1-213 | 4.27 | 78.5 | Green | 209 |
| Example 50 | 1-217 | 4.19 | 80.5 | Green | 208 |
| Example 51 | 1-221 | 4.14 | 81.6 | Green | 204 |
| Example 52 | 1-223 | 4.10 | 82.5 | Green | 206 |
| Example 53 | 1-231 | 4.11 | 84.0 | Green | 215 |
| Example 54 | 1-233 | 4.15 | 83.1 | Green | 216 |
| Example 55 | 1-235 | 4.20 | 80.9 | Green | 220 |
| Example 56 | 1-238 | 4.19 | 83.2 | Green | 225 |
| Example 57 | 1-239 | 4.18 | 85.3 | Green | 230 |
| Example 58 | 1-241 | 4.19 | 79.2 | Green | 206 |
| Example 59 | 1-242 | 4.20 | 76.6 | Green | 198 |
| Example 60 | 1-249 | 4.20 | 77.9 | Green | 201 |
| Example 61 | 1-251 | 4.18 | 78.4 | Green | 198 |
| Example 62 | 1-255 | 4.24 | 79.1 | Green | 205 |
| Example 63 | 1-263 | 4.15 | 80.6 | Green | 211 |
| Example 64 | 1-268 | 4.23 | 76.9 | Green | 198 |
| Example 65 | 1-271 | 4.25 | 80.4 | Green | 207 |
| Example 66 | 1-279 | 4.23 | 82.1 | Green | 215 |
| Example 67 | 1-286 | 4.22 | 81.7 | Green | 209 |
| Example 68 | 1-293 | 4.18 | 83.2 | Green | 221 |
| Example 69 | 1-299 | 4.14 | 84.0 | Green | 229 |
| Example 70 | 1-307 | 4.19 | 80.4 | Green | 213 |
| Example 71 | 1-312 | 4.24 | 78.6 | Green | 210 |
| Example 72 | 1-313 | 4.20 | 80.5 | Green | 225 |
| Example 73 | 1-322 | 4.10 | 83.3 | Green | 217 |
| Example 74 | 1-329 | 4.12 | 84.0 | Green | 224 |
| Example 75 | 1-331 | 4.20 | 84.1 | Green | 211 |
| Example 76 | 1-337 | 4.09 | 83.6 | Green | 225 |
| Example 77 | 1-343 | 4.15 | 83.1 | Green | 241 |
| Example 78 | 1-347 | 4.08 | 82.6 | Green | 216 |
| Example 79 | 1-358 | 4.21 | 84.8 | Green | 221 |
| Example 80 | 1-361 | 4.30 | 76.8 | Green | 191 |
| Example 81 | 1-366 | 4.28 | 75.4 | Green | 188 |
| Example 82 | 1-372 | 4.06 | 85.8 | Green | 229 |
| Example 83 | 1-378 | 4.04 | 83.9 | Green | 228 |
| Example 84 | 1-388 | 4.33 | 73.8 | Green | 182 |
| Example 85 | 1-394 | 4.16 | 79.5 | Green | 214 |
| Example 86 | 1-403 | 4.14 | 81.5 | Green | 207 |
| Example 87 | 1-407 | 4.10 | 82.2 | Green | 210 |
| Example 88 | 1-412 | 4.05 | 82.5 | Green | 233 |
| Example 89 | 1-423 | 4.08 | 82.8 | Green | 241 |
| Example 90 | 1-439 | 4.05 | 83.6 | Green | 233 |
| Example 91 | 1-448 | 4.11 | 84.0 | Green | 240 |
| Example 92 | 1-451 | 4.16 | 82.5 | Green | 233 |
| Example 93 | 1-457 | 4.10 | 81.9 | Green | 221 |
| Example 94 | 1-459 | 4.16 | 80.4 | Green | 208 |
| Example 95 | 1-468 | 4.10 | 83.1 | Green | 227 |
| Example 96 | 1-472 | 4.12 | 85.1 | Green | 220 |
| Example 97 | 1-481 | 4.22 | 74.1 | Green | 194 |
| Example 98 | 1-483 | 4.25 | 75.1 | Green | 204 |
| Example 99 | 1-484 | 4.20 | 75.2 | Green | 203 |
| Example 100 | 1-487 | 4.30 | 74.9 | Green | 212 |
| Example 101 | 1-489 | 4.15 | 77.2 | Green | 215 |
| Example 102 | 1-494 | 4.11 | 76.5 | Green | 195 |
| Example 103 | 1-496 | 4.20 | 78.6 | Green | 201 |
| Example 104 | 1-497 | 4.28 | 75.7 | Green | 202 |
| Example 105 | 1-500 | 4.17 | 79.1 | Green | 234 |
| Example 106 | 1-503 | 4.20 | 81.2 | Green | 251 |
| Example 107 | 1-505 | 4.12 | 84.3 | Green | 231 |
| Example 108 | 1-510 | 4.20 | 79.4 | Green | 224 |
| Example 109 | 1-518 | 4.20 | 83.9 | Green | 237 |
| Example 179 | 1-529 | 4.13 | 89 | Green | 291 |
| Example 180 | 1-541 | 4.18 | 91 | Green | 284 |
| Example 181 | 1-559 | 4.11 | 93 | Green | 293 |
| Example 182 | 1-575 | 4.13 | 88 | Green | 287 |
| Example 183 | 1-587 | 4.17 | 93 | Green | 281 |
| Example 184 | 1-595 | 4.15 | 87 | Green | 291 |
| Comparative Example 1 | A | 6.51 | 23.5 | Green | 29 |
| Comparative Example 2 | B | 6.44 | 23.4 | Green | 24 |
| Comparative Example 3 | C | 6.45 | 23.9 | Green | 24 |
| Comparative Example 4 | D | 4.58 | 68.3 | Green | 79 |
| Comparative Example 5 | E | 5.00 | 44.2 | Green | 96 |
| Comparative Example 6 | F | 4.94 | 43.9 | Green | 90 |
| Comparative Example 7 | G | 5.04 | 43.5 | Green | 106 |
| Comparative Example 8 | H | 4.95 | 42.9 | Green | 101 |
| Comparative Example 9 | I | 4.60 | 69.1 | Green | 139 |
| Comparative Example 10 | J | 4.51 | 68.2 | Green | 123 |
| Comparative Example 11 | K | 4.64 | 69.7 | Green | 131 |
| Comparative Example 12 | L | 4.46 | 67.4 | Green | 94 |
| Comparative Example 13 | M | 4.49 | 68.4 | Green | 128 |
| Comparative Example 14 | N | 4.52 | 69.7 | Green | 135 |
| Comparative Example 15 | O | 4.44 | 70.5 | Green | 116 |
| Comparative Example 16 | P | 4.40 | 69.4 | Green | 117 |
| Comparative Example 17 | Q | 4.47 | 72.1 | Green | 60 |
| Comparative Example 18 | R | 4.40 | 70.3 | Green | 107 |
| Comparative Example 19 | S | 4.62 | 54.9 | Green | 72 |
| Comparative Example 20 | 2-3 | 5.21 | 47.0 | Green | 55 |
| Comparative Example 21 | 2-4 | 5.75 | 41.1 | Green | 49 |
| Comparative Example 22 | 2-5 | 5.71 | 42.6 | Green | 51 |
| Comparative Example 23 | 2-7 | 5.48 | 45.3 | Green | 56 |
| Comparative Example 24 | 2-31 | 5.75 | 41.2 | Green | 41 |
| Comparative Example 25 | 2-32 | 5.48 | 40.2 | Green | 54 |
| Comparative Example 26 | 2-42 | 5.52 | 41.0 | Green | 53 |
| Comparative Example 27 | 3-2 | 5.83 | 42.9 | Green | 45 |
| Comparative Example 28 | 3-3 | 5.84 | 43.0 | Green | 45 |
| Comparative Example 29 | 2-98 | 5.39 | 45.1 | Green | 59 |
| Comparative Example 30 | 2-106 | 5.41 | 44.8 | Green | 62 |
| Comparative Example 31 | 2-110 | 5.35 | 48.2 | Green | 68 |
| Comparative Example 32 | 2-114 | 5.32 | 49.6 | Green | 69 |
| Comparative Example 33 | 3-106 | 5.47 | 46.2 | Green | 53 |
| Comparative Example 34 | 3-111 | 5.51 | 45.3 | Green | 55 |
| Comparative Example 35 | 3-117 | 5.43 | 47.5 | Green | 57 |
| Comparative Example 36 | 3-118 | 5.42 | 46.8 | Green | 59 |

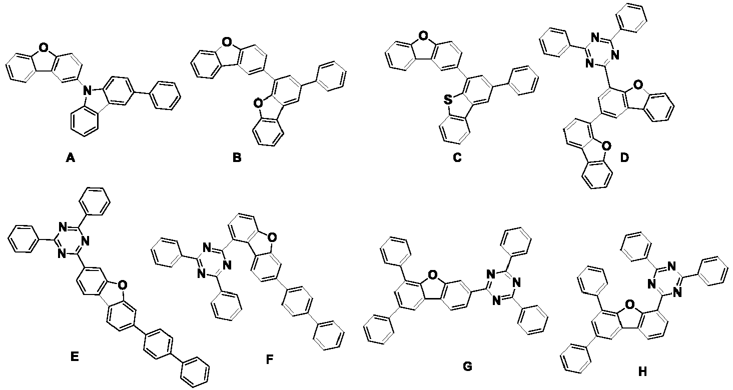

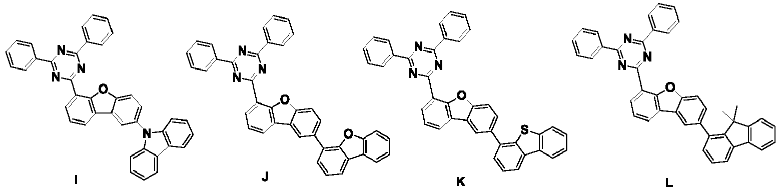

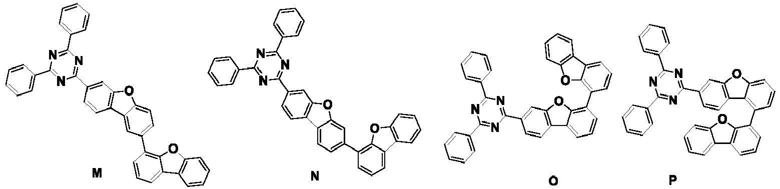

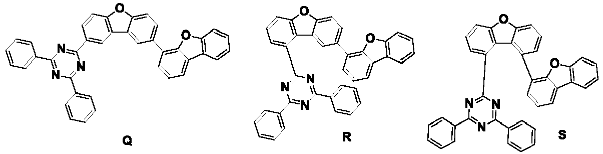

As can be seen in Table 19, in Comparative Compounds A, B, and C, carbazole, dibenzofuran, and dibenzothiophene, in which phenyl is each substituted at position 2 of dibenzofuran, are substituted. It can be seen that the service life in the case of a carbazole group is almost similar to that in the case of dibenzofuran and dibenzothiophene, although the driving voltage is relatively better.

In the case of Comparative Compound D, dibenzofuran and diphenyltriazine are substituted at positions 2 and 4 of dibenzofuran. Comparative Compound D has excellent driving voltage and efficiency, but an insufficient service life compared to Comparative Compounds E, F, G, and H, and shows an insufficient service life compared to Comparative Compounds I, J and K with similar driving voltages and efficiencies. This is because when only one side of the dibenzofuran core is utilized, the LUMO/HOMO levels are not properly separated and are mixed with each other, and thus structural stability deteriorates compared to Comparative Compounds E, F, G, and H.

Compared to Comparative Compounds E, F, G, and H, Comparative Compounds I, J, and K show improved driving voltage, service life, and efficiency. Substituents constituting the HOMO level in the corresponding compound are biphenyl, diphenyl, carbazole, dibenzofuran, and dibenzothiophene, respectively. A phenyl group has relatively poor properties as an electron donor compared to carbazole, dibenzofuran, and dibenzothiophene. Therefore, since the property as an electron donor is weakened toward the side of triazine, which is an electron withdrawing unit, and the flow of intramolecular electrons deteriorates, a stronger voltage is required, but the light emitting efficiency deteriorates, and non-radiative recombination occurs due to the accumulation of charges in the light emitting layer due to strong voltage, thereby having a low service life.

In Comparative Compounds I, J, K, and L, L is a structure in which dimethyl fluorine, which is a ring group which does not include a heteroatom, is substituted. This structure exhibits low voltage and a similar level of efficiency, but a low service life due to weak binding force at the methyl-bonded position of dimethylfluorene. Dibenzofuran, dibenzothiophene, or carbazole, which has N, O, and S substituted at the same position and thus has strong resonance structures, has enhanced structural stability, and thus has longer service life characteristics.

Although Comparative Compounds I, J, and K have similar tendencies, it can be seen that the stable electron donor properties of a cabazole group favor long service life properties. However, although dibenzofuran and dibenzothiophene are combined, a life deviation of about 10% is shown, but it can be confirmed that rather a similar efficiency is maintained, and the driving voltage is slightly improved.

In terms of the properties and structural stability as the electron donor, the characteristics of the carbazole group are excellent compared to those of dibenzofuran or dibenzothiophene, but the strong electronegativity of O and S atoms has an advantage in increasing the charge mobility of materials. Therefore, electrons move faster and a lower driving voltage is shown. However, as the mobility of electrons increases, the stability of the molecule itself is affected, so that when electrons gather in a positionally vulnerable part, there is a disadvantage in that radicalization progresses, and thus the deterioration in the molecule may progress.

In the case of Comparative Compounds J, M, N, O, P, Q, R, and S, it can be seen that there are changes due to the binding positions of triazine and dibenzofuran. It can be seen that the relative absolute service life values increase for Comparative Compounds J, M, and N, which have more enhanced orthogonality. It can be seen that in the case of Comparative Compounds O, P, and R, the service life may be decreased by providing stronger steric hindrance to the molecular structure, but the driving voltage or efficiency may be improved.

Comparative compound Q is a material in which a triazine group is substituted at position 2 of dibenzofuran. Although the driving voltage and efficiency are excellent, it can be seen that the service life is only at a half level. When a triazine group is introduced into the corresponding position, Comparative Compound Q has a high T1 level, and thus may be advantageous in terms of efficiency, but due to the characteristic that the heteroatom of dibenzofuran/dibenzothiophene is substituted, position 2 is the same as the substitution of the para position of O and S. This position is a position where dibenzofuran and dibenzothiophene are susceptible to deterioration due to radicalization, and when an acceptor that attracts electrons is substituted, radicalization proceeds more easily, so that this position shows a tendency that the service life rapidly decreases.

Comparative Compound S is a material in which positions 1 and 9 are substituted, and is in the form with the greatest steric hindrance. In this case, steric hindrance exacerbates structural distortions, showing a deterioration in structural stability. Neither the HOMO level nor the LUMO level spreads widely, and the levels are narrowly maintained in the substituent, and accordingly, the orbital is not extended to the substituted phenyl group of the central dibenzofuran or triazine, so that low efficiency and service life are exhibited due to an unstable form.

Comparative Examples 20 to 36 in Table 19 are all P-type hosts (donor hosts) centered on a carbazole group, which is a strong electron donor. Comparative Examples 20 to 36 have a strong electron donor tendency, but are composed of an arylene group whose electron acceptor properties deteriorate severely compared to a triazine group, and a substituted dibenzothiophene group, and thus show high driving voltage and low efficiency and service life.

Ultimately, as can be seen in Table 19, according to the present invention, a host material with long service life and high efficiency may be obtained by enhancing the low-power properties of dibenzofuran and dibenzothiophene and simultaneously introducing an aryl substituent into the material to expand the HOMO level and enhance the structural stability of the material.

As shown in Examples 1 to 109 and 179 to 184 in Table 19, it could be confirmed that in the heterocyclic compound represented by Chemical Formula 1 according to the present invention into which Ar of Chemical Formula 1 is introduced, the driving voltage, efficiency and service life are all improved compared to Comparative Examples 10 to 16 (Comparative Compounds J, K, M, N, O, and P). In the case of dibenzofuran/dibenzothiophene to which triazine is bonded, the LUMO settles around the triazine. In this case, the LUMO is extended and affects the dibenzofuran/dibenzothiophene core (hereinafter referred to as the core) located at the center of the compound due to the effect of O and S, which are atoms with strong electronegativity.

As can be seen from the results in Table 19, it could be confirmed that the organic electroluminescence device using a light emitting layer material of the organic electroluminescence device of the present invention had a low driving voltage, an enhanced light emitting efficiency, and a significantly improved service life compared to those in Comparative Compounds J, K, M, N, O, P, Q, R, S, and T.

A condensed cyclic group introduced into a triazine group may enhance the strong electron withdrawing properties of the triazine. Regardless of the presence or absence of heteroatoms, the introduction of multiple condensed rings with a stable structure may secure a wide conjugation region to expand the LUMO level. The expanded LUMO level plays a role in maintaining sufficient current efficiency even at low voltage by making electron mobility stronger, and adjustments to the suitable level of T1 energy level required for red light emission are also simultaneously made.

Since various colors may be implemented in the same structure depending on whether or not the substituent is introduced into the condensed ring based on an existing structural body, the extensibility of the corresponding structural body is infinite, and there is an advantage in that a device in the similar form can be constructed even though RGB method or various device structures such as 2-stack and 3-stack are introduced.

Since it is possible to prepare a material with an appropriate work function depending on the position of the substituent, the material can be used as an element for adjusting gap energy and energy levels such as HOMO and LUMO and adjusting the electron mobility of the entire device even though a new device will be constructed in the future.

When heteroarylene is introduced into a specific substituent of dibenzofuran/dibenzothiophene, the conjugation region of the material itself is expanded, but since the material is connected by a single bond, the expansion of the HOMO level to a certain level and the energy level are affected, but there is no major effect or there is a minor synergistic effect on the T1 energy levels of the material This is because the material is composed of a structure having a similar electron withdrawing or electron donating tendency of the substituents substituted at this position and the T1 energy levels of dibenzofuran and dibenzothiophene themselves are high (dibenzofuran = 3.14 eV and dibenzothiophene = 3.01 eV). The above-described heteroarylene group, which is a relatively weak donor and acceptor compared to a carbazole group/dibenzofuran/dibenzothiophene/an amine group, a triazine group/a pyrimidine group/a pyridine group/an imidazole group, or the like, rather has a close relationship with a substituent which the heteroarylene group is substituted with. In addition, it is possible to finely adjust the T1 energy level through an effect of breaking the pi-pi conjugation (structural steric hindrance) depending on the binding position. For this reason, the conjugation region of the substituted unit is expanded, and the work function of a certain level is not significantly affected. Therefore, since the maintenance of the intrinsic T1 energy level of dibenzofuran/dibenzothiophene of a central structure is not significantly affected, it is possible to exhibit excellent efficiency and long service life characteristics that do not hinder its use as a phosphorescent green host.

Further, it was confirmed that Examples 97 to 109 and Examples 179 to 184, which are materials which are additionally substituted with deuterium, are observed had an effect of further improving the service life compared to Examples 1, 4, 5, 9, 12, 16, 22, 32, 33, 40, 42, 46, 53, 58, 66, 78, 79, and 86, which are materials which are not substituted. This is due to the weak atomic vibration of deuterium, which has an increased atomic weight relative to hydrogen, and a decrease in the departure rate. However, it was confirmed that the higher the substitution rate of deuterium, the greater the influence thereof, and the driving voltage and current efficiency are not significantly affected.

In addition, the dibenzofuran/dibenzothiophene bonded to the opposite side of the core of Chemical Formula 1 of the present invention or the cyclic group such as another aryl, arylene, and heteroarylene has relatively strong electron donor properties, so that the HOMO is positioned. However, the region occupied by HOMO in the entire compound becomes a form which is not extended to the core, and is restricted to an arylene or heterocyclic group substituted for the core. Therefore, the region has a molecular structure with a very strong acceptor tendency, and such a structure has characteristics that electrons move rapidly during the driving of the device and it is advantageous to construct a device with a lower driving voltage.

Since more electrons are attracted, the electrical stress applied to the compound also increases correspondingly. However, the core unit has two substituents, and in particular, due to the influence of the triazine unit, the electrons of the entire core becomes insufficient, so radicalization is less likely to occur.

Triazine and a cyclic group substituted with the triazine also have insufficient electrons due to the strong electron withdrawing tendency of triazine, so the deterioration phenomenon due to chemical properties is not significant even during the driving of the device. Unlike triazine, dibenzofuran/dibenzothiophene opposite to the core have high stability of the molecule itself due to the resonance effect of the condensed cyclic group, but the radicalization or decomposition of the benzene ring may be sufficiently caused by the heat generated during the driving of the device and the energy of the supplied electric current. In particular, the P (para) position of O and S atoms is a position where radicalization is likely to occur, and the energy transferred by non-radiative recombination of TTA, TPA and the like, which occurs during the driving of the device transfers energy enough to disintegrate the bonds of the molecular structure. The deterioration of the light emitting layer caused by this is insufficient in life performance when the cabazole group, which has relatively high stability, is substituted compared to Comparative Example T.

However, the stability of the molecular structure may be enhanced by introducing an aryl group to extend the conjugation region of dibenzofuran/dibenzothiophene. In addition, as the conjugation region widens, the region of the HOMO level present at the corresponding position also widens and has a more stable region.

Dibenzofuran/dibenzothiophene introduced into the host plays a role as a weak donor compared to the carbazole group mainly used in the bipolar host in the related art. It is needless to say that the dibenzofuran/dibenzothiophene has a strong acceptor tendency, and thus has low voltage characteristics, and it was possible to pursue higher efficiency depending on the device structure. However, the flow of excessive electric current and the disintegration of the intramolecular balance aggravated the stability of the entire light emitting layer, causing fatal weaknesses in the service life. By substituting an additional heteroaryl group and extending the structure, the HOMO level distributed in a narrow region is expanded compared to the LUMO level distributed in a wide region due to the influence of a strong acceptor, thereby enhancing the electrical stability of the compound. In this case, since an acceptor unit maintains the same substituent as the existing one, it is possible to maintain the fast electrical characteristics and simultaneously enhance the stability of the unstable HOMO level, so that a long-service life host with low voltage and high efficiency characteristics can be obtained.

### <Experimental Example 2> - Manufacture of organic light emitting device

A glass substrate, in which indium tin oxide (ITO) was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then subjected to ultraviolet ozone (UVO) treatment for 5 minutes by using UV in a UV washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

The hole injection layer 4,4',4"-tris[2-naphthyl(phenyl)amino] triphenylamine (2-TNATA) and the hole transport layer N,N'-diphenyl-(1,1'-biphenyl)-4,4'-diamine (NPB), which are common layers, were formed on the ITO transparent electrode (positive electrode).

A light emitting layer was thermally vacuum deposited thereon as follows. The light emitting layer was pre-mixed by pre-mixing one type of compound described in Chemical Formula 1 and one type of compound described in Chemical Formula 2 or 3 as hosts as in the following Table 20, and then deposited to have a thickness of 400 Å from one common container, and deposited by doping the host with Ir(ppy)₃ a green phosphorescent dopant in an amount of 7%. Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then aluminum (Al) negative electrode was deposited to have a thickness of 1,200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁶ to 10⁻⁸ torr for each material, and used for the manufacture of OLED.

For the organic electroluminescence device manufactured as described above, electroluminescence (EL) characteristics were measured by M7000 manufactured by McScience Inc., and based on the measurement result thereof, T90 was measured by a service life measurement equipment (M6000) manufactured by McScience Inc., when the reference luminance was 6,000 cd/m².

**[Table 20]**

| | Light emitting layer Compoun d | | Ratiο | Driving voltag e (V) | Efficiency (cd/A) | Light emission color | Service life (T90) |
|---|---|---|---|---|---|---|---|
| Exampl e 110 | 1-1 | 3-2 | 1:5 | 5.57 | 75.4 | Green | 200 |
| Exampl e 111 | | | 1:3 | 4.45 | 78.5 | Green | 242 |
| Exampl e 112 | | | 1:2 | 4.18 | 86.0 | Green | 311 |
| Exampl e 113 | | | 1:1 | 4.01 | 81.5 | Green | 265 |
| Exampl e 114 | | | 2:1 | 4.12 | 79.8 | Green | 221 |
| Exampl e 115 | | | 3:1 | 4.80 | 78.1 | Green | 210 |
| Exampl e 116 | | | 5:1 | 5.36 | 73.4 | Green | 195 |
| Exampl e 117 | | 2-3 | 1:2 | 4.26 | 86.4 | Green | 337 |
| Exampl e 118 | | | 1:1 | 4.02 | 82.5 | Green | 270 |
| Exampl e 119 | 1-2 | 2-4 | 1:2 | 4.26 | 89.6 | Green | 352 |
| Exampl e 120 | | | 1:1 | 4.04 | 83.7 | Green | 277 |
| Exampl e 121 | 1-14 | 2-5 | 1:2 | 4.19 | 90.6 | Green | 347 |
| Exampl e 122 | | | 1:1 | 3.99 | 82.9 | Green | 269 |
| Exampl e 123 | 1-25 | 2-31 | 1:2 | 4.28 | 90.2 | Green | 362 |
| Exampl e 124 | | | 1:1 | 4.00 | 82.4 | Green | 281 |
| Exampl e 125 | 1-31 | 2-7 | 1:2 | 4.20 | 90.9 | Green | 381 |
| Exampl e 126 | | | 1:1 | 4.08 | 85.3 | Green | 284 |
| Exampl e 127 | 1-54 | 2-4 | 1:2 | 4.18 | 90.3 | Green | 366 |
| Exampl e 128 | | | 1:1 | 3.98 | 84.9 | Green | 277 |
| Exampl e 129 | 1-73 | 2-31 | 1:2 | 4.19 | 91.5 | Green | 394 |
| Exampl e 130 | | | 1:1 | 3.96 | 84.5 | Green | 296 |
| Exampl e 131 | 1-88 | 2-32 | 1:2 | 4.20 | 88.4 | Green | 371 |
| Exampl e 132 | | | 1:1 | 4.02 | 82.7 | Green | 281 |
| Exampl e 133 | 1-102 | 2-42 | 1:2 | 4.11 | 90.9 | Green | 409 |
| Exampl e 134 | | | 1:1 | 3.91 | 84.3 | Green | 281 |
| Exampl e 135 | 1-119 | 2-31 | 1:2 | 4.22 | 91.0 | Green | 388 |
| Exampl e 136 | | | 1:1 | 4.03 | 84.6 | Green | 279 |
| Exampl e 137 | 1-139 | 2-42 | 1:2 | 4.18 | 88.2 | Green | 371 |
| Exampl e 138 | | | 1:1 | 3.99 | 83.5 | Green | 288 |
| Exampl e 139 | 1-153 | 2-5 | 1:2 | 4.12 | 92.5 | Green | 415 |
| Exampl e 140 | | | 1:1 | 3.94 | 85.2 | Green | 294 |
| Exampl e 141 | 1-164 | 2-7 | 1:2 | 4.29 | 94.6 | Green | 429 |
| Exampl e 142 | | | 1:1 | 4.10 | 86.9 | Green | 301 |
| Exampl e 143 | 1-178 | 2-32 | 1:2 | 4.14 | 90.8 | Green | 388 |
| Exampl e 144 | | | 1:1 | 3.92 | 82.8 | Green | 289 |
| Exampl e 145 | 1-207 | 3-3 | 1:2 | 4.23 | 95.7 | Green | 415 |
| Exampl e 146 | | | 1:1 | 3.91 | 87.3 | Green | 301 |
| Exampl e 147 | 1-223 | 3-2 | 1:2 | 4.06 | 96.4 | Green | 367 |
| Exampl e 148 | | | 1:1 | 3.88 | 88.9 | Green | 294 |
| Exampl e 149 | 1-239 | 2-31 | 1:2 | 4.20 | 98.2 | Green | 422 |
| Exampl e 150 | | | 1:1 | 3.95 | 90.2 | Green | 304 |
| Exampl e 151 | 1-263 | 2-32 | 1:2 | 4.22 | 97.5 | Green | 366 |
| Exampl e 152 | | | 1:1 | 3.93 | 90.1 | Green | 304 |
| Exampl e 153 | 1-299 | 2-7 | 1:2 | 4.10 | 100.4 | Green | 371 |
| Exampl e 154 | | | 1:1 | 3.95 | 91.2 | Green | 288 |
| Exampl e 155 | 1-322 | 2-31 | 1:2 | 4.10 | 98.2 | Green | 388 |
| Exampl e 156 | | | 1:1 | 3.86 | 88.5 | Green | 299 |
| Exampl e 157 | 1-337 | 3-3 | 1:2 | 4.10 | 97.5 | Green | 391 |
| Exampl e 158 | | | 1:1 | 3.88 | 89.1 | Green | 300 |
| Exampl e 159 | 1-347 | 2-32 | 1:2 | 4.05 | 96.4 | Green | 401 |
| Exampl e 160 | | | 1:1 | 3.85 | 89.8 | Green | 291 |
| Exampl e 161 | 1-378 | 3-3 | 1:2 | 4.00 | 95.2 | Green | 388 |
| Exampl e 162 | | | 1:1 | 3.81 | 88.3 | Green | 303 |
| Exampl e 163 | 1-412 | 2-42 | 1:2 | 4.02 | 99.6 | Green | 389 |
| Exampl e 164 | | | 1:1 | 3.83 | 90.0 | Green | 308 |
| Exampl e 165 | 1-439 | 2-7 | 1:2 | 4.06 | 103.2 | Green | 375 |
| Exampl e 166 | | | 1:1 | 3.78 | 91.3 | Green | 291 |
| Exampl e 167 | 1-468 | 2-5 | 1:2 | 4.06 | 99.6 | Green | 390 |
| Exampl e 168 | | | 1:1 | 3.87 | 90.4 | Green | 284 |
| Exampl e 169 | 1-472 | 2-31 | 1:2 | 4.10 | 97.3 | Green | 372 |
| Exampl e 170 | | | 1:1 | 3.90 | 91.4 | Green | 288 |
| Exampl e 171 | 1-487 | 2-7 | 1:2 | 4.21 | 91.0 | Green | 451 |
| Exampl e 172 | | | 1:1 | 4.06 | 85.2 | Green | 336 |
| Exampl e 173 | 1-489 | 2-4 | 1:2 | 4.19 | 89.8 | Green | 442 |
| Exampl e 174 | | | 1:1 | 3.98 | 84.0 | Green | 321 |
| Exampl e 175 | 1-496 | 2-42 | 1:2 | 4.17 | 87.4 | Green | 464 |
| Exampl e 176 | | | 1:1 | 3.96 | 82.9 | Green | 330 |
| Exampl e 177 | 1-500 | 2-32 | 1:2 | 4.12 | 90.9 | Green | 458 |
| Exampl e 178 | | | 1:1 | 3.93 | 82.4 | Green | 341 |
| Exampl e 185 | 1-500 | 2-110 | 1:2 | 4.01 | 99.5 | Green | 472 |
| Exampl e 186 | | | 1:1 | 3.90 | 91.4 | Green | 381 |
| Exampl e 187 | 1-529 | 2-4 | 1:2 | 4.09 | 92.7 | Green | 467 |
| Exampl e 188 | | | 1:1 | 3.95 | 86.2 | Green | 376 |
| Exampl e 189 | 1-529 | 2-98 | 1:2 | 4.02 | 99.8 | Green | 475 |
| Exampl e 190 | | | 1:1 | 3.91 | 91.6 | Green | 388 |
| Exampl e 191 | 1-529 | 2-110 | 1:2 | 3.92 | 110.2 | Green | 502 |
| Exampl e 192 | | | 1:1 | 3.87 | 105.4 | Green | 483 |
| Exampl e 193 | 1-541 | 2-114 | 1:2 | 3.93 | 111.4 | Green | 504 |
| Exampl e 194 | | | 1:1 | 3.87 | 107.8 | Green | 481 |
| Exampl e 195 | 1-496 | 3-3 | 1:2 | 4.10 | 91.5 | Green | 461 |
| Exampl e 196 | | | 1:1 | 3.91 | 83.9 | Green | 348 |
| Exampl e 197 | 1-500 | 3-117 | 1:2 | 4.06 | 100.3 | Green | 477 |
| Exampl e 198 | | | 1:1 | 3.89 | 92.5 | Green | 384 |
| Exampl e 199 | 1-541 | 3-3 | 1:2 | 4.06 | 93.2 | Green | 469 |
| Exampl e 200 | | | 1:1 | 3.98 | 87.8 | Green | 377 |
| Exampl e 201 | 1-559 | 3-106 | 1:2 | 4.00 | 100.5 | Green | 478 |
| Exampl e 202 | | | 1:1 | 3.89 | 92.0 | Green | 389 |
| Exampl e 203 | 1-559 | 3-117 | 1:2 | 3.96 | 111.4 | Green | 514 |
| Exampl e 204 | | | 1:1 | 3.91 | 106.2 | Green | 489 |
| Exampl e 205 | 1-575 | 3-118 | 1:2 | 3.95 | 112.8 | Green | 513 |
| Exampl e 206 | | | 1:1 | 3.90 | 108.2 | Green | 488 |
| Exampl e 207 | 1-1 | 2-98 | 1:2 | 4.22 | 92.2 | Green | 446 |
| Exampl e 208 | | | 1:1 | 4.08 | 86.5 | Green | 325 |
| Exampl e 209 | 1-14 | 2-114 | 1:2 | 4.12 | 93.6 | Green | 455 |
| Exampl e 210 | | | 1:1 | 3.98 | 87.4 | Green | 363 |
| Exampl e 211 | 1-54 | 3-106 | 1:2 | 4.11 | 92.7 | Green | 457 |
| Exampl e 212 | | | 1:1 | 3.92 | 84.5 | Green | 344 |
| Exampl e 213 | 1-119 | 3-118 | 1:2 | 4.09 | 92.3 | Green | 467 |
| Exampl e 214 | | | 1:1 | 3.99 | 86.6 | Green | 374 |

In an exemplary embodiment of the present application, referring to the results of Examples 110 to 178 and 185 to 214 of Table 20, when the compound represented by Chemical Formula 1 (n-type) according to the present invention was used together with the compound represented by Chemical Formula 2 or 3, better efficiency and service life effects are exhibited. This result is because an exciplex phenomenon occurs when two compounds are simultaneously included.

The exciplex phenomenon is a phenomenon in which energy with a magnitude of the HOMO energy level of the donor (p-host) and the LUMO energy level of the acceptor (n-host) is released due to an electron exchange between two molecules. When the exciplex phenomenon between two molecules occurs, a reverse intersystem crossing (RISC) occurs, and the internal quantum efficiency of fluorescence can be increased to 100% due to the RISC. When a donor (p-host) with good ability to transport holes and an acceptor (n-host) with good ability to transport electrons are used as hosts of the light emitting layer, holes are injected into the p-host, and electrons are injected into the n-host, and in this case, excitons are not quenched by intermolecular electron exchange, and the lifetime of excitons that can hold energy increases. As a result, the overall electric current efficiency may be improved and the service life of the device may be improved. In the present invention, it could be confirmed that the donor role and the acceptor role exhibit excellent device characteristics when the compound of Chemical Formula 2 or 3 and the compound of Chemical Formula 1 are used as hosts of the light emitting device, respectively.

The pre-mixing according to the present invention means that before the heterocyclic compound of Chemical Formula 1 and the heterocyclic compound of Chemical Formula 2 or 3 are deposited onto an organic material layer, the materials are first mixed and the mixture is contained in one common container and mixed. Since one deposition source is used instead of using two or three types of deposition sources during the pre-mixing, there is an advantage in that the process is more simplified.

During the pre-mixing described above, materials need to be mixed by confirming the thermal characteristics inherent to each material. In this case, when the pre-mixed host material is deposited from one deposition source, the deposition conditions including the deposition rate may be significantly affected by the thermal characteristics inherent to the material. When the thermal characteristics of two or more pre-mixed materials are not similar and significantly differ, repeatability and reproducibility cannot be maintained in the vapor deposition process, meaning that a completely uniform OLED device cannot be manufactured in a single deposition process.

In order to overcome this problem, the electrical characteristics of the material can be controlled by utilizing the appropriate combination of the basic structure of each material and the substituent, and simultaneously, the thermal characteristics can also be adjusted according to the form of the molecular structure. Therefore, it is possible not only to improve the performance of the device by utilizing the C-N bonds of carbazole as in Chemical Formula 2 and various substituents in the triazine group or dibenzofurane/dibenzothiophene of Chemical Formula 1 in addition to the basic skeleton, but also to secure diversity of various host-host pre-mixing deposition processes by adjusting thermal characteristics of each material. Through this, there is an advantage in that it is possible to ensure the diversity of the pre-mixing deposition process utilizing not only two compounds as hosts but also 3 to 4 or more types of host materials.

The mixing of Chemical Formula 1 and Chemical Formula 2 may mix two or more materials (3, 4 types, and the like), and the above experimental example is only a representative example, and is not limited thereto.

By mixing Chemical Formula 1 with Chemical Formula 2 or Chemical Formula 3, the effect of partially improving the current efficiency of the light emitting layer can be obtained, and a device with long service life characteristics can be constructed. Referring to the results of Table 20, better efficiency and service life effects are exhibited when the compound of Chemical Formula 1 and the compound of Chemical Formula 2 or 3 are simultaneously included. This is due to the exciplex phenomenon that occurs when two or more materials are mixed.

When the exciplex phenomenon occurs, the driving voltage is increased in some cases. This is due to an imbalance between holes and electrons in the light emitting layer of the device. This is a problem caused by deviations in hole and electron mobility of each material between mixed hosts. Therefore, a device which exhibits optimum performance can be constructed only by properly maintaining the balance of electron flow within the device. This problem could be solved by adjusting the ratio between an acceptor and a donor.

## Claims

1. A heterocyclic compound represented by the following Chemical Formula 1: 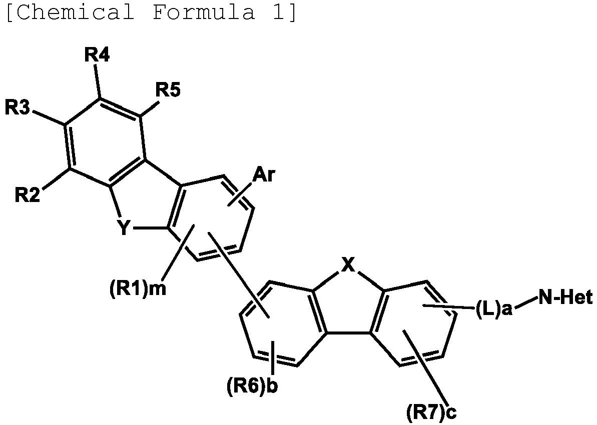 wherein, in Chemical Formula 1,
X and Y are the same as or different from each other, and are each independently O; or S,
N-Het is a monocyclic or polycyclic heterocyclic group which is substituted or unsubstituted and includes one or more N's,
R1 to R7 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
L is a direct bond; a substituted or unsubstituted C6 to C60 arylene group; or a substituted or unsubstituted C2 to C60 heteroarylene group,
Ar is a substituted or unsubstituted C1 to C60 alkyl group; a substituted or unsubstituted C6 to C60 aryl group; or a substituted or unsubstituted C2 to C60 heteroaryl group,
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
a deuterium content of the heterocyclic compound of Chemical Formula 1 is 0% to 100%,
a is an integer from 0 to 4,
b and c are an integer from 0 to 3,
m is an integer from 0 to 2, and
when a, b and c are each 2 or higher, substituents in the parenthesis are the same as or different from each other, and when m is 2, substituents in the parenthesis are the same as or different from each other.

2. The heterocyclic compound of claim 1, wherein Chemical Formula 1 is represented by any one of the following Chemical Formulae 4 to 7: 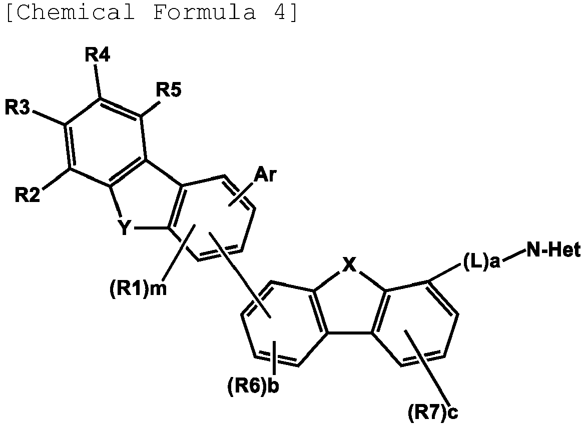 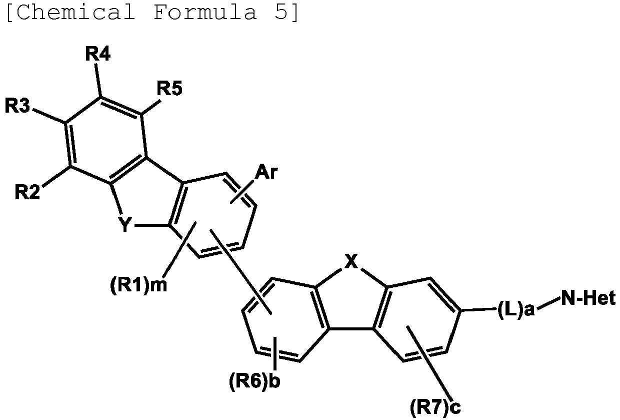 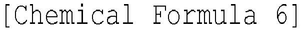 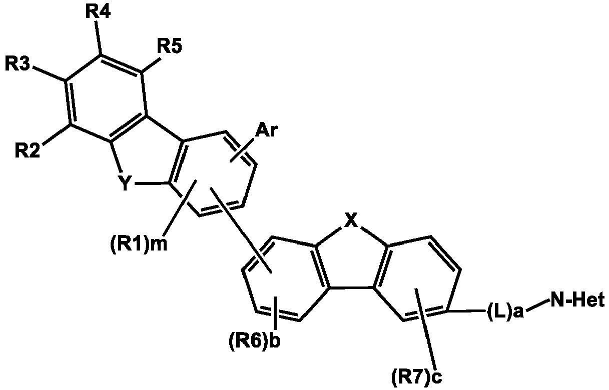 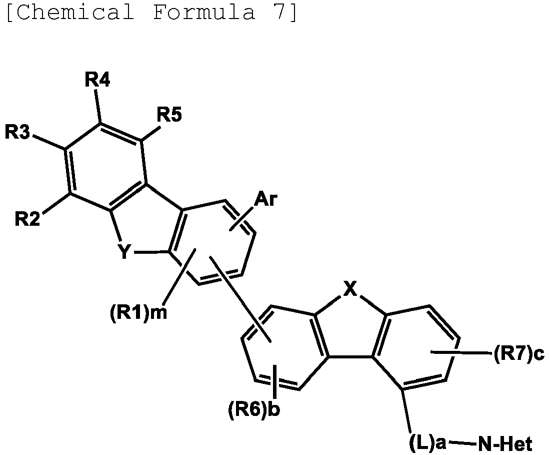 in Chemical Formulae 4 to 7,
the definition of each substituent is the same as the definition in Chemical Formula 1.

3. The heterocyclic compound of claim 1, wherein Chemical Formula 1 is represented by any one of the following Chemical Formulae 8 to 11: 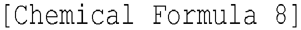 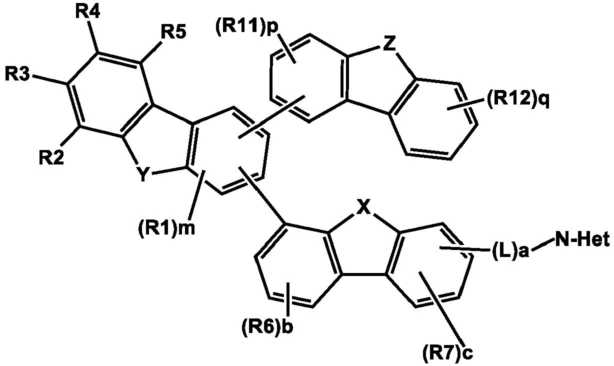 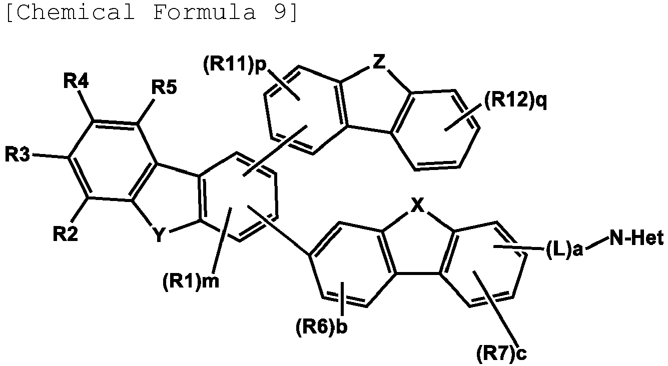 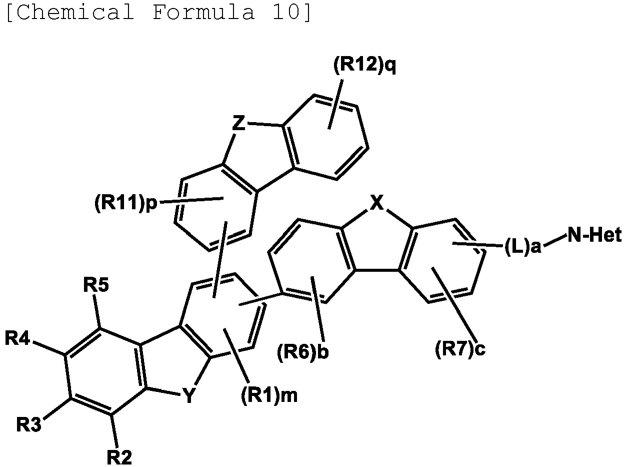 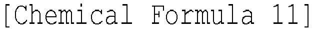 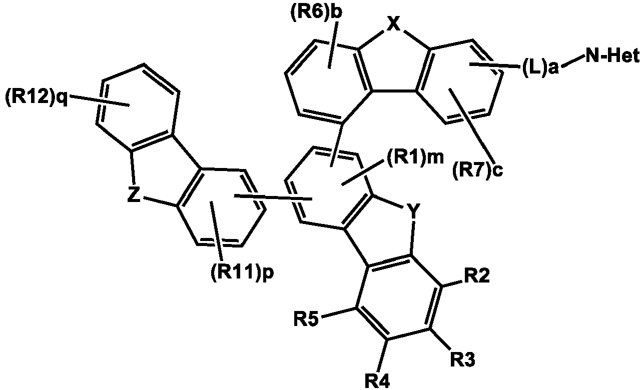 in Chemical Formulae 8 to 11,
the definitions of R1 to R7, X, Y, L, N-Het, a, b, c and m are the same as the definitions in Chemical Formula 1,
Z is O; or S,
R11 and R12 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
a deuterium content of the heterocyclic compound of Chemical Formulae 8 to 11 is 0% to 100%,
q is an integer of 0 to 4,
p is an integer from 0 to 3, and
when p and q are each an integer of 2 or higher, substituents in the parenthesis are the same as or different from each other.

4. The heterocyclic compound of claim 1, wherein in 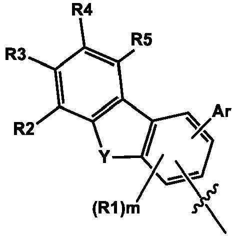 of Chemical Formula 1, a linking group linked to 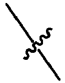 and a substituent of -Ar are bonded to a metha; or para position each other.

5. The heterocyclic compound of claim 1, wherein Chemical Formula 1 is represented by any one of the following compounds:

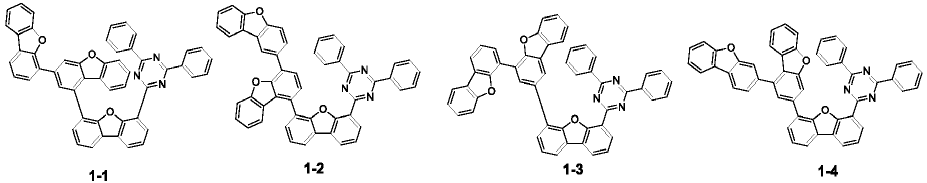

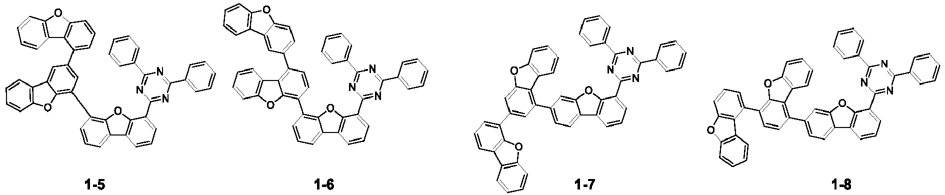

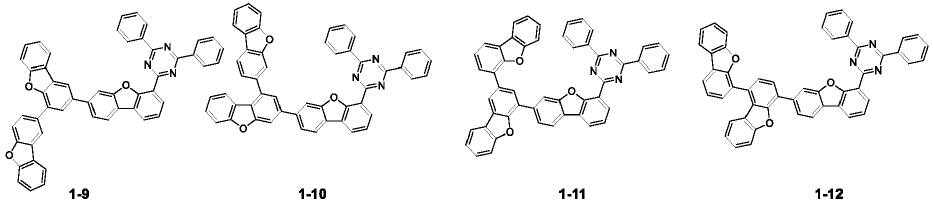

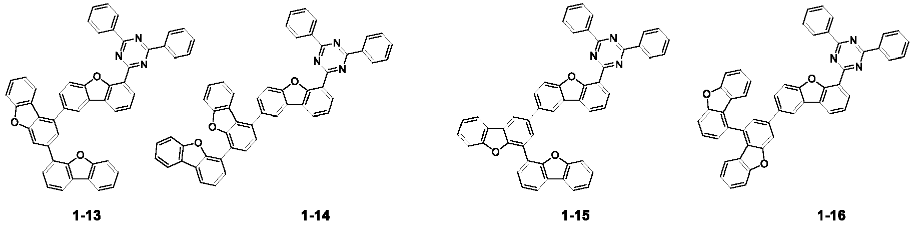

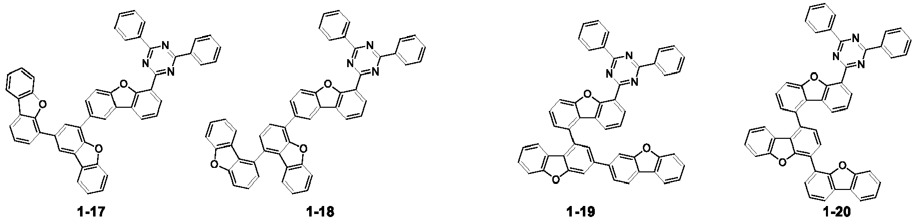

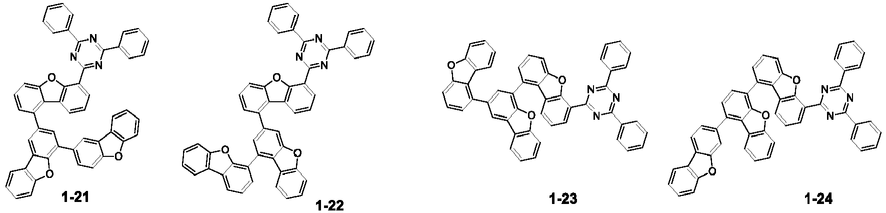

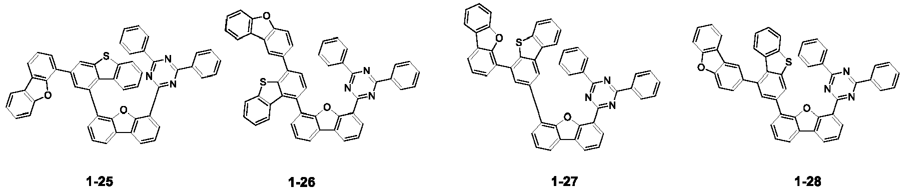

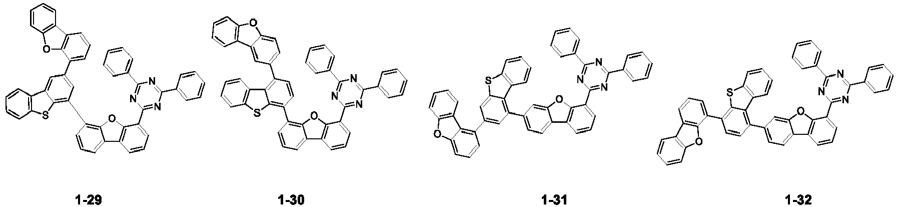

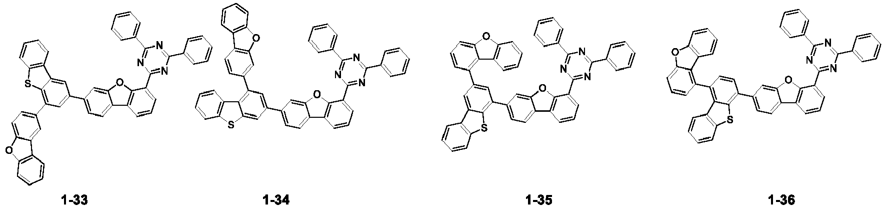

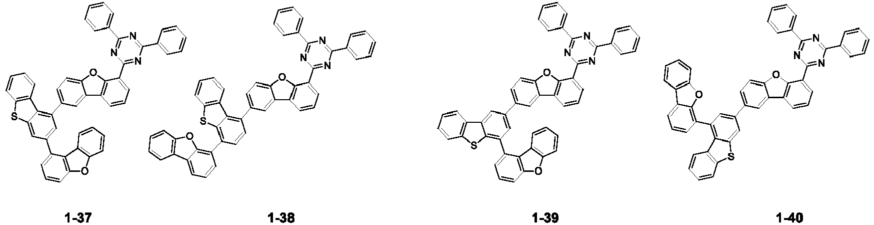

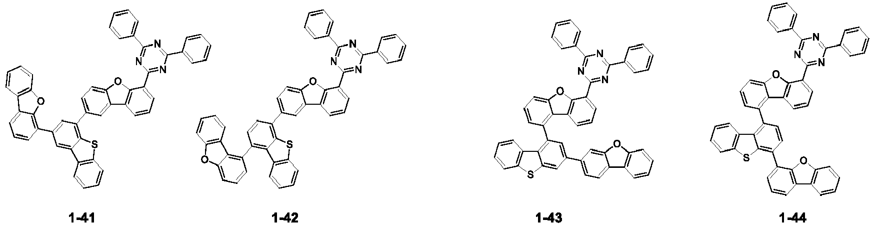

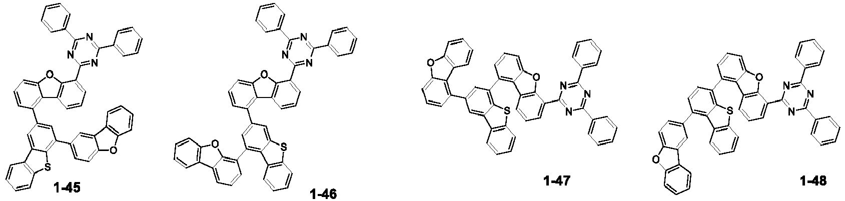

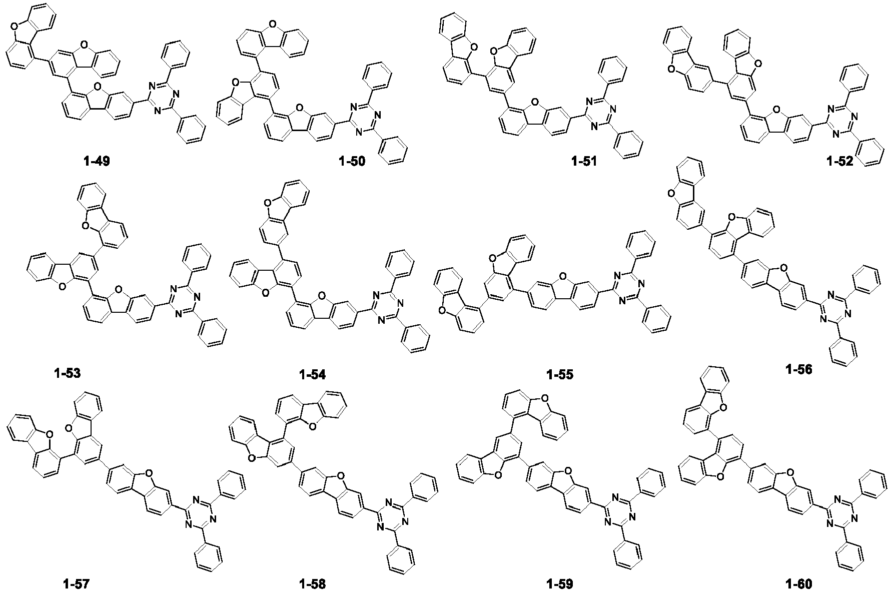

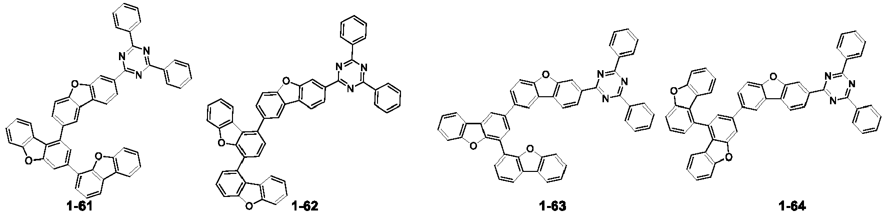

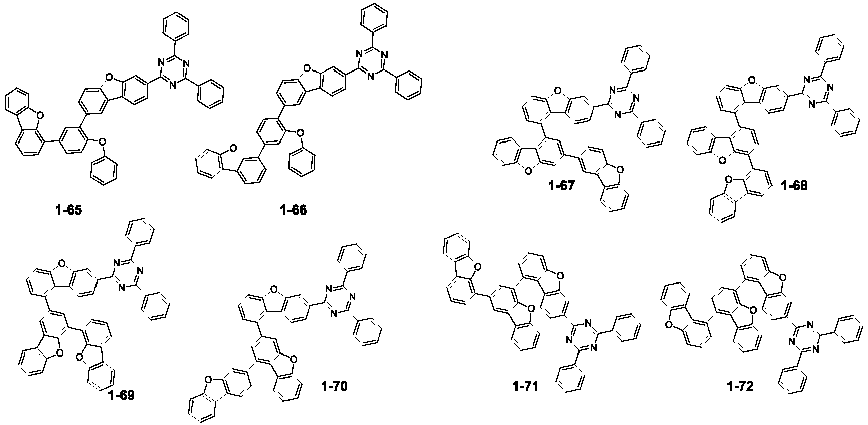

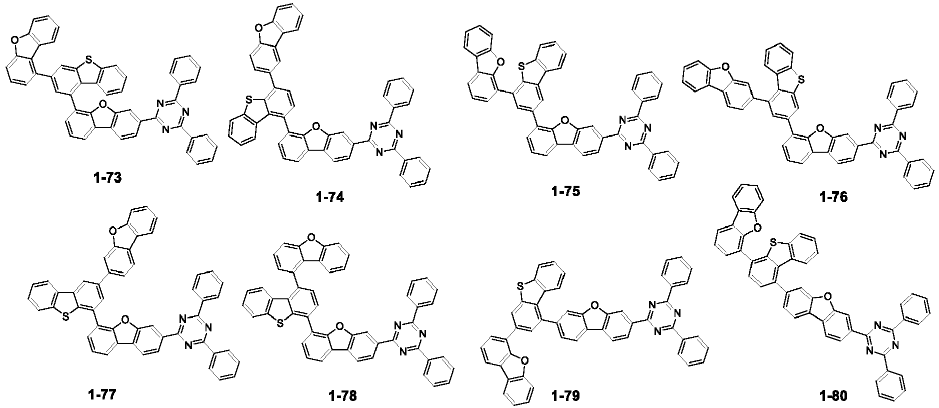

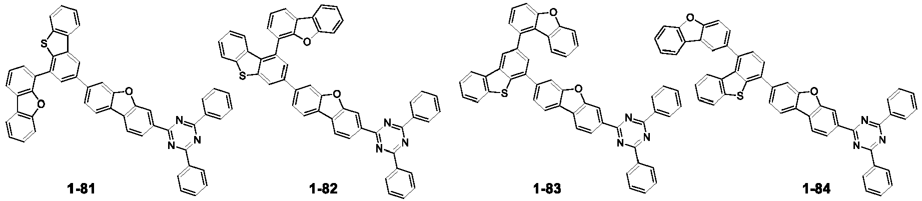

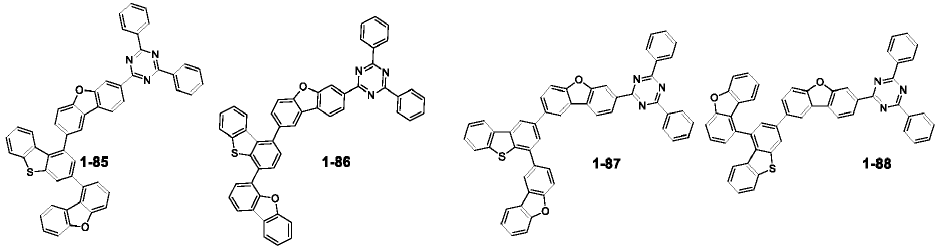

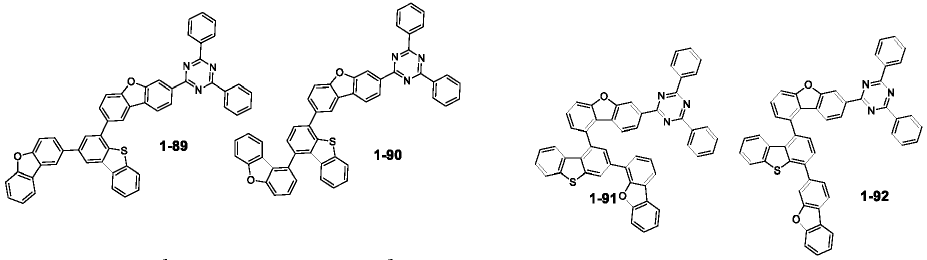

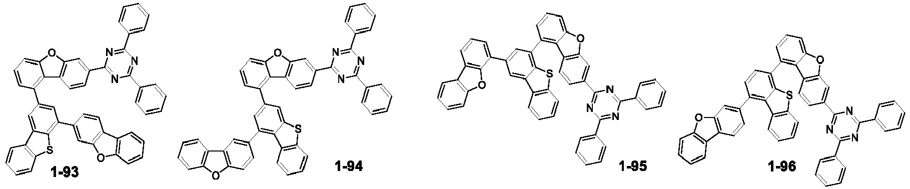

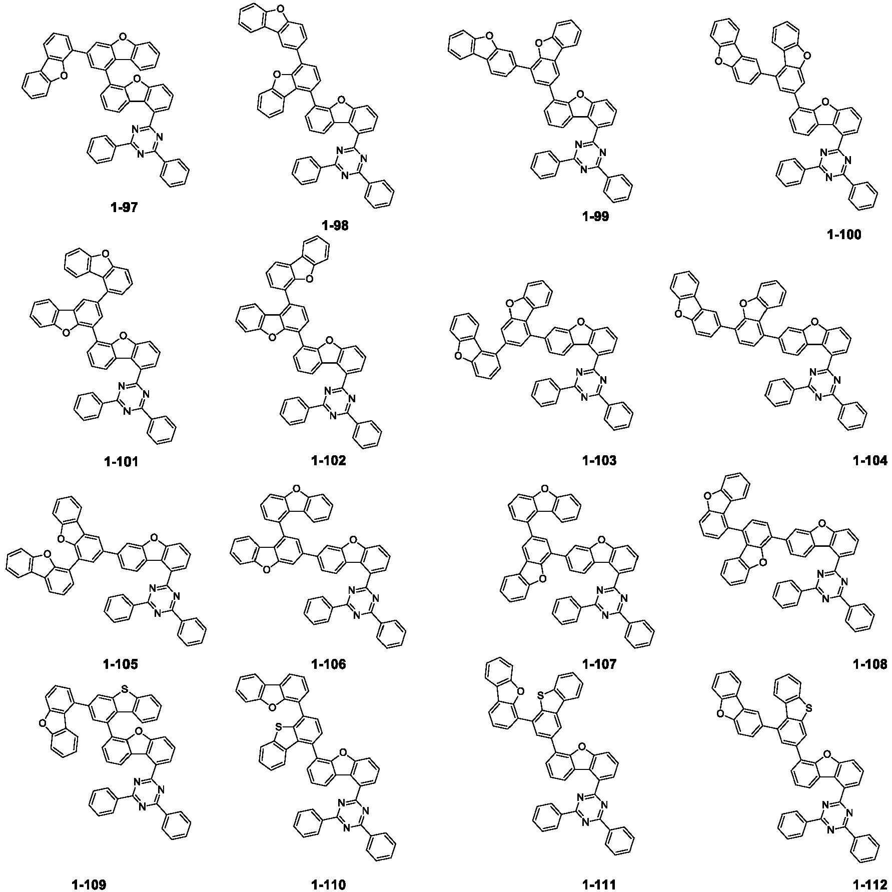

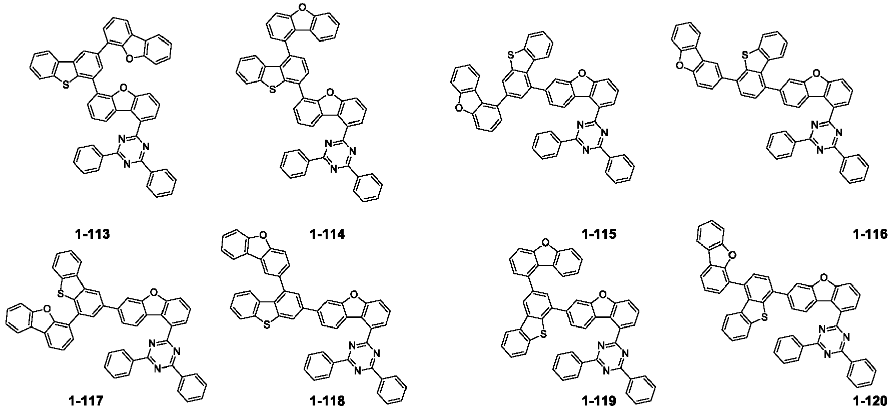

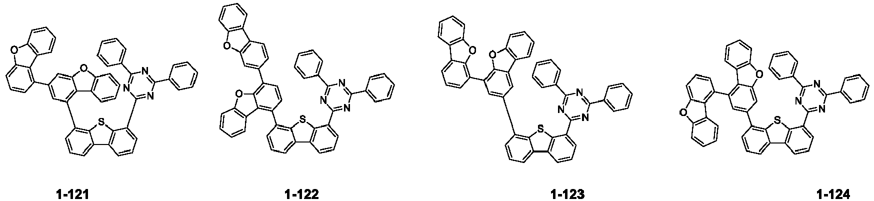

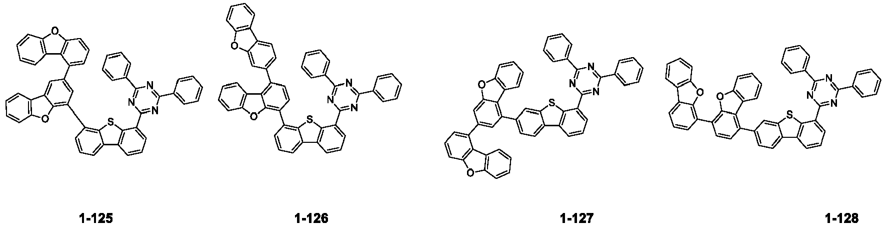

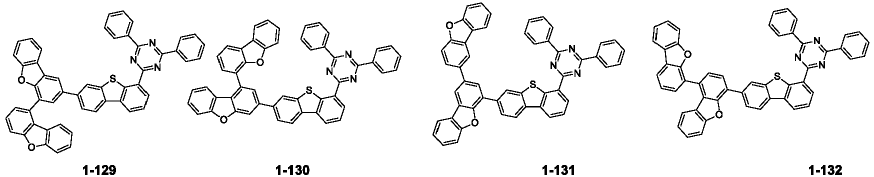

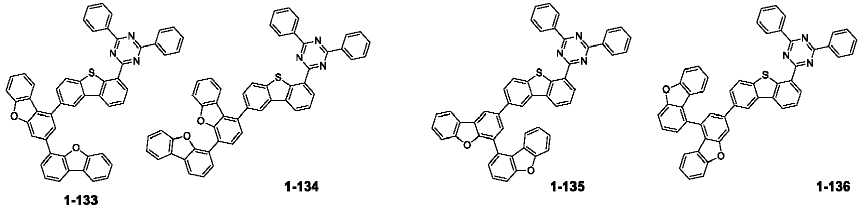

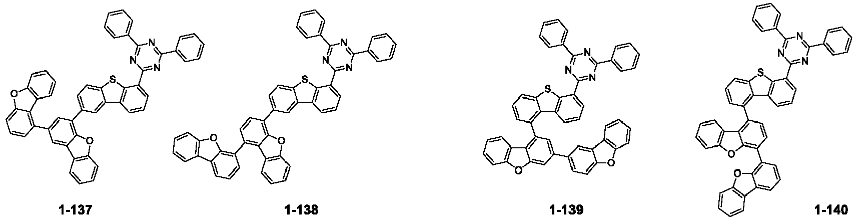

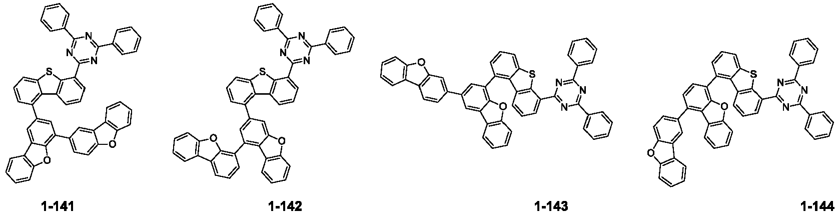

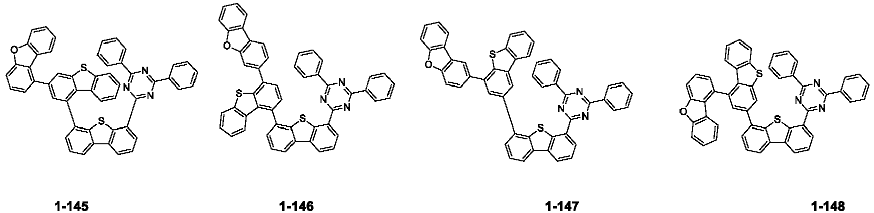

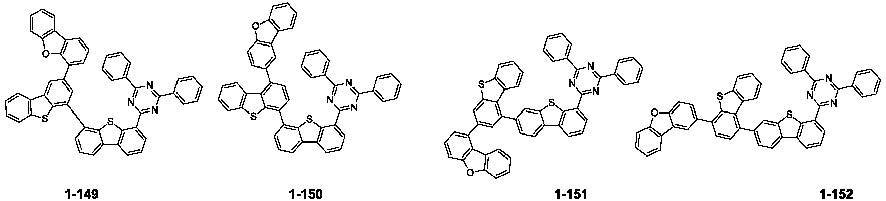

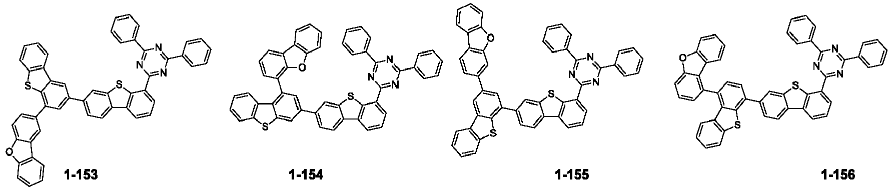

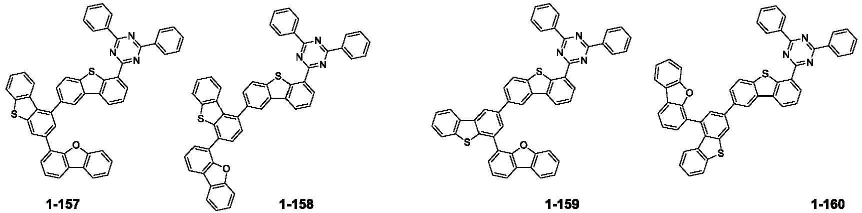

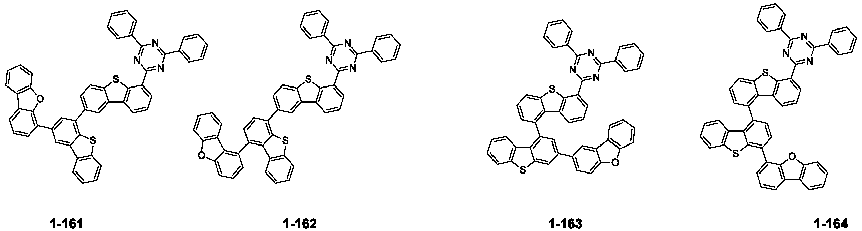

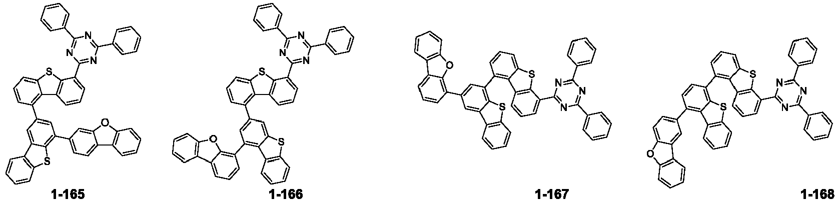

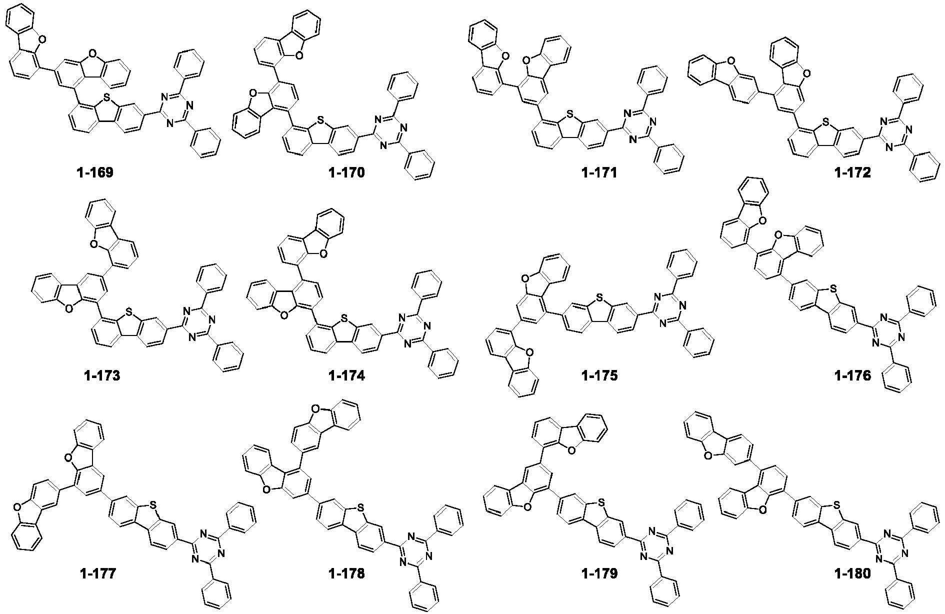

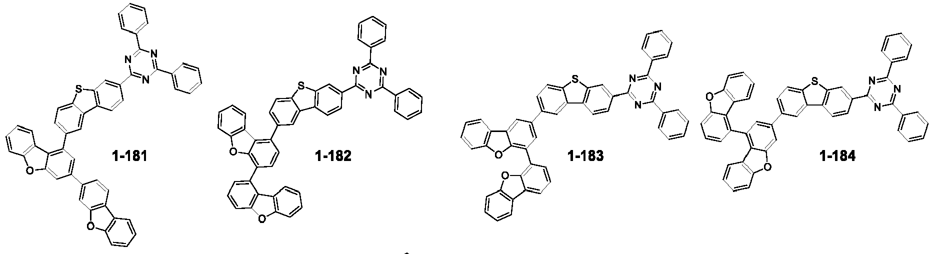

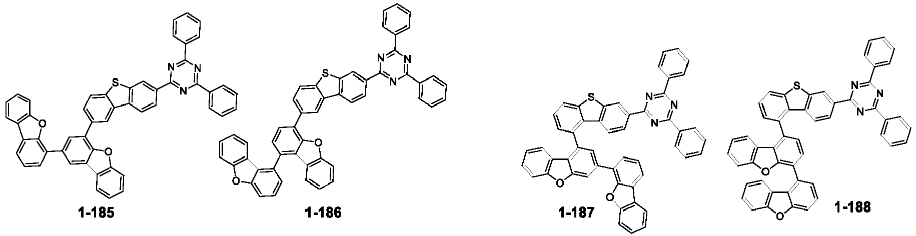

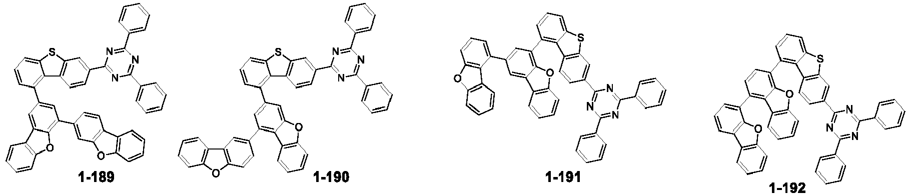

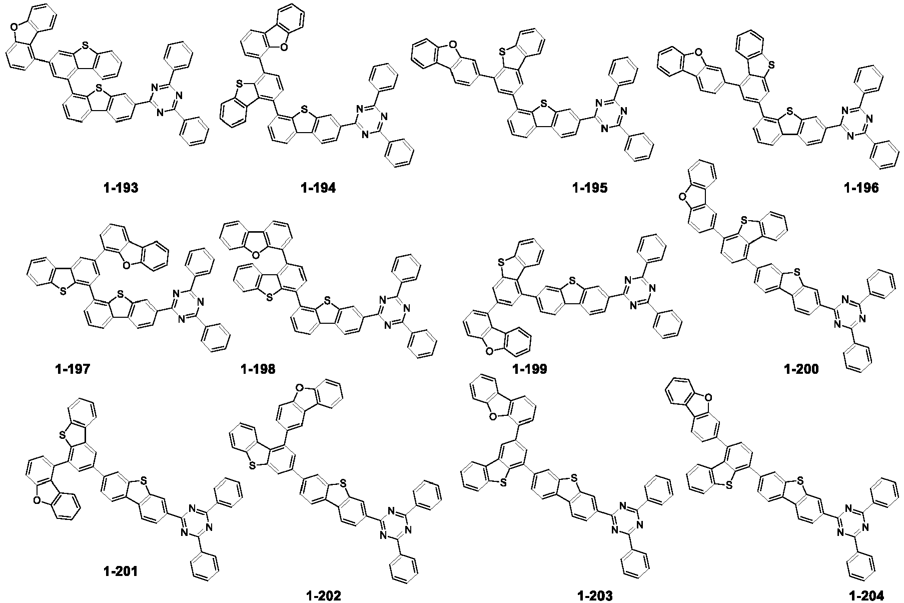

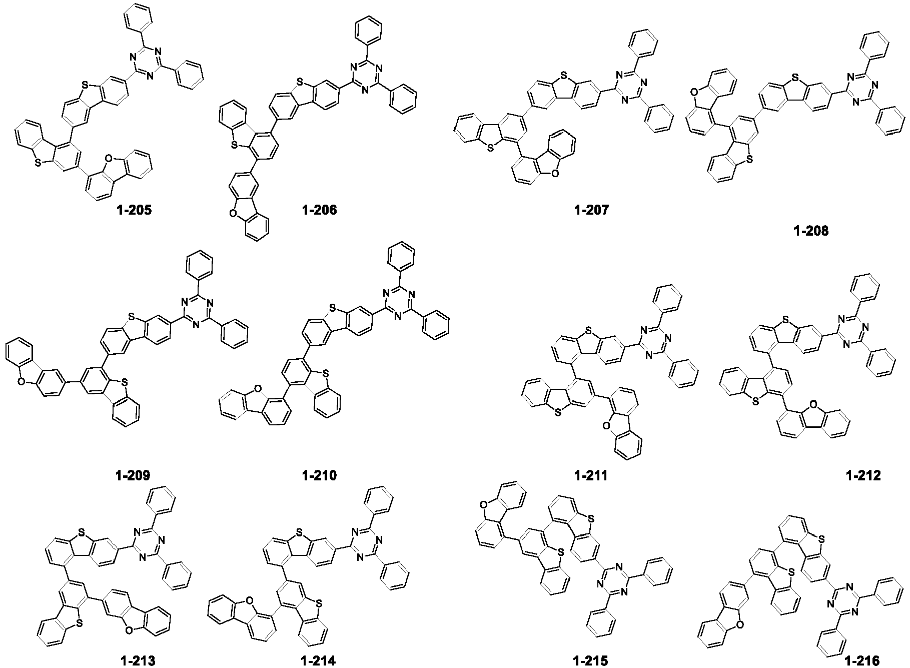

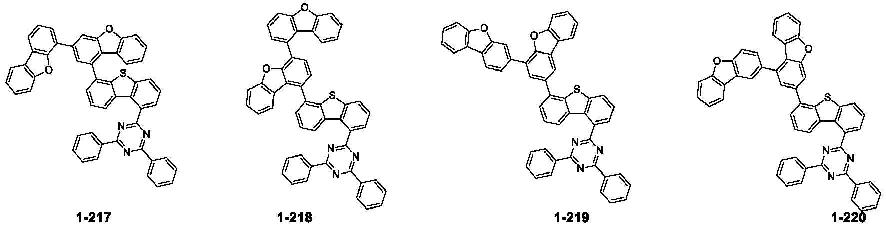

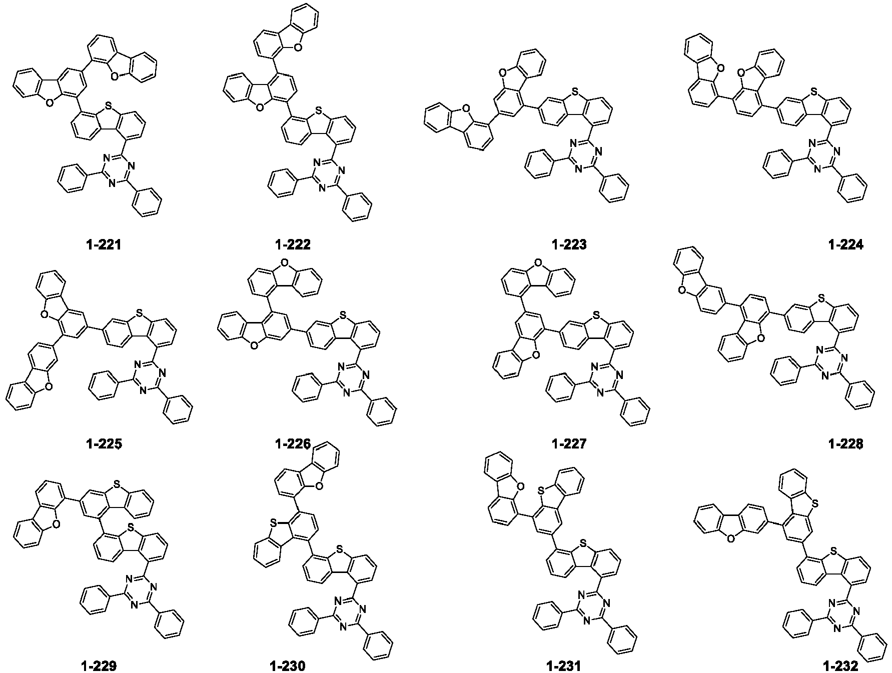

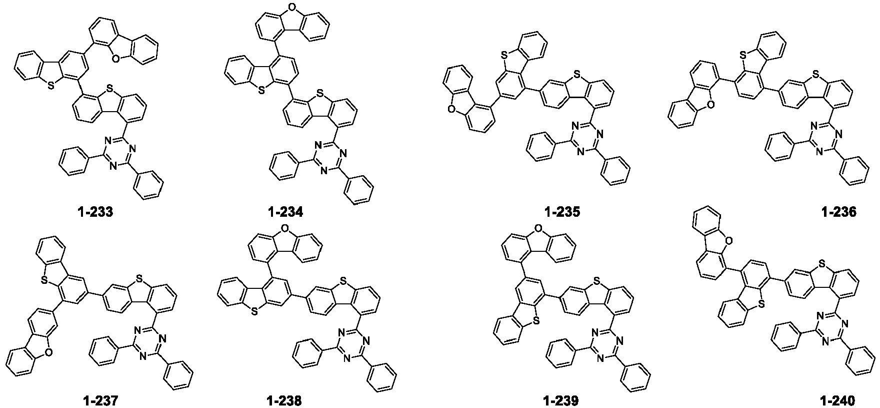

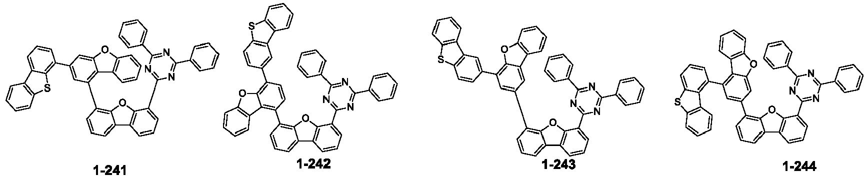

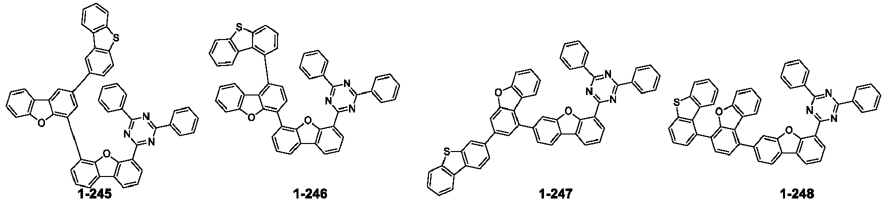

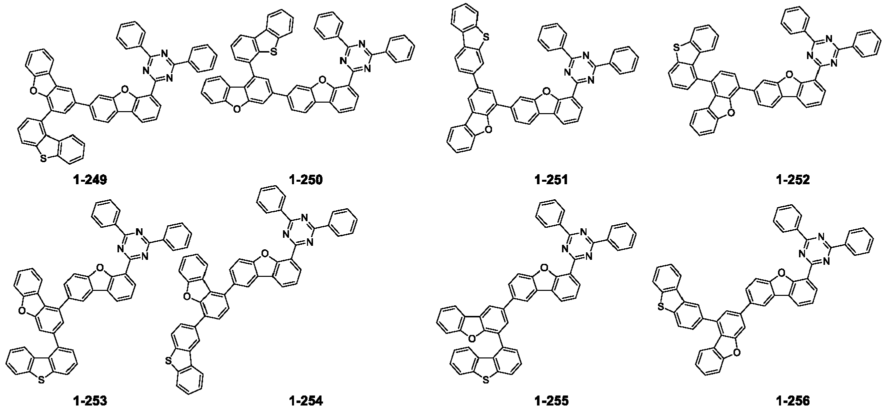

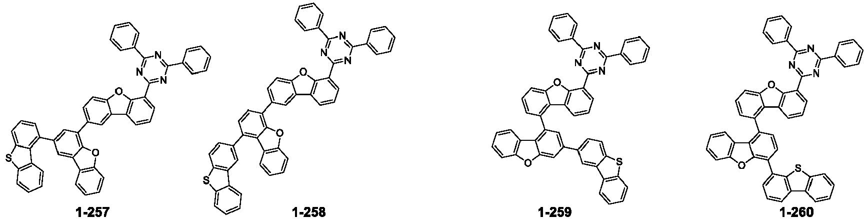

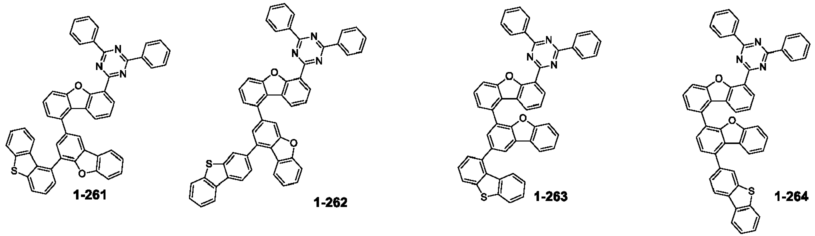

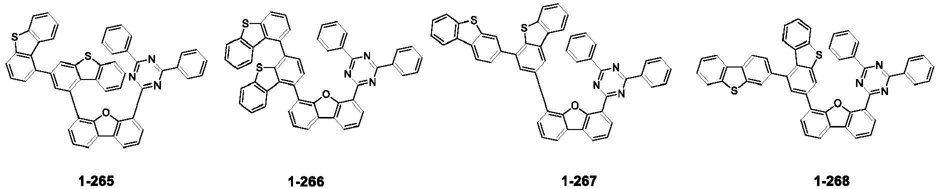

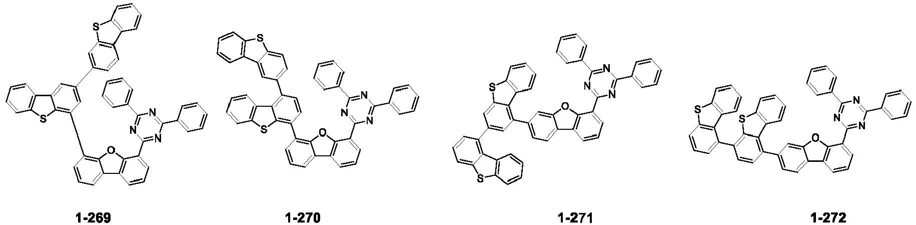

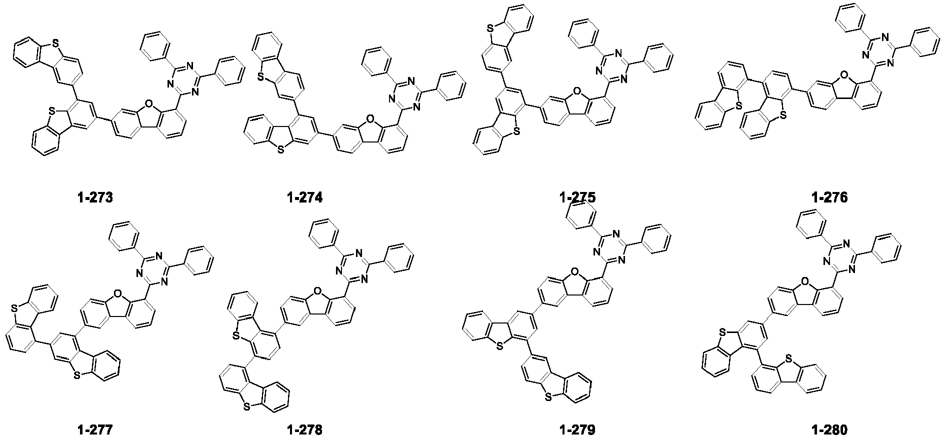

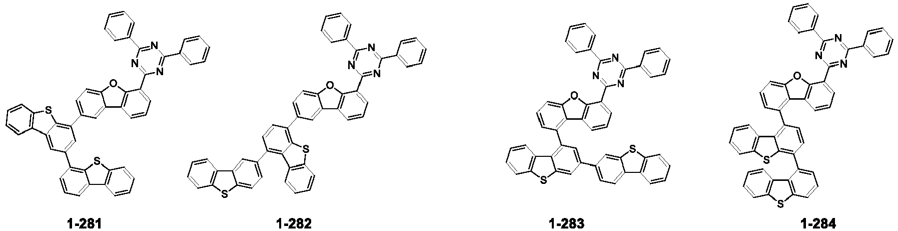

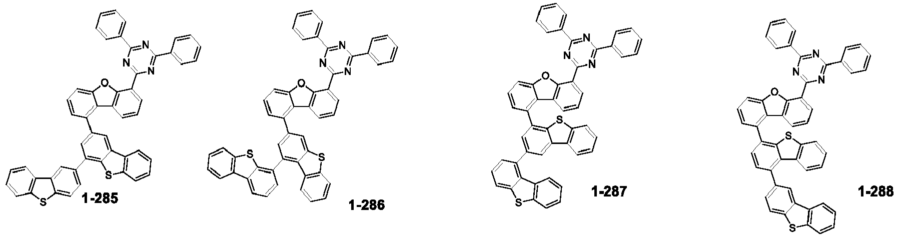

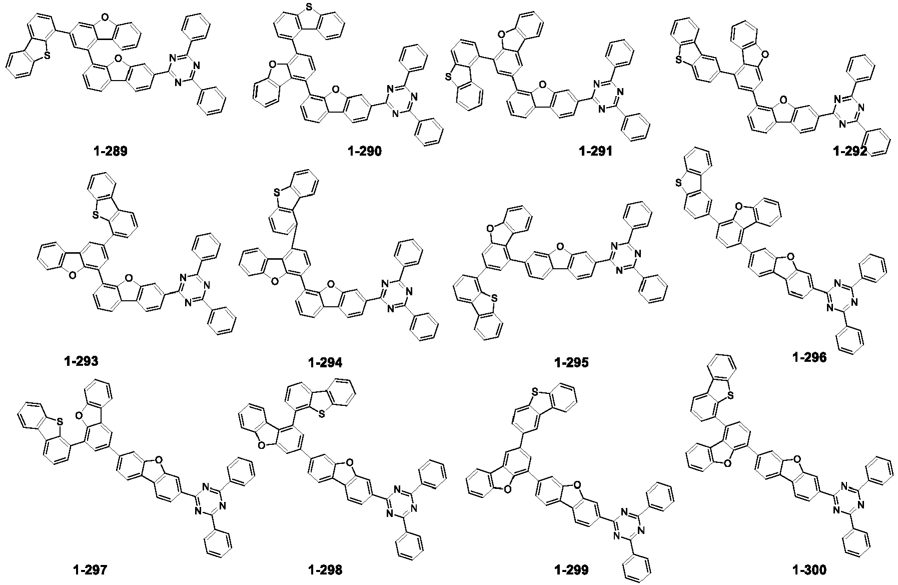

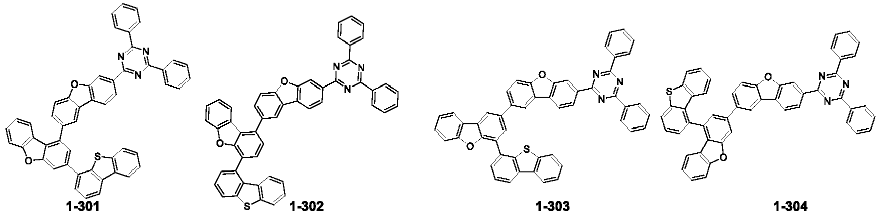

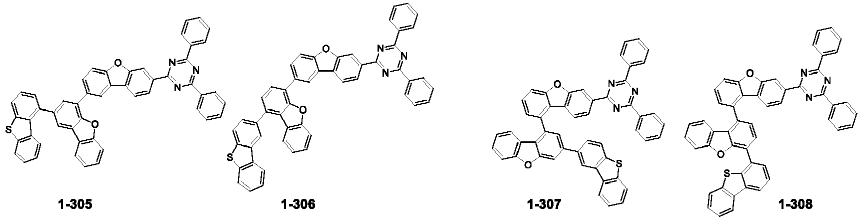

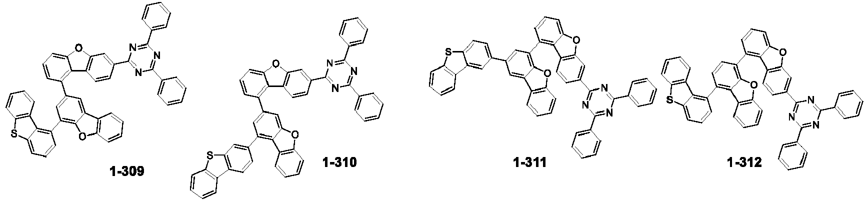

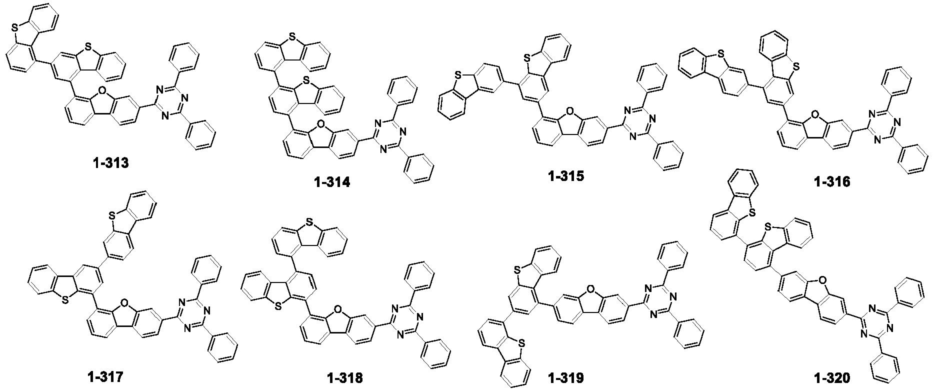

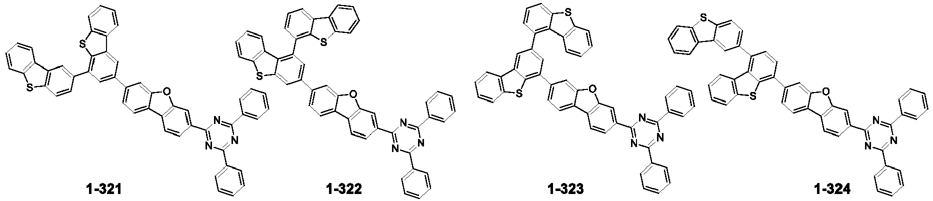

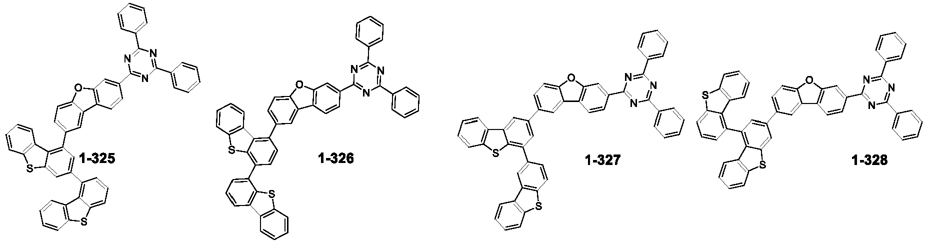

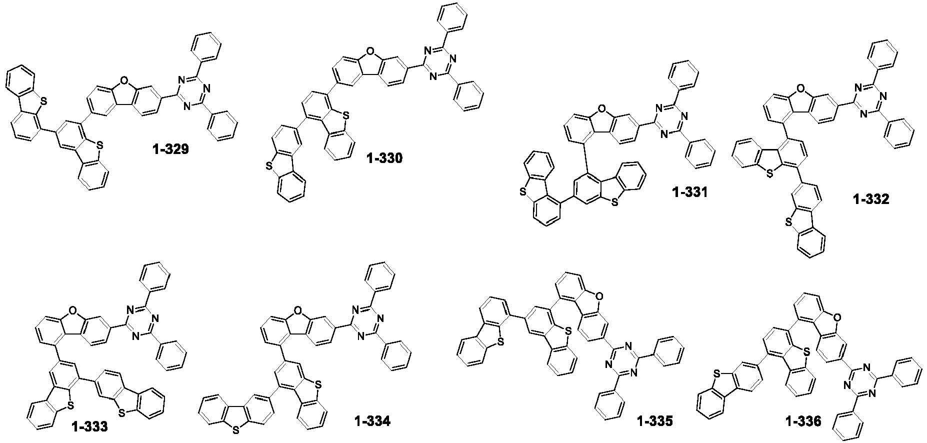

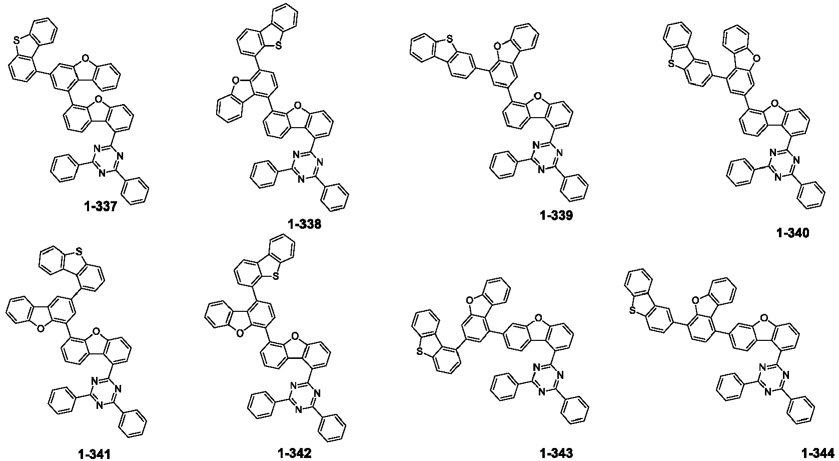

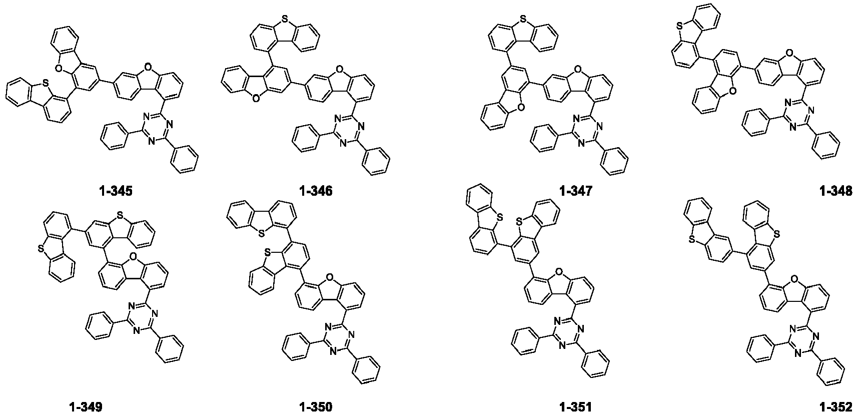

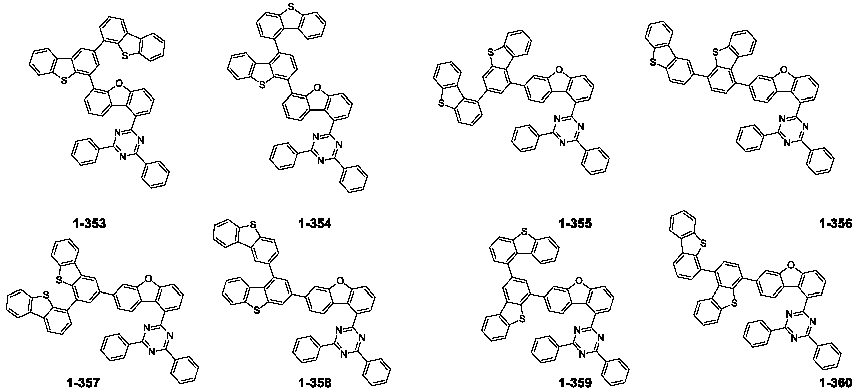

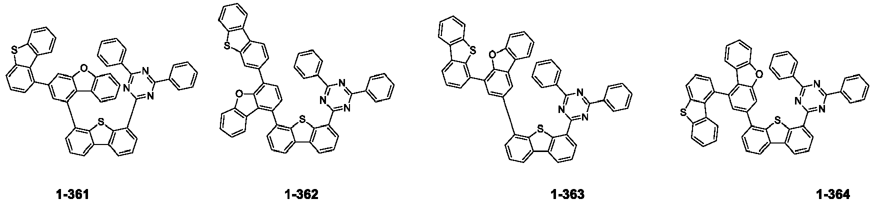

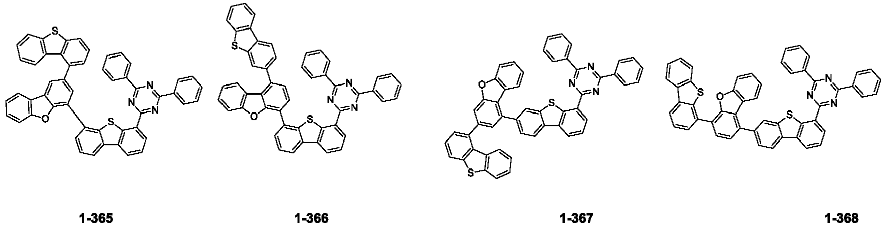

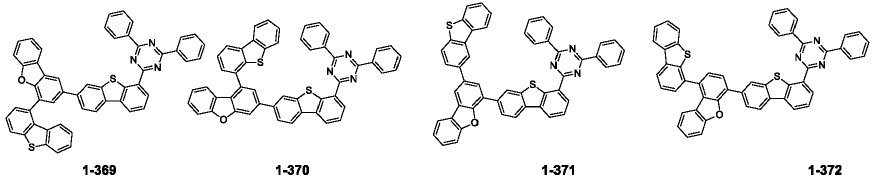

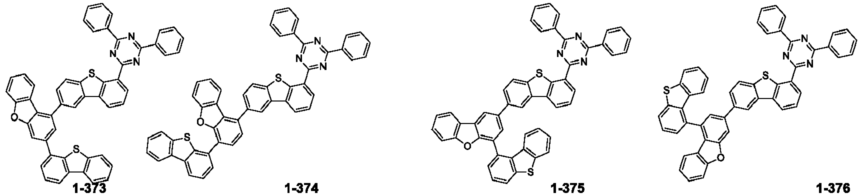

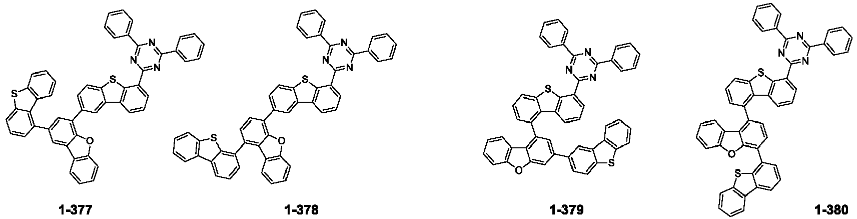

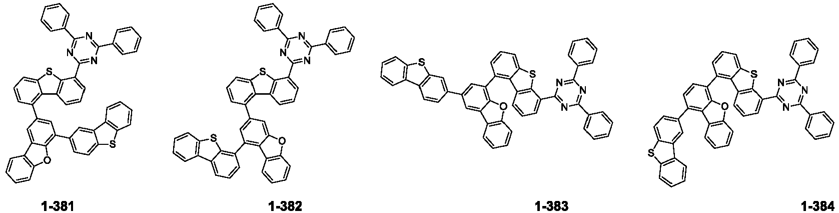

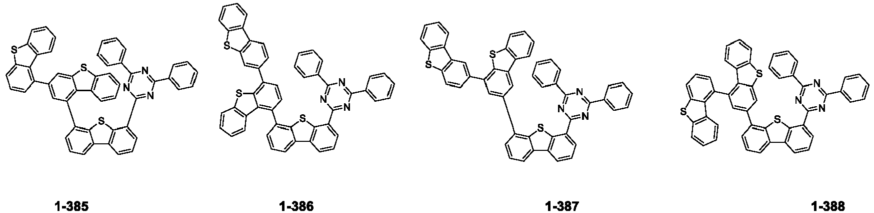

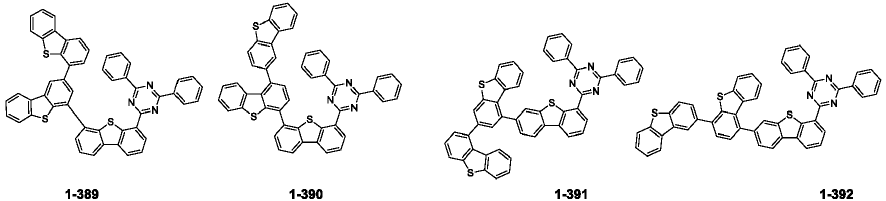

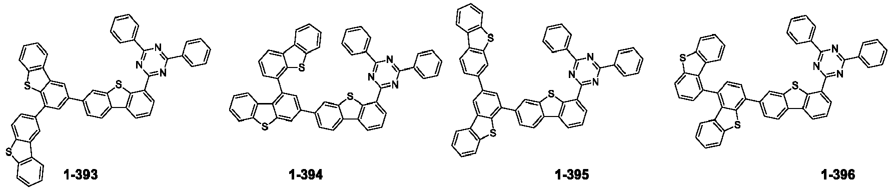

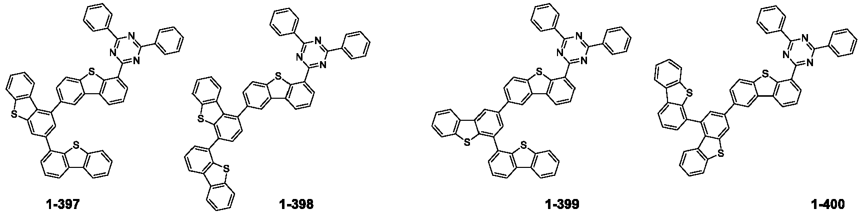

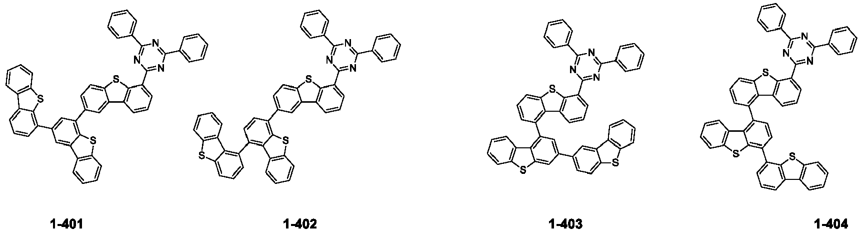

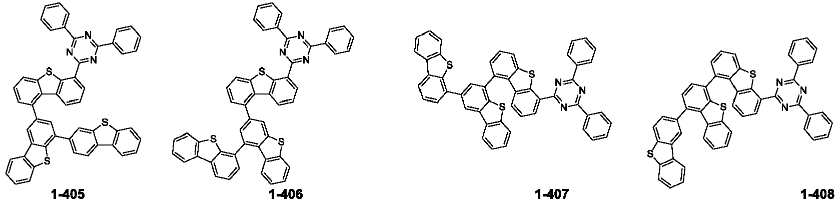

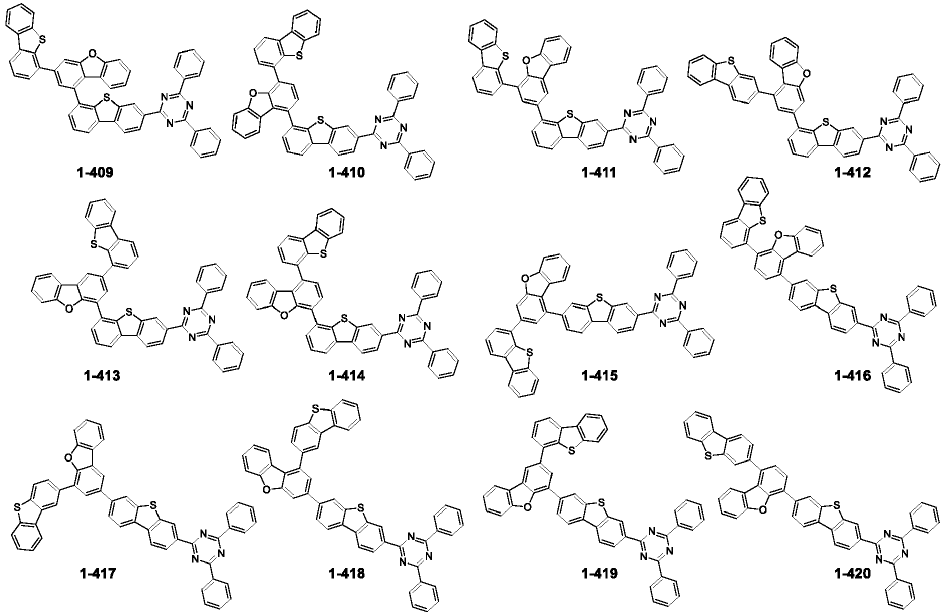

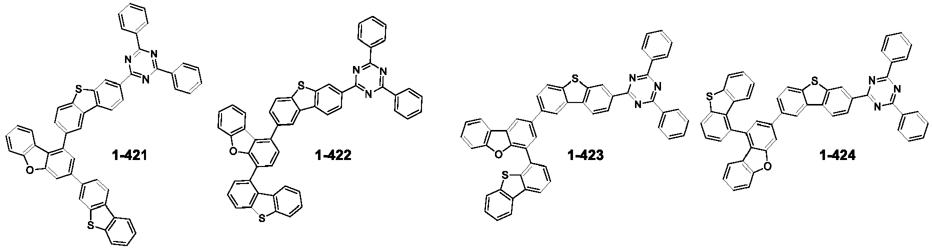

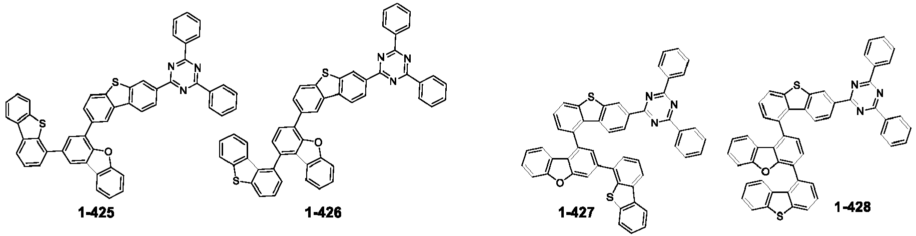

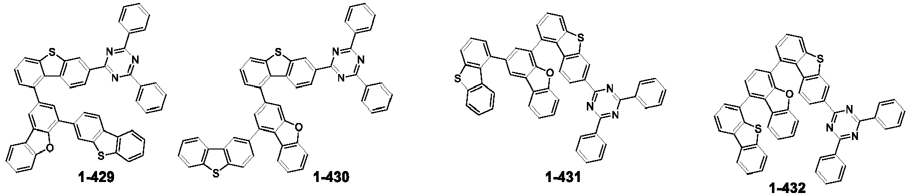

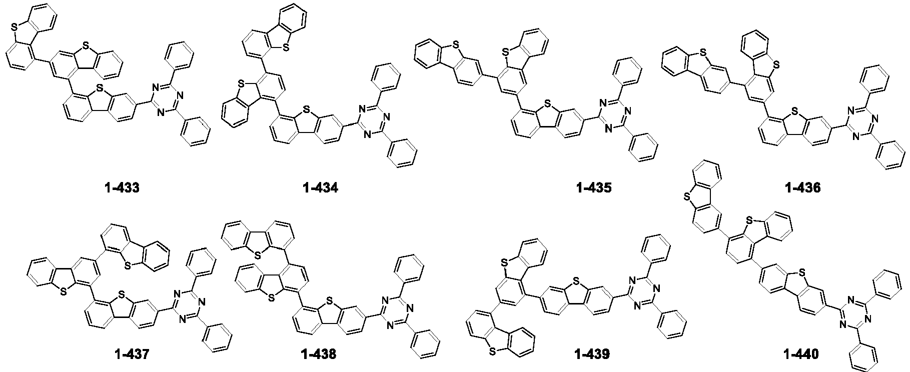

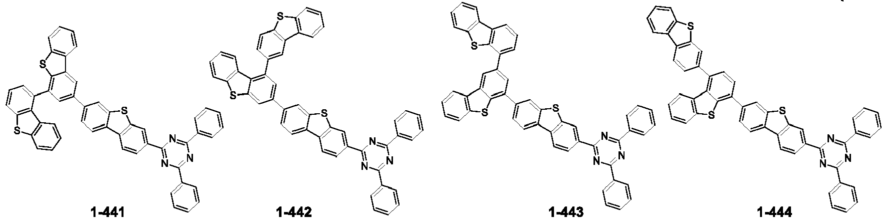

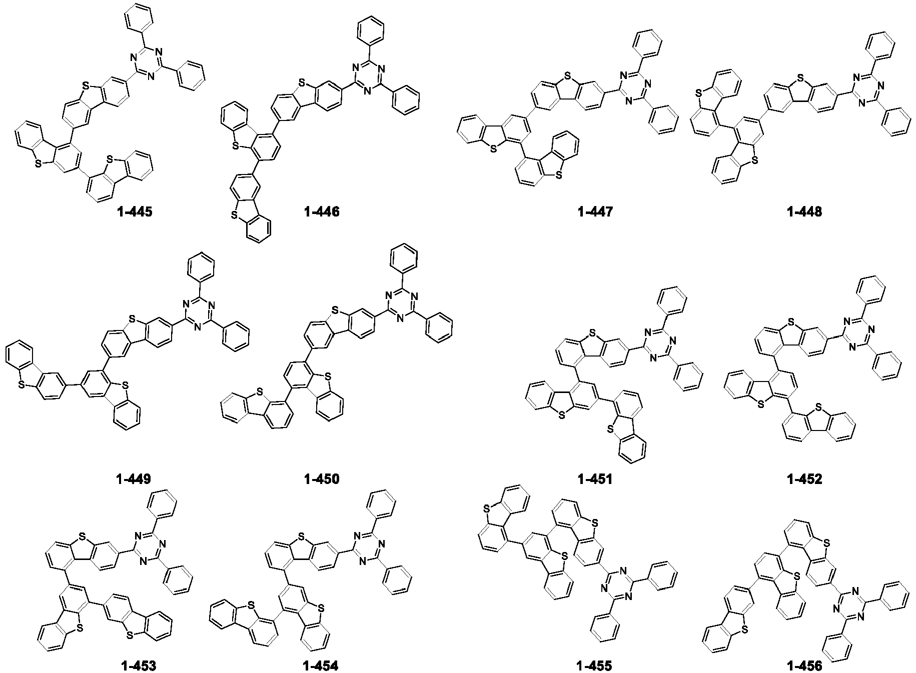

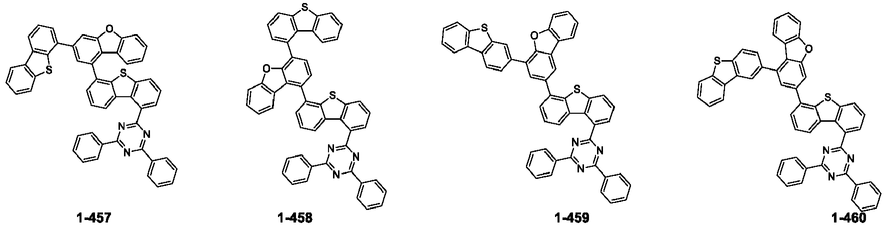

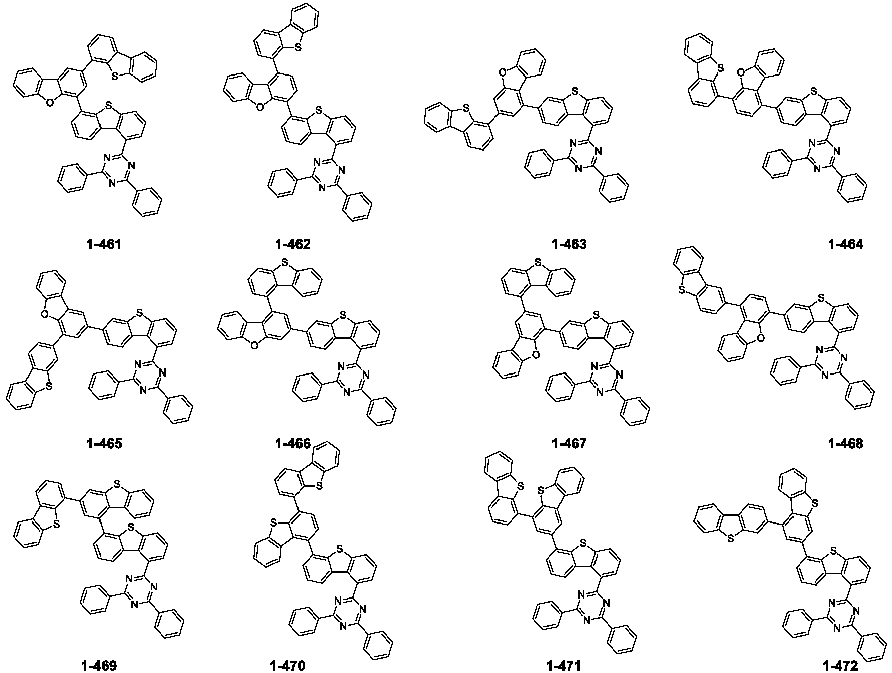

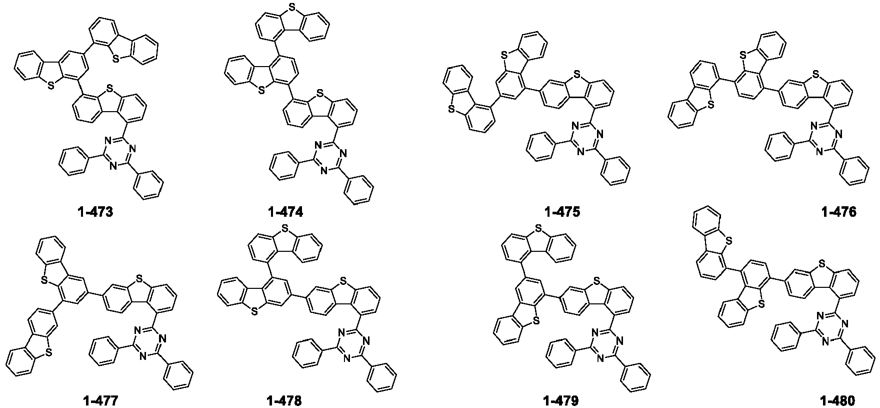

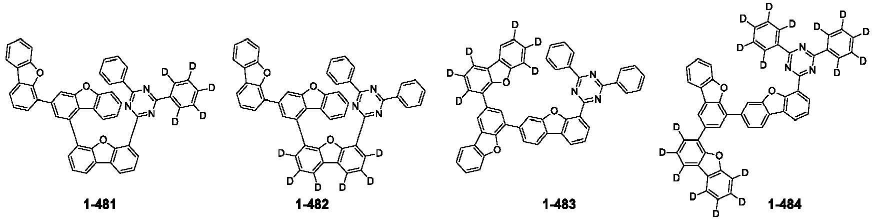

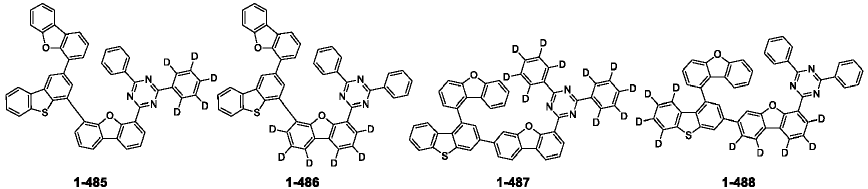

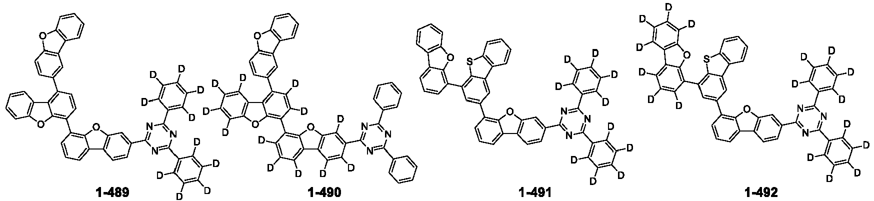

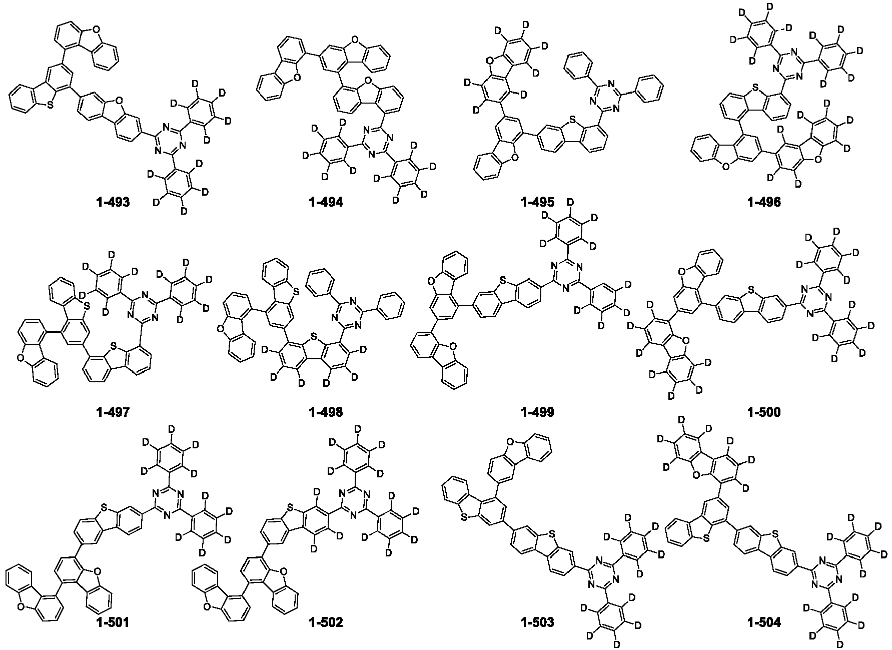

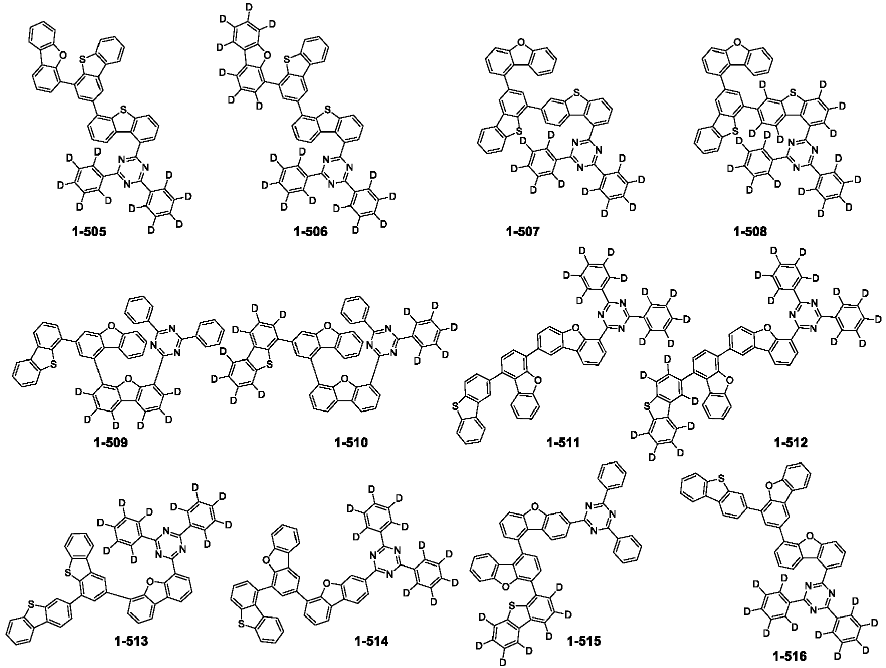

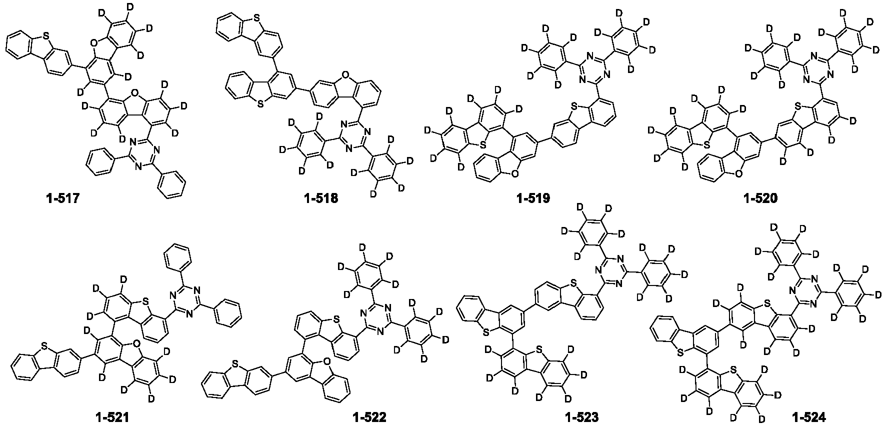

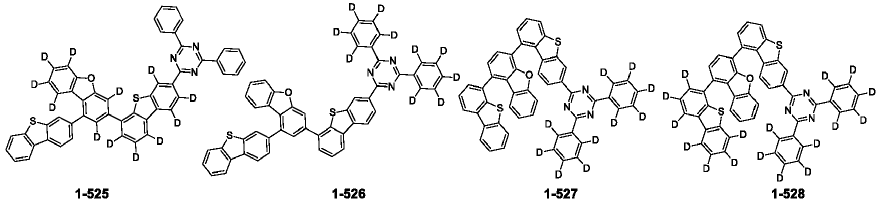

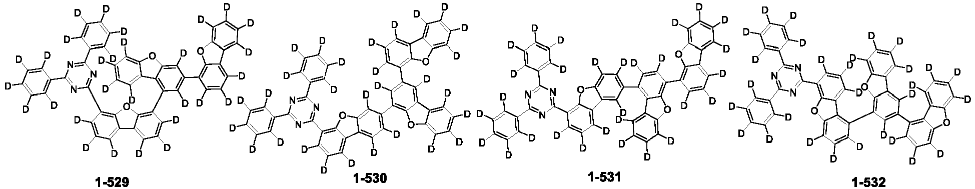

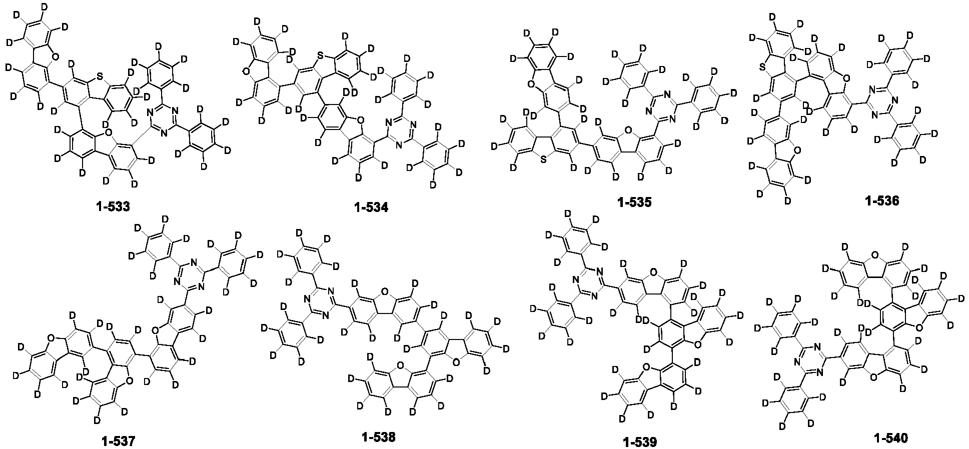

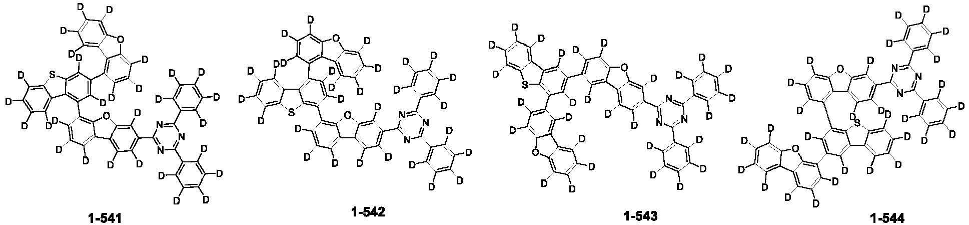

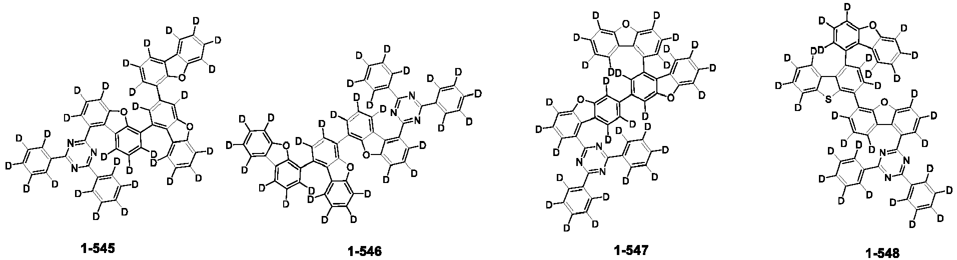

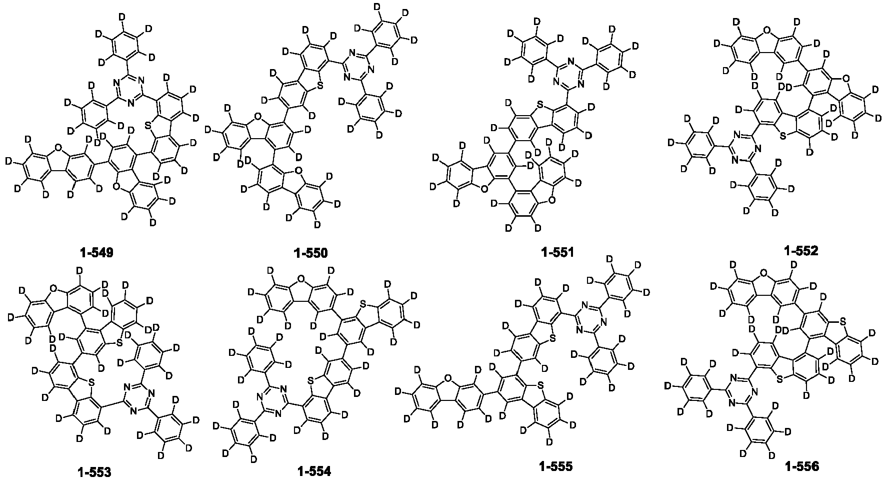

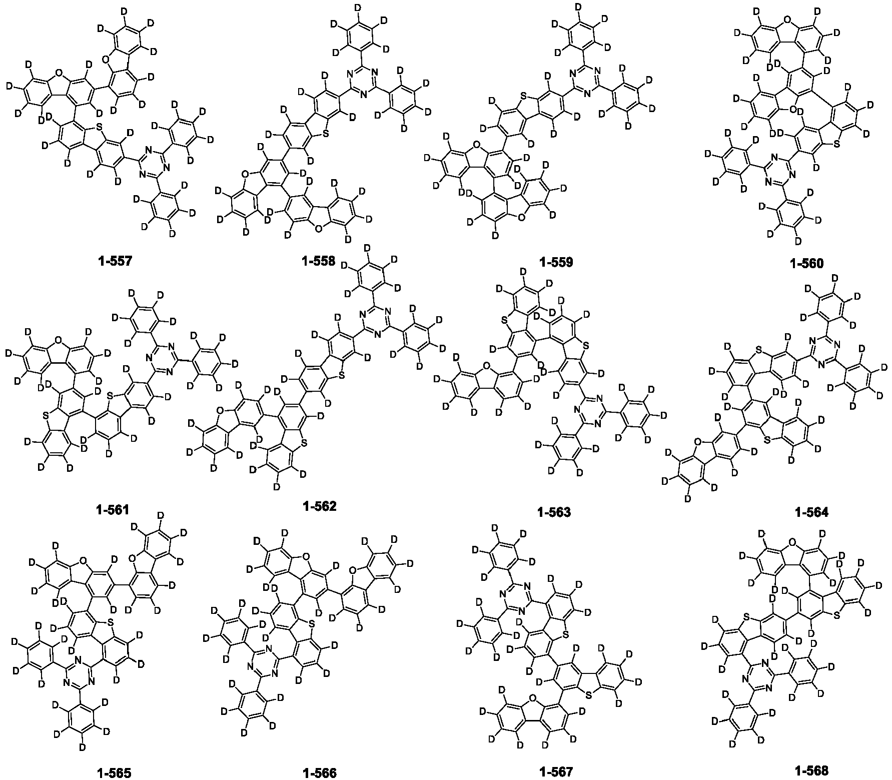

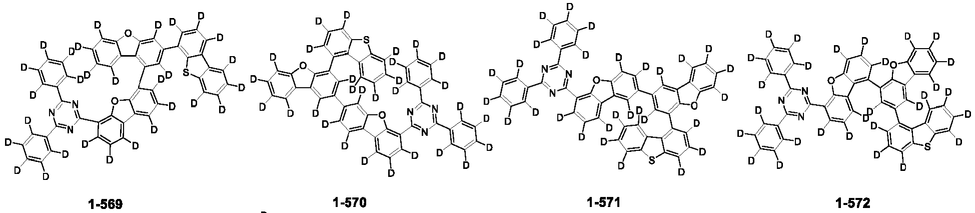

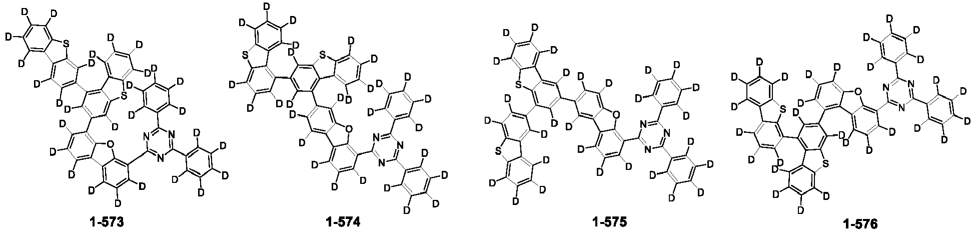

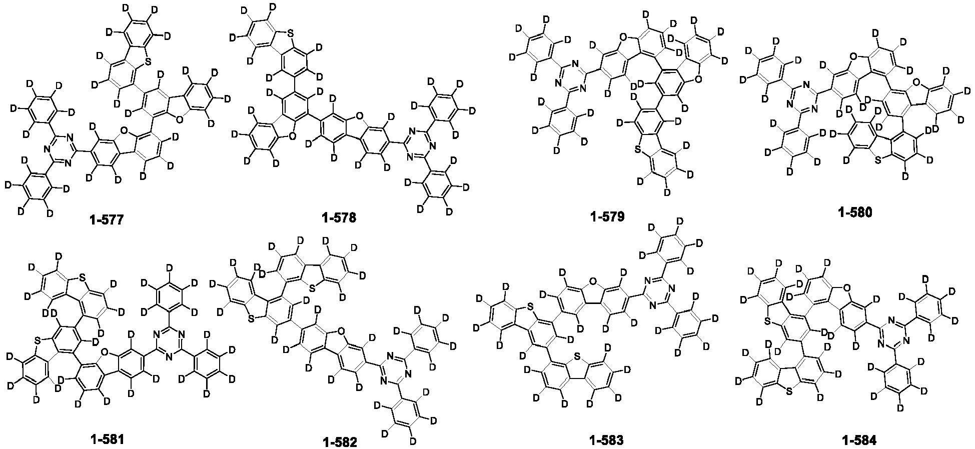

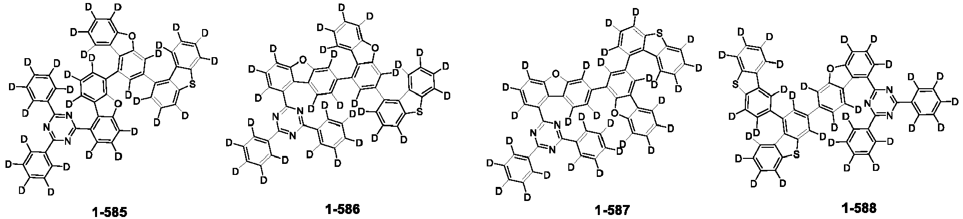

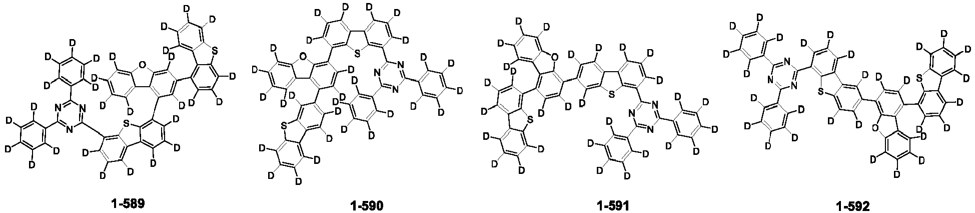

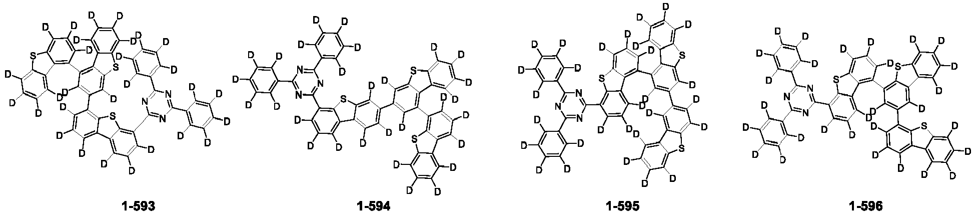

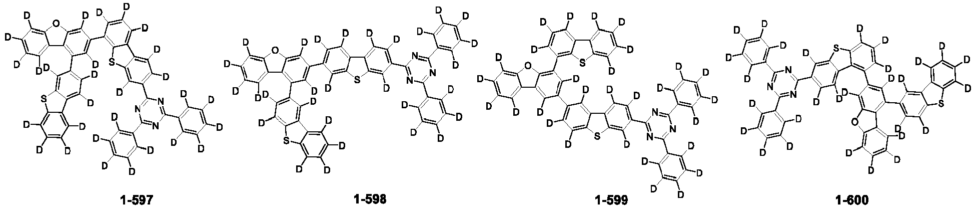

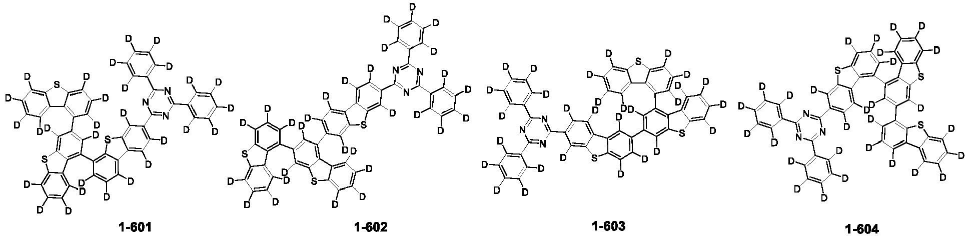

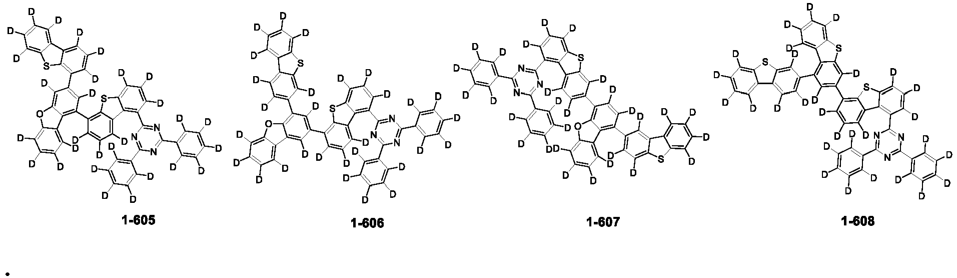

6. An organic light emitting device comprising:
a first electrode;
a second electrode provided to face the first electrode; and
an organic material layer having one or more layers provided between the first electrode and the second electrode,
wherein one or more layers of the organic material layer comprise the heterocyclic compound of any one of claims 1 to 5.

7. The organic light emitting device of claim 6, wherein the organic material layer comprising the heterocyclic compound further comprises a heterocyclic compound represented by the following Chemical Formula 2; or a heterocyclic compound represented by the following Chemical Formula 3: 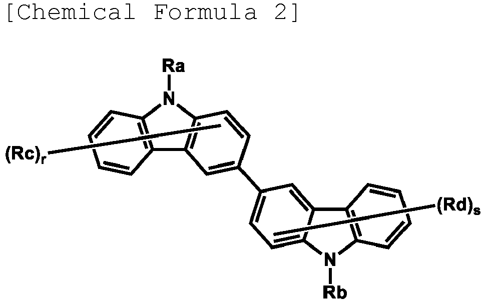 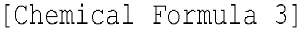 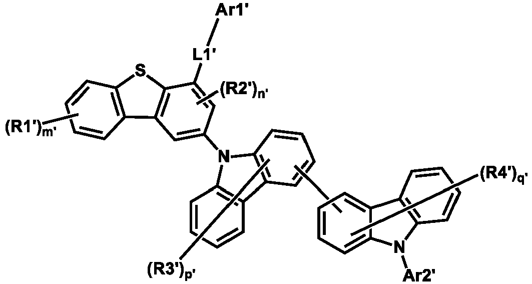 in Chemical Formulae 2 and 3,
Rc, Rd and R1' to R4' are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring,
Ra and Rb are the same as or different from each other, and are each independently a substituted or unsubstituted C6 to C60 aryl group; or a substituted or unsubstituted C2 to C60 heteroaryl group,
L1' is a direct bond; or a substituted or unsubstituted C6 to C60 arylene group,
Ar1' is a substituted or unsubstituted C6 to C60 aryl group; or a substituted or unsubstituted C2 to C60 heteroaryl group including at least one of S and O,
Ar2' is a substituted or unsubstituted C6 to C60 aryl group; or a substituted or unsubstituted C2 to C60 heteroaryl group,
m' is an integer from 0 to 4,
n' is an integer from 0 to 2,
p', q', r and s are an integer from 0 to 7, and
when m', p', q', n', r and s are each an integer of 2 or higher, substituents in the parenthesis are the same as or different from each other.

8. The organic light emitting device of claim 7, wherein the heterocyclic compound represented by Chemical Formula 2 and the heterocyclic compound represented by Chemical Formula 3 have a deuterium content of 0% to 100%.

9. The organic light emitting device of claim 7, wherein the heterocyclic compound represented by Chemical Formula 2 is any one selected from the following compounds:

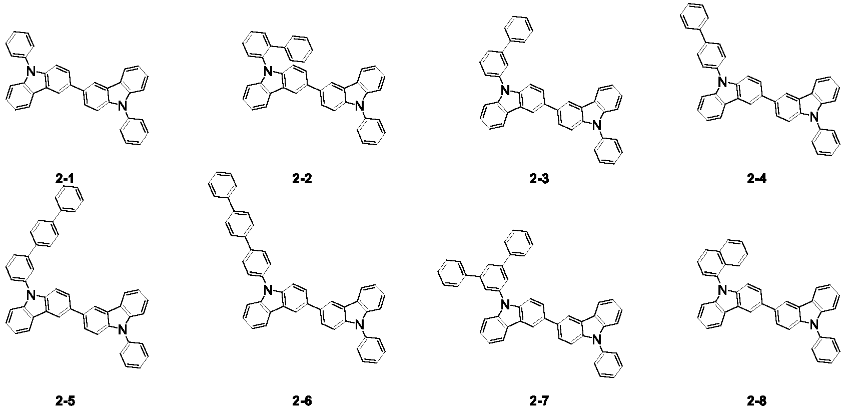

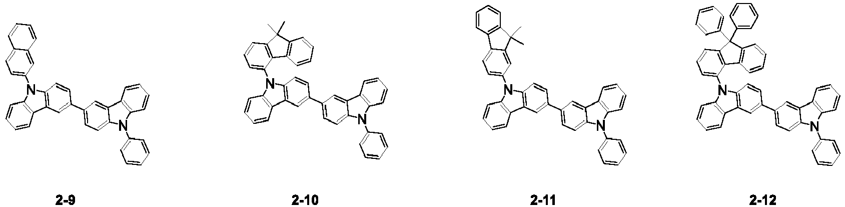

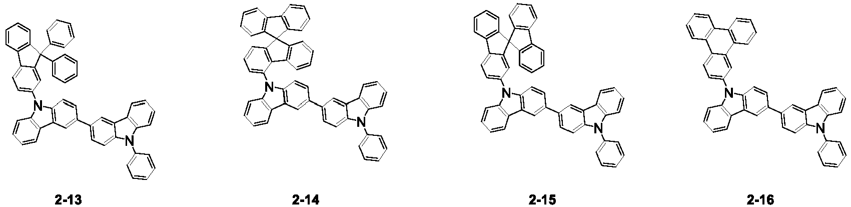

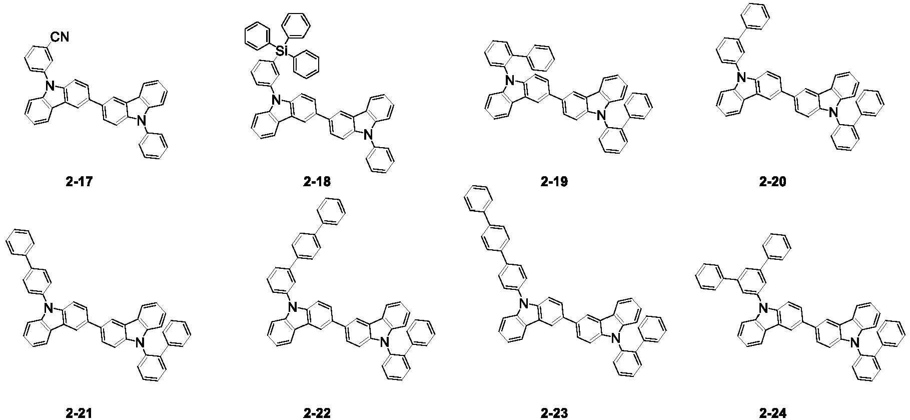

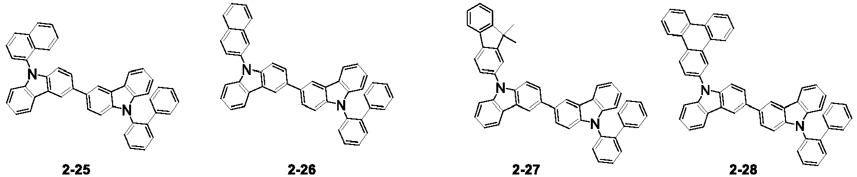

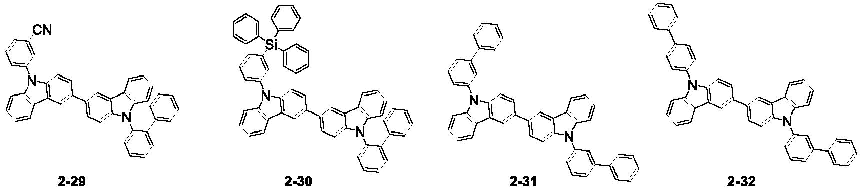

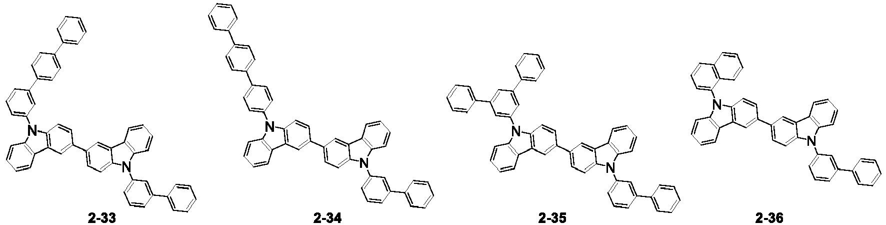

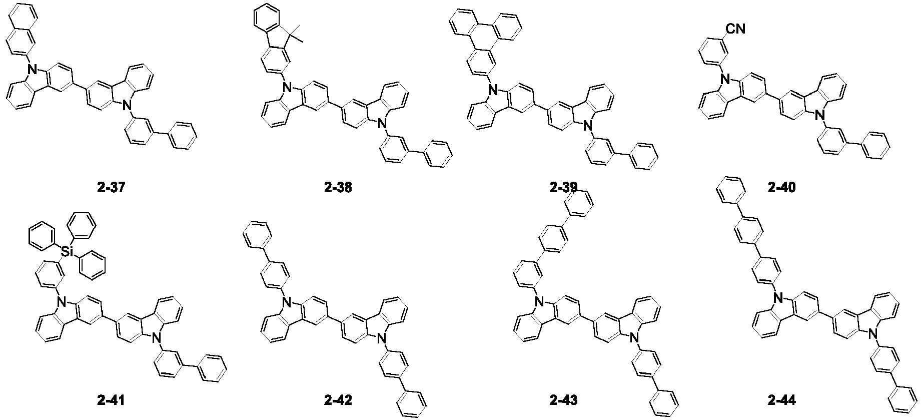

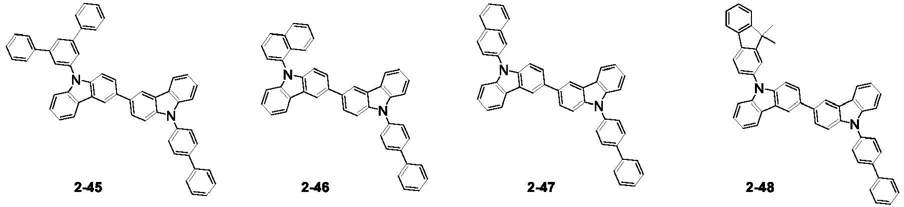

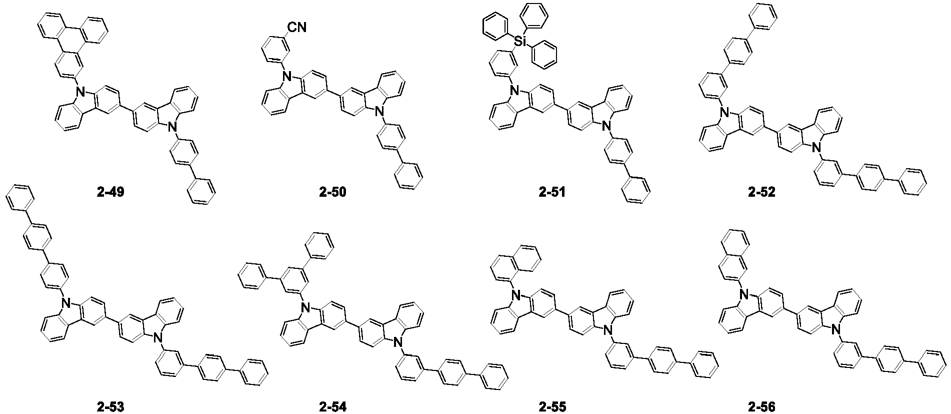

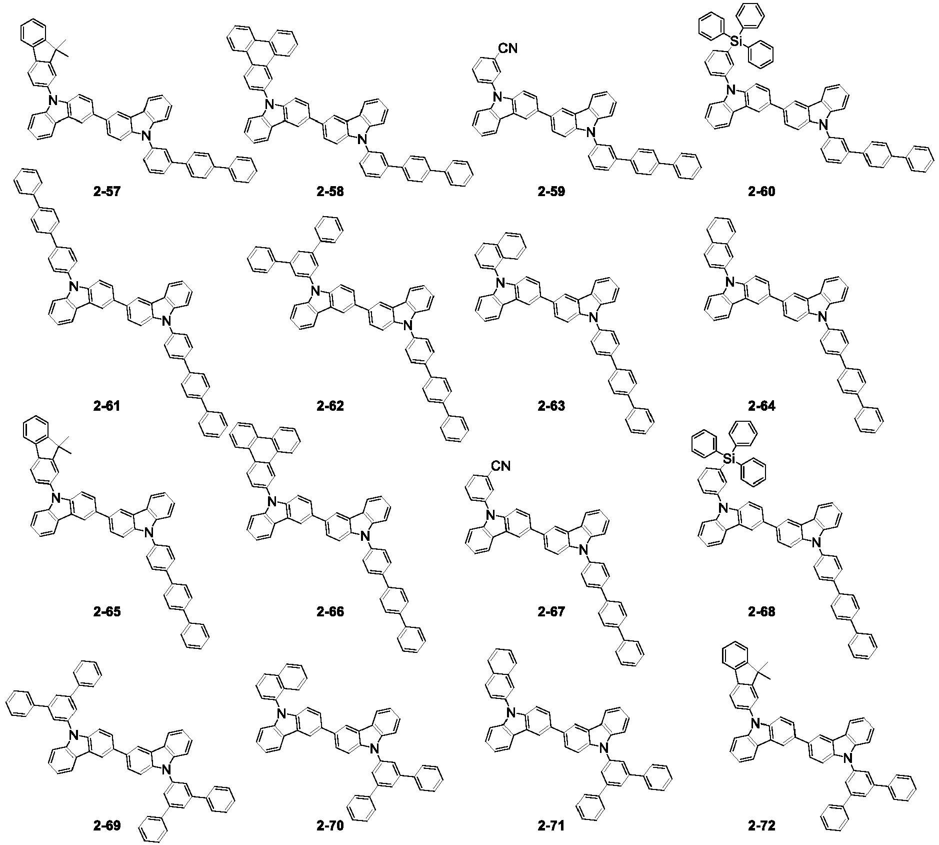

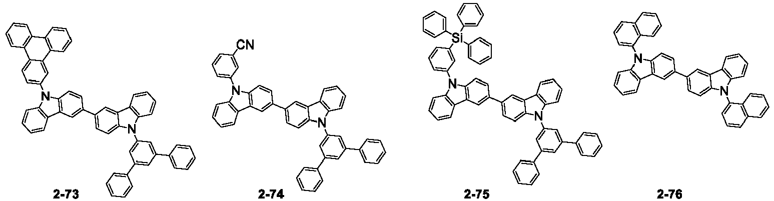

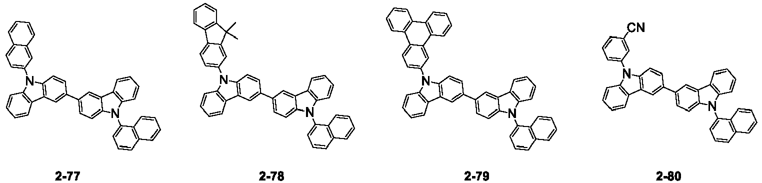

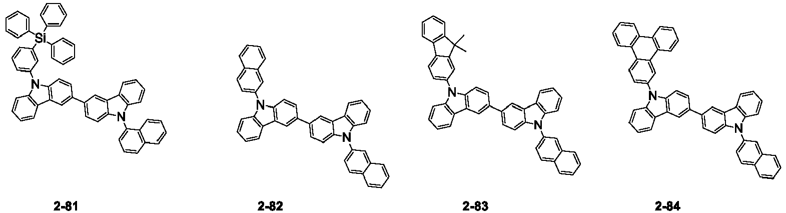

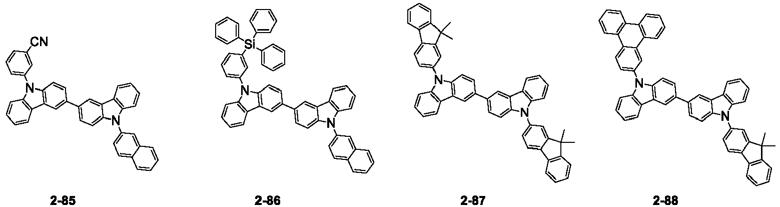

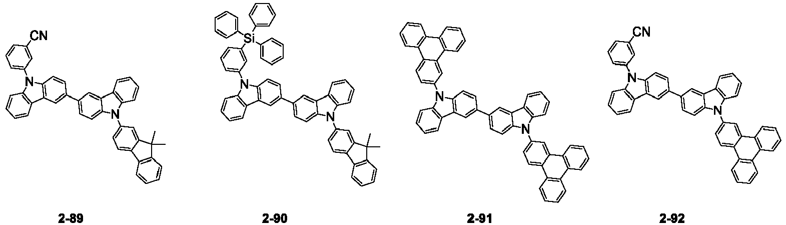

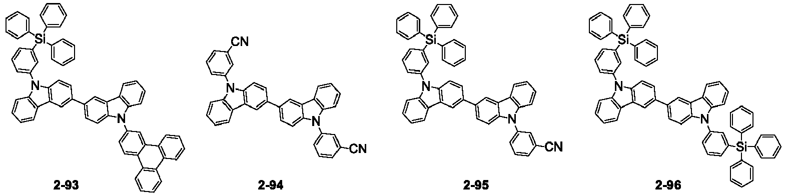

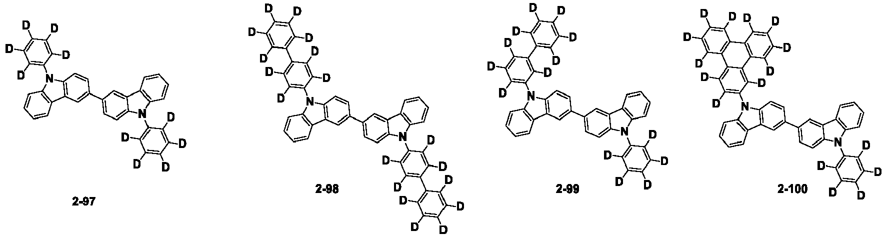

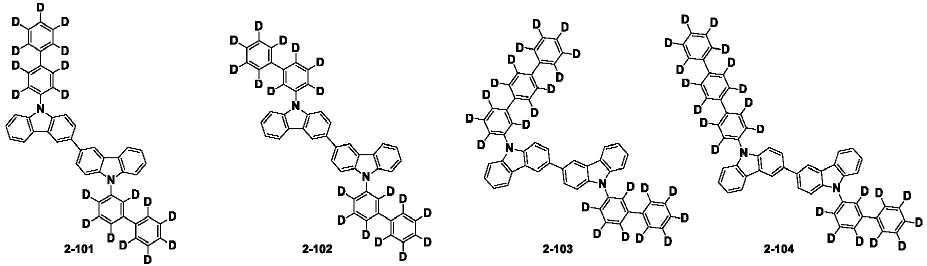

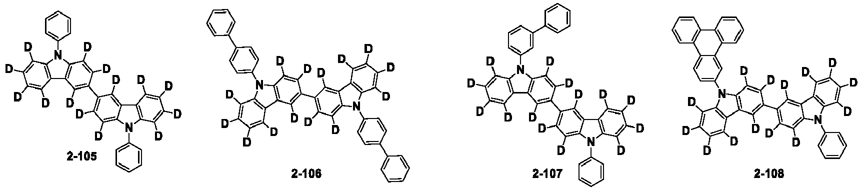

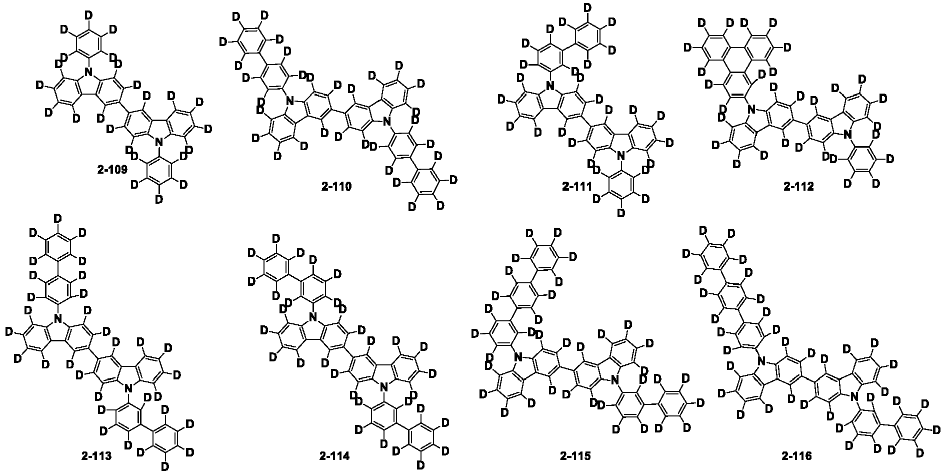

10. The organic light emitting device of claim 7, wherein the heterocyclic compound represented by Chemical Formula 3 is any one selected from the following compounds:

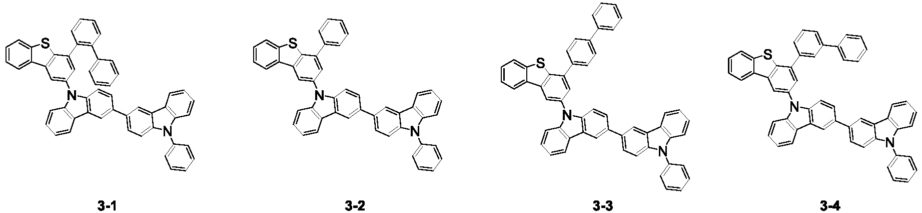

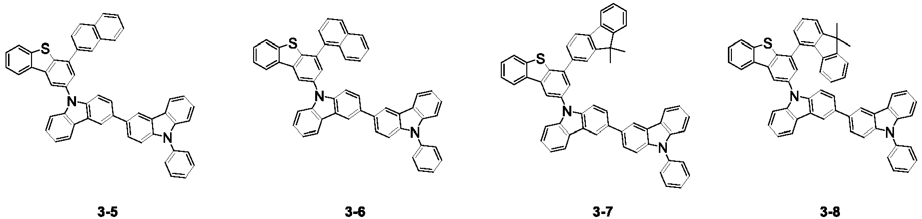

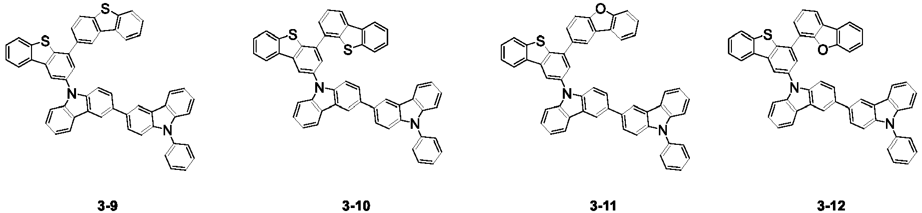

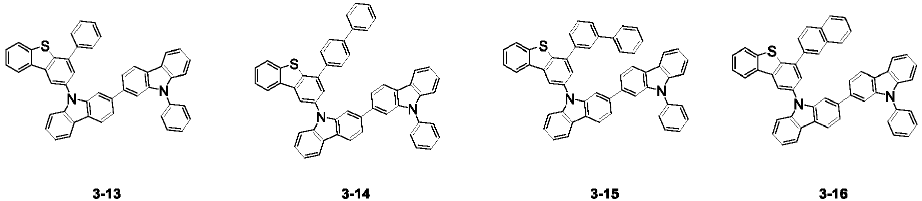

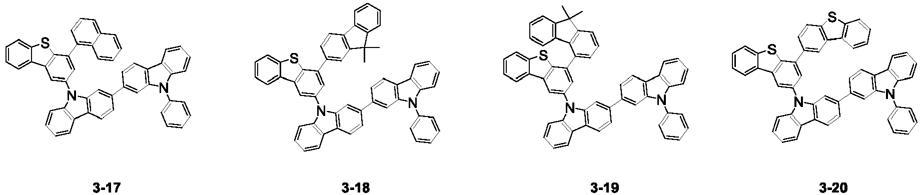

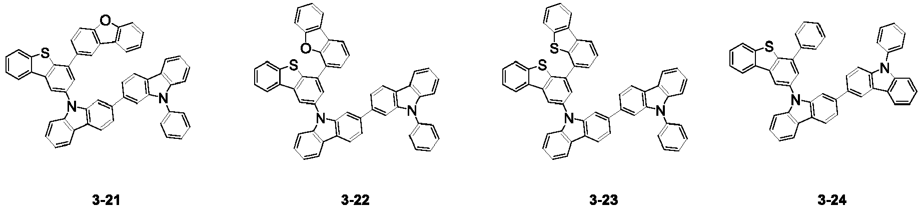

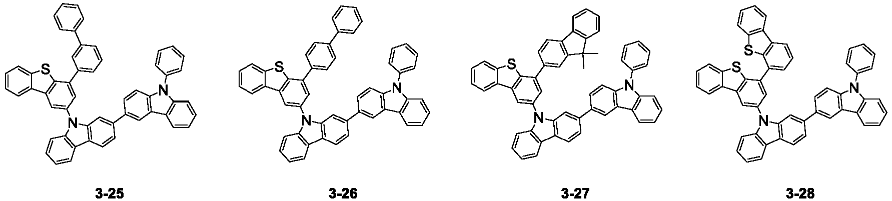

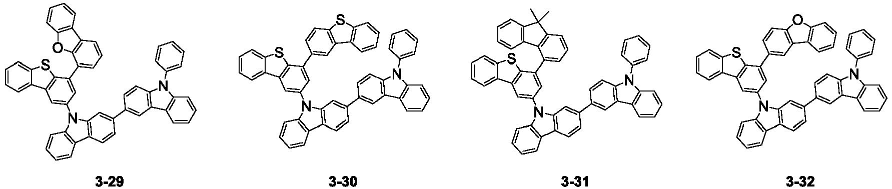

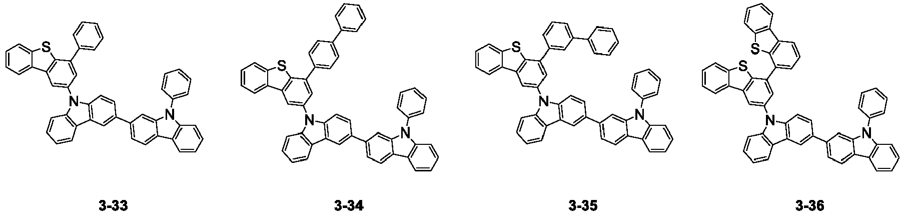

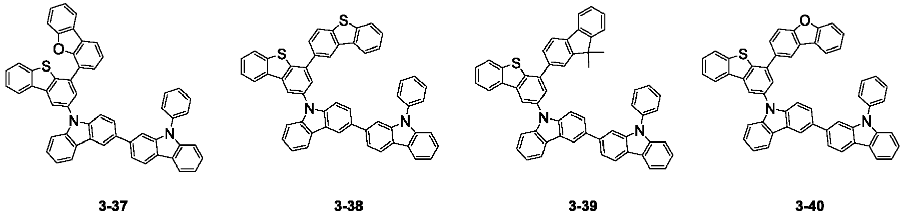

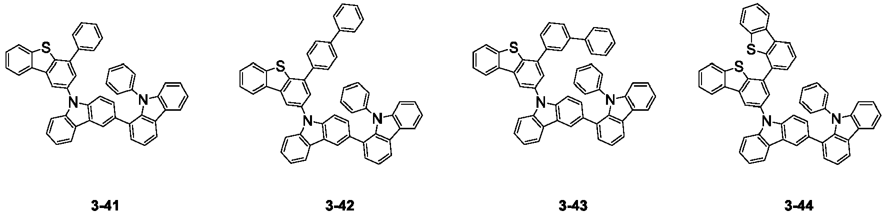

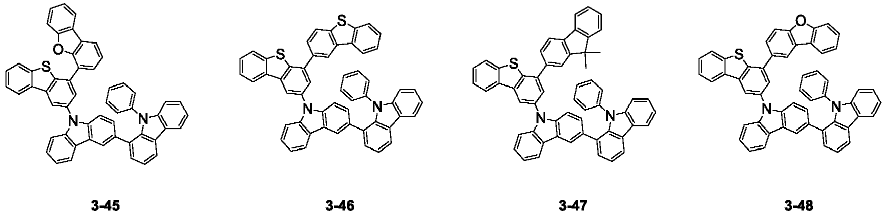

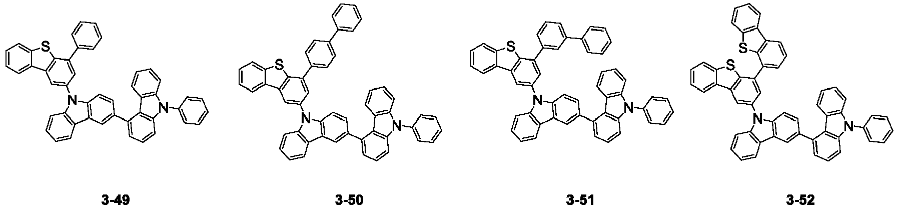

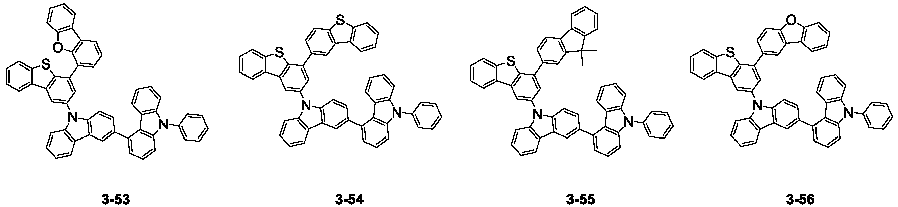

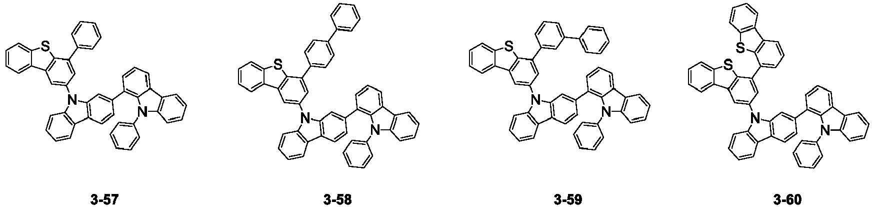

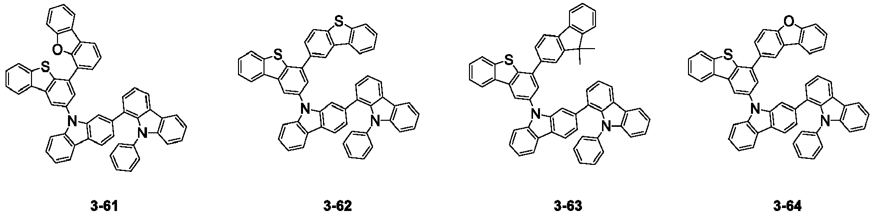

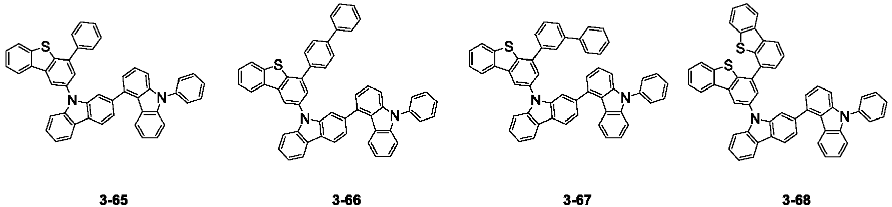

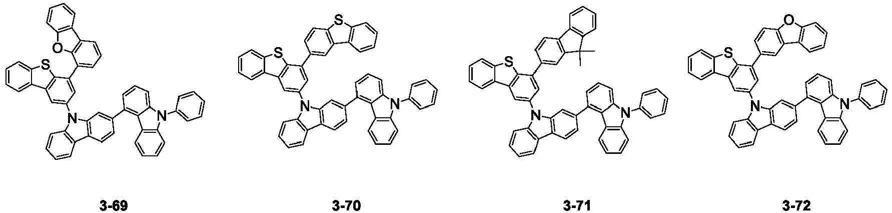

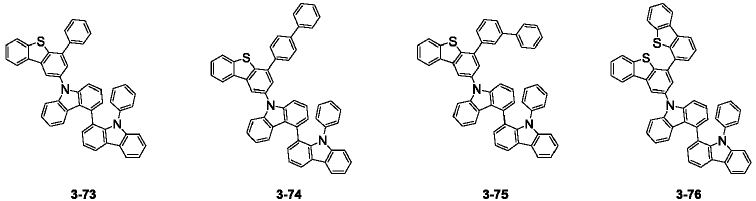

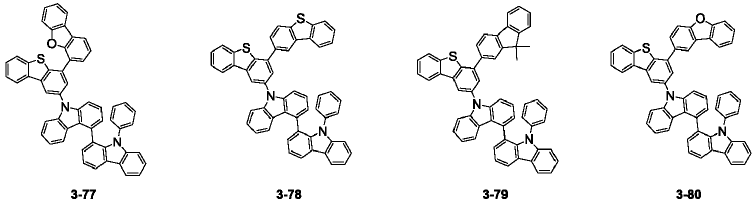

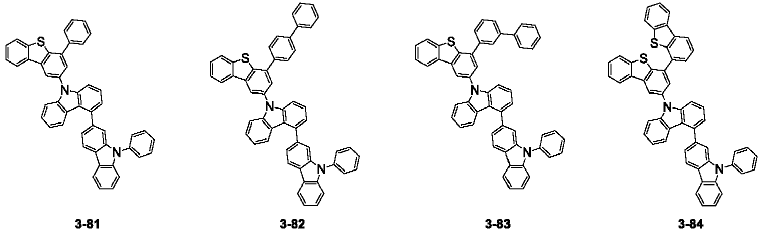

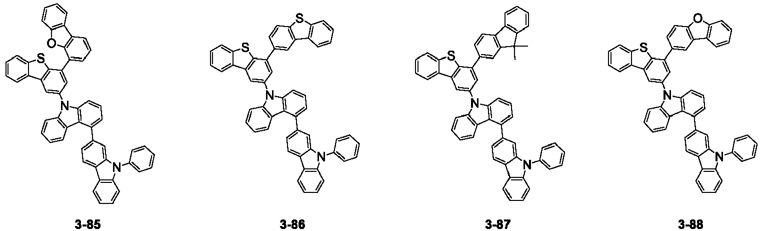

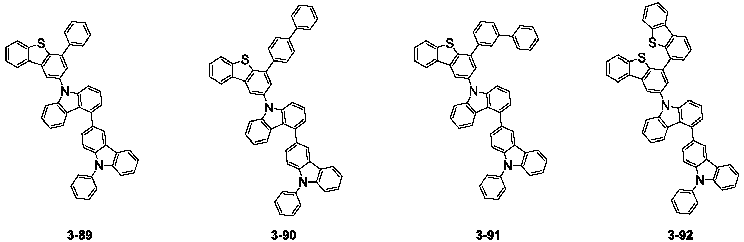

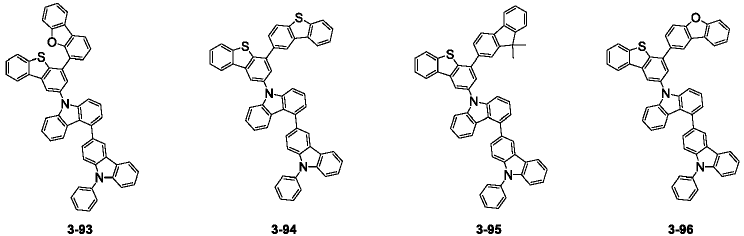

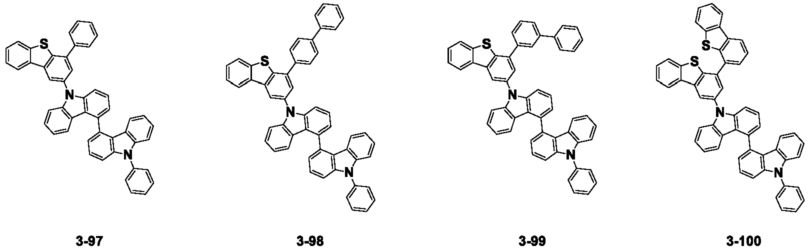

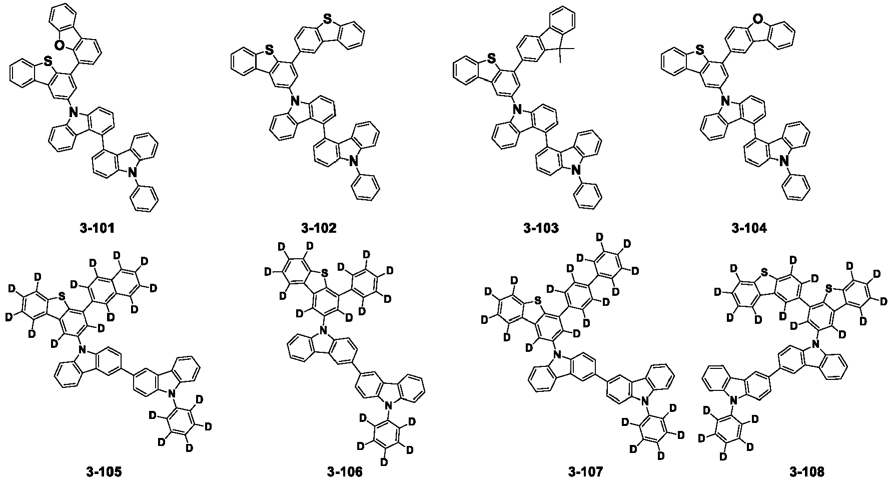

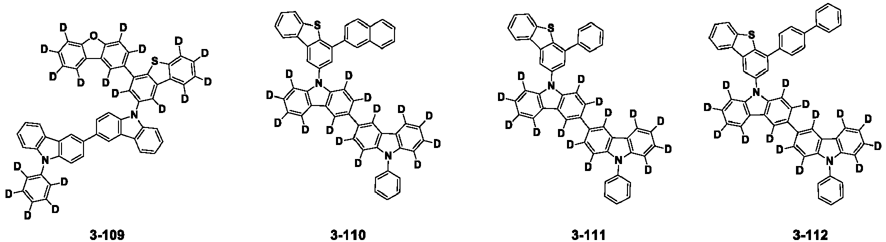

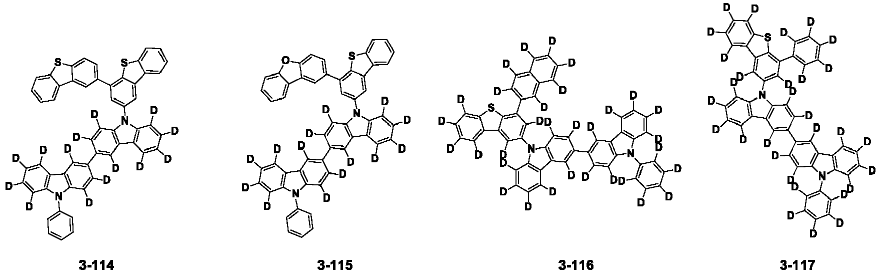

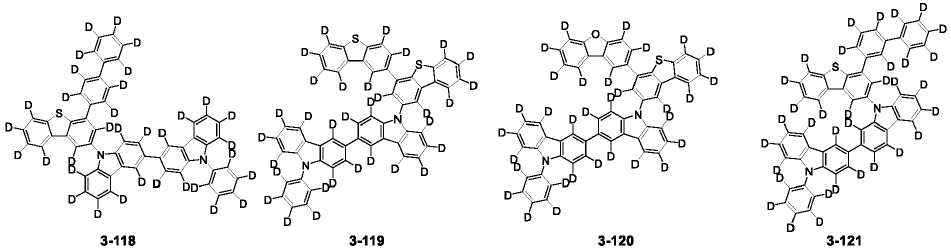

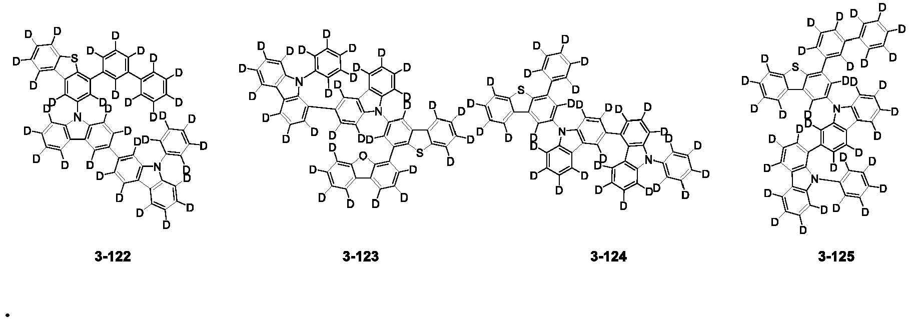

11. The organic light emitting device of claim 6, wherein the organic material layer comprises a light emitting layer and the light emitting layer comprises the heterocyclic compound.

12. The organic light emitting device of claim 6, wherein the organic material layer comprises a light emitting layer, the light emitting layer comprises a host material, and the host material comprises the heterocyclic compound.

13. The organic light emitting device of claim 6, further comprising one or two or more layers selected from the group consisting of a light emitting layer, a hole injection layer, a hole transport layer, an electron injection layer, an electron transport layer, an electron blocking layer and a hole blocking layer.

14. A composition for an organic material layer of an organic light emitting device, comprising the heterocyclic compound according to any one of claims 1 to 5; and a heterocyclic compound represented by the following Chemical Formula 2 or a heterocyclic compound represented by the following Chemical Formula 3: 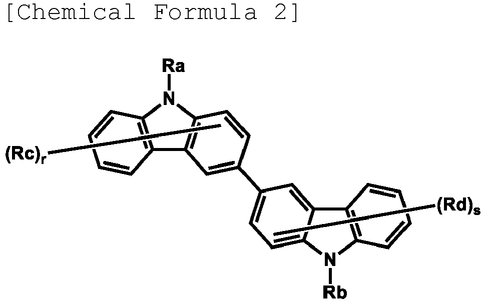 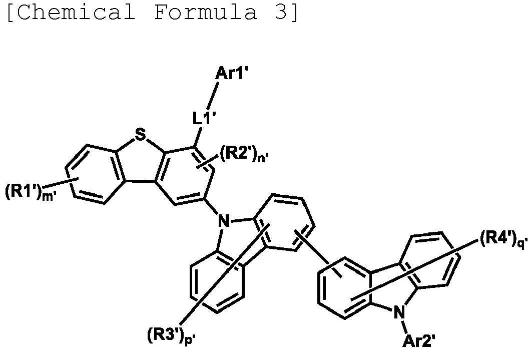 in Chemical Formulae 2 and 3,
Rc, Rd and R1' to R4' are the same as or different from each other, and are each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted C6 to C60 aromatic hydrocarbon ring or a substituted or unsubstituted C2 to C60 hetero ring,
Ra and Rb are the same as or different from each other, and are each independently a substituted or unsubstituted C6 to C60 aryl group; or a substituted or unsubstituted C2 to C60 heteroaryl group,
L1' is a direct bond; or a substituted or unsubstituted C6 to C60 arylene group,
Ar1' is a substituted or unsubstituted C6 to C60 aryl group; or a substituted or unsubstituted C2 to C60 heteroaryl group including at least one of S and O,
Ar2' is a substituted or unsubstituted C6 to C60 aryl group; or a substituted or unsubstituted C2 to C60 heteroaryl group,
m' is an integer from 0 to 4,
n' is an integer from 0 to 2,
p', q', r and s are an integer from 0 to 7, and
when m', p', q', n', r and s are each an integer of 2 or higher, substituents in the parenthesis are the same as or different from each other.

15. The composition for the organic material layer of the organic light emitting device of claim 14, wherein a weight ratio of the heterocyclic compound : the heterocyclic compound represented by Chemical Formula 2 or the heterocyclic compound represented by Chemical Formula 3 in the composition is 1 : 10 to 10 : 1.
